# EUROPEAN PATENT APPLICATION

(11) **EP 4 700 015 A1**
(43) Date of publication of application: **25.02.2026**
(21) Application number: 24792100.0
(22) Date of filing: 19.04.2024
(51) Int. Cl.: C07D 207/273, C07D 207/26, C07D 207/263, C07C 39/04, A61K 31/05, A61K 31/40, A61K 31/4015

(54) **HETEROCYCLIC RING-SUBSTITUTED AROMATIC COMPOUND, PREPARATION METHOD THEREFOR, AND USE THEREOF**

(30) Priority: 19.04.2023 CN 202310419575
(71) Applicant: Shanghai Henlius Biotech, Inc., Shanghai 201210 (CN); Shanghai Henlius Biologics Co., Ltd., Shanghai 201616 (CN)
(72) Inventor: CHI, Yushi, Shanghai 201210 (CN); LIU, Rui, Shanghai 201210 (CN); GU, Boqi, Shanghai 201210 (CN); SHAN, Yongqiang, Shanghai 201210 (CN); YAN, Kun, Shanghai 201210 (CN)
(74) Representative: Plougmann Vingtoft a/s
(86) International application number: PCT/CN2024/088700
(87) International publication number: WO 2024/217515

(57) **Abstract**

The present disclosure relates to a substituted aromatic compound represented by general formula (I), a preparation method therefor, a pharmaceutical composition comprising the compound, and use thereof for treating and/or preventing a disease or symptom in which a Kv1.3 potassium channel plays a role.

## Description

### Technical Field

The present disclosure belongs to the field of medicine, and relates to a substituted aromatic compound, a preparation method therefor and the use thereof. Specifically, the present disclosure relates to a substituted aromatic compound represented by formula (I), a preparation method therefor, a pharmaceutical composition comprising the compound, and the use thereof for treating and/or preventing a disease or symptom in which a Kv1.3 potassium channel plays a role.

### Background Art

Voltage-gated Kv1.3 potassium (K⁺) channel is expressed in the central nervous system and lymphocytes, regulating physiological processes such as neurotransmitter release and immune cell activation. In the immune system, activated effector memory T cells upregulate the expression of Kv1.3 potassium channel (Wulff et al., 2003). Kv1.3 channel can regulate the membrane potential of effector memory T cells, affect its calcium signalling, and thereby continuously promote its TCR-dependent cell activation, proliferation, and inflammatory cytokine secretion (such as interferon-γ and interleukin-2). In contrast, sustained activation of naive T cells and central memory T cells relies more on KCa3.1 channel than Kv1.3 potassium channel. Similarly, Kv1.3 is expressed at low level in naive B cells and early memory B cells, but is highly expressed in memory B cells that have undergone antibody class switching.

Autoimmune diseases such as rheumatoid arthritis, multiple sclerosis and psoriasis threaten the health of millions of people worldwide and share similar pathogenic mechanisms, namely the breakdown of the body's tolerance to autoantigens, leading to the immune system misidentifying self-substances as foreign. Autoantigen-specific T cells survive negative selection during thymic development due to genetic factors or exogenous induction, and are activated by autoantigens after entering the peripheral circulation. After activation, autoreactive T cells differentiate from the initial state into persistently activated memory T cells due to repeated self-antigen stimulation, and promote inflammatory damage by rapidly migrating to tissues, secreting inflammatory cytokines, and exhibiting immediate effector functions (Sallusto et al., 1999). Repeated antigen stimulation plays a key role in this process. One of the characteristics of differentiated effector memory T cells is that they do not need to return to lymph nodes to be activated by antigens. A therapy specifically targeting effector memory T cell is a promising approach for the prevention and treatment of autoimmune disease.

In addition to autoimmune diseases, a large number of studies have shown that Kv1.3 potassium channel also plays an important role in the occurrence and development of diseases such as gastrointestinal diseases (such as inflammatory bowel disease and ulcerative colitis) (Koch Hansen et al., 2014), Alzheimer's disease (Rangaraju et al., 2015), and obesity (Upadhyay et al., 2013). Thus, a compound as a selective Kv1.3 channel inhibitor has great potential for the treatment of various diseases.

### Summary of the Invention

The present application relates to the compound of formula (I) as defined herein, which can selectively inhibit Kv1.3 channel. The present application is characterised by a method for treating or preventing a disease or symptom in which a Kv1.3 potassium channel plays a role, comprising administering to a subject in need thereof a therapeutically effective amount of the compound of formula (I) as defined herein (including formula I-1 to I-6) or a pharmaceutically acceptable salt, an ester, a stereoisomer, a tautomer, a polymorph, a hydrate, a solvate, an N-oxide, an isotopically labelled compound, a metabolite or a prodrug thereof. The method of the present application can be used to treat a disease or symptom in which a Kv1.3 potassium channel plays a role by inhibiting the activity of Kv1.3 potassium channel.

A first aspect of the present application relates to a compound of formula (I): 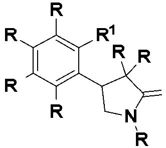 or a pharmaceutically acceptable salt, an ester, a stereoisomer, a tautomer, a polymorph, a hydrate, a solvate, an N-oxide, an isotopically labelled compound, a metabolite or a prodrug thereof, wherein R¹, R², R³, R⁴, R⁵, R⁶, R⁷ and R⁸ are as described in detail herein below.

Another aspect of the present application relates to a pharmaceutical composition, wherein the pharmaceutical composition comprises the compound of formula (I) or the pharmaceutically acceptable salt, the ester, the stereoisomer, the tautomer, the polymorph, the hydrate, the solvate, the N-oxide, the isotopically labelled compound, the metabolite or the prodrug thereof, and one or more pharmaceutically acceptable carriers, diluents or excipients.

Another aspect of the present application relates to a method for inhibiting Kv1.3 potassium channel. The method comprises administering to a subject in need thereof the compound of formula (I) or the pharmaceutically acceptable salt, the ester, the stereoisomer, the tautomer, the polymorph, the hydrate, the solvate, the N-oxide, the isotopically labelled compound, the metabolite or the prodrug thereof or the pharmaceutical composition comprising same.

Another aspect of the present application relates to a method for treating a disease or symptom in which a Kv1.3 potassium channel plays a role. The method comprises administering to a subject in need thereof the compound of formula (I) or the pharmaceutically acceptable salt, the ester, the stereoisomer, the tautomer, the polymorph, the hydrate, the solvate, the N-oxide, the isotopically labelled compound, the metabolite or the prodrug thereof or the pharmaceutical composition comprising same. The disease or symptom is selected from inflammatory disease or autoimmune disease, transplant rejection, neurological disorder or neurodegenerative disease, cardiovascular disease, metabolic disorder, nephrosis, gastrointestinal disorder, or oncological condition. Preferably, the inflammatory disease is selected from atopic dermatitis, arthritis, and psoriasis. The autoimmune disease is selected from rheumatoid arthritis, multiple sclerosis, systemic lupus erythematosus or type I diabetes. The neurodegenerative disease is Alzheimer's disease. The cardiovascular disease is ischemic stroke. The metabolic disorder is selected from obesity or type II diabetes. The nephrosis is selected from chronic kidney disease, nephritis or chronic renal failure. The gastrointestinal disorder is inflammatory bowel disease.

The present application further provides a compound and a composition with improved potency and/or safety profiles compared with known Kv1.3 potassium channel inhibitors.

The details of the present application are set forth in the accompanying description below. Although methods and materials similar or equivalent to those described herein may be used in the practice or testing of the present application, illustrative methods and materials are now described. Other features, objects, and advantages of the present application will be apparent from the description and the claims. In the description and the appended claims, singular forms also include plural forms, unless the context clearly dictates otherwise. Unless otherwise defined, all technical and scientific terms used herein have the same meanings as commonly understood by those of ordinary skill in the field to which the present application belongs. All patents and publications cited in the description are incorporated herein by reference in their entirety.

The contents of all references (including literature references, granted patents, published patent applications, and co-pending patent applications) cited throughout the present application are expressly incorporated herein by reference in their entirety. Unless defined otherwise, all technical and scientific terms used herein have the meanings as commonly understood by those of ordinary skill in the art.

### Detailed Description of Embodiments

### Definitions

Unless stated to the contrary, the terms used in the description and claims have the following meanings.

The term "alkyl" refers to a saturated aliphatic hydrocarbon group, which is a straight chain or branched chain group containing 1 to 20 (e.g., 1, 2, 3, 4, 5, 6, 7, 8, 9, 10, 11, 12, 13, 14, 15, 16, 17, 18, 19 or 20) carbon atoms (i.e., C₁₋₂₀ alkyl), preferably alkyl containing 1 to 12 (e.g., 1, 2, 3, 4, 5, 6, 7, 8, 9, 10, 11 and 12) carbon atoms (i.e., C₁₋₂₀ alkyl), more preferably alkyl containing 1 to 6 carbon atoms (i.e., C₁₋₆ alkyl). Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, their respective branched chain isomers, etc. The alkyl may be substituted or unsubstituted. When substituted, it may be substituted at any available point of attachment. The substituent is preferably selected from one or more of a deuterium atom, halogen, alkoxy, haloalkyl, haloalkoxy, cycloalkyloxy, heterocyclyloxy, hydroxyl, hydroxyalkyl, cyano, amino, nitro, cycloalkyl, heterocyclyl, aryl and heteroaryl.

The term "alkylene" refers to a saturated straight chain or branched chain aliphatic hydrocarbon group, which is a residue derived by removing two hydrogen atoms from the same carbon atom or two different carbon atoms of a parent alkane, and has 1 to 20 (e.g., 1, 2, 3, 4, 5, 6, 7, 8, 9, 10, 11, 12, 13, 14, 15, 16, 17, 18, 19 or 20) carbon atoms (i.e., C₁₋₂₀ alkylene), preferably 1 to 12 carbon atoms (i.e., C₁₋₁₂ alkylene), more preferably 1 to 6 carbon atoms (i.e., C₁₋₆ alkylene). Non-limiting examples include: methylene (-CH₂-), 1,1-ethylene (-CH(CH₃)-), 1,2-ethylene (-CH₂CH₂)-, 1,1-propylene (-CH(CH₂CH₃)-), 1,2-propylene (-CH₂CH(CH₃)-), 1,3-propylene (-CH₂CH₂CH₂-), 1,4-butylene (-CH₂CH₂CH₂CH₂-), etc. The alkylene may be substituted or unsubstituted. When substituted, it may be substituted at any available point of attachment. The substituent is preferably selected from one or more of a deuterium atom, halogen, alkoxy, haloalkyl, haloalkoxy, cycloalkyloxy, heterocyclyloxy, hydroxyl, hydroxyalkyl, cyano, amino, nitro, cycloalkyl, heterocyclyl, aryl and heteroaryl.

The term "heteroalkylene" refers to an alkylene group in which one or more - CH₂- groups are replaced by one or more selected from N, O, S, S(O) and S(O)₂, wherein the alkyl is as defined above. The heteroalkylene may be substituted or unsubstituted. When substituted, substitution with a substituent may be performed at any available point of attachment. The substituent is preferably selected from one or more of a deuterium atom, halogen, alkoxy, haloalkyl, haloalkoxy, cycloalkyloxy, heterocyclyloxy, hydroxyl, hydroxyalkyl, cyano, amino, nitro, cycloalkyl, heterocyclyl, aryl and heteroaryl.

The term "alkenyl" refers to an alkyl compound containing at least one carbon-carbon double bond in the molecule, wherein the alkyl is as defined above, and the alkenyl has 2 to 12 (e.g., 2, 3, 4, 5, 6, 7, 8, 9, 10, 11 or 12) carbon atoms (i.e., C₂₋₁₂ alkenyl). The alkenyl is preferably an alkenyl group having 2 to 6 carbon atoms (i.e., C₂₋₆ alkenyl). The alkenyl may be substituted or unsubstituted. When substituted, the substituent is preferably selected from one or more of a deuterium atom, alkoxy, halogen, haloalkyl, haloalkoxy, cycloalkyloxy, heterocyclyloxy, hydroxyl, hydroxyalkyl, cyano, amino, nitro, cycloalkyl, heterocyclyl, aryl and heteroaryl.

The term "alkynyl" refers to an alkyl compound containing at least one carbon-carbon triple bond in the molecule, wherein the alkyl is as defined above, and the alkynyl has 2 to 12 (e.g., 2, 3, 4, 5, 6, 7, 8, 9, 10, 11 or 12) carbon atoms (i.e., C₂₋₁₂ alkynyl). The alkynyl is preferably an alkynyl group having 2 to 6 carbon atoms (i.e., C₂₋₆ alkynyl). The alkynyl may be substituted or unsubstituted. When substituted, the substituent is preferably selected from one or more of a deuterium atom, alkoxy, halogen, haloalkyl, haloalkoxy, cycloalkyloxy, heterocyclyloxy, hydroxyl, hydroxyalkyl, cyano, amino, nitro, cycloalkyl, heterocyclyl, aryl and heteroaryl.

The term "cycloalkyl" refers to a saturated or partially unsaturated monocyclic or polycyclic hydrocarbon substituent, and the cycloalkyl ring contains 3 to 20 (e.g., 3, 4, 5, 6, 7, 8, 9, 10, 11, 12, 13, 14, 15, 16, 17, 18, 19 or 20) carbon atoms (i.e., 3- to 20-membered cycloalkyl), preferably 3 to 12 (e.g., 3, 4, 5, 6, 7, 8, 9, 10, 11 or 12) carbon atoms (i.e., 3- to 12-membered cycloalkyl), preferably 3 to 8 (e.g., 3, 4, 5, 6, 7 and 8) carbon atoms (i.e., 3- to 8-membered cycloalkyl), further preferably 4 to 7 (e.g., 4, 5, 6 and 7) carbon atoms (i.e., 4- to 7-membered cycloalkyl), more preferably 3 to 6 (e.g., 3, 4, 5 and 6) carbon atoms (i.e., 3- to 6-membered cycloalkyl). Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc.; and polycyclic cycloalkyl includes spirocycloalkyl, fused cycloalkyl and bridged cycloalkyl.

The term "spirocycloalkyl" refers to a 5- to 20-membered polycyclic group (e.g., 5, 6, 7, 8, 9, 10, 11, 12, 13, 14, 15, 16, 17, 18, 19 or 20 ring atoms, i.e., 5- to 20-membered spirocycloalkyl) in which the monocyclic rings share one carbon atom (referred to as a spiro atom), and which may contain one or more double bonds. Preferably, the spirocycloalkyl is 6- to 14-membered (i.e., 6- to 14-membered spirocycloalkyl); more preferably, it is 7- to 10-membered (e.g., 7-, 8-, 9-, or 10-membered, i.e., 7- to 10-membered spirocycloalkyl). According to the number of shared spiro atoms between the rings, the spirocycloalkyl is divided into monospirocycloalkyl, bispirocycloalkyl or polyspirocycloalkyl, preferably monospirocycloalkyl and bispirocycloalkyl. More preferably, the group is 3-membered/5-membered, 3-membered/6-membered, 4-membered/4-membered, 4-membered/5-membered, 4-membered/6-membered, 5-membered/5-membered or 5-membered/6-membered monospirocycloalkyl. Non-limiting examples of spirocycloalkyl include:

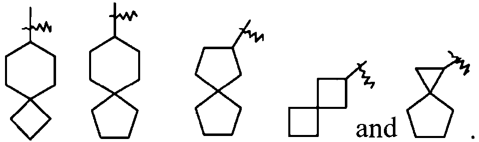

The term "fused cycloalkyl" refers to a 5- to 20-membered all-carbon polycyclic group (e.g., 5, 6, 7, 8, 9, 10, 11, 12, 13, 14, 15, 16, 17, 18, 19 or 20 ring atoms, i.e., 5- to 20-membered fused cycloalkyl), wherein each ring in the system shares a pair of adjacent carbon atoms with other rings in the system, wherein one or more rings may contain one or more double bonds. Preferably, the fused cycloalkyl is 6- to 14-membered (i.e., 6- to 14-membered fused cycloalkyl); more preferably, it is 7- to 10-membered (e.g., 7-, 8-, 9-, or 10-membered, i.e., 7- to 10-membered fused cycloalkyl). According to the number of constituent rings, it can be divided into bicyclic, tricyclic, tetracyclic or polycyclic fused cycloalkyl, preferably bicyclic or tricyclic, more preferably 3-membered/4-membered, 3-membered/5-membered, 3-membered/6-membered, 4-membered/4-membered, 4-membered/5-membered, 4-membered/6-membered, 5-membered/4-membered, 5-membered/5-membered, 5-membered/6-membered, 6-membered/3-membered, 6-membered/4-membered, 6-membered/5-membered, and 6-membered/6-membered bicyclic fused cycloalkyl. Non-limiting examples of fused cycloalkyl include:

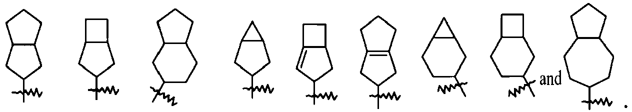

The term "bridged cycloalkyl" refers to a 5- to 20-membered all-carbon polycyclic group (e.g., 5, 6, 7, 8, 9, 10, 11, 12, 13, 14, 15, 16, 17, 18, 19 or 20 carbon atoms, i.e., 5- to 20-membered bridged cycloalkyl) in which any two rings share two carbon atoms that are not directly connected, and which may contain one or more double bonds. Preferably, the bridged cycloalkyl is 6- to 14-membered (i.e., 6- to 14-membered bridged cycloalkyl); more preferably, it is 7- to 10-membered (e.g., 7-, 8-, 9-, or 10-membered, i.e., 7- to 10-membered bridged cycloalkyl). According to the number of constituent rings, it can be divided into bicyclic, tricyclic, tetracyclic or polycyclic bridged cycloalkyl, preferably bicyclic, tricyclic or tetracyclic, more preferably bicyclic or tricyclic. Non-limiting examples of bridged cycloalkyl include:

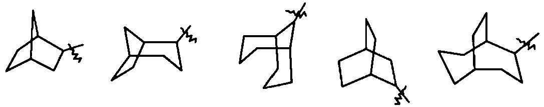

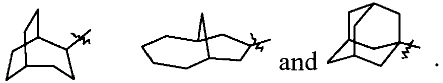

The cycloalkyl ring includes a cycloalkyl group as described above (including monocyclic cycloalkyl, spirocycloalkyl, fused cycloalkyl and bridged cycloalkyl) fused to an aryl, heteroaryl or heterocycloalkyl ring, wherein the ring connected to the parent structure is cycloalkyl, and non-limiting examples include 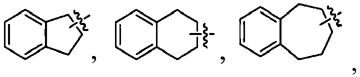 etc.; preferably

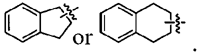

The cycloalkyl may be substituted or unsubstituted. When substituted, it may be substituted at any available point of attachment. The substituent is preferably selected from one or more of a deuterium atom, halogen, alkyl, alkoxy, haloalkyl, haloalkoxy, cycloalkyloxy, heterocyclyloxy, hydroxyl, hydroxyalkyl, cyano, amino, nitro, cycloalkyl, heterocyclyl, aryl and heteroaryl.

The term "alkoxy" refers to -O-(alkyl), wherein the alkyl is as defined above. Non-limiting examples of alkoxy include: methoxy, ethoxy, propoxy and butoxy. The alkoxy may be optionally substituted or unsubstituted. When substituted, the substituent is preferably one or more groups independently selected from a deuterium atom, halogen, alkoxy, haloalkyl, haloalkoxy, cycloalkyloxy, heterocyclyloxy, hydroxyl, hydroxyalkyl, cyano, amino, nitro, cycloalkyl, heterocyclyl, aryl and heteroaryl.

The term "heterocyclyl" refers to a saturated or partially unsaturated monocyclic or polycyclic substituent, comprising 3 to 20 ring atoms (e.g., 3, 4, 5, 6, 7, 8, 9, 10, 11, 12, 13, 14, 15, 16, 17, 18, 19 or 20 ring atoms, i.e., 3- to 20-membered heterocyclyl), wherein one or more ring atoms are heteroatoms selected from nitrogen, oxygen and sulphur, the sulphur may be optionally oxidized (i.e., to form sulfoxide or sulfone), but excluding the ring moiety of -O-O-, -O-S- or -S-S-, and the remaining ring atoms are carbon. Preferably, the heterocyclyl comprises 3 to 12 (e.g., 3, 4, 5, 6, 7, 8, 9, 10, 11 and 12) ring atoms (i.e., 3- to 12-membered heterocyclyl), wherein 1-4 (e.g., 1, 2, 3 and 4) ring atoms are heteroatoms; more preferably, the heterocyclyl comprises 3 to 8 ring atoms (e.g., 3, 4, 5, 6, 7 and 8, i.e., 3- to 8-membered heterocyclyl), wherein 1-3 (e.g., 1, 2 and 3) ring atoms are heteroatoms; further preferably, the heterocyclyl comprises 4 to 7 ring atoms (e.g., 4, 5, 6 and 7, i.e., 4- to 7-membered heterocyclyl), wherein 1-3 (e.g., 1, 2 and 3) ring atoms are heteroatoms; more preferably, the heterocyclyl comprises 3 to 6 ring atoms (e.g., 3, 4, 5 and 6, i.e., 3- to 6-membered heterocyclyl), wherein 1-3 (e.g., 1, 2 and 3) ring atoms are heteroatoms; most preferably, the heterocyclyl comprises 5 or 6 ring atoms (i.e., 5-membered or 6-membered heterocyclyl), wherein 1-2 (e.g., 1 and 2) ring atoms are heteroatoms. Non-limiting examples of monocyclic heterocyclyl include pyrrolidyl, tetrahydropyranyl, 1,2,3,6-tetrahydropyridyl, piperidyl, piperazinyl, morpholinyl, thiomorpholinyl, homopiperazinyl, etc. Polycyclic heterocyclyl includes spiro heterocyclyl, fused heterocyclyl and bridged heterocyclyl.

The term "spiro heterocyclyl" refers to a 5- to 20-membered polycyclic heterocyclic group (e.g., 5, 6, 7, 8, 9, 10, 11, 12, 13, 14, 15, 16, 17, 18, 19 or 20 ring atoms, i.e., 5- to 20-membered spiro heterocyclyl) in which the monocyclic rings share one atom (referred to as a spiro atom), wherein one or more ring atoms are heteroatoms selected from nitrogen, oxygen and sulphur, the sulphur may be optionally oxidized (i.e., to form sulfoxide or sulfone), and the remaining ring atoms are carbon. The spiro heterocyclyl may contain one or more double bonds. Preferably, the spiro heterocyclyl is 6- to 14-membered (i.e., 6- to 14-membered spiro heterocyclyl); more preferably, it is 7- to 10-membered (e.g., 7-, 8-, 9-, or 10-membered, i.e., 7- to 10-membered spiro heterocyclyl). According to the number of shared spiro atoms between the rings, the spiro heterocyclyl is divided into monospiro heterocyclyl, bispiro heterocyclyl or polyspiro heterocyclyl, preferably monospiro heterocyclyl and bispiro heterocyclyl. More preferably, the group is 3-membered/5-membered, 3-membered/6-membered, 4-membered/4-membered, 4-membered/5-membered, 4-membered/6-membered, 5-membered/5-membered, or 5-membered/6-membered monospiro heterocyclyl. Non-limiting examples of spiro heterocyclyl include:

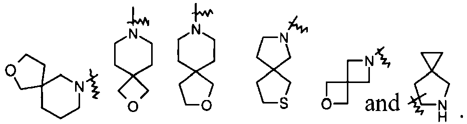

The term "fused heterocyclyl" refers to a 5- to 20-membered polycyclic heterocyclic group (e.g., 5, 6, 7, 8, 9, 10, 11, 12, 13, 14, 15, 16, 17, 18, 19 or 20 ring atoms, i.e., 5- to 20-membered fused heterocyclyl), wherein each ring in the system shares a pair of adjacent atoms with other rings in the system, and one or more rings may contain one or more double bonds, wherein one or more ring atoms are heteroatoms selected from nitrogen, oxygen and sulphur, the sulphur may be optionally oxidized (i.e., to form sulfoxide or sulfone), and the remaining ring atoms are carbon. Preferably, the fused heterocyclyl is 6- to 14-membered (i.e., 6- to 14-membered fused heterocyclyl); more preferably, it is 7- to 10-membered (e.g., 7-, 8-, 9-, or 10-membered, i.e., 7- to 10-membered fused heterocyclyl). According to the number of constituent rings, it can be divided into bicyclic, tricyclic, tetracyclic or polycyclic fused heterocyclyl, preferably bicyclic or tricyclic, more preferably 3-membered/4-membered, 3-membered/5-membered, 3-membered/6-membered, 4-membered/4-membered, 4-membered/5-membered, 4-membered/6-membered, 5-membered/4-membered, 5-membered/5-membered, 5-membered/6-membered, 6-membered/3-membered, 6-membered/4-membered, 6-membered/5-membered, and 6-membered/6-membered bicyclic fused heterocyclyl. Non-limiting examples of fused heterocyclyl include:

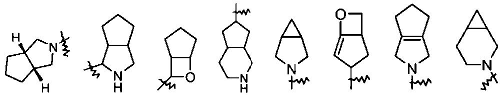

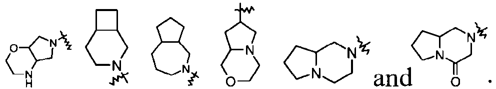

The term "bridged heterocyclyl" refers to a 5- to 14-membered polycyclic heterocyclic group (e.g., 5, 6, 7, 8, 9, 10, 11, 12, 13 or 14 ring atoms, i.e., 5- to 14-membered bridged heterocyclyl) in which any two rings share two atoms that are not directly connected, and which may contain one or more double bonds, wherein one or more ring atoms are heteroatoms selected from nitrogen, oxygen and sulphur, the sulphur may be optionally oxidized (i.e., to form sulfoxide or sulfone), and the remaining ring atoms are carbon. Preferably, the bridged heterocyclyl is 6-to 14-membered (i.e., 6- to 14-membered bridged heterocyclyl); more preferably, it is 7- to 10-membered (e.g., 7-, 8-, 9-, or 10-membered, i.e., 7- to 10-membered bridged heterocyclyl). According to the number of constituent rings, it can be divided into bicyclic, tricyclic, tetracyclic or polycyclic bridged heterocyclyl, preferably bicyclic, tricyclic or tetracyclic, more preferably bicyclic or tricyclic. Non-limiting examples of bridged heterocyclyl include:

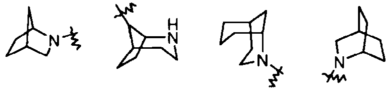

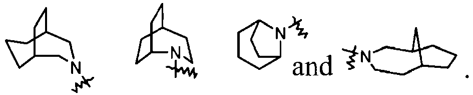

The heterocyclyl ring includes a heterocyclyl group as described above (including monocyclic heterocyclyl, spiro heterocyclyl, fused heterocyclyl and bridged heterocyclyl) fused to an aryl, heteroaryl or cycloalkyl ring, wherein the ring connected to the parent structure is heterocyclyl, and non-limiting examples thereof include:

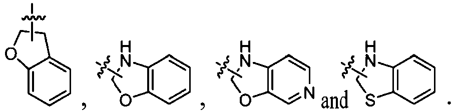

The heterocyclyl may be substituted or unsubstituted. When substituted, it may be substituted at any available point of attachment. The substituent is preferably selected from one or more of a deuterium atom, halogen, alkyl, alkoxy, haloalkyl, haloalkoxy, cycloalkyloxy, heterocyclyloxy, hydroxyl, hydroxyalkyl, cyano, amino, nitro, cycloalkyl, heterocyclyl, aryl and heteroaryl.

The term "aryl" refers to a 6- to 14-membered (e.g., 6, 7, 8, 9, 10, 11, 12, 13, or 14 ring atoms, i.e., 6- to 14-membered aryl) all-carbon monocyclic or fused polycyclic (fused polycyclic rings share adjacent pairs of carbon atoms) group having a conjugated π electron system, preferably 6- to 10-membered (i.e., 6- to 10-membered aryl), such as phenyl and naphthyl. The aryl ring includes an aryl ring as described above fused to a heteroaryl, heterocyclyl or cycloalkyl ring, wherein the ring connected to the parent structure is the aryl ring, and non-limiting examples thereof include:

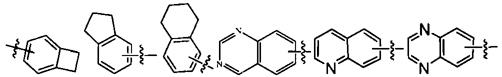

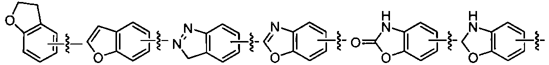

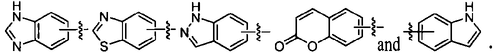

The aryl may be substituted or unsubstituted. When substituted, it may be substituted at any available point of attachment. The substituent is preferably selected from one or more of a deuterium atom, halogen, alkyl, alkoxy, haloalkyl, haloalkoxy, cycloalkyloxy, heterocyclyloxy, hydroxyl, hydroxyalkyl, cyano, amino, nitro, cycloalkyl, heterocyclyl, aryl and heteroaryl.

The term "heteroaryl" refers to a heteroaromatic system containing 1 to 4 (e.g., 1, 2, 3 and 4) heteroatoms and 5 to 14 ring atoms (e.g., 5, 6, 7, 8, 9, 10, 11, 12, 13 or 14 ring atoms, i.e., 5- to 14-membered heteroaryl), wherein the heteroatoms are selected from oxygen, sulphur and nitrogen. The heteroaryl is preferably 5- to 10-membered (e.g., 5-, 6-, 7-, 8-, 9- or 10-membered, i.e., 5- to 10-membered heteroaryl), more preferably 5- or 6-membered (i.e., 5- or 6-membered heteroaryl), such as furyl, thienyl, pyridyl, pyrrolyl, N-alkylpyrrolyl, pyrimidyl, pyrazinyl, pyridazinyl, imidazolyl, pyrazolyl, triazolyl and tetrazolyl. The heteroaryl ring includes a heteroaryl group as described above fused to an aryl, heterocyclyl or cycloalkyl ring, wherein the ring connected to the parent structure is the aryl ring, and non-limiting examples thereof include:

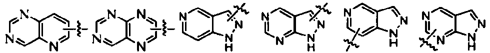

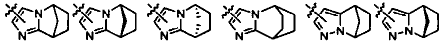

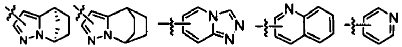

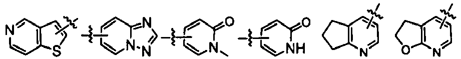

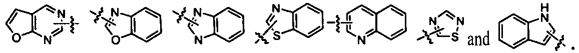

The heteroaryl may be substituted or unsubstituted. When substituted, it may be substituted at any available point of attachment. The substituent is preferably selected from one or more of a deuterium atom, halogen, alkyl, alkoxy, haloalkyl, haloalkoxy, cycloalkyloxy, heterocyclyloxy, hydroxyl, hydroxyalkyl, cyano, amino, nitro, cycloalkyl, heterocyclyl, aryl and heteroaryl.

The above-mentioned cycloalkyl, heterocyclyl, aryl and heteroaryl groups include residues derived by removing one hydrogen atom from a parent ring atom, or residues derived by removing two hydrogen atoms from the same or two different ring atoms of the parent, i.e., "divalent cycloalkyl", "divalent heterocyclyl" ( 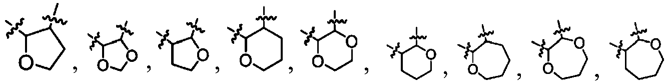 etc.), "arylene" and "heteroarylene".

The term "alkylthio" refers to alkyl-S-, wherein the alkyl is as defined above.

The term "haloalkyl" refers to alkyl substituted with one or more halogen, wherein the alkyl is as defined above.

The term "haloalkoxy" refers to alkoxy substituted with one or more halogen, wherein the alkoxy is as defined above.

The term "alkoxyalkyl" refers to alkyl substituted with one or more alkoxy groups, wherein the alkyl and alkoxy are as defined above.

The term "hydroxyalkyl" refers to alkyl substituted with one or more hydroxyl groups, wherein the alkyl is as defined above.

The term "halogen" refers to fluorine, chlorine, bromine or iodine.

The term "hydroxyl" refers to -OH.

The term "sulfhydryl" refers to -SH.

The term "amino" refers to -NH₂.

The term "cyano" refers to -CN.

The term "nitro" refers to -NO₂.

The term "oxo group" or "oxo" refers to "=O".

The term "carbonyl" refers to C=O.

The term "aldehyde group" refers to -C(O)H.

The term "carboxyl" refers to -C(O)OH.

The term "carboxylate group" refers to -C(O)O(alkyl), - C(O)O(cycloalkyl)(alkyl)C(O)O- or (cycloalkyl)C(O)O-, wherein the alkyl and cycloalkyl are as defined above.

In another aspect, the compounds of the present disclosure may exist in specific geometric or stereoisomeric forms. The present disclosure contemplates all such compounds, including cis and trans isomers, (-)- and (+)-enantiomers, (R)- and (S)-enantiomers, diastereomers, (D)-isomers, (L)-isomers, and racemic mixtures and other mixtures thereof, such as enantiomerically or diastereomerically enriched mixtures, all of which fall within the scope of the present disclosure. Additional asymmetric carbon atoms may be present in a substituent such as alkyl. All these isomers and mixtures thereof are included in the scope of the present disclosure. Optically active (R)- and (S)-isomers and D and L isomers can be prepared using chiral synthesis or chiral reagents or other conventional techniques. If a particular enantiomer of a compound of the present disclosure is desired, it can be prepared by asymmetric synthesis or derivatization with a chiral auxiliary, wherein the resulting diastereomeric mixture is separated and the auxiliary groups are cleaved to provide pure desired enantiomers. Alternatively, where the molecule contains a basic functional group (such as an amino group) or an acidic functional group (such as a carboxyl group), diastereomeric salts can be formed with an appropriate optically active acid or base, followed by resolution of the diastereomers using conventional methods well known in the art, and subsequent recovery of the pure enantiomers. Furthermore, separation of enantiomers and diastereomers is frequently accomplished by using chromatography, which uses chiral stationary phases, optionally in combination with chemical derivatization methods (e.g., formation of carbamates from amines).

In the chemical structure of the compound of the present disclosure, the bond 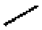 represents an unspecified configuration, that is, if there is a chiral isomer in the chemical structure, the bond 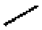 may be 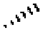 or 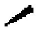 , or comprises both configurations. In the chemical structure of the compound of the present disclosure, the bond 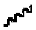 represents an unspecified configuration, that is, the bond may be in the Z configuration or the E configuration, or comprises both configurations.

In addition, the compounds and intermediates of the present disclosure may also exist in different tautomeric forms, and all such forms are embraced within the scope of the present disclosure. The term "tautomer" or "tautomeric form" refers to structural isomers of different energies which are interconvertible via a low energy barrier. For example, proton tautomers (also known as prototropic tautomers) include interconversions via migration of a proton, such as keto-enol and imine-enamine isomerizations. An example of lactam-lactim equilibrium is between A and B as shown below.

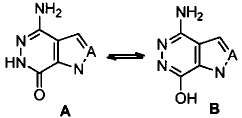

All compounds in the present disclosure can be drawn as type A or type B. All tautomeric forms are within the scope of the present disclosure. The naming of compounds does not exclude any tautomers.

The present disclosure also includes some isotopically-labelled compounds of the present disclosure which are identical to those recited herein, but have one or more atoms replaced by an atom having an atomic mass or mass number different from the atomic mass or mass number usually found in nature. Examples of isotopes that can be incorporated into compounds of the present disclosure include isotopes of hydrogen, carbon, nitrogen, oxygen, phosphorus, sulphur, fluorine, iodine, and chlorine, such as ²H , ³H, ¹¹C, ¹³C, ¹⁴C, ¹³N, ¹⁵N, ¹⁵O, ¹⁷O, ¹⁸O, ³¹P, ³²P, ³⁵S, ¹⁸F, ¹²³I, ¹²⁵I and ³⁶Cl, respectively.

The compounds of the present disclosure may contain unnatural proportions of atomic isotopes at one or more of the atoms constituting the compound. For example, compounds can be labelled with radioactive isotopes, such as tritium (³H), and hydrogen can be substituted with heavy hydrogen to form deuterated drugs. The bond formed by deuterium and carbon is stronger than the bond formed by ordinary hydrogen and carbon. Compared with undeuterated drugs, deuterated drugs have reduced toxic and side effects, increased drug stability, enhanced efficacy, prolonged biological half-life of drugs and other advantages. All isotopic variations of the compounds of the present disclosure, whether radioactive or not, are intended to be encompassed within the scope of the present disclosure.

Furthermore, substitution with heavier isotopes (such as deuterium, i.e., ²H) may afford certain therapeutic advantages resulting from greater metabolic stability, for example, increased *in vivo* half-life or reduced dosage requirements, and hence may be preferred in some circumstances, wherein the deuterium substitution may be partial or complete, and partial deuterium substitution means that at least one hydrogen is substituted with at least one deuterium.

Unless otherwise specified, when a position is specifically designated as deuterium (D), it should be understood that the position has a deuterium abundance at least 1000 times greater than the natural abundance of deuterium (which is 0.015%) (i.e., at least 10% deuterium incorporation). For the compounds in the examples, the deuterium abundance greater than the natural abundance may be at least 1000 times the natural abundance, at least 2000 times the natural abundance, at least 3000 times the natural abundance, at least 4000 times the natural abundance, at least 5000 times the natural abundance, at least 6000 times the natural abundance, or higher. The present disclosure further includes compounds of formula (I) (including formula I-1 to I-4) in various deuterated forms. Each available hydrogen atom attached to a carbon atom can be independently replaced by a deuterium atom. Those skilled in the art can synthesise compounds of formula (I) (including formulas I-1 to I-6) in deuterated forms with reference to relevant literatures. The compounds of formula (I) (including formulas I-1 to I-6) in deuterated forms can be prepared using commercially available deuterated starting materials, or can be synthesised with deuterated reagents using conventional techniques. The deuterated reagents include, but are not limited to, deuterated borane, solution of trideuterated borane in tetrahydrofuran, deuterated lithium aluminium hydride, deuterated iodoethane, deuterated iodomethane, etc.

"Optional" or "optionally" means that the event or circumstance subsequently described may but not necessarily occur, and the description includes the case where the event or circumstance occurs or does not occur. For example, "heterocyclyl optionally substituted with alkyl" means that the alkyl may but not necessarily be present, and the description includes the case where the heterocyclyl is substituted with alkyl and the case where the heterocyclyl is not substituted with alkyl.

"Substituted" means that one or more hydrogen atoms, preferably 1-5, more preferably 1-3 hydrogen atoms in the group are each independently substituted with a corresponding number of substituents. Those skilled in the art can determine the possible or impossible substitutions (by experiment or theory) without paying too much effort. For example, an amino or hydroxyl group having a free hydrogen may be unstable when combined with the carbon atoms having an unsaturated (e.g., olefinic) bond.

"Pharmaceutical composition" denotes a mixture containing one or more compounds described herein or physiologically/pharmaceutically acceptable salts or prodrugs thereof and other chemical components, as well as other components, such as physiologically/pharmaceutically acceptable carriers and excipients. The purpose of a pharmaceutical composition is to facilitate the administration to an organism, which will enhance the absorption of active ingredients and thus exert biological activities.

"Pharmaceutically acceptable salts" refer to salts of the compounds of the present disclosure, which are safe and effective when used in mammals, and have appropriate biological activity. The salts can be prepared separately during the final isolation and purification of the compounds, or by reacting a suitable group with a suitable base or acid. Bases commonly used to form pharmaceutically acceptable salts include inorganic bases such as sodium hydroxide and potassium hydroxide, and organic bases such as ammonia. Acids commonly used to form pharmaceutically acceptable salts include inorganic acids and organic acids.

With respect to drugs or pharmacologically active agents, the term "therapeutically effective amount", "inhibitory effective amount" or "prophylactically effective amount" refers to an amount of the drug or agent sufficient to achieve or partially achieve the desired effect. Determination of the effective amount, varying from person to person, depends on the age and general condition of a recipient and also depends on a specific active substance. The appropriate effective amount in individual cases can be determined by those skilled in the art according to routine experiments.

As used herein, the term "pharmaceutically acceptable" refers to compounds, materials, compositions, and/or dosage forms that are, within the scope of sound medical judgment, suitable for use in contact with patient tissues without excessive toxicity, irritation, allergic response, or other problems or complications, at a reasonable benefit/risk ratio, and effective for the intended use.

As used herein, the singular forms "a", "an" and "the" include plural referents and vice versa, unless otherwise clearly indicated in the context.

When the term "about" is applied to a parameter such as pH, concentration, and temperature, it indicates that the parameter may vary within ±10%, and sometimes more preferably within ±5%. As will be understood by those skilled in the art, when the parameters are not critical, the numbers are generally given for the purpose of illustration only rather than limitation.

### Compounds of the present disclosure

In some aspects, the present disclosure provides a compound of formula I or a pharmaceutically acceptable salt, an ester, a stereoisomer, a tautomer, a polymorph, a hydrate, a solvate, an N-oxide, an isotopically labelled compound, a metabolite or a prodrug thereof: 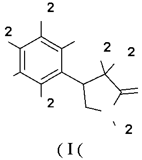 wherein:
R¹, R², R³ and R⁴ are the same or different, and are each independently selected from a hydrogen atom, halogen, C₁₋₆ alkyl, C₁₋₆ haloalkyl, C₁₋₆ alkoxy, C₁₋₆ haloalkoxy and C₂₋₆ alkenyl;
R⁵ is OR^{a}, wherein the R^{a} is selected from a hydrogen atom, -C(=O)R^{5A} and - C(=O)-NR^{5A}R^{5B}; R^{5A} and R^{5B} are the same or different, and are each independently selected from a hydrogen atom, alkyl, haloalkyl, cycloalkyl, halocycloalkyl and alkenyl;
R⁶ is -(CR^{b}R^{c})ₙR^{d} or -CH₂CR^{b}R^{c}CH₂R^{d};
R^{b} and R^{c} are the same or different, and are each independently selected from a hydrogen atom, halogen, amino, hydroxyl, C₁₋₆ alkyl, C₁₋₆ haloalkyl, 3- to 8-membered cycloalkyl, 3- to 8-membered halocycloalkyl, C₁₋₆ alkoxy, C₁₋₆ haloalkoxy, -NH(C₁₋₆ alkyl), -N(C₁₋₆ alkyl)₂ and C₂₋₆ alkenyl;
R^{d} is selected from hydroxyl, amino, hydroxyamino, -OR^{6A}, -O-NHR^{6A}, - NHR^{6A}, -NR^{6A}-OR^{6B}, -C(=O)R^{6A}, -C(=O)NR^{6A}OR^{6B}, -C(=NH)R^{6A}, - C(=NH)NR^{6A}R^{6B}, -NH-C(=O)R^{6A}, -S(=O)(=NH)R^{6A}, -S(=O)₂R^{6A}, -NH-S(=O)₂R^{6A}, -C(=O)-NR^{6A}R^{6B}, -C(=S)-NR^{6A}R^{6B}, -P(=O)R^{6A}R^{6B}, 3- to 8-membered cycloalkyl, 3- to 8-membered heterocyclyl, 6- to 12-membered spirocyclyl, 6- to 12-membered spiro heterocyclyl, a 5- to 12-membered bridged ring group, 5- to 12-membered bridged heterocyclyl, 6- to 10-membered aryl, 5- to 10-membered heteroaryl containing at least one nitrogen atom and 6- to 12-membered fused heterocyclyl, wherein the 3- to 8-membered cycloalkyl, 3- to 8-membered heterocyclyl, 6- to 12-membered spirocyclyl, 6- to 12-membered spiro heterocyclyl, 5- to 12-membered bridged ring group, 5- to 12-membered bridged heterocyclyl, 6- to 10-membered aryl, 5- to 10-membered heteroaryl containing at least one nitrogen atom and 6- to 12-membered fused heterocyclyl are optionally substituted with one or more R^{6C},
R^{6A} and R^{6B} are the same or different, and are each independently selected from a hydrogen atom, amino, C₁₋₆ alkyl, C₁₋₆ haloalkyl, C₂₋₆ alkenyl, 3- to 8-membered cycloalkyl, 3- to 8-membered heterocyclyl, 6- to 10-membered aryl and 5- to 10-membered heteroaryl, wherein the amino, C₁₋₆ alkyl, C₁₋₆ haloalkyl, C₂₋₆ alkenyl, 3- to 8-membered cycloalkyl, 3- to 8-membered heterocyclyl, 6- to 10-membered aryl and 5- to 10-membered heteroaryl are optionally substituted with one or more substituents selected from halogen, hydroxyl, amino, cyano, C₁₋₆ alkyl, C₁₋₆ haloalkyl, 3- to 8-membered cycloalkyl, 3- to 8-membered halocycloalkyl, C₁₋₆ alkoxy, 3- to 8-membered cycloalkoxy and C₂₋₆ alkenyl;
R^{6C} is selected from halogen, amino, hydroxyl, cyano, C₁₋₆ alkyl, C₁₋₆ haloalkyl, C₁₋₆ alkoxy, 3- to 8-membered cycloalkoxy, C₁₋₆ haloalkoxy, 3- to 8-membered halocycloalkoxy, C₂₋₆ alkenyl, 3- to 8-membered cycloalkyl, 3- to 8-membered heterocyclyl, 6- to 10-membered aryl, 5- to 10-membered heteroaryl, oxo, -C(=O)R^{6C1}, -NH-C(=O)R^{6C1}, -S(=O)(=NH)R^{6C1}, -S(=O)₂R^{6C1}, -NH-S(=O)₂R^{6C1}, -C(=O)-NR^{6C1}R^{6C2}, -P(=O) R^{6C1}R^{6C2} and -C₁₋₆ alkyl- P(=O) R^{6C1}R^{6C2}, wherein the C₁₋₆ alkyl, C₁₋₆ haloalkyl, C₁₋₆ alkoxy, 3- to 8-membered cycloalkoxy, C₁₋₆ haloalkoxy, 3- to 8-membered halocycloalkoxy, C₂₋₆ alkenyl, 3- to 8-membered cycloalkyl, 3- to 8-membered heterocyclyl, 6- to 10-membered aryl and 5- to 10-membered heteroaryl are optionally substituted with one or more substituents selected from: halogen, hydroxyl, amino, cyano, C₁₋₆ alkyl, C₁₋₆ haloalkyl, 3- to 8-membered cycloalkyl, 3- to 8-membered halocycloalkyl, C₁₋₆ alkoxy, 3- to 8-membered cycloalkoxy and C₂₋₆ alkenyl;
R^{6C1} and R^{6C2} are the same or different, and are each independently selected from a hydrogen atom, C₁₋₆ alkyl, C₁₋₆ haloalkyl, 3- to 8-membered cycloalkyl and 3- to 8-membered heterocyclyl;
n is selected from 0, 1, 2, 3 and 4;
R⁷ and R⁸ are the same or different, and are each independently selected from a hydrogen atom, C₁₋₆ alkyl, halogen and C₁₋₆ haloalkyl; alternatively, R⁷ and R⁸, together with the carbon atom to which they are attached, form 3- to 6-membered cycloalkyl or 3- to 6-membered heterocyclyl, wherein the cycloalkyl and heterocyclyl are optionally substituted with one or more substituents selected from: halogen, hydroxyl, amino, C₁₋₆ alkyl, C₁₋₆ haloalkyl, C₁₋₆ alkoxy, C₁₋₆ haloalkoxy, - C(=O)C₁₋₆ alkyl, -C(=O)C₁₋₆ haloalkyl, -S(=O)C₁₋₆ alkyl, or -S(=O)C₁₋₆ haloalkyl;
X is O, S or Se.

In some embodiments of formula (I), X is S. In some embodiments of formula (I), R¹ is hydrogen, halogen or C₁₋₆ alkyl. In some embodiments of formula (I), R¹ is hydrogen. In some embodiments of formula (I), R¹ is F. In some embodiments of formula (I), R¹ is Cl. In some embodiments of formula (I), R¹ is methyl. In some embodiments of formula (I), R² is halogen or C₂₋₆ alkenyl. In some embodiments of formula (I), R² is Cl. In some embodiments of formula (I), R² is vinyl. In some embodiments of formula (I), R³ is hydrogen or halogen. In some embodiments of formula (I), R³ is hydrogen. In some embodiments of formula (I), R³ is Cl. In some embodiments of formula (I), R⁴ is hydrogen. In some embodiments of formula (I), R⁵ is hydroxyl or a dimethylcarbamate group. In some embodiments of formula (I), R⁷ and R⁸ are each halogen; alternatively, R⁷ and R⁸, together with the carbon atom to which they are attached, form 3- to 6-membered cycloalkyl. In some embodiments of formula (I), R⁷ and R⁸ are both fluorine. In some embodiments of formula (I), R⁷ and R⁸, together with the carbon atom to which they are attached, form cyclopropyl.

In some aspects, the present disclosure provides a compound of formula (I-1) or a pharmaceutically acceptable salt, an ester, a stereoisomer, a tautomer, a polymorph, a hydrate, a solvate, an N-oxide, an isotopically labelled compound, a metabolite or a prodrug thereof: 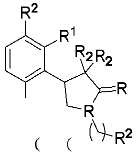 wherein:
n is selected from 0, 1, 2 and 3;
R¹, R², R⁷, R⁸, R^{a} and R^{d} are as defined herein.

In some embodiments of formula (I-1), R¹ and R² are the same or different, and are each independently selected from a hydrogen atom, C₁₋₄ alkyl, C₂₋₆ alkenyl and halogen. In some embodiments of formula (I-1), R¹ and R² are the same or different, and are each independently selected from hydrogen, methyl, a chlorine atom and a fluorine atom. In some embodiments of formula (I), R¹ is hydrogen. In some embodiments of formula (I), R¹ is F. In some embodiments of formula (I), R¹ is Cl. In some embodiments of formula (I), R¹ is methyl. In some embodiments of formula (I), R² is halogen or C₂₋₆ alkenyl. In some embodiments of formula (I), R² is Cl. In some embodiments of formula (I), R² is vinyl. In some embodiments of formula (I-1), R¹ and R² are both Cl.

In some embodiments of formula (I-1), R^{a} is selected from a hydrogen atom, - C(=O)C₁₋₆ alkyl, -C(=O)-NH(C₁₋₆ alkyl) and -C(=O)-N(C₁₋₆ alkyl)₂. In some embodiments of formula (I-1), R^{a} is selected from a hydrogen atom, - C(=O)CH(CH₃)₂ and -C(=O)-N(CH₃)₂. In some embodiments of formula (I-1), R^{a} is a hydrogen atom. In some embodiments of formula (I-1), R^{a} is -C(=O)CH(CH₃)₂. In some embodiments of formula (I-1), R^{a} is -C(=O)-N(CH₃)₂.

In some embodiments of formula (I-1), R⁷ and R⁸ are both selected from hydrogen atoms, fluorine atoms and methyl; alternatively, R⁷ and R⁸, together with the carbon atom to which they are attached, form 3- to 6-membered cycloalkyl or 3- to 6-membered heterocyclyl. In some embodiments of formula (I-1), R⁷ and R⁸ are both hydrogen or fluorine, or, together with the carbon atom to which they are attached, form cyclopropyl. In some embodiments of formula (I-1), R⁷ and R⁸ are both hydrogen. In some embodiments of formula (I-1), R⁷ and R⁸ are both fluorine. In some embodiments of formula (I-1), R⁷ and R⁸, together with the carbon atom to which they are attached, form cyclopropyl.

In some embodiments of formula (I-1), when n is 1, 2 or 3, R^{d} is selected from hydroxyl, amino, hydroxyamino, -OR^{6A}, -O-NHR^{6A}, -NHR^{6A}, -NR^{6A}-OR^{6B}, - C(=O)R^{6A}, -C(=O)NR^{6A}OR^{6B}, -C(=NH)R^{6A}, -C(=NH)NR^{6A}R^{6B}, -NH-C(=O)R^{6A}, - S(=O)(=NH)R^{6A}, -S(=O)₂R^{6A}, -NH-S(=O)₂R^{6A}, -C(=O)-NR^{6A}R^{6B}, -C(=S)-NR^{6A}R^{6B}, or -P(=O)R^{6A}R^{6B}. In some embodiments of formula (I-1), R^{6A} and R^{6B} are the same or different, and are each independently selected from a hydrogen atom, amino, C₁₋₆ alkyl, C₁₋₆ haloalkyl, 3- to 8-membered cycloalkyl and 3- to 8-membered heterocyclyl. In some embodiments of formula (I-1), R^{6A} and R^{6B} are the same or different, and are each independently selected from a hydrogen atom, amino, C₁₋₆ alkyl and C₁₋₆ haloalkyl. In some embodiments of formula (I-1), R^{6A} and R^{6B} are the same or different, and are each independently selected from a hydrogen atom, amino, methyl and trifluoroethyl. In a further embodiment of formula (I-1), R^{d} is selected from hydroxyl, amino, -NHCH₃, -NHOCH₃, - NCH₃OCH₃, -NHOCH₂CF₃, -NHS(=O)₂CH₃, -C(=O)NH₂, -S(=O)₂NH₂, - S(=O)(=NH)CH₃ and -S(=O)₂CH₃. In a further embodiment of formula (I-1), R^{d} is hydroxyl. In a further embodiment of formula (I-1), R^{d} is amino. In a further embodiment of formula (I-1), R^{d} is -NHCH₃. In a further embodiment of formula (I-1), R^{d} is -NHOCH₃. In a further embodiment of formula (I-1), R^{d} is - NCH₃OCH₃. In a further embodiment of formula (I-1), R^{d} is -NHOCH₂CF₃. In a further embodiment of formula (I-1), R^{d} is -NHS(=O)₂CH₃. In a further embodiment of formula (I-1), R^{d} is -C(=O)NH₂. In a further embodiment of formula (I-1), R^{d} is -S(=O)₂NH₂. In a further embodiment of formula (I-1), R^{d} is - S(=O)(=NH)CH₃. In a further embodiment of formula (I-1), R^{d} is -S(=O)₂CH₃.

In some embodiments of formula (I-1), when n is 0, R^{d} is selected from - C(=O)R^{6A} and -S(=O)₂R^{6A}, wherein R^{6A} is selected from C₁₋₆ alkyl, C₁₋₆ haloalkyl, 3- to 8-membered cycloalkyl and 3- to 8-membered heterocyclyl. In some embodiments of formula (I-1), the C₁₋₆ alkyl, 3- to 8-membered cycloalkyl, and 3-to 8-membered heterocyclyl are optionally substituted with one or more substituents selected from halogen, hydroxyl, amino, C₁₋₆ alkyl, C₁₋₆ haloalkyl and C₁₋₆alkoxy. In some embodiments of formula (I-1), R^{6A} is selected from - C₂H₄NH₂, 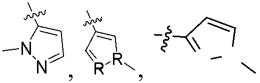 and cyclopropyl. In a further embodiment of formula (I-1), R^{d} is selected from -C(=O)C₂H₄NH₂, 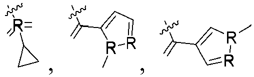 and

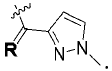

In some aspects, the present disclosure provides a compound of formula (I-2) or a pharmaceutically acceptable salt, an ester, a stereoisomer, a tautomer, a polymorph, a hydrate, a solvate, an N-oxide, an isotopically labelled compound, a metabolite or a prodrug thereof: 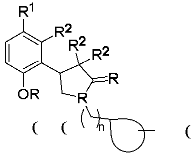 wherein:
ring A is selected from 3- to 8-membered cycloalkyl, 3- to 8-membered heterocyclyl, 6- to 12-membered spirocyclyl, 6- to 12-membered spiro heterocyclyl, a 5- to 12-membered bridged ring group, 5- to 12-membered bridged heterocyclyl, 6- to 10-membered aryl, 5- to 10-membered heteroaryl containing at least one nitrogen atom and 6- to 12-membered fused heterocyclyl;
k is selected from 0, 1, 2, 3 and 4;
n is selected from 0, 1, 2 and 3;
R¹, R², R⁷, R⁸, R^{a} and R^{6C} are as defined herein.

In some embodiments of formula (I-2), R¹ and R² are the same or different, and are each independently selected from a hydrogen atom, C₁₋₄ alkyl, C₂₋₆ alkenyl and halogen. In some embodiments of formula (I-2), R¹ and R² are the same or different, and are each independently selected from halogen and vinyl. In some embodiments of formula (I-2), R¹ is F. In some embodiments of formula (I-2), R¹ is Cl. In some embodiments of formula (I-2), R¹ is methyl. In some embodiments of formula (i-2), R² is Cl. In some embodiments of formula (I-2), R² is vinyl. In some embodiments of formula (I-2), R¹ and R² are both Cl.

In some embodiments of formula (I-2), R^{a} is selected from a hydrogen atom, - C(=O)C₁₋₆ alkyl, -C(=O)-NH(C₁₋₆ alkyl) and -C(=O)-N(C₁₋₆ alkyl)₂. In some embodiments of formula (I-2), R^{a} is selected from a hydrogen atom, - C(=O)CH(CH₃)₂ and -C(=O)-N(CH₃)₂. In some embodiments of formula (I-2), R^{a} is a hydrogen atom.

In some embodiments of formula (I-2), R⁷ and R⁸ are both selected from hydrogen atoms, fluorine atoms and methyl; alternatively, R⁷ and R⁸, together with the carbon atom to which they are attached, form 3- to 6-membered cycloalkyl or 3- to 6-membered heterocyclyl. In some embodiments of formula (I-2), R⁷ and R⁸ are both hydrogen atoms or fluorine atoms, or, together with the carbon atom to which they are attached, form cyclopropyl. In some embodiments of formula (I-2), R⁷ and R⁸ are both hydrogen. In some embodiments of formula (I-2), R⁷ and R⁸ are both fluorine. In some embodiments of formula (I-2), R⁷ and R⁸, together with the carbon atom to which they are attached, form cyclopropyl.

In some embodiments of formula (I-2), n is 0 or 1, and ring A is selected from 3- to 8-membered cycloalkyl, 3- to 8-membered heterocyclyl, 6- to 12-membered spirocyclyl, 6- to 12-membered spiro heterocyclyl, 5- to 12-membered bridged heterocyclyl, 6- to 10-membered aryl, 5- to 10-membered heteroaryl containing at least one nitrogen atom and 6- to 12-membered fused heterocyclyl. Preferably, in some embodiments of formula (I-2), ring A is selected from

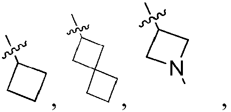

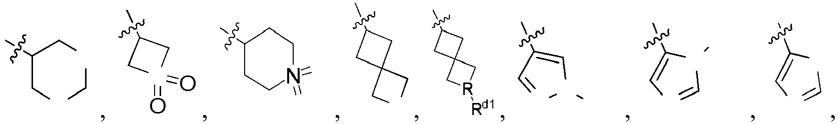

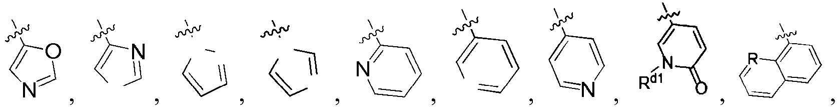

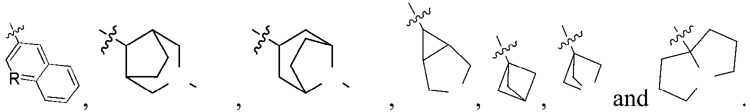

In some embodiments of formula (I-2), R^{d1} is selected from a hydrogen atom, C₁₋₆ alkyl, C₁₋₆ haloalkyl, 3- to 10-membered cycloalkyl, 3- to 10-membered heterocycloalkyl, -C(=O)C₁₋₆ alkyl, -C(=O)C₁₋₆ haloalkyl, -C(=O)C₃₋₆ cycloalkyl, - S(=O)₂C₁₋₆ alkyl, -S(=O)₂C₃₋₆ cycloalkyl, C₂₋₆ alkenyl and -C₁₋₆ alkyl-P(=O)(C₁₋₆ alkyl)₂. Preferably, in some embodiments of formula (I-2), R^{d1} is selected from a hydrogen atom, C₁₋₆ alkyl, 3- to 6-membered cycloalkyl, 3- to 6-membered heterocycloalkyl, C₁₋₆ haloalkyl, -S(=O)₂C₁₋₆ alkyl, -S(=O)₂C₃₋₆ cycloalkyl and C₂₋₆ alkenyl. More preferably, in some embodiments of formula (I-2), R^{d1} is selected from a hydrogen atom, methyl, cyclopropyl, oxetanyl, difluoroethyl, trifluoroethyl, vinyl, allyl, -S(=O)₂CH₃ and -S(=O)₂ cyclopropyl.

In some embodiments of formula (I-2), R^{6C} is selected from halogen, amino, hydroxyl, cyano, C₁₋₆ alkyl, C₁₋₆ haloalkyl, C₁₋₆ alkoxy, 3- to 8-membered cycloalkoxy, C₁₋₆ haloalkoxy, 3- to 8-membered halocycloalkoxy, C₂₋₆ alkenyl, oxo, -C(=O)R^{6C1}, -NH-C(=O)R^{6C1}, -S(=O)(=NH)R^{6C1}, -S(=O)₂R^{6C1}, -NH-S(=O)₂R^{6C1}, - C(=O)-NR^{6C1}R^{6C2}, -P(=O) R^{6C1}R^{6C2} and -C₁₋₆ alkyl- P(=O) R^{6C1}R^{6C2}; wherein R^{6C1} and R^{6C2} are the same or different, and are each independently selected from a hydrogen atom, C₁₋₆ alkyl, C₁₋₆ haloalkyl, 3- to 8-membered cycloalkyl and 3- to 8-membered heterocyclyl. In some embodiments of formula (I-2), R^{6C} is selected from halogen, amino, hydroxyl, C₁₋₆ alkyl, C₁₋₆ haloalkyl and -S(=O)₂ C₁₋₆ alkyl. In some embodiments of formula (I-2), R^{6C} is selected from a fluorine atom, amino, hydroxyl, methyl, trifluoromethyl and -S(=O)₂CH₃.

In some embodiments of formula (I-2), k is 4. In some embodiments of formula (I-2), k is 3. In some embodiments of formula (I-2), k is 2. In some embodiments of formula (I-2), k is 1. In some embodiments of formula (I-2), k is 0.

In some embodiments of formula (I-2), structural unit 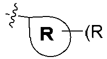 is selected from

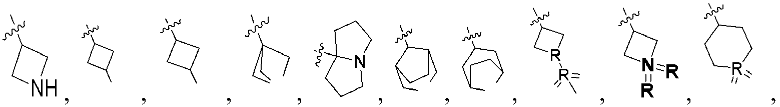

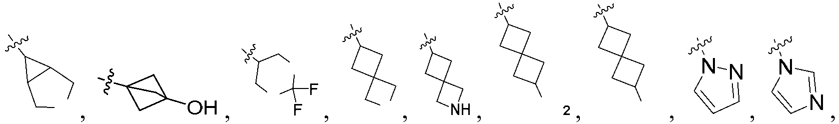

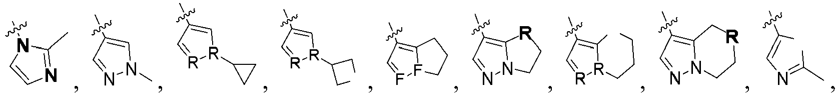

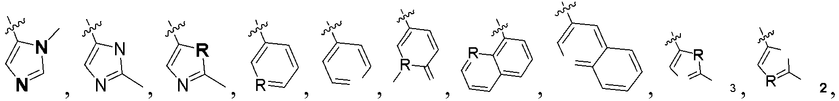

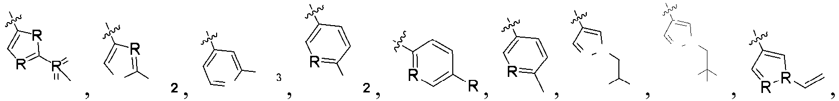

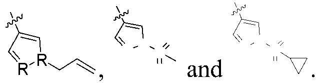

In some aspects, the present disclosure provides a compound of formula (I-3) or a pharmaceutically acceptable salt, an ester, a stereoisomer, a tautomer, a polymorph, a hydrate, a solvate, an N-oxide, an isotopically labelled compound, a metabolite or a prodrug thereof: 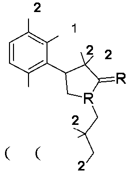 wherein:
R¹, R², R⁷, R⁸, R^{a}, R^{b}, R^{c} and R^{d} are as defined herein.

In some embodiments of formula (I-3), R¹ and R² are the same or different, and are each independently selected from a hydrogen atom, C₁₋₄ alkyl, C₂₋₆ alkenyl and halogen. In some embodiments of formula (I-3), R¹ and R² are the same or different, and are each independently selected from methyl, vinyl and halogen. In some embodiments of formula (I-3), R¹ and R² are the same or different, and are each independently selected from halogen and vinyl. In some embodiments of formula (I-3), R¹ is F. In some embodiments of formula (I-3), R¹ is Cl. In some embodiments of formula (I-3), R¹ is methyl. In some embodiments of formula (I-3), R² is Cl. In some embodiments of formula (I-3), R² is vinyl. In some embodiments of formula (I-3), R¹ and R² are both Cl.

In some embodiments of formula (I-3), R^{a} is selected from a hydrogen atom, - C(=O)C₁₋₆ alkyl, -C(=O)-NH(C₁₋₆ alkyl) and -C(=O)-N(C₁₋₆ alkyl)₂. In some embodiments of formula (I-3), R^{a} is selected from a hydrogen atom, - C(=O)CH(CH₃)₂ and -C(=O)-N(CH₃)₂. In some embodiments of formula (I-3), R^{a} is a hydrogen atom. In some embodiments of formula (I-3), R^{a} is -C(=O)CH(CH₃)₂. In some embodiments of formula (I-3), R^{a} is -C(=O)-N(CH₃)₂.

In some embodiments of formula (I-3), R⁷ and R⁸ are both selected from hydrogen atoms, fluorine atoms and methyl; alternatively, R⁷ and R⁸, together with the carbon atom to which they are attached, form 3- to 6-membered cycloalkyl or 3- to 6-membered heterocyclyl. In some embodiments of formula (I-3), Rand R⁸ are both hydrogen or fluorine; alternatively, R⁷ and R⁸, together with the carbon atom to which they are attached, form cyclopropyl. In some embodiments of formula (I-3), R⁷ and R⁸ are both hydrogen. In some embodiments of formula (I-1), R⁷ and R⁸ are both fluorine. In some embodiments of formula (I-3), R⁷ and R⁸, together with the carbon atom to which they are attached, form cyclopropyl.

In some embodiments of formula (I-3), R^{b} and R^{c} are the same or different, and are each independently selected from a hydrogen atom, halogen, amino, hydroxyl, C₁₋₄ alkyl, C₁₋₄ haloalkyl, 3- to 6-membered cycloalkyl, 3- to 6-membered halocycloalkyl, C₁₋₄ alkoxy, C₁₋₄ haloalkoxy, -NH(C₁₋₄ alkyl), -N(C₁₋₄ alkyl)₂ and C₂₋₄ alkenyl; alternatively, R^{b} and R^{c}, together with the carbon atom to which they are attached, form C₂₋₄ alkenyl. In some embodiments of formula (I-3), R^{b} and R^{c} are the same or different, and are each independently selected from a hydrogen atom, halogen, hydroxyl and C₁₋₄alkyl; alternatively, R^{b} and R^{c}, together with the carbon atom to which they are attached, form vinyl. In some embodiments of formula (I-3), R^{b} and R^{c} are the same or different, and are each independently selected from a hydrogen atom, a fluorine atom, methyl and hydroxyl; alternatively, R^{b} and R^{c}, together with the carbon atom to which they are attached, form vinyl. In some embodiments of formula (I-3), R^{b} and R^{c} are the same or different, and are each independently selected from a hydrogen atom, a fluorine atom, and hydroxyl. In some embodiments of formula (I-3), R^{b} and R^{c} are the same or different, and are each independently selected from a hydrogen atom, and hydroxyl. In some embodiments of formula (I-3), R^{b} and R^{c} are the same or different, and are each independently selected from a hydrogen atom, and a fluorine atom. In some embodiments of formula (I-3), R^{b} and R^{c} are both fluorine atoms, or one of R^{b} and R^{c} is a fluorine atom or hydroxyl and the other is a hydrogen atom. In some embodiments of formula (I-3), R^{b} and R^{c} are both fluorine atoms, or one of R^{b} and R^{c} is hydroxyl and the other is a hydrogen atom. In some embodiments of formula (I-3), R^{b} and R^{c} are both fluorine atoms. In some embodiments of formula (I-3), R^{b} and R^{c} are both hydrogen atoms. In some embodiments of formula (I-3), R^{b} and R^{c}, together with the carbon atom to which they are attached, form vinyl.

In some embodiments of formula (I-3), R^{d} is selected from hydroxyl, amino, hydroxyamino, -OR^{6A}, -O-NHR^{6A}, -NHR^{6A} -NR^{6A}-OR^{6B}, -C(=O)R^{6A}, - C(=O)NR^{6A}OR^{6B}, -C(=NH)R^{6A}, -C(=NH)NR^{6A}R^{6B}, -NH-C(=O)R^{6A}, - S(=O)(=NH)R^{6A}, -S(=O)₂R^{6A}, -NH-S(=O)₂R^{6A}, -C(=O)-NR^{6A}R^{6B}, -C(=S)-NR^{6A}R^{6B}, or -P(=O)R^{6A}R^{6B}. In some embodiments of formula (I-3), R^{6A} and R^{6B} are the same or different, and are each independently selected from a hydrogen atom, amino, C₁₋₆ alkyl, C₁₋₆ haloalkyl, 3- to 8-membered cycloalkyl and 3- to 8-membered heterocyclyl. In some embodiments of formula (I-3), R^{6A} and R^{6B} are the same or different, and are each independently selected from a hydrogen atom, amino, C₁₋₆ alkyl and C₁₋₆ haloalkyl. In some embodiments of formula (I-3), R^{6A} and R^{6B} are the same or different, and are each independently selected from a hydrogen atom, amino, methyl and trifluoroethyl. In a further embodiment of formula (I-3), R^{d} is selected from hydroxyl, amino, hydroxyamino, -NHCH₃, - NHOCH₃, -NCH₃OCH₃, -NHOCH₂CF₃, -NHS(=O)₂CH₃, -C(=O)NH₂, - S(=O)₂NH₂, -S(=O)(=NH)CH₃ and -S(=O)₂CH₃. In a further embodiment of formula (I-3), R^{d} is selected from hydroxyl, amino and hydroxyamino. In a further embodiment of formula (I-3), R^{d} is hydroxyl. In a further embodiment of formula (I-3), R^{d} is amino. In a further embodiment of formula (I-3), R^{d} is -NHCH₃. In a further embodiment of formula (I-3), R^{d} is -NHOCH₃. In a further embodiment of formula (I-3), R^{d} is -NCH₃OCH₃. In a further embodiment of formula (I-3), R^{d} is - NHOCH₂CF₃. In a further embodiment of formula (I-3), R^{d} is -NHS(=O)₂CH₃. In a further embodiment of formula (I-3), R^{d} is -C(=O)NH₂. In a further embodiment of formula (I-3), R^{d} is -S(=O)₂NH₂. In a further embodiment of formula (I-3), R^{d} is - S(=O)(=NH)CH₃. In a further embodiment of formula (I-3), R^{d} is -S(=O)₂CH₃.

In some aspects, the present disclosure provides a compound of formula (I-4) or a pharmaceutically acceptable salt, an ester, a stereoisomer, a tautomer, a polymorph, a hydrate, a solvate, an N-oxide, an isotopically labelled compound, a metabolite or a prodrug thereof: 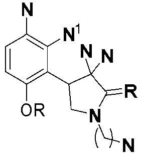 wherein:
R¹ and R² are the same or different, and are each independently selected from a hydrogen atom, C₁₋₄ alkyl, C₂₋₆ alkenyl and halogen;
R^{a} is selected from a hydrogen atom, -C(=O)C₁₋₆ alkyl, -C(=O)-NH(C₁₋₆ alkyl) and -C(=O)-N(C₁₋₆ alkyl)₂;
R⁷ and R⁸ are both selected from hydrogen atoms and fluorine atoms; alternatively, R⁷ and R⁸, together with the carbon atom to which they are attached, form 3- to 6-membered cycloalkyl or 3- to 6-membered heterocyclyl;
R^{d} is selected from hydroxyamino, -O-NHR^{6A}, -NR^{6A}-OR^{6B}, - C(=O)NR^{6A}OR^{6B}, -C(=S)-NR^{6A}R^{6B}, -C(=NH)R^{6A}, -C(=NH)NR^{6A}R^{6B}, - S(=O)(=NH)R^{6A}, -S(=O)₂R^{6A}, -NH-S(=O)₂R^{6A} and -P(=O) R^{6A} R^{6B};
R^{6A}, R^{6B} and n are as defined herein.

In some embodiments of formula (I-4), R¹ and R² are the same or different, and are each independently selected from halogen and vinyl. In some embodiments of formula (I-4), R¹ is F. In some embodiments of formula (I-4), R¹ is Cl. In some embodiments of formula (I-4), R¹ is methyl. In some embodiments of formula (I-4), R² is Cl. In some embodiments of formula (I-4), R² is vinyl. In some embodiments of formula (I-4), R¹ and R² are both Cl.

In some embodiments of formula (I-4), R^{a} is selected from a hydrogen atom, - C(=O)CH(CH₃)₂ and -C(=O)-N(CH₃)₂. Preferably, in some embodiments of formula (I-4), R^{a} is a hydrogen atom.

In some embodiments of formula (I-4), R⁷ and R⁸ are both hydrogen atoms or fluorine atoms, or, together with the carbon atom to which they are attached, form cyclopropyl. Preferably, in some embodiments of formula (I-4), R⁷ and R⁸ are both hydrogen atoms. In some embodiments of formula (I-4), R⁷ and R⁸ are both fluorine. In some embodiments of formula (I-4), R⁷ and R⁸, together with the carbon atom to which they are attached, form cyclopropyl.

In some embodiments of formula (I-4), when n is 2 or 3, R^{d} is selected from hydroxyamino, -O-NHR^{6A}, -NR^{6A}-OR^{6B}, -C(=O)NR^{6A}OR^{6B}, -C(=S)-NR^{6A}R^{6B}, - C(=NH)NR^{6A}R^{6B}, -S(=O)(=NH)R^{6A}, -S(=O)₂R^{6A} and -NH-S(=O)₂R^{6A} Preferably, in some embodiments of formula (I-4), R^{6A} and R^{6B} are the same or different, and are each independently selected from a hydrogen atom, amino, C₁₋₆ alkyl, C₁₋₆ haloalkyl and 3- to 8-membered cycloalkyl. More preferably, in some embodiments of formula (I-4), R^{6A} and R^{6B} are the same or different, and are each independently selected from a hydrogen atom, amino, methyl, trifluoroethyl and cyclopropyl. Further preferably, in some embodiments of formula (I-4), R^{d} is selected from -NHOH, -ONH₂, -NHOCH₃, -NCH₃OCH₃, -NHOCH₂CF₃, - C(=S)NH₂, -C(=NH)NH₂, -C(=O)NHOH, -C(=O)NCH₃OH, -C(=O)NHOCH₃, - NHS(=O)₂CH₃, -S(=O)₂NH₂, -S(=O)(=NH)CH₃ and -S(=O)₂CH₃.

In some embodiments of formula (I-4), when n is 0, R^{d} is selected from - C(=O)R^{6A} and -S(=O)₂R^{6A}; R^{6A} is selected from C₁₋₆ alkyl, 3- to 8-membered cycloalkyl and 3- to 8-membered heterocyclyl, wherein the C₁₋₆ alkyl, 3- to 8-membered cycloalkyl, and 3- to 8-membered heterocyclyl are optionally substituted with one or more substituents selected from halogen, hydroxyl, amino, C₁₋₆ alkyl, C₁₋₆ haloalkyl and C₁₋₆ alkoxy. Preferably, in some embodiments of formula (I-4), R^{6A} is selected from - C₂H₄NH₂, 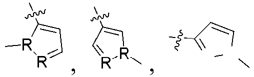 and cyclopropyl. More preferably, in some embodiments of formula (I-3), R^{d} is selected from -C(=O)C₂H₄NH₂,

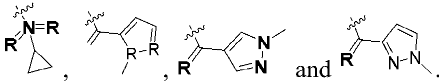

In some aspects, the present disclosure provides a compound of formula (I-5) or a pharmaceutically acceptable salt, an ester, a stereoisomer, a tautomer, a polymorph, a hydrate, a solvate, an N-oxide, an isotopically labelled compound, a metabolite or a prodrug thereof: 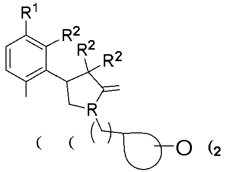 wherein:
R¹ and R² are the same or different, and are each independently selected from a hydrogen atom, C₁₋₄ alkyl, C₂₋₆ alkenyl and halogen;
R^{a} is selected from a hydrogen atom, -C(=O)C₁₋₆ alkyl, -C(=O)-NH(C₁₋₆ alkyl) and -C(=O)-N(C₁₋₆ alkyl)₂;
R⁷ and R⁸ are both selected from hydrogen atoms and fluorine atoms; alternatively, R⁷ and R⁸, together with the carbon atom to which they are attached, form 3- to 6-membered cycloalkyl or 3- to 6-membered heterocyclyl;
ring A is selected from 3- to 8-membered cycloalkyl, 3- to 8-membered heterocyclyl, 6- to 12-membered spirocyclyl, 6- to 12-membered spiro heterocyclyl, a 5- to 12-membered bridged ring group, 5- to 12-membered bridged heterocyclyl, 6- to 10-membered aryl, 5- to 10-membered heteroaryl containing at least one nitrogen atom and 6- to 12-membered fused heterocyclyl;
k is selected from 0, 1, 2, 3 and 4.
n is selected from 0, 1, 2 and 3.
R^{6C} is as defined herein.

In some embodiments of formula (I-5), R¹ and R² are the same or different, and are each independently selected from halogen and vinyl. In some embodiments of formula (I-5), R¹ is F. In some embodiments of formula (I-5), R¹ is Cl. In some embodiments of formula (I-5), R¹ is methyl. In some embodiments of formula (I-5), R² is Cl. In some embodiments of formula (I-5), R² is vinyl. In some embodiments of formula (I-5), R¹ and R² are both Cl.

In some embodiments of formula (I-5), R^{a} is selected from a hydrogen atom, - C(=O)CH(CH₃)₂ and -C(=O)-N(CH₃)₂. In some embodiments of formula (I-5), R^{a} is a hydrogen atom. In some embodiments of formula (I-5), R^{a} is -C(=O)CH(CH₃)₂. In some embodiments of formula (I-5), R^{a} is -C(=O)-N(CH₃)₂.

In some embodiments of formula (I-5), R⁷ and R⁸ are both hydrogen or fluorine, or, together with the carbon atom to which they are attached, form cyclopropyl. In some embodiments of formula (I-5), R⁷ and R⁸ are both hydrogen. In some embodiments of formula (I-5), R⁷ and R⁸ are both fluorine. In some embodiments of formula (I-5), R⁷ and R⁸, together with the carbon atom to which they are attached, form cyclopropyl.

In some embodiments of formula (I-5), n is 0 or 1, and ring A is selected from 6- to 12-membered spirocyclyl, 6- to 12-membered spiro heterocyclyl, a 5- to 12-membered bridged ring group and 5- to 12-membered bridged heterocyclyl. Preferably, in some embodiments of formula (I-5), ring A is selected from 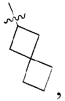 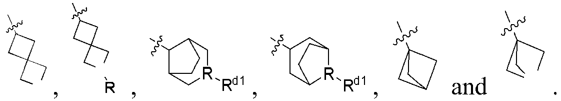 In some embodiments of formula (I-5), R^{d1} is selected from a hydrogen atom, C₁₋₆ alkyl, C₁₋₆ haloalkyl, 3- to 10-membered cycloalkyl, -C(=O)C₁₋₆ alkyl, -C(=O)C₁₋₆ haloalkyl, -C(=O)C₃₋₆ cycloalkyl, -S(=O)₂C₁₋₆ alkyl, -S(=O)₂C₃₋₆ cycloalkyl, C₂₋₆ alkenyl and -C₁₋₆ alkyl-P(=O)(C₁₋₆ alkyl)₂. Preferably, in some embodiments of formula (I-5), R^{d1} is selected from a hydrogen atom, C₁₋₆ alkyl, -S(=O)₂C₁₋₆ alkyl, -S(=O)₂C₃₋₆ cycloalkyl, C₂₋₆ alkenyl and -C₁₋₆ alkyl- P(=O)(C₁₋₆ alkyl)₂. More preferably, in some embodiments of formula (I-5), R^{d1} is selected from a hydrogen atom.

In some embodiments of formula (I-5), R^{6C} is selected from halogen, amino, hydroxyl, cyano, C₁₋₆ alkyl, C₁₋₆ haloalkyl, C₁₋₆ alkoxy, 3- to 8-membered cycloalkoxy, C₁₋₆ haloalkoxy, 3- to 8-membered halocycloalkoxy, C₂₋₆ alkenyl, oxo, -C(=O)R^{6C1}, -NH-C(=O)R^{6C1}, -S(=O)(=NH)R^{6C1}, -S(=O)₂R^{6C1}, -NH-S(=O)₂R^{6C1}, - C(=O)-NR^{6C1}R^{6C2}, -P(=O) R^{6C1}R^{6C2} and -C₁₋₆ alkyl- P(=O) R^{6C1}R^{6C2}; R^{6C1} and R^{6C2} are the same or different, and are each independently selected from a hydrogen atom, C₁₋₆ alkyl, C₁₋₆ haloalkyl, 3- to 8-membered cycloalkyl and 3- to 8-membered heterocyclyl; R^{6C} is selected from halogen, amino, hydroxyl, C₁₋₆ alkyl, C₁₋₆ haloalkyl and -S(=O)₂ C₁₋₆ alkyl. Preferably, in some embodiments of formula (I-5), R^{6C} is selected from amino, hydroxyl, -S(=O)₂NH₂ and -S(=O)₂CH₃.

In some embodiments of formula (I-5), structural unit 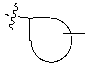 is selected from 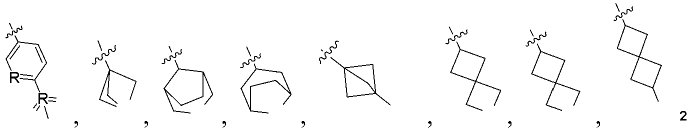 and

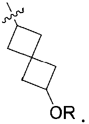

In some embodiments of formula (I-5), n is 0 or 1, and ring A is selected from 3- to 8-membered cycloalkyl, 3- to 8-membered heterocyclyl, 6- to 10-membered aryl, 5- to 10-membered heteroaryl containing at least one nitrogen atom and 6- to 12-membered fused heterocyclyl. Preferably, in some embodiments of formula (I-5), ring A is selected from 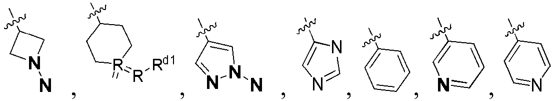 and 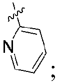 R^{d1} is selected from a hydrogen atom, 3- to 6-membered cycloalkyl, - S(=O)₂C₁₋₆ alkyl, -S(=O)₂C₃₋₆ cycloalkyl, C₂₋₆ alkenyl and -C₁₋₆ alkyl-P(=O)(C₁₋₆ alkyl)₂; more preferably, R^{d1} is selected from cyclopropyl, vinyl, allyl, -S(=O)₂CH₃, -S(=O)₂ cyclopropyl and -CH₂- P(=O)(CH₃)₂.

In some embodiments of formula (I-5), k is 4. In some embodiments of formula (I-5), k is 3. In some embodiments of formula (I-5), k is 2. In some embodiments of formula (I-5), k is 1. In some embodiments of formula (I-5), k is 0.

In some embodiments of formula (I-5), R^{6C} is selected from C₂₋₆ alkenyl, - S(=O)(=NH)R^{6C1}, -S(=O)₂R^{6C1}, -NH-S(=O)₂R^{6C1}, -P(=O) R^{6C1}R^{6C2} and -C₁₋₆ alkyl-P(=O) R^{6C1}R^{6C2}; R^{6C1} and R^{6C2} are the same or different, and are each independently selected from a hydrogen atom, C₁₋₆ alkyl, C₁₋₆ haloalkyl, 3- to 8-membered cycloalkyl and 3- to 8-membered heterocyclyl. Preferably, in some embodiments of formula (I-5), R^{6C} is selected from -S(=O)₂NH₂ and -S(=O)₂ C₁₋₆ alkyl. More preferably, in some embodiments of formula (I-5), R^{6C} is selected from -S(=O)₂NH₂ and -S(=O)₂CH₃.

In some embodiments of formula (I-5), structural unit 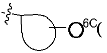 is selected from

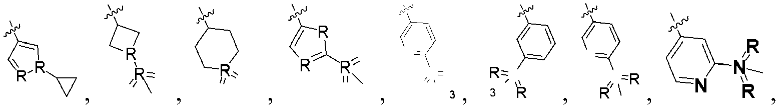

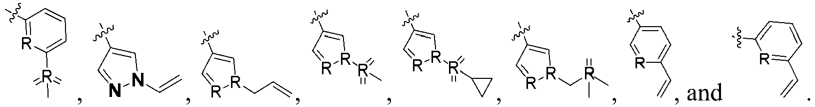

In some aspects, the present disclosure provides a compound of formula (I-6) or a pharmaceutically acceptable salt, an ester, a stereoisomer, a tautomer, a polymorph, a hydrate, a solvate, an N-oxide, an isotopically labelled compound, a metabolite or a prodrug thereof: 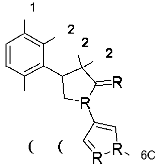
wherein R¹ and R² are the same or different, and are each independently selected from a hydrogen atom, C₁₋₄ alkyl, C₂₋₆ alkenyl and halogen;
Rₐ is selected from a hydrogen atom, -C(=O)C₁₋₆ alkyl, -C(=O)-NH(C₁₋₆ alkyl) and -C(=O)-N(C₁₋₆ alkyl)₂;
R⁷ and R⁸ are both selected from hydrogen atoms, fluorine atoms and methyl; alternatively, R⁷ and R⁸, together with the carbon atom to which they are attached, form 3- to 6-membered cycloalkyl or 3- to 6-membered heterocyclyl;
R^{6D} is selected from C₁₋₆ alkyl, C₁₋₆ haloalkyl, C₂₋₆ alkenyl, 3- to 8-membered cycloalkyl, 3- to 8-membered halocycloalkyl, 3- to 8-membered heterocyclyl, - S(=O)₂-C₁₋₆ alkyl or -S(=O)₂-3- to 8-membered cycloalkyl, wherein the C₁₋₆ alkyl, C₁₋₆ haloalkyl, C₂₋₆ alkenyl, 3- to 8-membered cycloalkyl, 3- to 8-membered halocycloalkyl, 3- to 8-membered heterocyclyl, -S(=O)₂-C₁₋₆ alkyl, and -S(=O)₂-3-to 8-membered cycloalkyl are optionally substituted with one or more substituents selected from: halogen, hydroxyl, amino, cyano, C₁₋₄ alkyl, C₁₋₄ haloalkyl, or C₁₋₄ alkoxy.

In some embodiments of formula (I-6), R¹ and R² are the same or different, and are each independently selected from methyl, a chlorine atom and a fluorine atom. In a further embodiment of formula (I-6), R¹ is a chlorine atom or a fluorine atom, and R² is a chlorine atom or a fluorine atom. In a further embodiment of formula (I-6), R¹ and R² are both chlorine atoms.

In some embodiments of formula (I-6), Rₐ is selected from a hydrogen atom, - C(=O)CH(CH₃)₂ and -C(=O)-N(CH₃)₂. In a further embodiment of formula (I-6), Rₐ is a hydrogen atom.

In some embodiments of formula (I-6), R⁷ and R⁸ are both hydrogen atoms or fluorine atoms; alternatively, R⁷ and R⁸, together with the carbon atom to which they are attached, form cyclopropyl. In a further embodiment of formula (I-6), R⁷ and R⁸ are both hydrogen atoms.

In some embodiments of formula (I-6), R^{6D} is selected from C₁₋₆ alkyl, C₁₋₆ haloalkyl, C₂₋₆ alkenyl, 3- to 8-membered cycloalkyl and -S(=O)₂ -C₁₋₆ alkyl. In a further embodiment of formula (I-6), R^{6D} is selected from methyl, cyclopropyl, vinyl and -S(=O)₂-CH₃. In a further embodiment of formula (I-6), R^{6D} is selected from methyl and cyclopropyl. In a further embodiment of formula (I-6), R^{6D} is cyclopropyl. In a further embodiment of formula (I-6), R^{6D} is cyclopropyl.

In some embodiments, the present disclosure provides compounds of Table 1 below or a pharmaceutically acceptable salt, an ester, a stereoisomer, a tautomer, a polymorph, a hydrate, a solvate, an N-oxide, an isotopically labelled compound, a metabolite or a prodrug thereof:

**Table 1. Typical compounds of the present disclosure:**

| Compound No. | Structure | Chemical name |
|---|---|---|
| 1 | 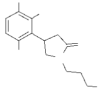 | rac-4-(2,3-Dichloro-6-hydroxyphenyl)-1-(3-hydroxypropyl)pyrrolidine-2-thione |
| 2 | 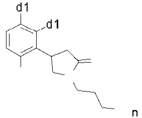 | rac-1-(3-Aminopropyl)-4-(2,3-dichloro-6-hydroxyphenyl)pyrrolidine-2-thione |
| 3 | 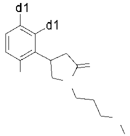 | rac-4-(2,3-Dichloro-6-hydroxyphenyl)-1-(3-(methylamino)propyl)pyrrolidine-2-thione |
| 4 | 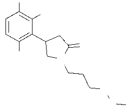 | rac-4-(2,3-Dichloro-6-hydroxyphenyl)-1-(3-(methoxyamino)propyl)pyrrolidine-2-thione |
| 5 | 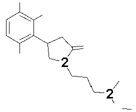 | rac-4-(2,3-Dichloro-6-hydroxyphenyl)-1-(3-(methoxy(methyl)amino)propyl)pyrrolidine-2-thione |
| 6 | 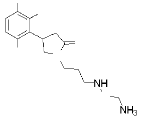 | rac-4-(2,3-Dichloro-6-hydroxyphenyl)-1-(3-((2,2,2-trifluoroethoxy)amino)propyl)pyrrolidine-2-thione |
| 7 | 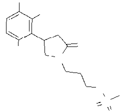 | rac-*N*-(3-(4-(2,3-Dichloro-6-hydroxyphenyl)-2-thiopyrrolidin-1-yl)propyl)methanesulfonamide |
| 8 | 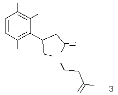 | rac-3-(4-(2,3-Dichloro-6-hydroxyphenyl)-2-thiopyrrolidin-1-yl)propionamide |
| 9 | 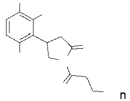 | rac-3-Amino-1-(4-(2,3-dichloro-6-hydroxyphenyl)-2-thiopyrrolidin-1-yl)propan-1-one |
| 10 | 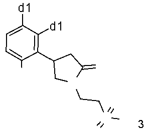 | rac-2-(4-(2,3-Dichloro-6-hydroxyphenyl)-2-thiopyrrolidin-1-yl)ethane-1-sulfonamide |
| 11 | 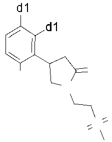 | rac- (2-(4-(2,3-Dichloro-6-hydroxyphenyl)-2-thiopyrrolidin-1-yl)ethyl)(imino)(methyl)-λ⁶-sulfanone |
| 12 | 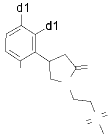 | rac-4-(2,3-Dichloro-6-hydroxyphenyl)-1-(2-(methylsulfonyl)ethyl)pyrrolidine-2-thione |
| 13 | 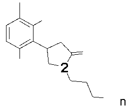 | rac-1-(3-Aminopropyl)-4-(2-chloro-6-hydroxy-3-methylphenyl)pyrrolidine-2-thione |
| 14 | 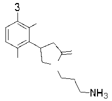 | rac-1-(3-Aminopropyl)-4-(2-chloro-3-fluoro-6-hydroxyphenyl)pyrrolidine-2-thione |
| 15 | 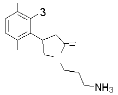 | rac-1-(3-Aminopropyl)-4-(3-chloro-2-fluoro-6-hydroxyphenyl)pyrrolidine-2-thione |
| 16 | 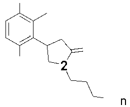 | rac-1-(3-Aminopropyl)-4-(3-chloro-6-hydroxy-2-methylphenyl)pyrrolidine-2-thione |
| 17 | 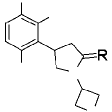 | rac-1-(Azetidin-3-yl)-4-(2,3-dichloro-6-hydroxyphenyl)pyrrolidine-2-thione |
| 18 | 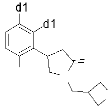 | rac-1-(Azetidin-3-ylmethyl)-4-(2,3-dichloro-6-hydroxyphenyl)pyrrolidine-2-thione |
| 19 | 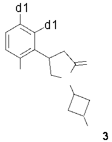 | rac-1-(3-Aminocyclobutyl)-4-(2,3-dichloro-6-hydroxyphenyl)pyrrolidine-2-thione |
| 20 | 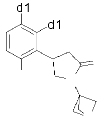 | rac-1-(1-Azabicyclo[1.1.1]pentan-3-yl)-4-(2,3-dichloro-6-hydroxyphenyl)pyrrolidine-2-thione |
| 21 | 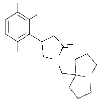 | rac-4-(2,3-Dichloro-6-hydroxyphenyl)-1-((tetrahydro-1H-pyrrolizin-7a(5H)-yl)methyl)pyrrolidine-2-thione |
| 22 | 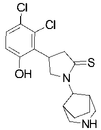 | rac-1-(3-Azabicyclo[3.2.1]octan-8-yl)-4-(2,3-dichloro-6-hydroxyphenyl)pyrrolidine-2-thione |
| 23 | 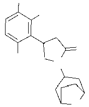 | rac-1-(8-Azabicyclo[3.2.1]octan-3-yl)-4-(2,3-dichloro-6-hydroxyphenyl)pyrrolidine-2-thione |
| 24 | 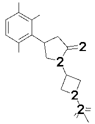 | rac-4-(2,3-Dichloro-6-hydroxyphenyl)-1-(1-(methylsulfonyl)azetidin-3-yl)pyrrolidine-2-thione |
| 25 | 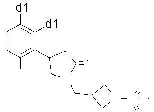 | rac-4-(2,3-Dichloro-6-hydroxyphenyl)-1-((1-(methylsulfonyl)azetidin-3-yl)methyl)pyrrolidine-2-thione |
| 26 | 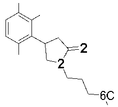 | rac-4-(2,3-Dichloro-6-hydroxyphenyl)-1-(3-(hydroxyamino)propyl)pyrrolidine-2-thione |
| 27 | 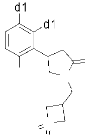 | rac-4-(2,3-Dichloro-6-hydroxyphenyl)-1-((1,1-dioxothietan-3-yl)methyl)pyrrolidine-2-thione |
| 28 | 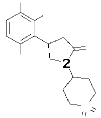 | rac-4-(2,3-Dichloro-6-hydroxyphenyl)-1-(1,1-dioxotetrahydro-2H-thiopyran-4-yl)pyrrolidine-2-thione |
| 29 | 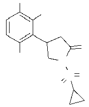 | rac-1-(Cyclopropylsulfonyl)-4-(2,3-dichloro-6-hydroxyphenyl)pyrrolidine-2-thione |
| 30 | 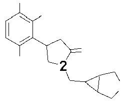 | rac-1-((3-Oxabicyclo[3.1.0]hexan-6-yl)methyl)-4-(2,3-dichloro-6-hydroxyphenyl)pyrrolidine-2-thione |
| 31 | 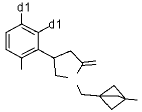 | rac-4-(2,3-Dichloro-6-hydroxyphenyl)-1-((3-hydroxybicyclo[1.1.1]pentan-1-yl)methyl)pyrrolidine-2-thione |
| 32 | 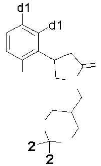 | rac-4-(2,3-Dichloro-6-hydroxyphenyl)-1-((2,2-difluoro-1,3-dioxan-5-yl)methyl)pyrrolidine-2-thione |
| 33 | 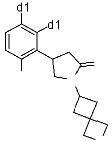 | rac-4-(2,3-Dichloro-6-hydroxyphenyl)-1-(2-oxaspiro[3.3]heptan-6-yl)pyrrolidine-2-thione |
| 34 | 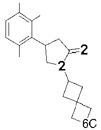 | rac-4-(2,3-Dichloro-6-hydroxyphenyl)-1-(2-azaspiro[3.3]heptan-6-yl)pyrrolidine-2-thione |
| 35 | 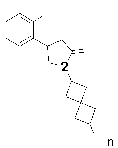 | rac-1-(6-Aminospiro[3.3]heptan-2-yl)-4-(2,3-dichloro-6-hydroxyphenyl)pyrrolidine-2-thione |
| 36 | 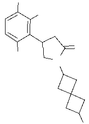 | rac-4-(2,3-Dichloro-6-hydroxyphenyl)-1-(6-hydroxyspiro[3.3]heptan-2-yl)pyrrolidine-2-thione |
| 37 | 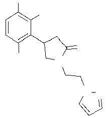 | rac-1-(2-(1H-Pyrazol-1-yl)ethyl)-4-(2,3-dichloro-6-hydroxyphenyl)pyrrolidine-2-thione |
| 38 | 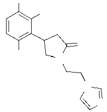 | rac-1-(2-(1H-Imidazol-1-yl)ethyl)-4-(2,3-dichloro-6-hydroxyphenyl)pyrrolidine-2-thione |
| 39 | 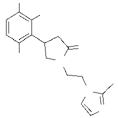 | rac-4-(2,3-Dichloro-6-hydroxyphenyl)-1-(2-(2-methyl-1H-imidazol-1-yl)ethyl)pyrrolidine-2-thione |
| 40 | 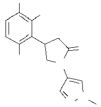 | rac-4-(2,3-Dichloro-6-hydroxyphenyl)-1-(1-methyl-1H-pyrazol-4-yl)pyrrolidine-2-thione |
| 41 | 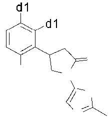 | rac-4-(2,3-Dichloro-6-hydroxyphenyl)-1-(2-methyl-1H-imidazol-5-yl)pyrrolidine-2-thione |
| 42 | 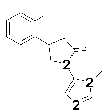 | rac-4-(2,3-Dichloro-6-hydroxyphenyl)-1-(1-methyl-1H-imidazol-5-yl)pyrrolidine-2-thione |
| 43 | 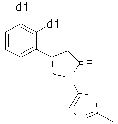 | rac-4-(2,3-Dichloro-6-hydroxyphenyl)-1-(2-methylthiazol-5-yl)pyrrolidine-2-thione |
| 44 | 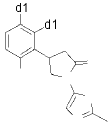 | rac-4-(2,3-Dichloro-6-hydroxyphenyl)-1-(2-methyloxazol-5-yl)pyrrolidine-2-thione |
| 45 | 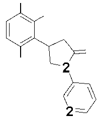 | rac-4-(2,3-Dichloro-6-hydroxyphenyl)-1-(pyridin-3-yl)pyrrolidine-2-thione |
| 46 | 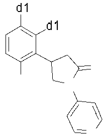 | rac-4-(2,3-Dichloro-6-hydroxyphenyl)-1-(pyridin-4-yl)pyrrolidine-2-thione |
| 47 | 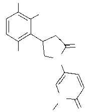 | rac-5-(4-(2,3-Dichloro-6-hydroxyphenyl)-2-thiopyrrolidin-1-yl)-1-methylpyridin-2(1H)-one |
| 48 | 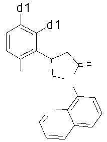 | rac-4-(2,3-Dichloro-6-hydroxyphenyl)-1-(quinolin-8-yl)pyrrolidine-2-thione |
| 49 | 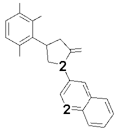 | rac-4-(2,3-Dichloro-6-hydroxyphenyl)-1-(quinolin-3-yl)pyrrolidine-2-thione |
| 50 | 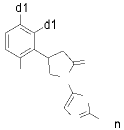 | rac-4-(2,3-Dichloro-6-hydroxyphenyl)-1-(2-(trifluoromethyl)thiazol-5-yl)pyrrolidine-2-thione |
| 51 | 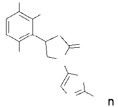 | rac-4-(2,3-Dichloro-6-hydroxyphenyl)-1-(2-(trifluoromethyl)-1H-imidazol-5-yl)pyrrolidine-2-thione |
| 52 | 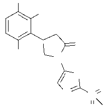 | rac-4-(2,3-Dichloro-6-hydroxyphenyl)-1-(2-(methylsulfonyl)thiazol-5-yl)pyrrolidine-2-thione |
| 53 | 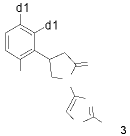 | rac-1-(2-Aminooxazol-4-yl)-4-(2,3-dichloro-6-hydroxyphenyl)pyrrolidine-2-thione |
| 54 | 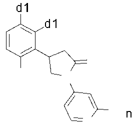 | rac-1-(2-Aminopyridin-4-yl)-4-(2,3-dichloro-6-hydroxyphenyl)pyrrolidine-2-thione |
| 55 | 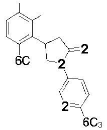 | rac-1-(6-Aminopyridin-3-yl)-4-(2,3-dichloro-6-hydroxyphenyl)pyrrolidine-2-thione |
| 56 | 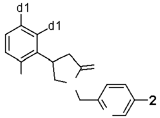 | rac-4-(2,3-Dichloro-6-hydroxyphenyl)-1-((5-fluoropyridin-2-yl)methyl)pyrrolidine-2-thione |
| 57 | 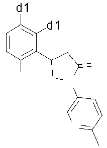 | rac-4-(2,3-Dichloro-6-hydroxyphenyl)-1-(6-methylpyridin-3-yl)pyrrolidine-2-thione |
| 58 | 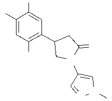 | rac-4-(4,5-Dichloro-2-hydroxyphenyl)-1-(1-methyl-1H-pyrazol-4-yl)pyrrolidine-2-thione |
| 59 | 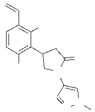 | rac-4-(2-Chloro-6-hydroxy-3-vinylphenyl)-1-(1-methyl-1H-pyrazol-4-yl)pyrrolidine-2-thione |
| 60 | 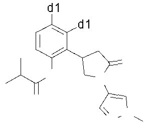 | rac-3,4-Dichloro-2-(1-(1-methyl-1H-pyrazol-4-yl)-5-thiopyrrolidin-3-yl)phenyl isobutyrate |
| 61 | 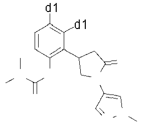 | rac-3,4-Dichloro-2-(1-(1-methyl-1H-pyrazol-4-yl)-5-thiopyrrolidin-3-yl)phenyl dimethylcarbamate |
| 62 | 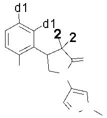 | rac-4-(2,3-Dichloro-6-hydroxyphenyl)-3,3-difluoro-1-(1-methyl-1H-pyrazol-4-yl)pyrrolidine-2-thione |
| 63 | 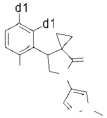 | rac-7-(2,3-Dichloro-6-hydroxyphenyl)-5-(1-methyl-1H-pyrazol-4-yl)-5-azaspiro[2.4]heptane-4-thione |
| 64 | 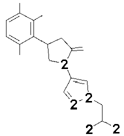 | rac-4-(2,3-Dichloro-6-hydroxyphenyl)-1-(1-(2,2-difluoroethyl)-1H-pyrazol-4-yl)pyrrolidine-2-thione |
| 65 | 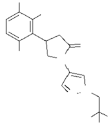 | rac-4-(2,3-Dichloro-6-hydroxyphenyl)-1-(1-(2,2,2-trifluoroethyl)-1H-pyrazol-4-yl)pyrrolidine-2-thione |
| 66 | 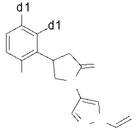 | rac-4-(2,3-Dichloro-6-hydroxyphenyl)-1-(1-vinyl-1H-pyrazol-4-yl)pyrrolidine-2-thione |
| 67 | 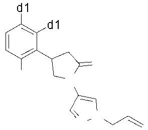 | rac-1-(1-Allyl-1H-pyrazol-4-yl)-4-(2,3-dichloro-6-hydroxyphenyl)pyrrolidine-2-thione |
| 68 | 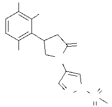 | rac-4-(2,3-Dichloro-6-hydroxyphenyl)-1-(1-(methylsulfonyl)-1H-pyrazol-4-yl)pyrrolidine-2-thione |
| 69 | 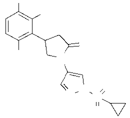 | rac-1-(1-(Cyclopropylsulfonyl)-1H-pyrazol-4-yl)-4-(2,3-dichloro-6-hydroxyphenyl)pyrrolidine-2-thione |
| 70 | 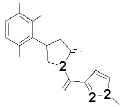 | rac- (4-(2,3-Dichloro-6-hydroxyphenyl)-2-thiopyrrolidin-1-yl)(1-methyl-1H-pyrazol-3-yl)methanone |
| 71 | 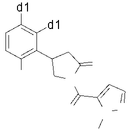 | rac- (4-(2,3-Dichloro-6-hydroxyphenyl)-2-thiopyrrolidin-1-yl)(1-methyl-1H-pyrazol-5-yl)methanone |
| 72 | 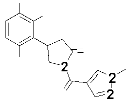 | rac- (4-(2,3-Dichloro-6-hydroxyphenyl)-2-thiopyrrolidin-1-yl)(1-methyl-1H-pyrazol-4-yl)methanone |
| 73 | 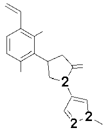 | rac-4-(2-Chloro-6-hydroxy-3-vinylphenyl)-1-(1-methyl-1H-pyrazol-4-yl)pyrrolidin-2-one |
| 74 | 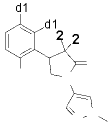 | rac-4-(2,3-Dichloro-6-hydroxyphenyl)-3,3-difluoro-1-(1-methyl-1H-pyrazol-4-yl)pyrrolidin-2-one |
| 75 | 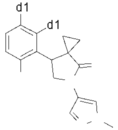 | rac-7-(2,3-Dichloro-6-hydroxyphenyl)-5-(1-methyl-1H-pyrazol-4-yl)-5-azaspiro[2.4]heptan-4-one |
| 76 | 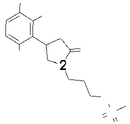 | rac-N-(3-(4-(2,3-Dichloro-6-hydroxyphenyl)-2-oxopyrrolidin-1-yl)propyl)methanesulfonamide |
| 77 | 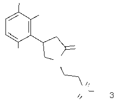 | rac-2-(4-(2,3-Dichloro-6-hydroxyphenyl)-2-oxopyrrolidin-1-yl)ethane-1-sulfonamide |
| 78 | 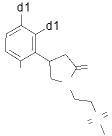 | rac-4-(2,3-Dichloro-6-hydroxyphenyl)-1-(2-(S-methylsulfonylimino)ethyl)pyrrolidin-2-one |
| 79 | 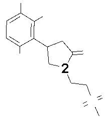 | rac-4-(2,3-Dichloro-6-hydroxyphenyl)-1-(2-(methylsulfonyl)ethyl)pyrrolidin-2-one |
| 80 | 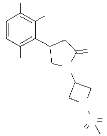 | rac-4-(2,3-Dichloro-6-hydroxyphenyl)-1-(1-(methylsulfonyl)azetidin-3-yl)pyrrolidin-2-one |
| 81 | 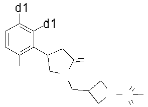 | rac-4-(2,3-Dichloro-6-hydroxyphenyl)-1-((1-(methylsulfonyl)azetidin-3-yl)methyl)pyrrolidin-2-one |
| 82 | 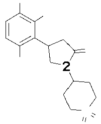 | rac-4-(2,3-Dichloro-6-hydroxyphenyl)-1-(1-imino-1-oxohexahydro-116-thiopyran-4-yl)pyrrolidin-2-one |
| 83 | 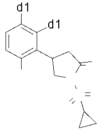 | rac-1-(Cyclopropylsulfonyl)-4-(2,3-dichloro-6-hydroxyphenyl)pyrrolidin-2-one |
| 84 | 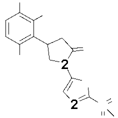 | rac-4-(2,3-Dichloro-6-hydroxyphenyl)-1-(2-(methylsulfonyl)thiazol-5-yl)pyrrolidin-2-one |
| 85 | 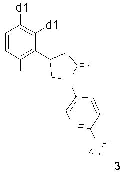 | rac-5-(4-(2,3-Dichloro-6-hydroxyphenyl)-2-oxopyrrolidin-1-yl)pyridine-2-sulfonamide |
| 86 | 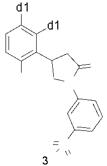 | rac-3-(4-(2,3-Dichloro-6-hydroxyphenyl)-2-oxopyrrolidin-1-yl)benzenesulfonamide |
| 87 | 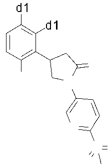 | rac-4-(2,3-Dichloro-6-hydroxyphenyl)-1-(6-(methylsulfonyl)pyridin-3-yl)pyrrolidin-2-one |
| 88 | 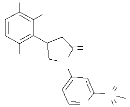 | rac-4-(2,3-Dichloro-6-hydroxyphenyl)-1-(2-(methylsulfonyl)pyridin-4-yl)pyrrolidin-2-one |
| 89 | 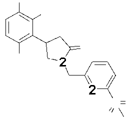 | rac-4-(2,3-Dichloro-6-hydroxyphenyl)-1-((6-(methylsulfonyl)pyridin-2-yl)methyl)pyrrolidin-2-one |
| 90 | 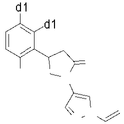 | rac-4-(2,3-Dichloro-6-hydroxyphenyl)-1-(1-vinyl-1H-pyrazol-4-yl)pyrrolidin-2-one |
| 91 | 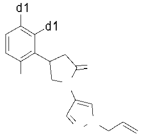 | rac-1-(1-Allyl-1H-pyrazol-4-yl)-4-(2,3-dichloro-6-hydroxyphenyl)pyrrolidin-2-one |
| 92 | 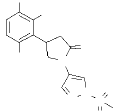 | rac-4-(2,3-Dichloro-6-hydroxyphenyl)-1-(1-(methylsulfonyl)-1H-pyrazol-4-yl)pyrrolidin-2-one |
| 93 | 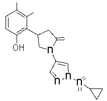 | rac-1-(1-(Cyclopropylsulfonyl)-1H-pyrazol-4-yl)-4-(2,3-dichloro-6-hydroxyphenyl)pyrrolidin-2-one |
| 94 | 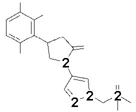 | rac-4-(2,3-Dichloro-6-hydroxyphenyl)-1-(1-((dimethylphosphoryl)methyl)-1H-pyrazol-4-yl)pyrrolidin-2-one |
| 95 | 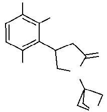 | rac-1-(1-Azabicyclo[1.1.1]pentan-3-yl)-4-(2,3-dichloro-6-hydroxyphenyl)pyrrolidin-2-one |
| 96 | 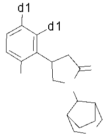 | rac-1-(3-Azabicyclo[3.2.1]octan-8-yl)-4-(2,3-dichloro-6-hydroxyphenyl)pyrrolidin-2-one |
| 97 | 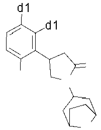 | rac-1-(8-Azabicyclo[3.2.1]octan-3-yl)-4-(2,3-dichloro-6-hydroxyphenyl)pyrrolidin-2-one |
| 98 | 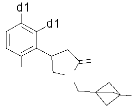 | rac-4-(2,3-Dichloro-6-hydroxyphenyl)-1-(3-hydroxybicyclo[1.1.1]pentan-1-yl)methyl)pyrrolidin-2-one |
| 99 | 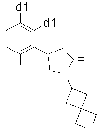 | rac-4-(2,3-Dichloro-6-hydroxyphenyl)-1-(2-oxaspiro[3.3]heptan-6-yl)pyrrolidin-2-one |
| 100 | 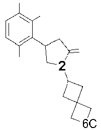 | rac-4-(2,3-Dichloro-6-hydroxyphenyl)-1-(2-azaspiro[3.3]heptan-6-yl)pyrrolidin-2-one |
| 101 | 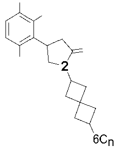 | rac-1-(6-Aminospiro[3.3]heptan-2-yl)-4-(2,3-dichloro-6-hydroxyphenyl)pyrrolidin-2-one |
| 102 | 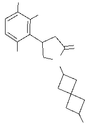 | rac-4-(2,3-Dichloro-6-hydroxyphenyl)-1-(6-hydroxyspiro[3.3]heptan-2-yl)pyrrolidin-2-one |
| 103 | 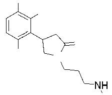 | rac-4-(2,3-Dichloro-6-hydroxyphenyl)-1-(3-(hydroxyamino)propyl)pyrrolidin-2-one |
| 104 | 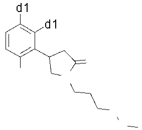 | rac-4-(2,3-Dichloro-6-hydroxyphenyl)-1-(3-(methoxyamino)propyl)pyrrolidin-2-one |
| 105 | 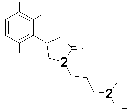 | rac-4-(2,3-Dichloro-6-hydroxyphenyl)-1-(3-(methoxy(methyl)amino)propyl)pyrrolidin-2-one |
| 106 | 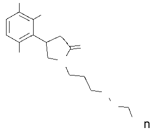 | rac-4-(2,3-Dichloro-6-hydroxyphenyl)-1-(3-((2,2,2-trifluoroethoxy)amino)propyl)pyrrolidin-2-one |
| 107 | 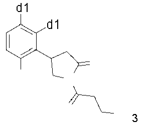 | rac-1-(3-Aminopropanoyl)-4-(2,3-dichloro-6-hydroxyphenyl)pyrrolidin-2-one |
| 108 | 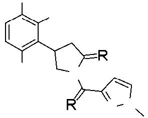 | rac-4-(2,3-Dichloro-6-hydroxyphenyl)-1-(1-methyl-1H-pyrazole-3-carbonyl)pyrrolidin-2-one |
| 109 | 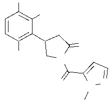 | rac-4-(2,3-Dichloro-6-hydroxyphenyl)-1-(1-methyl-1H-pyrazole-5-carbonyl)pyrrolidin-2-one |
| 110 | 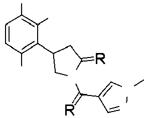 | rac-4-(2,3-Dichloro-6-hydroxyphenyl)-1-(1-methyl-1H-pyrazole-4-carbonyl)pyrrolidin-2-one |
| 111 | 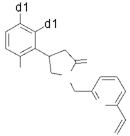 | rac-4-(2,3-Dichloro-6-hydroxyphenyl)-1-((6-vinylpyridin-2-yl)methyl)pyrrolidin-2-one |
| 112 | 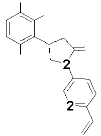 | rac-4-(2,3-Dichloro-6-hydroxyphenyl)-1-(6-vinylpyridin-3-yl)pyrrolidin-2-one |
| 113 | 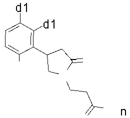 | rac-3-(4-(2,3-Dichloro-6-hydroxyphenyl)-2-oxopyrrolidin-1-yl)propanethioamide |
| 114 | 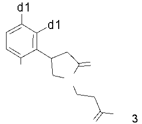 | rac-3-(4-(2,3-Dichloro-6-hydroxyphenyl)-2-oxopyrrolidin-1-yl)propionimide |
| 115 | 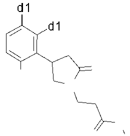 | rac-3-(4-(2,3-Dichloro-6-hydroxyphenyl)-2-oxopyrrolidin-1-yl)-N-hydroxypropionamide |
| 116 | 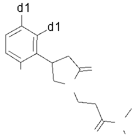 | rac-3-(4-(2,3-Dichloro-6-hydroxyphenyl)-2-oxopyrrolidin-1-yl)-N-hydroxy-N-methylpropionamide |
| 117 | 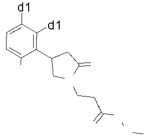 | rac-3-(4-(2,3-Dichloro-6-hydroxyphenyl)-2-oxopyrrolidin-1-yl)-N-methoxypropionamide |
| 118 | 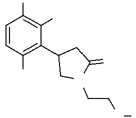 | rac-4-(2,3-Dichloro-6-hydroxyphenyl)-1-(2-(hydroxyamino)ethyl)pyrrolidin-2-one |
| 119 | 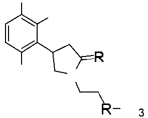 | rac-1-(2-(Aminooxy)ethyl)-4-(2,3-dichloro-6-hydroxyphenyl)pyrrolidin-2-one |
| 120 | 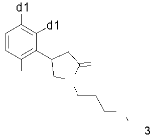 | rac-1-(3-(Aminooxy)propyl)-4-(2,3-dichloro-6-hydroxyphenyl)pyrrolidin-2-one |
| 121 | 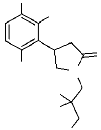 | rac-4-(2,3-Dichloro-6-hydroxyphenyl)-1-(2,2-difluoro-3-hydroxypropyl)pyrrolidine-2-thione |
| 122 | 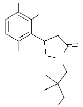 | rac-4-(2,3-Dichloro-6-hydroxyphenyl)-1-(3-hydroxy-2,2-dimethylpropyl)pyrrolidine-2-thione |
| 123 | 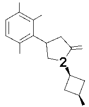 | rac-4-(2,3-Dichloro-6-hydroxyphenyl)-1-((1s,3s)-3-hydroxycyclobutyl)pyrrolidine-2-thione |
| 124 | 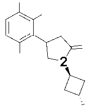 | rac-4-(2,3-Dichloro-6-hydroxyphenyl)-1-((1r,3r)-3-hydroxycyclobutyl)pyrrolidine-2-thione |
| 125 | 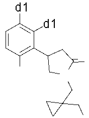 | rac-4-(2,3-Dichloro-6-hydroxyphenyl)-1-((1-(hydroxymethyl)cyclopropyl)methyl)pyrrolidine-2-thione |
| 126 | 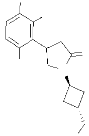 | rac-4-(2,3-Dichloro-6-hydroxyphenyl)-1-((1r,3r)-3-(hydroxymethyl)cyclobutyl)pyrrolidine-2-thione |
| 127 | 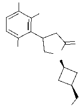 | rac-4-(2,3-Dichloro-6-hydroxyphenyl)-1-((1s,3s)-3-(hydroxymethyl)cyclobutyl)pyrrolidine-2-thione |
| 128 | 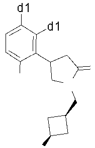 | rac-4-(2,3-Dichloro-6-hydroxyphenyl)-1-(((1s,3s)-3-hydroxycyclobutyl)methyl)pyrrolidine-2-thione |
| 129 | 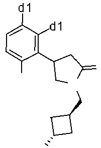 | rac-4-(2,3-Dichloro-6-hydroxyphenyl)-1-(((1r,3r)-3-hydroxycyclobutyl)methyl)pyrrolidine-2-thione |
| 130 | 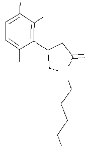 | rac-4-(2,3-Dichloro-6-hydroxyphenyl)-1-(4-hydroxybutyl)pyrrolidine-2-thione |
| 131 | 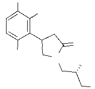 | rac-4-(2,3-Dichloro-6-hydroxyphenyl)-1-((S)-2,3-dihydroxypropyl)pyrrolidine-2-thione |
| 132 | 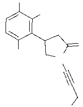 | rac-4-(2,3-Dichloro-6-hydroxyphenyl)-1-(3-hydroxyprop-1-yn-1-yl)pyrrolidine-2-thione |
| 133 | 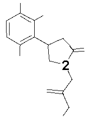 | rac-4-(2,3-Dichloro-6-hydroxyphenyl)-1-(2-(hydroxymethyl)allyl)pyrrolidine-2-thione |
| 134 | 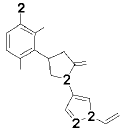 | rac-4-(2-Chloro-3-fluoro-6-hydroxyphenyl)-1-(1-vinyl-1H-pyrazol-4-yl)pyrrolidine-2-thione |
| 135 | 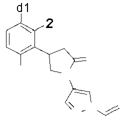 | rac-4-(3-Chloro-2-fluoro-6-hydroxyphenyl)-1-(1-vinyl-1H-pyrazol-4-yl)pyrrolidine-2-thione |
| 136 | 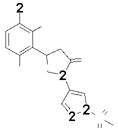 | rac-4-(2-Chloro-3-fluoro-6-hydroxyphenyl)-1-(1-(methylsulfonyl)-1H-pyrazol-4-yl)pyrrolidine-2-thione |
| 137 | 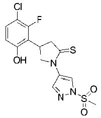 | rac-4-(3-Chloro-2-fluoro-6-hydroxyphenyl)-1-(1-(methylsulfonyl)-1H-pyrazol-4-yl)pyrrolidine-2-thione |
| 138 | 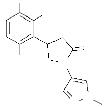 | rac-4-(2-Chloro-3-fluoro-6-hydroxyphenyl)-1-(1-methyl-1H-pyrazol-4-yl)pyrrolidine-2-thione |
| 139 | 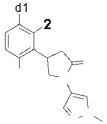 | rac-4-(3-Chloro-2-fluoro-6-hydroxyphenyl)-1-(1-methyl-1H-pyrazol-4-yl)pyrrolidine-2-thione |
| 140 | 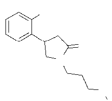 | rac-4-(2-Chloro-3-fluoro-6-hydroxyphenyl)-1-(3-(hydroxyamino)propyl)pyrrolidine-2-thione |
| 141 | 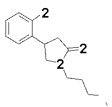 | rac-4-(3-Chloro-2-fluoro-6-hydroxyphenyl)-1-(3-(hydroxyamino)propyl)pyrrolidine-2-thione |
| 142 | 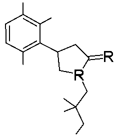 | rac-4-(2-Chloro-3-fluoro-6-hydroxyphenyl)-1-(2,2-difluoro-3-hydroxypropyl)pyrrolidine-2-thione |
| 143 | 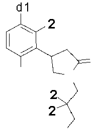 | rac-4-(3-Chloro-2-fluoro-6-hydroxyphenyl)-1-(2,2-difluoro-3-hydroxypropyl)pyrrolidine-2-thione |
| 144 | 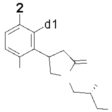 | rac-4-(2-Chloro-3-fluoro-6-hydroxyphenyl)-1-((S)-2,3-dihydroxypropyl)pyrrolidine-2-thione |
| 145 | 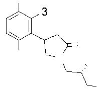 | rac-4-(3-Chloro-2-fluoro-6-hydroxyphenyl)-1-((S)-2,3-dihydroxypropyl)pyrrolidine-2-thione |
| 146 | 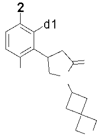 | rac-4-(2-Chloro-3-fluoro-6-hydroxyphenyl)-1-(2-azaspiro[3.3]heptan-6-yl)pyrrolidine-2-thione |
| 147 | 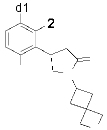 | rac-4-(3-Chloro-2-fluoro-6-hydroxyphenyl)-1-(2-azaspiro[3.3]heptan-6-yl)pyrrolidine-2-thione |
| 148 | 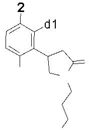 | rac-4-(2-Chloro-3-fluoro-6-hydroxyphenyl)-1-(3-hydroxypropyl)pyrrolidine-2-thione |
| 149 | 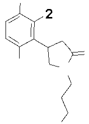 | rac-4-(3-Chloro-2-fluoro-6-hydroxyphenyl)-1-(3-hydroxypropyl)pyrrolidine-2-thione |
| 150 | 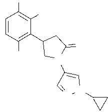 | rac-1-(1-Cyclopropyl-1H-pyrazol-4-yl)-4-(2,3-dichloro-6-hydroxyphenyl)pyrrolidine-2-thione |
| 151 | 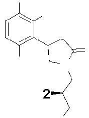 | rac-4-(2,3-Dichloro-6-hydroxyphenyl)-1-((S)-2-fluoro-3-hydroxypropyl)pyrrolidine-2-thione |
| 152 | 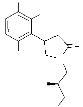 | rac-4-(2-Chloro-3-fluoro-6-hydroxyphenyl)-1-((S)-2-fluoro-3-hydroxypropyl)pyrrolidine-2-thione |
| 153 | 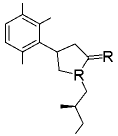 | rac-4-(3-Chloro-2-fluoro-6-hydroxyphenyl)-1-((S)-2-fluoro-3-hydroxypropyl)pyrrolidine-2-thione |
| 154 | 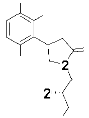 | rac-4-(2,3-Dichloro-6-hydroxyphenyl)-1-((R)-2-fluoro-3-hydroxypropyl)pyrrolidine-2-thione |
| 155 | 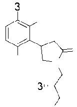 | rac-4-(2-Chloro-3-fluoro-6-hydroxyphenyl)-1-((R)-2-fluoro-3-hydroxypropyl)pyrrolidine-2-thione |
| 156 | 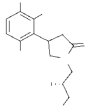 | rac-4-(3-Chloro-2-fluoro-6-hydroxyphenyl)-1-((R)-2-fluoro-3-hydroxypropyl)pyrrolidine-2-thione |
| 157 | 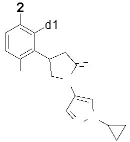 | rac-4-(2-Chloro-3-fluoro-6-hydroxyphenyl)-1-(1-cyclopropyl-1H-pyrazol-4-yl)pyrrolidine-2-thione |
| 158 | 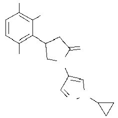 | rac-4-(3-Chloro-2-fluoro-6-hydroxyphenyl)-1-(1-cyclopropyl-1H-pyrazol-4-yl)pyrrolidine-2-thione |
| 159 | 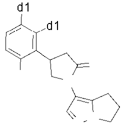 | rac-4-(2,3-Dichloro-6-hydroxyphenyl)-1-(5,6-dihydro-4H-pyrrolo[1,2-b]pyrazol-3-yl)pyrrolidine-2-thione |
| 160 | 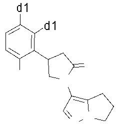 | rac-4-(2,3-Dichloro-6-hydroxyphenyl)-1-(5,6-dihydro-4H-pyrrolo[1,2-b]pyrazol-3-yl)pyrrolidin-2-one |
| 161 | 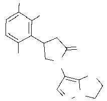 | rac-4-(2,3-Dichloro-6-hydroxyphenyl)-1-(2,3-dihydropyrazolo[5,1-b]oxazol-7-yl)pyrrolidine-2-thione |
| 162 | 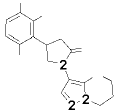 | rac-4-(2,3-Dichloro-6-hydroxyphenyl)-1-(6,7-dihydro-5H-pyrazolo[5,1-b][1,3]oxazin-3-yl)pyrrolidine-2-thione |
| 163 | 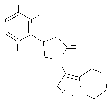 | rac-4-(2,3-Dichloro-6-hydroxyphenyl)-1-(6,7-dihydro-4H-pyrazolo[5,1-c][1,4]oxazin-3-yl)pyrrolidine-2-thione |
| 164 | 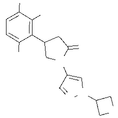 | rac-4-(2,3-Dichloro-6-hydroxyphenyl)-1-(1-(oxetan-3-yl)-1H-pyrazol-4-yl)pyrrolidine-2-thione |
| 165 | 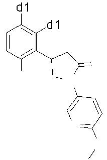 | rac-4-(2,3-Dichloro-6-hydroxyphenyl)-1-(6-methoxypyridin-3-yl)pyrrolidine-2-thione |
| 166 | 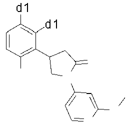 | rac-4-(2,3-Dichloro-6-hydroxyphenyl)-1-(2-methoxypyridin-4-yl)pyrrolidine-2-thione |
| 167 | 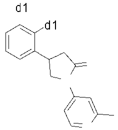 | rac-4-(2,3-Dichloro-6-hydroxyphenyl)-1-(2-methylpyridin-4-yl)pyrrolidine-2-thione |
| 168 | 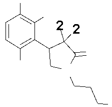 | rac-4-(2,3-Dichloro-6-hydroxyphenyl)-3,3-difluoro-1-(3-hydroxypropyl)pyrrolidin-2-one |
| 169 | 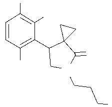 | rac-7-(2,3-Dichloro-6-hydroxyphenyl)-5-(3-hydroxypropyl)-5-azaspiro[2.4]heptan-4-one |
| 1-1 | 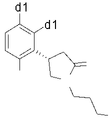 | (*S*)-4-(2,3-Dichloro-6-hydroxyphenyl)-1-(3-hydroxypropyl)pyrrolidine-2-thione |
| 2-1 | 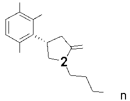 | (*S*)-1-(3-Aminopropyl)-4-(2,3-dichloro-6-hydroxyphenyl)pyrrolidine-2-thione |
| 3-1 | 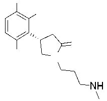 | (*S*)-4-(2,3-Dichloro-6-hydroxyphenyl)-1-(3-(methylamino)propyl)pyrrolidine-2-thione |
| 4-1 | 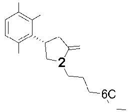 | (*S*)-4-(2,3-Dichloro-6-hydroxyphenyl)-1-(3-(methoxyamino)propyl)pyrrolidine-2-thione |
| 5-1 | 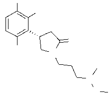 | (*S*)-4-(2,3-Dichloro-6-hydroxyphenyl)-1-(3-(methoxy(methyl)amino)propyl)pyrrolidine-2-thione |
| 6-1 | 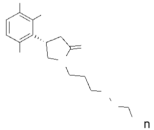 | (*S*)-4-(2,3-Dichloro-6-hydroxyphenyl)-1-(3-((2,2,2-trifluoroethoxy)amino)propyl)pyrrolidine-2-thione |
| 7-1 | 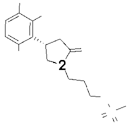 | (*S*)-*N*-(3-(4-(2,3-Dichloro-6-hydroxyphenyl)-2-thiopyrrolidin-1-yl)propyl)methanesulfonamide |
| 8-1 | 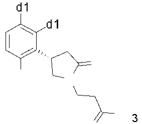 | (*S*)-3-(4-(2,3-Dichloro-6-hydroxyphenyl)-2-thiopyrrolidin-1-yl)propionamide |
| 9-1 | 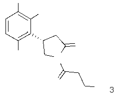 | (*S*)-3-Amino-1-(4-(2,3-dichloro-6-hydroxyphenyl)-2-thiopyrrolidin-1-yl)propan-1-one |
| 10-1 | 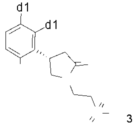 | (*S*)-2-(4-(2,3-Dichloro-6-hydroxyphenyl)-2-thiopyrrolidin-1-yl)ethane-1-sulfonamide |
| 11-1 | 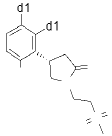 | (*S*)- (2-(4-(2,3-Dichloro-6-hydroxyphenyl)-2-thiopyrrolidin-1-yl)ethyl)(imino)(methyl)-λ⁶-sulfanone |
| 12-1 | 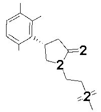 | (*S*)-4-(2,3-Dichloro-6-hydroxyphenyl)-1-(2-(methylsulfonyl)ethyl)pyrrolidine-2-thione |
| 13-1 | 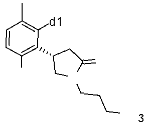 | (*S*)-1-(3-Aminopropyl)-4-(2-chloro-6-hydroxy-3-methylphenyl)pyrrolidine-2-thione |
| 14-1 | 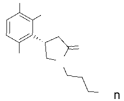 | (*S*)-1-(3-Aminopropyl)-4-(2-chloro-3-fluoro-6-hydroxyphenyl)pyrrolidine-2-thione |
| 15-1 | 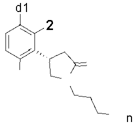 | (*S*)-1-(3-Aminopropyl)-4-(3-chloro-2-fluoro-6-hydroxyphenyl)pyrrolidine-2-thione |
| 16-1 | 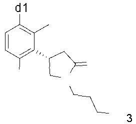 | (*S*)-1-(3-Aminopropyl)-4-(3-chloro-6-hydroxy-2-methylphenyl)pyrrolidine-2-thione |
| 17-1 | 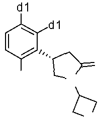 | (*S*)-1-(Azetidin-3-yl)-4-(2,3-dichloro-6-hydroxyphenyl)pyrrolidine-2-thione |
| 18-1 | 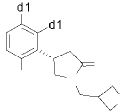 | (*S*)-1-(Azetidin-3-ylmethyl)-4-(2,3-dichloro-6-hydroxyphenyl)pyrrolidine-2-thione |
| 19-1 | 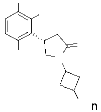 | (*S*)-1-(3-Aminocyclobutyl)-4-(2,3-dichloro-6-hydroxyphenyl)pyrrolidine-2-thione |
| 20-1 | 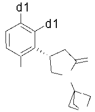 | (*S*)-1-(1-Azabicyclo[1.1.1]pentan-3-yl)-4-(2,3-dichloro-6-hydroxyphenyl)pyrrolidine-2-thione |
| 21-1 | 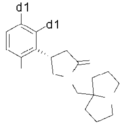 | (*S*)-4-(2,3-Dichloro-6-hydroxyphenyl)-1-((tetrahydro-1H-pyrrolizin-7a(5H)-yl)methyl)pyrrolidine-2-thione |
| 22-1 | 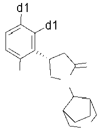 | (*S*)-1-(3-Azabicyclo[3.2.1]octan-8-yl)-4-(2,3-dichloro-6-hydroxyphenyl)pyrrolidine-2-thione |
| 23-1 | 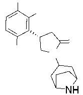 | (*S*)-1-(8-Azabicyclo[3.2.1]octan-3-yl)-4-(2,3-dichloro-6-hydroxyphenyl)pyrrolidine-2-thione |
| 24-1 | 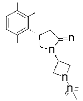 | (*S*)-4-(2,3-Dichloro-6-hydroxyphenyl)-1-(1-(methylsulfonyl)azetidin-3-yl)pyrrolidine-2-thione |
| 25-1 | 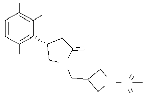 | (*S*)-4-(2,3-Dichloro-6-hydroxyphenyl)-1-((1-(methylsulfonyl)azetidin-3-yl)methyl)pyrrolidine-2-thione |
| 26-1 | 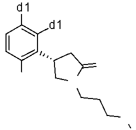 | (*S*)- 4-(2,3-Dichloro-6-hydroxyphenyl)-1-(3-(hydroxyamino)propyl)pyrrolidine-2-thione |
| 27-1 | 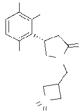 | (*S*)-4-(2,3-Dichloro-6-hydroxyphenyl)-1-((1,1-dioxothietan-3-yl)methyl)pyrrolidine-2-thione |
| 28-1 | 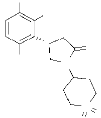 | (*S*)-4-(2,3-Dichloro-6-hydroxyphenyl)-1-(1,1-dioxotetrahydro-2H-thiapyran-4-yl)pyrrolidine-2-thione |
| 29-1 | 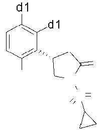 | (*S*)-1-(Cyclopropylsulfonyl)-4-(2,3-dichloro-6-hydroxyphenyl)pyrrolidine-2-thione |
| 30-1 | 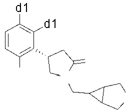 | (*S*)-1-((3-Oxabicyclo[3.1.0]hexan-6-yl)methyl)-4-(2,3-dichloro-6-hydroxyphenyl)pyrrolidine-2-thione |
| 31-1 | 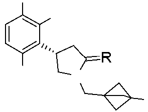 | (*S*)-4-(2,3-Dichloro-6-hydroxyphenyl)-1-((3-hydroxybicyclo[1.1.1]pentan-1-yl)methyl)pyrrolidine-2-thione |
| 32-1 | 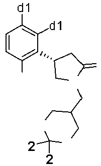 | (*S*)-4-(2,3-Dichloro-6-hydroxyphenyl)-1-((2,2-difluoro-1,3-dioxan-5-yl)methyl)pyrrolidine-2-thione |
| 33-1 | 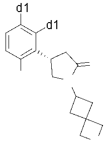 | (*S*)-4-(2,3-Dichloro-6-hydroxyphenyl)-1-(2-oxaspiro[3.3]heptan-6-yl)pyrrolidine-2-thione |
| 34-1 | 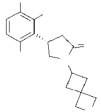 | (*S*)-4-(2,3-Dichloro-6-hydroxyphenyl)-1-(2-azaspiro[3.3]heptan-6-yl)pyrrolidine-2-thione |
| 35-1 | 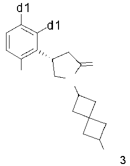 | (*S*)-1-(6-Aminospiro[3.3]heptan-2-yl)-4-(2,3-dichloro-6-hydroxyphenyl)pyrrolidine-2-thione |
| 36-1 | 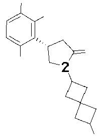 | (*S*)-4-(2,3-Dichloro-6-hydroxyphenyl)-1-(6-hydroxyspiro[3.3]heptan-2-yl)pyrrolidine-2-thione |
| 37-1 | 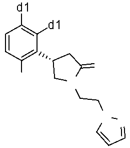 | (*S*)-1-(2-(1H-Pyrazol-1-yl)ethyl)-4-(2,3-dichloro-6-hydroxyphenyl)pyrrolidine-2-thione |
| 38-1 | 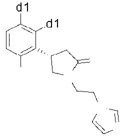 | (*S*)-1-(2-(1H-Imidazol-1-yl)ethyl)-4-(2,3-dichloro-6-hydroxyphenyl)pyrrolidine-2-thione |
| 39-1 | 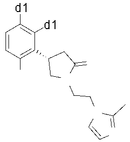 | (*S*)-4-(2,3-Dichloro-6-hydroxyphenyl)-1-(2-(2-methyl-1H-imidazol-1-yl)ethyl)pyrrolidine-2-thione |
| 40-1 | 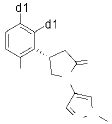 | (*S*)-4-(2,3-Dichloro-6-hydroxyphenyl)-1-(1-methyl-1H-pyrazol-4-yl)pyrrolidine-2-thione |
| 41-1 | 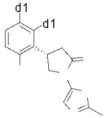 | (*S*)-4-(2,3-Dichloro-6-hydroxyphenyl)-1-(2-methyl-1H-imidazol-5-yl)pyrrolidine-2-thione |
| 42-1 | 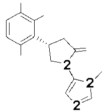 | (*S*)-4-(2,3-Dichloro-6-hydroxyphenyl)-1-(1-methyl-1H-imidazol-5-yl)pyrrolidine-2-thione |
| 43-1 | 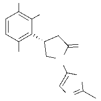 | (*S*)-4-(2,3-Dichloro-6-hydroxyphenyl)-1-(2-methylthiazol-5-yl)pyrrolidine-2-thione |
| 44-1 | 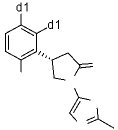 | (*S*)-4-(2,3-Dichloro-6-hydroxyphenyl)-1-(2-methyloxazol-5-yl)pyrrolidine-2-thione |
| 45-1 | 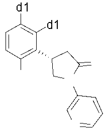 | (*S*)-4-(2,3-Dichloro-6-hydroxyphenyl)-1-(pyridin-3-yl)pyrrolidine-2-thione |
| 46-1 | 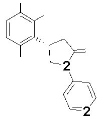 | (*S*)-4-(2,3-Dichloro-6-hydroxyphenyl)-1-(pyridin-4-yl)pyrrolidine-2-thione |
| 47-1 | 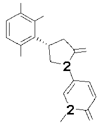 | (*S*)-5-(4-(2,3-Dichloro-6-hydroxyphenyl)-2-thiopyrrolidin-1-yl)-1-methylpyridin-2(1H)-one |
| 48-1 | 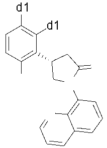 | (*S*)-4-(2,3-Dichloro-6-hydroxyphenyl)-1-(quinolin-8-yl)pyrrolidine-2-thione |
| 49-1 | 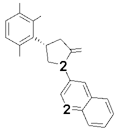 | (*S*)-4-(2,3-Dichloro-6-hydroxyphenyl)-1-(quinolin-3-yl)pyrrolidine-2-thione |
| 50-1 | 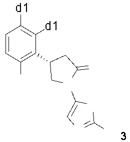 | (*S*)-4-(2,3-Dichloro-6-hydroxyphenyl)-1-(2-(trifluoromethyl)thiazol-5-yl)pyrrolidine-2-thione |
| 51-1 | 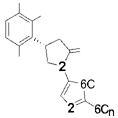 | (*S*)-4-(2,3-Dichloro-6-hydroxyphenyl)-1-(2-(trifluoromethyl)-1H-imidazol-5-yl)pyrrolidine-2-thione |
| 52-1 | 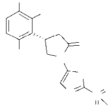 | (*S*)-4-(2,3-Dichloro-6-hydroxyphenyl)-1-(2-(methylsulfonyl)thiazol-5-yl)pyrrolidine-2-thione |
| 53-1 | 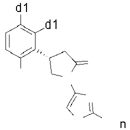 | (*S*)-1-(2-Aminooxazol-4-yl)-4-(2,3-dichloro-6-hydroxyphenyl)pyrrolidine-2-thione |
| 54-1 | 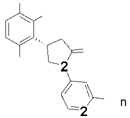 | (*S*)-1-(2-Aminopyridin-4-yl)-4-(2,3-dichloro-6-hydroxyphenyl)pyrrolidine-2-thione |
| 55-1 | 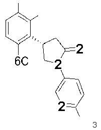 | (*S*)-1-(6-Aminopyridin-3-yl)-4-(2,3-dichloro-6-hydroxyphenyl)pyrrolidine-2-thione |
| 56-1 | 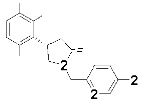 | (*S*)-4-(2,3-Dichloro-6-hydroxyphenyl)-1-((5-fluoropyridin-2-yl)methyl)pyrrolidine-2-thione |
| 57-1 | 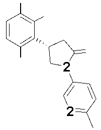 | (*S*)-4-(2,3-Dichloro-6-hydroxyphenyl)-1-(6-methylpyridin-3-yl)pyrrolidine-2-thione |
| 58-1 | 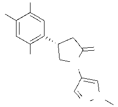 | (*S*)-4-(4,5-Dichloro-2-hydroxyphenyl)-1-(1-methyl-1H-pyrazol-4-yl)pyrrolidine-2-thione |
| 59-1 | 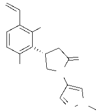 | (*S*)-4-(2-Chloro-6-hydroxy-3-vinylphenyl)-1-(1-methyl-1H-pyrazol-4-yl)pyrrolidine-2-thione |
| 60-1 | 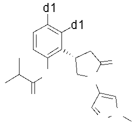 | (*S*)-3,4-Dichloro-2-(1-(1-methyl-1H-pyrazol-4-yl)-5-thiopyrrolidin-3-yl)phenyl isobutyrate |
| 61-1 | 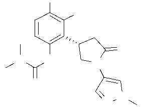 | (*S*)-3,4-Dichloro-2-(1-(1-methyl-1H-pyrazol-4-yl)-5-thiopyrrolidin-3-yl)phenyl dimethylcarbamate |
| 62-1 | 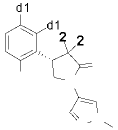 | (*S*)-4-(2,3-Dichloro-6-hydroxyphenyl)-3,3-difluoro-1-(1-methyl-1H-pyrazol-4-yl)pyrrolidine-2-thione |
| 63-1 | 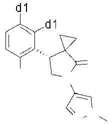 | (*S*)-7-(2,3-Dichloro-6-hydroxyphenyl)-5-(1-methyl-1H-pyrazol-4-yl)-5-azaspiro[2.4]heptane-4-thione |
| 64-1 | 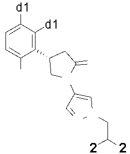 | (*S*)-4-(2,3-Dichloro-6-hydroxyphenyl)-1-(1-(2,2-difluoroethyl)-1H-pyrazol-4-yl)pyrrolidine-2-thione |
| 65-1 | 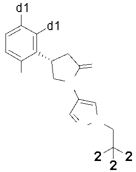 | (*S*)-4-(2,3-Dichloro-6-hydroxyphenyl)-1-(1-(2,2,2-trifluoroethyl)-1H-pyrazol-4-yl)pyrrolidine-2-thione |
| 66-1 | 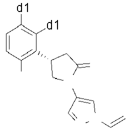 | (*S*)-4-(2,3-Dichloro-6-hydroxyphenyl)-1-(1-vinyl-1H-pyrazol-4-yl)pyrrolidine-2-thione |
| 67-1 | 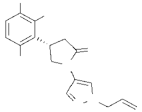 | (*S*)-1-(1-Allyl-1H-pyrazol-4-yl)-4-(2,3-dichloro-6-hydroxyphenyl)pyrrolidine-2-thione |
| 68-1 | 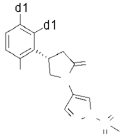 | (*S*)-4-(2,3-Dichloro-6-hydroxyphenyl)-1-(1-(methylsulfonyl)-1H-pyrazol-4-yl)pyrrolidine-2-thione |
| 69-1 | 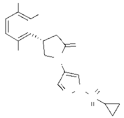 | (*S*)-1-(1-(Cyclopropylsulfonyl)-1H-pyrazol-4-yl)-4-(2,3-dichloro-6-hydroxyphenyl)pyrrolidine-2-thione |
| 70-1 | 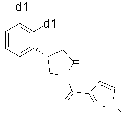 | (*S*)- (4-(2,3-Dichloro-6-hydroxyphenyl)-2-thiopyrrolidin-1-yl)(1-methyl-1H-pyrazol-3-yl)methanone |
| 71-1 | 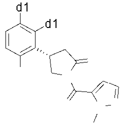 | (*S*)- (4-(2,3-Dichloro-6-hydroxyphenyl)-2-thiopyrrolidin-1-yl)(1-methyl-1H-pyrazol-5-yl)methanone |
| 72-1 | 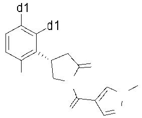 | (*S*)- (4-(2,3-Dichloro-6-hydroxyphenyl)-2-thiopyrrolidin-1-yl)(1-methyl-1H-pyrazol-4-yl)methanone |
| 73-1 | 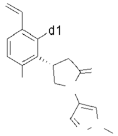 | (*S*)-4-(2-Chloro-6-hydroxy-3-vinylphenyl)-1-(1-methyl-1H-pyrazol-4-yl)pyrrolidin-2-one |
| 74-1 | 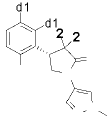 | (*S*)-4-(2,3-Dichloro-6-hydroxyphenyl)-3,3-difluoro-1-(1-methyl-1H-pyrazol-4-yl)pyrrolidin-2-one |
| 75-1 | 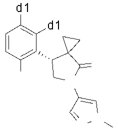 | (*S*)-7-(2,3-Dichloro-6-hydroxyphenyl)-5-(1-methyl-1H-pyrazol-4-yl)-5-azaspiro[2.4]heptan-4-one |
| 76-1 | 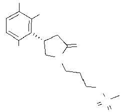 | (*S*)-N-(3-(4-(2,3-Dichloro-6-hydroxyphenyl)-2-oxopyrrolidin-1-yl)propyl)methanesulfonamide |
| 77-1 | 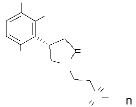 | (*S*)-2-(4-(2,3-Dichloro-6-hydroxyphenyl)-2-oxopyrrolidin-1-yl)ethane-1-sulfonamide |
| 78-1 | 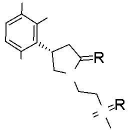 | (*S*)-4-(2,3-Dichloro-6-hydroxyphenyl)-1-(2-(S-methylsulfonylimino)ethyl)pyrrolidin-2-one |
| 79-1 | 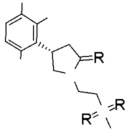 | (*S*)-4-(2,3-Dichloro-6-hydroxyphenyl)-1-(2-(methylsulfonyl)ethyl)pyrrolidin-2-one |
| 80-1 | 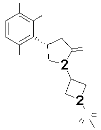 | (*S*)-4-(2,3-Dichloro-6-hydroxyphenyl)-1-(1-(methylsulfonyl)azetidin-3-yl)pyrrolidin-2-one |
| 81-1 | 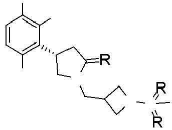 | (*S*)-4-(2,3-Dichloro-6-hydroxyphenyl)-1-((1-(methylsulfonyl)azetidin-3-yl)methyl)pyrrolidin-2-one |
| 82-1 | 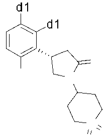 | (*S*)-4-(2,3-Dichloro-6-hydroxyphenyl)-1-(1-imino-1-oxohexahydro-1l6-thiopyran-4-yl)pyrrolidin-2-one |
| 83-1 | 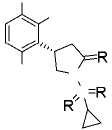 | (*S*)-1-(Cyclopropylsulfonyl)-4-(2,3-dichloro-6-hydroxyphenyl)pyrrolidin-2-one |
| 84-1 | 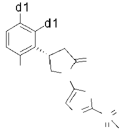 | (*S*)-4-(2,3-Dichloro-6-hydroxyphenyl)-1-(2-(methylsulfonyl)thiazol-5-yl)pyrrolidin-2-one |
| 85-1 | 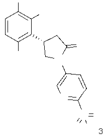 | (*S*)-5-(4-(2,3-Dichloro-6-hydroxyphenyl)-2-oxopyrrolidin-1-yl)pyridine-2-sulfonamide |
| 86-1 | 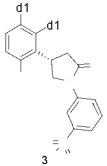 | (*S*)-3-(4-(2,3-Dichloro-6-hydroxyphenyl)-2-oxopyrrolidin-1-yl)benzenesulfonamide |
| 87-1 | 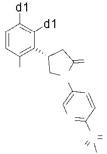 | (*S*)-4-(2,3-Dichloro-6-hydroxyphenyl)-1-(6-(methylsulfonyl)pyridin-3-yl)pyrrolidin-2-one |
| 88-1 | 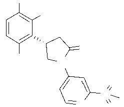 | (*S*)-4-(2,3-Dichloro-6-hydroxyphenyl)-1-(2-(methylsulfonyl)pyridin-4-yl)pyrrolidin-2-one |
| 89-1 | 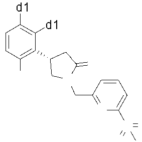 | (*S*)-4-(2,3-Dichloro-6-hydroxyphenyl)-1-((6-(methylsulfonyl)pyridin-2-yl)methyl)pyrrolidin-2-one |
| 90-1 | 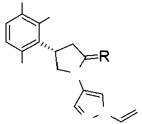 | (*S*)-4-(2,3-Dichloro-6-hydroxyphenyl)-1-(1-vinyl-1H-pyrazol-4-yl)pyrrolidin-2-one |
| 91-1 | 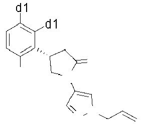 | (*S*)-1-(1-Allyl-1H-pyrazol-4-yl)-4-(2,3-dichloro-6-hydroxyphenyl)pyrrolidin-2-one |
| 92-1 | 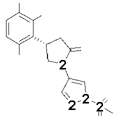 | (*S*)-4-(2,3-Dichloro-6-hydroxyphenyl)-1-(1-(methylsulfonyl)-1H-pyrazol-4-yl)pyrrolidin-2-one |
| 93-1 | 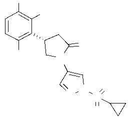 | (*S*)-1-(1-(Cyclopropylsulfonyl)-1H-pyrazol-4-yl)-4-(2,3-dichloro-6-hydroxyphenyl)pyrrolidin-2-one |
| 94-1 | 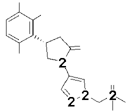 | (*S*)-4-(2,3-Dichloro-6-hydroxyphenyl)-1-(1-((dimethylphosphoryl)methyl)-1H-pyrazol-4-yl)pyrrolidin-2-one |
| 95-1 | 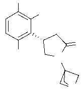 | (*S*)-1-(1-Azabicyclo[1.1.1]pentan-3-yl)-4-(2,3-dichloro-6-hydroxyphenyl)pyrrolidin-2-one |
| 96-1 | 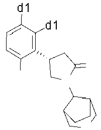 | (*S*)-1-(3-Azabicyclo[3.2.1]octan-8-yl)-4-(2,3-dichloro-6-hydroxyphenyl)pyrrolidin-2-one |
| 97-1 | 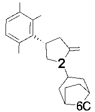 | (*S*)-1-(8-Azabicyclo[3.2.1]octan-3-yl)-4-(2,3-dichloro-6-hydroxyphenyl)pyrrolidin-2-one |
| 98-1 | 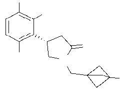 | (*S*)-4-(2,3-Dichloro-6-hydroxyphenyl)-1-(3-hydroxybicyclo[1.1.1]pentan-1-yl)methyl)pyrrolidin-2-one |
| 99-1 | 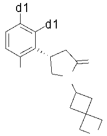 | (*S*)-4-(2,3-Dichloro-6-hydroxyphenyl)-1-(2-oxaspiro[3.3]heptan-6-yl)pyrrolidin-2-one |
| 100-1 | 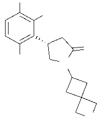 | (*S*)-4-(2,3-Dichloro-6-hydroxyphenyl)-1-(2-azaspiro[3.3]heptan-6-yl)pyrrolidin-2-one |
| 101-1 | 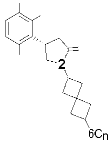 | (*S*)-1-(6-Aminospiro[3.3]heptan-2-yl)-4-(2,3-dichloro-6-hydroxyphenyl)pyrrolidin-2-one |
| 102-1 | 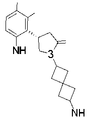 | (*S*)-4-(2,3-Dichloro-6-hydroxyphenyl)-1-(6-hydroxyspiro[3.3]heptan-2-yl)pyrrolidin-2-one |
| 103-1 | 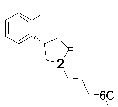 | (*S*)-4-(2,3-Dichloro-6-hydroxyphenyl)-1-(3-(hydroxyamino)propyl)pyrrolidin-2-one |
| 104-1 | 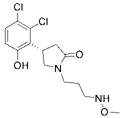 | (*S*)-4-(2,3-Dichloro-6-hydroxyphenyl)-1-(3-(methoxyamino)propyl)pyrrolidin-2-one |
| 105-1 | 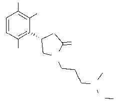 | (*S*)-4-(2,3-Dichloro-6-hydroxyphenyl)-1-(3-(methoxy(methyl)amino)propyl)pyrrolidin-2-one |
| 106-1 | 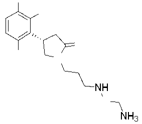 | (*S*)-4-(2,3-Dichloro-6-hydroxyphenyl)-1-(3-((2,2,2-trifluoroethoxy)amino)propyl)pyrrolidin-2-one |
| 107-1 | 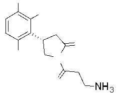 | (*S*)-1-(3-Aminopropanoyl)-4-(2,3-dichloro-6-hydroxyphenyl)pyrrolidin-2-one |
| 108-1 | 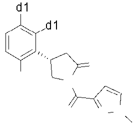 | (*S*)-4-(2,3-Dichloro-6-hydroxyphenyl)-1-(1-methyl-1H-pyrazole-3-carbonyl)pyrrolidin-2-one |
| 109-1 | 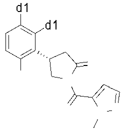 | (*S*)-4-(2,3-Dichloro-6-hydroxyphenyl)-1-(1-methyl-1H-pyrazole-5-carbonyl)pyrrolidin-2-one |
| 110-1 | 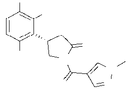 | (*S*)-4-(2,3-Dichloro-6-hydroxyphenyl)-1-(1-methyl-1H-pyrazole-4-carbonyl)pyrrolidin-2-one |
| 111-1 | 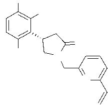 | (*S*)-4-(2,3-Dichloro-6-hydroxyphenyl)-1-((6-vinylpyridin-2-yl)methyl)pyrrolidin-2-one |
| 112-1 | 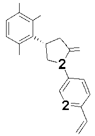 | (*S*)-4-(2,3-Dichloro-6-hydroxyphenyl)-1-(6-vinylpyridin-3-yl)pyrrolidin-2-one |
| 113-1 | 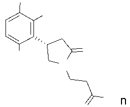 | (*S*)-3-(4-(2,3-Dichloro-6-hydroxyphenyl)-2-oxopyrrolidin-1-yl)propanethioamide |
| 114-1 | 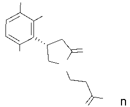 | (*S*)-3-(4-(2,3-Dichloro-6-hydroxyphenyl)-2-oxopyrrolidin-1-yl)propionimide |
| 115-1 | 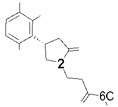 | (*S*)-3-(4-(2,3-Dichloro-6-hydroxyphenyl)-2-oxopyrrolidin-1-yl)-N-hydroxypropionamide |
| 116-1 | 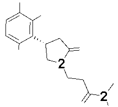 | (*S*)-3-(4-(2,3-Dichloro-6-hydroxyphenyl)-2-oxopyrrolidin-1-yl)-N-hydroxy-N-methylpropionamide |
| 117-1 | 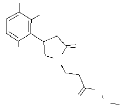 | (*S*)-3-(4-(2,3-Dichloro-6-hydroxyphenyl)-2-oxopyrrolidin-1-yl)-N-methoxypropionamide |
| 118-1 | 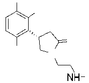 | (*S*)-4-(2,3-Dichloro-6-hydroxyphenyl)-1-(2-(hydroxyamino)ethyl)pyrrolidin-2-one |
| 119-1 | 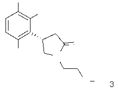 | (*S*)-1-(2-(Aminooxy)ethyl)-4-(2,3-dichloro-6-hydroxyphenyl)pyrrolidin-2-one |
| 120-1 | 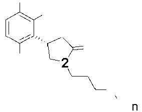 | (*S*)-1-(3-(Aminooxy)propyl)-4-(2,3-dichloro-6-hydroxyphenyl)pyrrolidin-2-one |
| 121-1 | 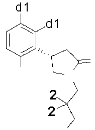 | (*S*)-4-(2,3-Dichloro-6-hydroxyphenyl)-1-(2,2-difluoro-3-hydroxypropyl)pyrrolidine-2-thione |
| 122-1 | 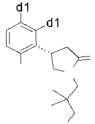 | (*S*)-4-(2,3-Dichloro-6-hydroxyphenyl)-1-(3-hydroxy-2,2-dimethylpropyl)pyrrolidine-2-thione |
| 123-1 | 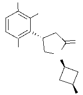 | (*S*)-4-(2,3-Dichloro-6-hydroxyphenyl)-1-((1s,3s)-3-hydroxycyclobutyl)pyrrolidine-2-thione |
| 124-1 | 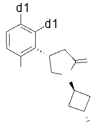 | (S)-4-(2,3-Dichloro-6-hydroxyphenyl)-1-((1r,3r)-3-hydroxycyclobutyl)pyrrolidine-2-thione |
| 125-1 | 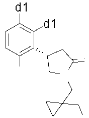 | (S)-4-(2,3-Dichloro-6-hydroxyphenyl)-1-((1-(hydroxymethyl)cyclopropyl)methyl)pyrrolidine-2-thione |
| 126-1 | 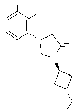 | (S)-4-(2,3-Dichloro-6-hydroxyphenyl)-1-((1r,3r)-3-(hydroxymethyl)cyclobutyl)pyrrolidine-2-thione |
| 127-1 | 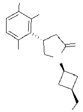 | (S)-4-(2,3-Dichloro-6-hydroxyphenyl)-1-((1s,3s)-3-(hydroxymethyl)cyclobutyl)pyrrolidine-2-thione |
| 128-1 | 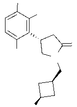 | (S)-4-(2,3-Dichloro-6-hydroxyphenyl)-1-(((1s,3s)-3-hydroxycyclobutyl)methyl)pyrrolidine-2-thione |
| 129-1 | 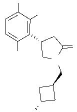 | (S)-4-(2,3-Dichloro-6-hydroxyphenyl)-1-(((1r,3r)-3-hydroxycyclobutyl)methyl)pyrrolidine-2-thione |
| 130-1 | 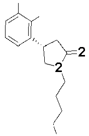 | (S)-4-(2,3-Dichloro-6-hydroxyphenyl)-1-(4-hydroxybutyl)pyrrolidine-2-thione |
| 131-1 | 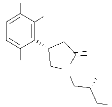 | (S)-4-(2,3-Dichloro-6-hydroxyphenyl)-1-((S)-2,3-dihydroxypropyl)pyrrolidine-2-thione |
| 132-1 | 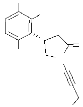 | (S)-4-(2,3-Dichloro-6-hydroxyphenyl)-1-(3-hydroxyprop-1-yn-1-yl)pyrrolidine-2-thione |
| 133-1 | 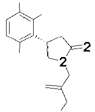 | (S)-4-(2,3-Dichloro-6-hydroxyphenyl)-1-(2-(hydroxymethyl)allyl)pyrrolidine-2-thione |
| 134-1 | 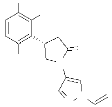 | (S)-4-(2-Chloro-3-fluoro-6-hydroxyphenyl)-1-(1-vinyl-1H-pyrazol-4-yl)pyrrolidine-2-thione |
| 135-1 | 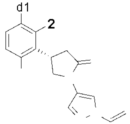 | (S)-4-(3-Chloro-2-fluoro-6-hydroxyphenyl)-1-(1-vinyl-1H-pyrazol-4-yl)pyrrolidine-2-thione |
| 136-1 | 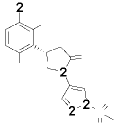 | (S)-4-(2-Chloro-3-fluoro-6-hydroxyphenyl)-1-(1-(methylsulfonyl)-1H-pyrazol-4-yl)pyrrolidine-2-thione |
| 137-1 | 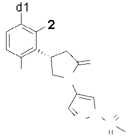 | (S)-4-(3-Chloro-2-fluoro-6-hydroxyphenyl)-1-(1-(methylsulfonyl)-1H-pyrazol-4-yl)pyrrolidine-2-thione |
| 138-1 | 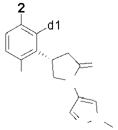 | (S)-4-(2-Chloro-3-fluoro-6-hydroxyphenyl)-1-(1-methyl-1H-pyrazol-4-yl)pyrrolidine-2-thione |
| 139-1 | 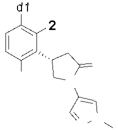 | (S)-4-(3-Chloro-2-fluoro-6-hydroxyphenyl)-1-(1-methyl-1H-pyrazol-4-yl)pyrrolidine-2-thione |
| 140-1 | 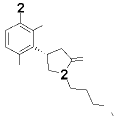 | (S)-4-(2-Chloro-3-fluoro-6-hydroxyphenyl)-1-(3-(hydroxyamino)propyl)pyrrolidine-2-thione |
| 141-1 | 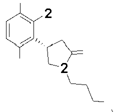 | (S)-4-(3-Chloro-2-fluoro-6-hydroxyphenyl)-1-(3-(hydroxyamino)propyl)pyrrolidine-2-thione |
| 142-1 | 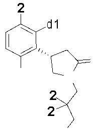 | (S)-4-(2-Chloro-3-fluoro-6-hydroxyphenyl)-1-(2,2-difluoro-3-hydroxypropyl)pyrrolidine-2-thione |
| 143-1 | 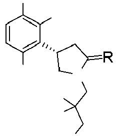 | (S)-4-(3-Chloro-2-fluoro-6-hydroxyphenyl)-1-(2,2-difluoro-3-hydroxypropyl)pyrrolidine-2-thione |
| 144-1 | 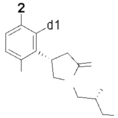 | (S)-4-(2-Chloro-3-fluoro-6-hydroxyphenyl)-1-((S)-2,3-dihydroxypropyl)pyrrolidine-2-thione |
| 145-1 | 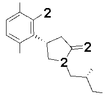 | (S)-4-(3-Chloro-2-fluoro-6-hydroxyphenyl)-1-((S)-2,3-dihydroxypropyl)pyrrolidine-2-thione |
| 146-1 | 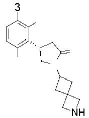 | (S)-4-(2-Chloro-3-fluoro-6-hydroxyphenyl)-1-(2-azaspiro[3.3]heptan-6-yl)pyrrolidine-2-thione |
| 147-1 | 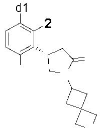 | (S)-4-(3-Chloro-2-fluoro-6-hydroxyphenyl)-1-(2-azaspiro[3.3]heptan-6-yl)pyrrolidine-2-thione |
| 148-1 | 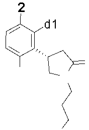 | (S)-4-(2-Chloro-3-fluoro-6-hydroxyphenyl)-1-(3-hydroxypropyl)pyrrolidine-2-thione |
| 149-1 | 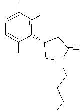 | (S)-4-(3-Chloro-2-fluoro-6-hydroxyphenyl)-1-(3-hydroxypropyl)pyrrolidine-2-thione |
| 150-1 | 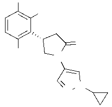 | (S)-1-(1-Cyclopropyl-1H-pyrazol-4-yl)-4-(2,3-dichloro-6-hydroxyphenyl)pyrrolidine-2-thione |
| 151-1 | 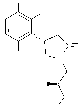 | (S)-4-(2,3-Dichloro-6-hydroxyphenyl)-1-((S)-2-fluoro-3-hydroxypropyl)pyrrolidine-2-thione |
| 152-1 | 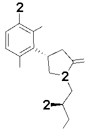 | (S)-4-(2-Chloro-3-fluoro-6-hydroxyphenyl)-1-((S)-2-fluoro-3-hydroxypropyl)pyrrolidine-2-thione |
| 153-1 | 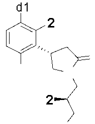 | (S)-4-(3-Chloro-2-fluoro-6-hydroxyphenyl)-1-((S)-2-fluoro-3-hydroxypropyl)pyrrolidine-2-thione |
| 154-1 | 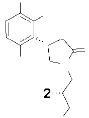 | (*S*)-4-(2,3-Dichloro-6-hydroxyphenyl)-1-((R)-2-fluoro-3-hydroxypropyl)pyrrolidine-2-thione |
| 155-1 | 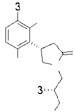 | (*S*)-4-(2-Chloro-3-fluoro-6-hydroxyphenyl)-1-((R)-2-fluoro-3-hydroxypropyl)pyrrolidine-2-thione |
| 156-1 | 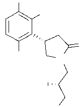 | (*S*)-4-(3-Chloro-2-fluoro-6-hydroxyphenyl)-1-((R)-2-fluoro-3-hydroxypropyl)pyrrolidine-2-thione |
| 157-1 | 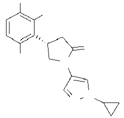 | (*S*)-4-(2-Chloro-3-fluoro-6-hydroxyphenyl)-1-(1-cyclopropyl-1H-pyrazol-4-yl)pyrrolidine-2-thione |
| 158-1 | 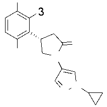 | (S)-4-(3-Chloro-2-fluoro-6-hydroxyphenyl)-1-(1-cyclopropyl-1H-pyrazol-4-yl)pyrrolidine-2-thione |
| 159-1 | 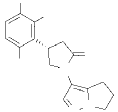 | (*S*)-4-(2,3-Dichloro-6-hydroxyphenyl)-1-(5,6-dihydro-4H-pyrrolo[1,2-b]pyrazol-3-yl)pyrrolidine-2-thione |
| 160-1 | 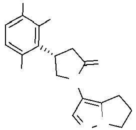 | (*S*)-4-(2,3-Dichloro-6-hydroxyphenyl)-1-(5,6-dihydro-4H-pyrrolo[1,2-b]pyrazol-3-yl)pyrrolidin-2-one |
| 161-1 | 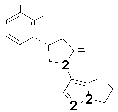 | (*S*)-4-(2,3-Dichloro-6-hydroxyphenyl)-1-(2,3-dihydropyrazolo[5,1-b]oxazol-7-yl)pyrrolidine-2-thione |
| 162-1 | 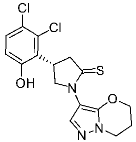 | (*S*)-4-(2,3-Dichloro-6-hydroxyphenyl)-1-(6,7-dihydro-5H-pyrazolo[5,1-b][1,3]oxazin-3-yl)pyrrolidine-2-thione |
| 163-1 | 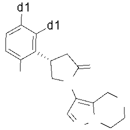 | (*S*)-4-(2,3-Dichloro-6-hydroxyphenyl)-1-(6,7-dihydro-4H-pyrazolo[5,1-c][1,4]oxazin-3-yl)pyrrolidine-2-thione |
| 164-1 | 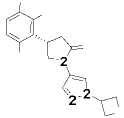 | (*S*)-4-(2,3-Dichloro-6-hydroxyphenyl)-1-(1-(oxetan-3-yl)-1H-pyrazol-4-yl)pyrrolidine-2-thione |
| 165-1 | 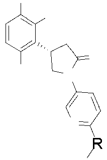 | (*S*)-4-(2,3-Dichloro-6-hydroxyphenyl)-1-(6-methoxypyridin-3-yl)pyrrolidine-2-thione |
| 166-1 | 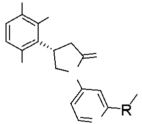 | (*S*)-4-(2,3-Dichloro-6-hydroxyphenyl)-1-(2-methoxypyridin-4-yl)pyrrolidine-2-thione |
| 167-1 | 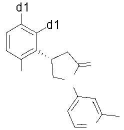 | (*S*)-4-(2,3-Dichloro-6-hydroxyphenyl)-1-(2-methylpyridin-4-yl)pyrrolidine-2-thione |
| 168-1 | 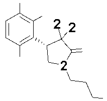 | (*S*)-4-(2,3-Dichloro-6-hydroxyphenyl)-3,3-difluoro-1-(3-hydroxypropyl)pyrrolidin-2-one |
| 169-1 | 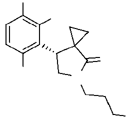 | (*S*)-7-(2,3-Dichloro-6-hydroxyphenyl)-5-(3-hydroxypropyl)-5-azaspiro[2.4]heptan-4-one |

The compound of formula I (including the compounds of any applicable subformula or Table 1 as described herein) may be present in the form of an enantiomer, a diastereomer, an atropisomer and/or a geometric isomer (if applicable) alone or as a mixture of stereoisomers (including racemic mixtures and a mixture enriched in one or more stereoisomers). In some embodiments, where applicable, the compound of formula I (including the compounds of any applicable subformula or Table 1 as described herein) may be present as a mixture of atropisomers in any ratio (including about 1 : 1). In some embodiments, where applicable, the compound of formula I (including the compounds of any applicable subformula or Table 1 as described herein) may be present as an isolated single atropisomer that is substantially free of other atropisomers (e.g., having less than 20%, less than 10%, less than 5%, less than 1%, or an undetectable amount of other atropisomers by weight, by HPLC area, or both).

In some embodiments, the above-mentioned compounds are substituted with an isotope. Preferably, in some embodiments, the isotope substitution is deuterium atom substitution.

Surprisingly, the compounds of the present disclosure exhibit significantly improved potency and/or safety profiles compared with Kv1.3 potassium channel inhibitors known in the prior art.

### Synthetic method of the compounds of the present disclosure

In view of the content of the present disclosure, those skilled in the art can readily synthesise the compounds of the present disclosure. Exemplary syntheses are shown in the Examples section.

The following synthetic methods for the compounds are illustrative. By using appropriate synthetic starting materials or intermediates, those skilled in the art can similarly use the synthetic method to synthesise other compounds.

To achieve the purpose of the present disclosure, the present disclosure adopts the following technical solutions:
a method for preparing the compound represented by general formula (I-1) or the pharmaceutically acceptable salt thereof of the present disclosure, the method comprising the following step: 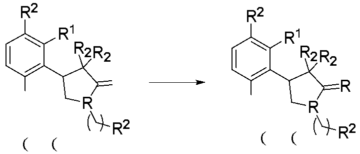
reacting a compound represented by general formula IA or a salt thereof with a thiocarbonylating reagent to obtain a compound represented by general formula (I-1) or a pharmaceutically acceptable salt thereof; optionally, the method further comprising the step of removing the phenolic hydroxyl protecting group R^{e} before, simultaneously with or after the reaction of the compound represented by general formula IA or the salt thereof with the thiocarbonylating reagent, wherein: the thiocarbonylating reagent is an oxygen-sulphur exchange reagent for converting carbonyl in an amide into thiocarbonyl, yielding a corresponding compound, preferably Lawesson's reagent;
R^{e} is a phenolic hydroxyl protecting group, preferably (trimethylsilyl)ethoxymethyl or allyl;
R¹, R², R⁷, R⁸, R^{a}, R^{d} and n are as defined herein;
a method for preparing the compound represented by general formula (I-2) or the pharmaceutically acceptable salt thereof of the present disclosure, the method comprising the following step: 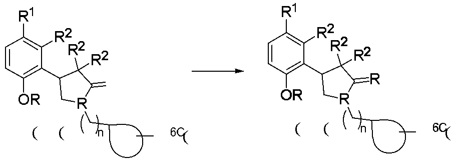
reacting a compound represented by general formula IB or a salt thereof with a thiocarbonylating reagent to obtain a compound represented by general formula (I-2) or a pharmaceutically acceptable salt thereof; optionally, the method further comprising the step of removing the phenolic hydroxyl protecting group R^{e} before, simultaneously with or after the reaction of the compound represented by general formula IB or the salt thereof with the thiocarbonylating reagent, wherein:
   the thiocarbonylating reagent is an oxygen-sulphur exchange reagent for converting carbonyl in an amide into thiocarbonyl, yielding a corresponding compound, preferably Lawesson's reagent;
   R^{e} is a phenolic hydroxyl protecting group, preferably (trimethylsilyl)ethoxymethyl or allyl;
   R¹, R², R⁷, R⁸, R^{a}, R^{6C}, ring A, n and k are as defined herein;
   a method for preparing the compound represented by general formula (I-3) or the pharmaceutically acceptable salt thereof of the present disclosure, the method comprising the following step: 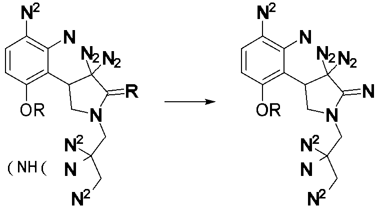
   reacting a compound represented by general formula IC or a salt thereof with a thiocarbonylating reagent to obtain a compound represented by general formula (I-3) or a pharmaceutically acceptable salt thereof; optionally, the method further comprising the step of removing the phenolic hydroxyl protecting group R^{e} before, simultaneously with or after the reaction of the compound represented by general formula IC or the salt thereof with the thiocarbonylating reagent, wherein: the thiocarbonylating reagent is an oxygen-sulphur exchange reagent for converting carbonyl in an amide into thiocarbonyl, yielding a corresponding compound, preferably Lawesson's reagent;
   R^{e} is a phenolic hydroxyl protecting group, preferably (trimethylsilyl)ethoxymethyl or allyl;
   R¹, R², R⁷, R⁸, R^{a}, R^{b}, R^{c} and R^{d} are as defined herein;
   a method for preparing the compound represented by general formula (I-6) or the pharmaceutically acceptable salt thereof of the present disclosure, the method comprising the following step: 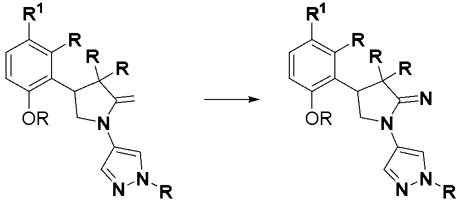
   reacting a compound represented by general formula ID or a salt thereof with a thiocarbonylating reagent to obtain a compound represented by general formula (I-6) or a pharmaceutically acceptable salt thereof; optionally, the method further comprising the step of removing the phenolic hydroxyl protecting group R^{e} before, simultaneously with or after the reaction of the compound represented by general formula ID or the salt thereof with the thiocarbonylating reagent, wherein: the thiocarbonylating reagent is an oxygen-sulphur exchange reagent for converting carbonyl in an amide into thiocarbonyl, yielding a corresponding compound, preferably Lawesson's reagent;
   R^{e} is a phenolic hydroxyl protecting group, preferably (trimethylsilyl)ethoxymethyl or allyl;
   R¹, R², R⁷, R⁸, R^{a} and R^{6D} are as defined herein.

The reaction of the above-mentioned scheme is preferably carried out in a solvent, and the solvent used includes, but is not limited to: ethylene glycol dimethyl ether, acetic acid, methanol, ethanol, acetonitrile, n-butanol, toluene, tetrahydrofuran, dichloromethane, petroleum ether, ethyl acetate, n-hexane, dimethyl sulfoxide, 1,4-dioxane, water, N,N-dimethylformamide, N,N-dimethylacetamide, 1,2-dibromoethane, pyridine and mixtures thereof.

It will be apparent to those skilled in the art that conventional protecting groups may be necessary to prevent certain functional groups from undergoing undesirable reactions. Suitable protecting groups for various functional groups and suitable conditions for protecting and deprotecting specific functional groups are well known in the art. For example, many protecting groups are described in "Protective Groups in Organic Synthesis", 4th ed. P.G.M.Wuts; T. W. Greene, John Wiley, 2007, and references cited therein. The reagents used in the reactions described herein are generally known compounds or can be prepared by known procedures or obvious modifications thereof. For example, some reagents suitable for the reactions described herein can be prepared by following the corresponding procedures described in WO 2019/103952, the content of which is incorporated herein by reference in its entirety. In addition, many reagents are available from commercial suppliers, such as Aldrich Chemical Co. (Milwaukee, Wisconsin, USA) and Sigma (St. Louis, Missouri, USA). Others may be prepared by procedures described in standard reference books or obvious modifications thereof, such as Fieser and Fieser's Reagents for Organic Synthesis, Volumes 1-15 (John Wiley and Sons,1991), Rodd's Chemistry of Carbon Compounds, Volumes 1-5 and Supplemental (Elsevier Science Publishers, 1989), Organic Reactions, Volumes 1-40 (John Wiley and Sons, 1991), March's Advanced Organic Chemistry, (Wiley,7th Edition), and Larock's Comprehensive Organic Transformations (Wiley-VCH,1999), and any updates available as of the present application.

### Pharmaceutical composition

Certain embodiments relate to a pharmaceutical composition comprising one or more compounds of the present disclosure.

The pharmaceutical composition may optionally comprise a pharmaceutically acceptable excipient. In some embodiments, the pharmaceutical composition comprises the compound of the present disclosure (such as, the compound of formula I (such as, formulas I-1 to I-4), any of Compound Nos. 1 to 120-1, or a pharmaceutically acceptable salt thereof) and a pharmaceutically acceptable excipient. The pharmaceutically acceptable excipient is known in the art. Non-limiting suitable excipients include, for example, encapsulating materials or additives, such as absorption enhancers, antioxidants, binders, buffers, carriers, coatings, colourants, diluents, disintegrants, emulsifiers, extenders, fillers, flavouring agents, humectants, lubricants, perfumes, preservatives, propellants, release agents, bactericides, sweeteners, solubilizers, wetting agents, and mixtures thereof. See also Remington's The Science and Practice of Pharmacy, 21st Edition, A. R. Gennaro (Lippincott, Williams & Wilkins, Baltimore, Md., 2005; incorporated herein by reference), which discloses various excipients used in formulating pharmaceutical compositions and known techniques for preparing pharmaceutical compositions.

The pharmaceutical composition can include any one or more compounds of the present disclosure. For example, in some embodiments, the pharmaceutical composition comprises, for example, a therapeutically effective amount of the compound of formula I (such as, formulas I-1 to I-6), any one of the compounds of Table 1, or a pharmaceutically acceptable salt thereof. In any of the embodiments described herein, the pharmaceutical composition can comprise a therapeutically effective amount of the compound selected from Table 1 or a pharmaceutically acceptable salt thereof.

The pharmaceutical composition can also be formulated for delivery via any known route of delivery, including but not limited to oral, parenteral, inhalation, and the like.

In some embodiments, the pharmaceutical composition can be formulated for oral administration. Oral formulations can be presented as: discrete units, such as capsules, pills, cachets, lozenges, or tablets, each containing a predetermined amount of the active compound; powders or granules; solutions or suspensions in aqueous or non-aqueous liquids; or oil-in-water or water-in-oil emulsions. Excipients used in preparing compositions for oral administration are known in the art. Non-limiting suitable excipients include, for example, agar, alginic acid, aluminium hydroxide, benzyl alcohol, benzyl benzoate, 1,3-butanediol, carbomer, castor oil, cellulose, cellulose acetate, cocoa butter, corn starch, corn oil, cottonseed oil, crospovidone, diglycerides, ethanol, ethylcellulose, ethyl laurate, ethyl oleate, fatty acid esters, gelatine, germ oil, glucose, glycerol, peanut oil, hydroxypropyl methylcellulose, isopropyl alcohol, isotonic saline, lactose, magnesium hydroxide, magnesium stearate, malt, mannitol, monoglycerides, olive oil, peanut oil, potassium phosphate, potato starch, povidone, propylene glycol, Ringer's solution, safflower oil, sesame oil, sodium carboxymethylcellulose, sodium phosphate, sodium lauryl sulphate, sodium sorbitol, soybean oil, stearic acid, stearyl fumarate, sucrose, surfactants, talc, tragacanth gum, tetrahydrofurfuryl alcohol, triglycerides, water, and mixtures thereof.

In some embodiments, the pharmaceutical composition is formulated for parenteral administration (such as, intravenous injection or infusion, or subcutaneous or intramuscular injection). Parenteral formulations can be, for example, aqueous solutions, suspensions, or emulsions. Excipients for preparing parenteral formulations are known in the art. Non-limiting suitable excipients include, for example, 1,3-butanediol, castor oil, corn oil, cottonseed oil, glucose, germ oil, peanut oil, liposomes, oleic acid, olive oil, peanut oil, Ringer's solution, safflower oil, sesame oil, soybean oil, U.S.P. or isotonic sodium chloride solution, water, and mixtures thereof.

In some embodiments, the pharmaceutical composition is formulated for inhalation administration. For example, inhalable formulations can be formulated as nasal sprays, dry powders, or aerosols that can be administered by a metered dose inhaler. Excipients for preparing inhalation formulations are known in the art. Non-limiting suitable excipients include, for example, lactose, talc, silicic acid, aluminium hydroxide, calcium silicate, and polyamide powder, and mixtures thereof. Sprays may also contain propellants, such as chlorofluorocarbons and volatile unsubstituted hydrocarbons such as butane and propane.

Pharmaceutical compositions may include various amounts of the compound of the present disclosure, depending on factors such as the intended use, potency and selectivity of the compound. In some embodiments, the pharmaceutical composition comprises a therapeutically effective amount of the compound of the present disclosure (e.g., the compound of formula I (such as, formulas I-1 to I-6), any one of the compounds of Table 1, or a pharmaceutically acceptable salt thereof). In some embodiments, the pharmaceutical composition comprises a therapeutically effective amount of the compound of the present disclosure and a pharmaceutically acceptable excipient. As used herein, the therapeutically effective amount of the compound of the present disclosure is an amount effective to treat the disease or symptom as described herein, such as multiple sclerosis, rheumatoid arthritis, inflammatory bowel disease, systemic lupus erythematosus, psoriasis, psoriatic arthritis, Crohn's disease, Sjogren's syndrome, and/or scleroderma, which may depend on the subject being treated, the disease or symptom being treated and its severity, the composition comprising the compound, the timing of administration, the route of administration, the duration of treatment, the potency of the compound (e.g., the potency for inhibiting Kv1.3), its clearance rate, and whether it is co-administered with another drug.

For veterinary use, the compound of the present disclosure can be administered in an appropriately acceptable formulation according to normal veterinary practice. Veterinarians can readily determine the most suitable administration regimen and administration route for a particular animal.

In some embodiments, all necessary ingredients for treating a disease or symptom in which Kv1.3 plays a role using the compound of the present invention, alone or in combination with another agent or intervention traditionally used to treat such disease or symptom, may be packaged into a kit. Specifically, in some embodiments, the present invention provides a kit for therapeutic intervention of a disease, comprising a set of packaged drugs comprising the compound disclosed herein, and a buffer and other components for preparing a deliverable form of the drug, and/or a device for delivering such drug, and/or any agent for combination therapy with the compound of the present disclosure, and/or instructions for use for treating the disease packaged with the drug. The instructions for use can be fixed in any tangible medium, such as printed paper, or computer-readable magnetic or optical media, or may indicate reference to a remote computer data source, such as World Wide Web pages accessible via the Internet.

### Treatment method/use

The compound of the present disclosure can be used as a therapeutically active substance for treating and/or preventing a disease or symptom in which Kv1.3 plays a role. In particular, the compound of the present disclosure that mediates signal transduction by acting on Kv1.3 can be used to treat a symptom associated with modulation of Kv1.3 function, particularly inhibition of Kv1.3 function. Such symptoms include these cytokine-related diseases in which the pathogenic mechanism is mediated by Kv1.3, including any of those known in the art and those described herein.

In some embodiments, the present disclosure provides a method for inhibiting Kv1.3-mediated cellular signalling, comprising contacting a cell with an effective amount of one or more compounds of the present disclosure (e.g., the compound of formula I (e.g., formulas I-1 to I-6), any one of the compounds of Table 1, or a pharmaceutically acceptable salt thereof).

In some embodiments, the present disclosure provides a method for inhibiting Kv1.3 function in a subject in need thereof, comprising administering to the subject an effective amount of one or more compounds of the present disclosure (e.g., the compound of formula I (e.g., formulas I-1 to I-6), any one of the compounds of Table 1, or a pharmaceutically acceptable salt thereof).

In some embodiments, the present disclosure provides a method for treating or preventing a disease or symptom mediated by Kv1.3 in a subject in need thereof, comprising administering to the subject an effective amount of one or more compounds of the present disclosure (e.g., the compound of formula I (e.g., formulas I-1 to I-6), any one of the compounds of Table 1, or a pharmaceutically acceptable salt thereof). Suitable diseases or symptoms mediated by Kv1.3 that can be treated with the method herein include any of those known in the art. Exemplary diseases or symptoms mediated by Kv1.3 that can be treated with the method herein also include, but are not limited to, those proliferative, metabolic, allergic, autoimmune and/or inflammatory diseases or symptoms described herein.

In some embodiments, the present disclosure provides a method for treating or preventing an autoimmune and/or inflammatory disease or symptom, such as those described herein in a subject in need thereof, comprising administering to the subject an effective amount of one or more compounds of the present disclosure (e.g., the compound of formula I (e.g., formulas I-1 to I-6), any one of the compounds of Table 1, or a pharmaceutically acceptable salt thereof).

In some embodiments, the present disclosure provides a method for treating or preventing a disease or symptom in a subject in need thereof, comprising administering to the subject an effective amount of one or more compounds of the present disclosure (e.g., the compound of formula I (e.g., formulas I-1 to I-6), any one of the compounds of Table 1, or a pharmaceutically acceptable salt thereof), wherein the disease or symptom can be one or more selected from the following diseases or symptoms: inflammatory disease or autoimmune disease, transplant rejection, neurological disorder or neurodegenerative disease, cardiovascular disease, metabolic disorder, nephrosis, gastrointestinal disorder, or oncological condition. Preferably, the inflammatory disease is selected from atopic dermatitis, arthritis, and psoriasis. The autoimmune disease is selected from rheumatoid arthritis, multiple sclerosis, systemic lupus erythematosus or type I diabetes. The neurodegenerative disease is Alzheimer's disease. The cardiovascular disease is ischemic stroke. The metabolic disorder is selected from obesity or type II diabetes. The nephrosis is selected from chronic kidney disease, nephritis or chronic renal failure. The gastrointestinal disorder is inflammatory bowel disease.

Administration herein is not limited to any particular route of administration. For example, in some embodiments, the administration can be oral, nasal, transdermal, pulmonary, inhaled, buccal, sublingual, intraperitoneal, subcutaneous, intramuscular, intravenous, rectal, intrapleural, intrathecal, and parenteral. In some embodiments, the administration is oral administration.

Administration regimen, including dosage, can vary and be adjusted depending on the subject being treated, the disease or symptom being treated and its severity, the composition comprising the compound, the time of administration, the route of administration, the duration of treatment, the potency of the compound, its clearance rate, and whether it is co-administered with another drug.

### Examples

The following examples are used to further describe the present disclosure, but these examples are not intended to limit the scope of the present disclosure.

The structures of compounds are determined by nuclear magnetic resonance (NMR) or/and mass spectrometry (MS). The NMR shift (δ) is given in the unit of 10⁻⁶ (ppm). NMR is determined with Bruker AVANCE NEO 500M/400M/300M NMR instrument; the solvents for determination are deuterated dimethylsulfoxide (DMSO-d₆), deuterated chloroform (CDCl₃) and deuterated methanol (CD₃OD); and the internal standard is tetramethylsilane (TMS).

MS is determined with Agilent 1200/1290 DAD-6110/6120 Quadrupole MS liquid chromatography-mass spectrometry (manufacturer: Agilent, MS model: 6110/6120 Quadrupole MS), waters ACQuity UPLC-QD/SQD (manufacturer: waters, MS model: waters ACQuity Qda Detector/waters SQ Detector), and THERMO Ultimate 3000-Q Exactive (manufacturer: THERMO, MS model: THERMO Q Exactive).

High performance liquid chromatography (HPLC) analysis is performed using Agilent HPLC 1200DAD, Agilent HPLC 1200VWD and Waters HPLC e2695-2489 high performance liquid chromatographs.

Chiral HPLC analysis is performed using Agilent 1260 DAD high performance liquid chromatograph.

Preparative high performance liquid chromatography is performed using Waters 2767, Waters 2767-SQ Detecor2, Shimadzu LC-20AP and Gilson-281 preparative chromatographs.

Chiral preparative chromatography is performed using Shimadzu LC-20AP preparative chromatograph.

The CombiFlash flash preparative instrument used is Combiflash Rf200 (TELEDYNE ISCO).

For thin layer chromatography, Yantai Huanghai HSGF254 silica gel plates are used. The specifications of silica gel plates applied in thin layer chromatography (TLC) are 0.15 mm-0.2 mm, while those used for separation and purification of products by thin layer chromatography are 0.4 mm-0.5 mm.

Silica gel column chromatography typically uses silica gel of 200-300 mesh or 300-400 mesh as a carrier.

The average kinase inhibition rate and IC₅₀ value are determined using NovoStar microplate reader (BMG Company, Germany).

The known starting materials of the present disclosure can be synthesised by or according to methods known in the art, or can be purchased from TiTan Technology (Shanghai) Co. Ltd., ABCR GmbH & Co. KG, Acros Organics, Aldrich Chemical Company, Accela ChemBio Inc., Bide Pharmatech Ltd., and other companies.

Unless otherwise specified in examples, reactions can all be carried out under argon or nitrogen atmosphere.

Argon or nitrogen atmosphere means that a reaction flask is connected to an argon or nitrogen balloon with a volume of about 1 L.

Hydrogen atmosphere means that a reaction flask is connected to a hydrogen balloon with a volume of about 1 L.

Pressurized hydrogenation reactions are performed using either Parr 3916EKX hydrogenation apparatus with Qinglan QL-500 hydrogen generator or HC2-SS hydrogenation apparatus.

Hydrogenation reactions are typically performed by evacuating the system and refilling it with hydrogen gas, repeating this operation three times.

Microwave reactions are performed using CEM Discover-S 908860 microwave reactor.

Unless otherwise specified in examples, the solution refers to an aqueous solution.

Unless otherwise specified in examples, the reaction temperature is room temperature, which is 20°C-30°C.

The reaction progress in the examples is monitored by thin layer chromatography (TLC). The developing solvent used for the reaction, the eluent system for column chromatography to purify compounds, and the developing solvent system for thin layer chromatography include: A: n-hexane or petroleum ether/ethyl acetate system, B: dichloromethane/methanol system. The volume ratio of the solvents is adjusted based on the polarity of the compounds, and a small amount of basic or acidic reagents such as triethylamine and acetic acid may also be added for adjustment.

### Example 1

### Synthesis of intermediate 1

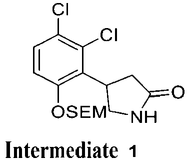

### 4-(2,3-Dichloro-6-((2-(trimethylsilyl)ethoxy)methoxy)phenyl)pyrrolidin-2-one

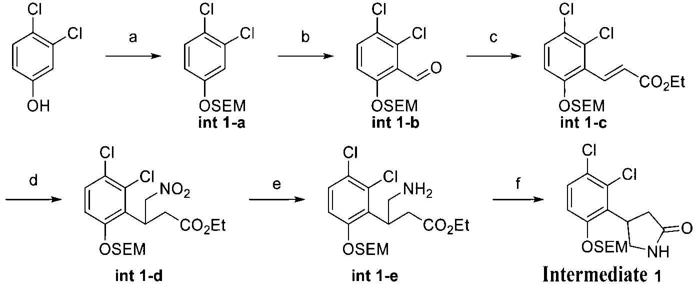

### Step a:

### (2-((3,4-Dichlorophenoxy)methoxy)ethyl)trimethylsilane

3.4-Dichlorophenol (20.18 g, 123.80 mmol) and potassium carbonate (34.22 g, 247.59 mmol) were added to a round-bottom flask and dissolved in N,N-dimethylformamide (100 mL). [2-(Chloromethoxy)ethyl]trimethylsilane (24.77 g, 148.57 mmol) was added portionwise at 0°C. The reaction mixture was stirred at 20°C for 24 hours. The mixture was diluted with water (200 mL x 3) and extracted with ethyl acetate (100 mL x 3). The combined organic layers were washed with sodium chloride aqueous solution and dried over anhydrous sodium sulphate. After filtration, the filtrate was concentrated under reduced pressure. The residue was subjected to flash silica gel column chromatography (petroleum ether : ethyl acetate = 100:1) to afford compound **int 1-a,** (2-((3,4-dichlorophenoxy)methoxy)ethyl)trimethylsilane (25.50 g, 70.2%).

¹H NMR (400 MHz, CDCl₃): *δ* 7.31 (d, 1H), 7.16 (d, 1H), 6.90 (dd, 1H), 5.18 (s, 2H), 3.75-3.71 (m, 2H), 0.97-0.92 (m, 2H), 0.01 (s, 9H).

### Step b:

### 2,3-Dichloro-6-{[2-(trimethylsilyl)ethoxy]methoxy}benzaldehyde

To a solution of [2-(3,4-dichlorophenoxymethoxy)ethyl]trimethylsilane (25.50 g, 86.95 mmol) in tetrahydrofuran (300 mL) was slowly added dropwise n-butyllithium (42 mL, 104.40 mmol, 2.5 M in hexane) at -78°C under nitrogen protection. After the dropwise addition was completed, the reaction solution was stirred for another 1 hour, and N,N-dimethylformamide (15.90 g, 217.54 mmol) was added dropwise at -78°C, followed by further stirring for 2 hours. The reaction mixture was quenched with ice water (200 mL) and extracted with ethyl acetate (100 mL x 3). The combined organic layers were washed with sodium chloride aqueous solution and dried over anhydrous sodium sulphate. After filtration, the filtrate was concentrated under reduced pressure. The residue was purified by flash silica gel column chromatography (petroleum ether : ethyl acetate = 100:1-10:1) to afford compound **int 1-b,** 2,3-dichloro-6-{[2-(trimethylsilyl)ethoxy]methoxy} benzaldehyde (25.00 g, 89.5%).

¹H NMR (400 MHz, CDCl₃): *δ* 10.36 (s, 1H), 7.84 (d, 1H), 7.34 (d, 1H), 5.41 (s, 2H), 3.76 (t, 2H), 0.91 (t, 2H), 0.01 (s, 9H).

### Step c:

### Ethyl (E)-3-(2,3-dichloro-6-((2-(trimethylsilyl)ethoxy)methoxy)phenyl)acrylate

Under nitrogen protection at 0°C, sodium hydride (6.24 g, 156.10 mmol, 60%) was added to a stirred solution of triethyl phosphonoacetate (26.16 g, 116.70 mmol) in tetrahydrofuran (250 mL), and the reaction solution was stirred at 0°C for 0.5 h, followed by the addition of 2,3-dichloro-6-{[2-(trimethylsilyl)ethoxy]methoxy}benzaldehyde (25.00 g, 77.82 mmol). The reaction solution was stirred at 20°C for 16 hours. The reaction mixture was quenched with water (100 mL) and extracted with ethyl acetate (50 mL x 3). The combined organic layers were washed with sodium chloride aqueous solution and dried over anhydrous sodium sulphate. After filtration, the filtrate was concentrated under reduced pressure. The residue was purified by flash silica gel column chromatography (petroleum ether : ethyl acetate = 10:1) to afford compound **int 1-c,** ethyl (*E*)-3-(2,3-dichloro-6-((2-(trimethylsilyl)ethoxy)methoxy)phenyl)acrylate (20.0 g, 65.7%).

¹H NMR (300 MHz, CDCl₃): *δ* 8.00 (d, 1H), 7.41 (d, 1H), 7.15 (d, 1H), 6.83 (d, 1H), 5.33 (s, 2H), 4.33 (q, 2H), 3.83-3.74 (m, 2H), 1.40 (t, 3H), 1.01-0.92 (m, 2H), 0.05 (s, 9H).

### Step d:

### Ethyl 3-(2,3-dichloro-6-((2-(trimethylsilyl)ethoxy)methoxy)phenyl)-4-nitrobutyrate

To a stirred solution of ethyl (*E*)-3-(2,3-dichloro-6-((2-(trimethylsilyl)ethoxy)methoxy)phenyl)acrylate (20.00 g, 51.10 mmol) in nitromethane (250 mL) was added 1,8-diazabicyclo[5.4.0]undec-7-ene (9.30 g, 61.09 mmol) at 20°C under nitrogen protection. The reaction solution was stirred at 60°C for another 16 h. The reaction mixture was poured into water (20 mL) and extracted with ethyl acetate (60 mL x 3). The combined organic layers were washed with sodium chloride aqueous solution and dried over anhydrous sodium sulphate. After filtration, the filtrate was concentrated under reduced pressure. The residue was purified by flash silica gel column chromatography (petroleum ether : ethyl acetate = 20:1-10:1) to afford compound **int 1-d,** ethyl 3-(2,3-dichloro-6-((2-(trimethylsilyl)ethoxy)methoxy)phenyl)-4-nitrobutyrate (19.25 g, 83.3%).

¹HNMR (400 MHz, CDCl₃): *δ* 7.31 (d, 1H), 7.05 (d, 1H), 5.23 (s, 2H), 4.90-4.82 (m, 3H), 4.08 (q, 2H), 3.78-3.73 (m, 2H), 2.86 (s, 2H), 1.17 (t, 3H), 0.97-0.93 (m, 2H), 0.01 (s, 9H).

### Step e:

### Ethyl 4-amino-3-(2,3-dichloro-6-((2-(trimethylsilyl)ethoxy)methoxy)phenyl) butyrate

To a stirred solution of ethyl 3-(2,3-dichloro-6-{[2-(trimethylsilyl)ethoxy]methoxy}phenyl)-4-nitrobutyrate (19.25 g, 42.55 mmol) in acetic acid (100 mL) was added portionwise zinc powder (41.80 g, 639.34 mmol) under nitrogen protection. The reaction solution was stirred at 20°C for 4 h. The reaction mixture was filtered, the filter cake was washed with ethyl acetate (50 mL x 3), and the filtrate was concentrated under reduced pressure. The residue was purified by flash silica gel column chromatography (dichloromethane : methanol = 20:1-10:1) to afford compound **int 1-e,** ethyl 4-amino-3-(2,3-dichloro-6-((2-(trimethylsilyl)ethoxy)methoxy)phenyl)butyrate (17.20 g, 95.7%).

¹HNMR (300 MHz, CDCl₃): *δ* 7.35 (d, 1H), 7.08 (d, 1H), 5.29-5.26 (m, 2H), 4.17-4.04 (m, 3H), 3.79 (t, 2H), 3.20-3.13 (m, 1H), 3.09-3.02 (m, 1H), 2.9-2.82 (m, 2H), 1.18 (t, 3H), 1.02-0.95 (m, 2H), 0.04 (s, 9H).

### Step f:

### 4-(2,3-Dichloro-6-((2-(trimethylsilyl)ethoxy)methoxy)phenyl)pyrrolidin-2-one

Ethyl 4-amino-3-(2,3-dichloro-6-{[2-(trimethylsilyl)ethoxy]methoxy}phenyl) butyrate (17.20 g, 40.72 mmol) and potassium carbonate (14.78 g, 106.94 mmol) were dissolved in methanol (150 mL) and stirred. The reaction solution was stirred at 20°C for 2 h. The resulting mixture was diluted with water (100 mL) and extracted with ethyl acetate (50 mL x 3). The combined organic layers were washed with sodium chloride aqueous solution and dried over anhydrous sodium sulphate. After filtration, the filtrate was concentrated under reduced pressure. The residue was purified by flash silica gel column chromatography (dichloromethane : methanol = 20:1) to afford compound **intermediate 1:** 4-(2,3-dichloro-6-((2-(trimethylsilyl)ethoxy)methoxy)phenyl)pyrrolidin-2-one (7.10 g, 46.3%).

¹HNMR (400 MHz, CDCl₃): *δ* 7.32 (d, 1H), 7.08 (d, 1H), 6.23 (s, 1H), 5.25 (s, 2H), 4.58-4.49 (m, 1H), 3.75-3.71 (m, 2H), 3.68-3.53 (m, 2H), 2.76 (dd, 1H), 2.58 (dd, 1H), 0.96-0.91 (m, 2H), 0.00 (s, 9H).

### Example 2

### Synthesis of intermediate 2 and intermediate 3

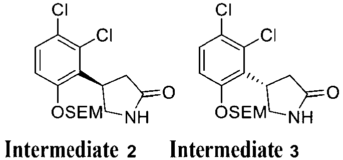

### (R)-4-(2,3-Dichloro-6-((2-(trimethylsilyl)ethoxy)methoxy)phenyl)pyrrolidin-2-one

### (S)-4-(2,3-Dichloro-6-((2-(trimethylsilyl)ethoxy)methoxy)phenyl)pyrrolidin-2-one

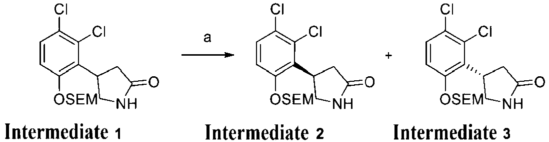

### Step a:

Intermediate 1: 4-(2,3-dichloro-6-((2-(trimethylsilyl)ethoxy)methoxy)phenyl) pyrrolidin-2-one was purified by preparative supercritical fluid chromatography (5.0 g, 13.29 mmol) under the purification conditions: chromatographic column: CHIRALPAK IH, 3 × 25 cm, 5 µm; mobile phase A: CO₂, mobile phase B: MeOH (with 0.2% DEA); flow rate: 60 mL/min; gradient: 25% B; detector: UV 214 nm; retention time 1: 4.14 min; retention time 2: 5.61 min. The faster-eluting enantiomer with a retention time of 4.14 min was identified as **intermediate 2:** (*R*)-4-(2,3-dichloro-6-((2-(trimethylsilyl)ethoxy)methoxy)phenyl)pyrrolidin-2-one (2.35 g, 47.0%).

¹HNMR (400 MHz,CDCl₃): *δ* 7.32 (d, 1H), 7.08 (d, 1H), 5.72 (bs, 1H), 5.25 (s, 2H), 4.59-4.50 (m, 1H), 3.75-3.71 (m, 2H), 3.69-3.58 (m, 2H), 2.75 (dd, 1H), 2.59 (dd, 1H), 0.96-0.91 (m, 2H), 0.00 (s, 9H).

The slower-eluting enantiomer with a retention time of 5.61 min was identified as **intermediate 3:** (*S*)-4-(2,3-dichloro-6-((2-(trimethylsilyl)ethoxy)methoxy) phenyl)pyrrolidin-2-one (2.30 g, 46.0%).

¹HNMR (400 MHz, CDCl₃): *δ* 7.32 (d, 1H), 7.08 (d, 1H), 5.72 (bs, 1H), 5.25 (s, 2H), 4.59-4.50 (m, 1H), 3.75-3.71 (m, 2H), 3.694-3.584 (m, 2H), 2.75 (dd, 1H), 2.59 (dd, 1H), 0.96-0.91 (m, 2H), 0.00 (s, 9H).

### Example 3

### Synthesis of intermediate 4

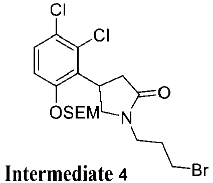

### 1-(3-Bromopropyl)-4-(2,3-dichloro-6-((2-(trimethylsilyl)ethoxy)methoxy) phenyl)pyrrolidin-2-one

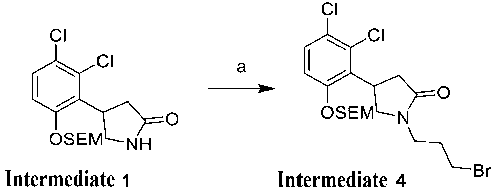

### Step a:

To a stirred solution of intermediate 1 (500.0 mg, 1.33 mmol) in toluene (25 mL) were added potassium hydroxide (149.0 mg, 2.66 mmol), tetrabutylammonium bromide (86.0 mg, 0.27 mmol) and 1,3-dibromopropane (805.0 mg, 3.99 mmol) at 20°C, and the reaction solution was stirred at 35°C for 16 h. The resulting mixture was diluted with water (100 mL) and extracted with ethyl acetate (100 mL x 3). The combined organic layers were washed with sodium chloride aqueous solution and dried over anhydrous sodium sulphate. After filtration, the filtrate was concentrated under reduced pressure. The residue was purified by flash silica gel column chromatography (petroleum ether : tetrahydrofuran = 7:3) to afford compound **intermediate 4:** 1-(3-bromopropyl)-4-(2,3-dichloro-6-((2-(trimethylsilyl)ethoxy) methoxy)phenyl)pyrrolidin-2-one (395.0 mg, 59.8%).
¹H NMR (400 MHz, CDCl₃) *δ* 7.31 (d, 1H), 7.08 (d, 1H), 5.24-5.18 (m, 2H), 4.46-4.36 (m, 1H), 3.75-3.68 (m, 2H), 3.68-3.63 (m, 1H), 3.60-3.55 (m, 1H), 3.49-3.40 (m, 4H), 2.79-2.64 (m, 2H), 2.17-2.10 (m, 2H), 0.93 (t, 2H), 0.01 (s, 9H);
MS m/z(ESI): 495.8 [M+H]⁺.

### Example 4

### Synthesis of compound 1 and compound 1-1

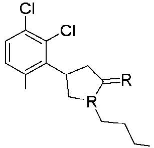

### 4-(2,3-Dichloro-6-hydroxyphenyl)-1-(3-hydroxypropyl)pyrrolidine-2-thione

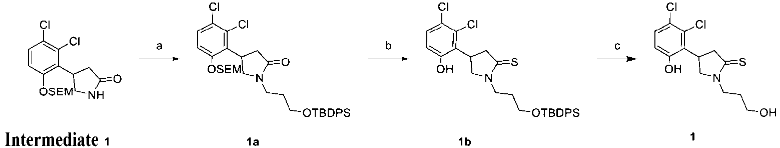

### Step a:

### 1-(3-((tert-Butyldiphenylsilyl)oxy)propyl)-4-(2,3-dichloro-6-((2-(trimethylsilyl)ethoxy)methoxy)phenyl)pyrrolidin-2-one

To a stirred solution of 4-(2,3-dichloro-6-((2-(trimethylsilyl)ethoxy)methoxy)phenyl)pyrrolidin-2-one (200.0 mg, 0.53 mmol) in tetrahydrofuran (2 mL) was added sodium hydride (85.0 mg, 2.13 mmol) at 0°C. The reaction solution was stirred at 0°C for 0.5 h. (3-Bromopropoxy)(tert-butyl)diphenylsilane (802.2 mg, 2.13 mmol) was added to the reaction solution, and the reaction solution was stirred at 60°C for 18 h. The resulting mixture was diluted with water and extracted with ethyl acetate (25 mL x 3). The combined organic layers were washed with sodium chloride aqueous solution and dried over anhydrous magnesium sulphate. After filtration, the filtrate was concentrated under reduced pressure. The residue was purified by flash silica gel column chromatography (petroleum ether : ethyl acetate = 3:2) to afford compound **1a:** 1-(3-((tert-butyldiphenylsilyl)oxy)propyl)-4-(2,3-dichloro-6-((2-(trimethylsilyl)ethoxy) methoxy)phenyl)pyrrolidin-2-one (120.0 mg, 33.7%).

1H NMR (400 MHz, CDCl₃): δ 7.68-7.65 (m, 4H), 7.47-7.31 (m, 7H), 7.08 (d, 1H), 5.16 (d, 2H), 4.35 (d, 1H), 3.78-3.37 (m, 8H), 2.76 (dd, 2H), 1.30-1.25 (m, 2H), 1.06 (s, 9H), 0.95-0.90 (m, 2H), 0.00 (s, 9H).

### Step b:

### 1-(3-(tert-Butyldiphenylsilyloxy)propyl)-4-(2,3-dichloro-6-hydroxyphenyl) pyrrolidine-2-thione

To a stirred solution of 1-(3-((tert-butyldiphenylsilyl)oxy)propyl)-4-(2,3-dichloro-6-((2-(trimethylsilyl)ethoxy)methoxy)phenyl)pyrrolidin-2-one (40.0 mg, 0.06 mmol) in tetrahydrofuran (3 mL) was added Lawesson's reagent (12.0 mg, 0.03 mmol) at 20°C. The reaction solution was stirred at 20°C for 18 h. The reaction solution was concentrated, and the residue was purified by flash silica gel column chromatography (petroleum ether : ethyl acetate = 7:3) to afford compound **1b:** 1-(3-(tert-butyldiphenylsilyloxy)propyl)-4-(2,3-dichloro-6-hydroxyphenyl) pyrrolidine-2-thione (40.0 mg, 83.0%).

MS m/z (ESI): 558.7 [M+H]⁺.

### Step c:

### 4-(2,3-Dichloro-6-hydroxyphenyl)-1-(3-hydroxypropyl)pyrrolidine-2-thione

To a stirred solution of 1-(3-(tert-butyldiphenylsilyloxy)propyl)-4-(2,3-dichloro-6-hydroxyphenyl)pyrrolidine-2-thione (55.0 mg, 0.10 mmol) in tetrahydrofuran (1 mL) was added tetrabutylammonium fluoride (0.3 mL, 0.98 mmol) at 0°C. The reaction solution was stirred at 20°C for 1 h. The reaction solution was concentrated, and the residue was purified by flash reverse-phase C18 column chromatography (water : acetonitrile = 6:4) to afford compound **1:** 4-(2,3-dichloro-6-hydroxyphenyl)-1-(3-hydroxypropyl)pyrrolidine-2-thione (20.0 mg, 63.4%).
¹H NMR (400 MHz, CD₃OD): *δ* 7.25 (d, 1H), 6.75 (d, 1H), 4.45-4.38 (m, 1H), 4.18-3.93 (m, 3H), 3.78-3.72 (m, 1H), 3.63 (t, 2H), 3.36 (d, 1H), 3.29-3.21 (m, 1H), 2.00-1.83 (m, 2H);
MS m/z (ESI): 320.0 [M+H]⁺.

Compound **1-1** was synthesised using intermediate **3** as the starting material and following the same synthetic route as compound **1.**

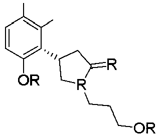

### Example 5

### Synthesis of compound 2

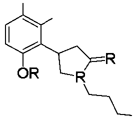

### 1-(3-Aminopropyl)-4-(2,3-dichloro-6-hydroxyphenyl)pyrrolidine-2-thione

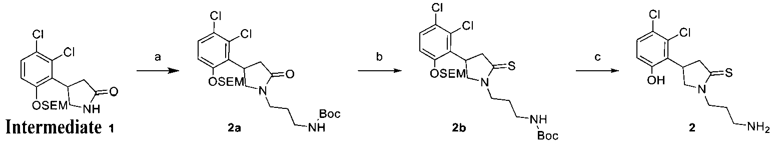

### Step a:

### tert-Butyl (3-(4-(2,3-dichloro-6-(2-(trimethylsilyl)ethoxy)methoxy)phenyl)-2-oxopyrrolidin-1-yl)propyl)carbamate

To a stirred solution of 4-(2,3-dichloro-6-((2-(trimethylsilyl)ethoxy)methoxy)phenyl)pyrrolidin-2-one (350.0 mg, 0.93 mmol) in tetrahydrofuran (15 mL) was added sodium hydride (148.0 mg, 3.70 mmol) at 25°C. The reaction solution was stirred at 0°C for 0.5 h. tert-Butyl (3-bromopropyl)carbamate (885.8 mg, 3.72 mmol) was added to the reaction solution, and the reaction solution was stirred at 50°C for 10 h. The resulting mixture was diluted with water and extracted with ethyl acetate (50 mL x 3). The combined organic layers were washed with sodium chloride aqueous solution and dried over anhydrous magnesium sulphate. After filtration, the filtrate was concentrated under reduced pressure. The residue was purified by flash silica gel column chromatography (petroleum ether : ethyl acetate = 1:1) to afford compound **2a:** (3-(4-(2,3-dichloro-6-(2-(trimethylsilyl)ethoxy)methoxy)phenyl)-2-oxopyrrolidin-1-yl)propyl)carbamate (430.0 mg, 86.7%).

¹H NMR (400 MHz, CDCl₃): *δ* 7.33 (s, 1H), 7.08 (d, 1H), 5.22 (d, 2H), 4.49-4.30 (m, 1H), 3.73-3.67 (m, 2H), 3.63-3.53 (m, 2H), 3.41 (t, 2H), 3.23-3.06 (m, 2H), 2.85-2.64 (m, 2H), 1.73-1.67 (m, 2H), 1.44 (s, 9H), 0.97-0.88 (m, 2H), 0.00 (s, 9H).

### Step b:

### tert-Butyl (3-(4-(2,3-dichloro-6-(2-(trimethylsilyl)ethoxy)methoxy)phenyl)-2-thiopyrrolidin-1-yl)propyl)carbamate

To a stirred solution of tert-butyl (3-(4-(2,3-dichloro-6-(2-(trimethylsilyl)ethoxy)methoxy)phenyl)-2-oxopyrrolidin-1-yl)propyl)carbamate (430.0 mg, 0.81 mmol) in dichloromethane (5 mL) was added Lawesson's reagent (161.8 mg, 0.40 mmol) at 20°C. The reaction solution was stirred at 20°C for 10 h. The resulting mixture was diluted with water and extracted with dichloromethane (20 mL x 3). The combined organic layers were washed with sodium chloride aqueous solution and dried over anhydrous sodium sulphate. After filtration, the filtrate was concentrated under reduced pressure. The residue was purified by flash silica gel column chromatography (petroleum ether : ethyl acetate = 1:1) to afford compound **2b:** tert-butyl (3-(4-(2,3-dichloro-6-(2-(trimethylsilyl)ethoxy) methoxy)phenyl)-2-thiopyrrolidin-1-yl)propyl)carbamate (349.0 mg, 78.8%).

¹H NMR (400 MHz, CDCl₃): δ 7.32 (d, 1H), 7.06 (d, 1H), 5.29 (s, 2H), 5.27-5.10 (m, 2H), 4.45 (s, 1H), 3.99-3.84 (m, 4H), 3.73-3.65 (m, 2H), 3.35 (dd, 2H), 3.16 (s, 2H), 1.85 (t, 2H), 1.44 (s, 9H), 0.96-0.88 (m, 2H), 0.00 (s, 9H).

### Step c:

### 1-(3-Aminopropyl)-4-(2,3-dichloro-6-hydroxyphenyl)pyrrolidine-2-thione

To a stirred solution of tert-butyl (3-(4-(2,3-dichloro-6-(2-(trimethylsilyl)ethoxy)methoxy)phenyl)-2-thiopyrrolidin-1-yl)propyl)carbamate (340.0 mg, 0.62 mmol) in dichloromethane (5 mL) was added hydrochloric acid (3.0 mL, 0.04 mmol) at 20°C. The reaction solution was stirred at 20°C for 1 h. The resulting mixture was diluted with saturated sodium bicarbonate aqueous solution and extracted with dichloromethane (15 mL x 3). The combined organic layers were washed with sodium chloride aqueous solution and dried over anhydrous sodium sulphate. After filtration, the filtrate was concentrated under reduced pressure to afford compound **2:** 1-(3-aminopropyl)-4-(2,3-dichloro-6-hydroxyphenyl)pyrrolidine-2-thione (75.4 mg, 38.2%).
¹H NMR (400 MHz, DMSO-*d*₆): *δ* 7.30-7.16 (m,1H), 6.75 (d, 1H), 5.35 (s, 3H), 4.24 (s, 1H), 3.99-3.90 (m, 3H), 3.66-3.60 (m, 1H), 3.19 (dd, 2H), 2.63 (t, 2H), 1.78-1.70 (m, 2H);
MS m/z (ESI): 319.1 [M+H]⁺.

### Example 6

### Synthesis of compound 26

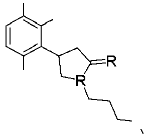

### 4-(2,3-Dichloro-6-hydroxyphenyl)-1-(3-(hydroxyamino)propyl)pyrrolidine-2-thione

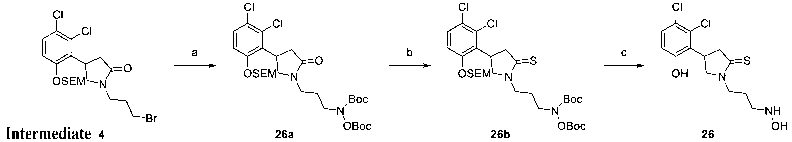

### Step a:

### tert-Butyl ((tert-butoxycarbonyl)oxy)(3-(4-(2,3-dichloro-6-((2-(trimethylsilyl)ethoxy)methoxy)phenyl)-2-oxopyrrolidin-1-yl)propyl)carbamate

To a stirred solution of intermediate 4 (300.0 mg, 0.60 mmol) in *N,N-*dimethylformamide (6 mL) were added tert-butyl ((tert-butoxycarbonyl)oxy)carbamate (422.1 mg, 1.81 mmol) and potassium carbonate (250.1 mg, 1.81 mmol) at 20°C. The reaction solution was stirred at 65°C for 2 h. After the reaction was completed, the resulting mixture was diluted with water and extracted with ethyl acetate (20 mL x 3). The combined organic layers were washed with sodium chloride aqueous solution and dried over anhydrous sodium sulphate. After filtration, the filtrate was concentrated under reduced pressure. The residue was purified by flash silica gel column chromatography (petroleum ether : tetrahydrofuran = 1:1) to afford compound **26a:** tert-butyl ((tert-butoxycarbonyl)oxy)(3-(4-(2,3-dichloro-6-((2-(trimethylsilyl)ethoxy)methoxy) phenyl)-2-oxopyrrolidin-1-yl)propyl)carbamate (300.0 mg, 77.0%).
¹H NMR (400 MHz, CDCl₃) *δ* 7.31 (d, 1H), 7.08 (d, 1H), 5.24-5.19 (m, 2H), 4.44-4.34 (m, 1H), 3.73-3.33 (m, 7H), 2.80-2.63 (m, 2H), 1.82-1.87 (m, 2H), 1.52 (s, 9H), 1.47 (s, 9H), 0.95-0.91 (m, 2H), 0.00 (s, 9H);
MS m/z (ESI): 649.1 [M+H]⁺.

### Step b:

### tert-Butyl ((tert-butoxycarbonyl)oxy)(3-(4-(2,3-dichloro-6-((2-(trimethylsilyl) ethoxy)methoxy)phenyl)-2-thiopyrrolidin-1-yl)propyl) carbamate

To a stirred solution of tert-butyl ((tert-butoxycarbonyl)oxy)(3-(4-(2,3-dichloro-6-((2-(trimethylsilyl)ethoxy)methoxy)phenyl)-2-oxopyrrolidin-1-yl)propyl)carbamate (300.0 mg, 0.46 mmol) in dichloromethane (5 mL) was added Lawesson's reagent (205.5 mg, 0.51 mmol) at 20°C. The reaction solution was stirred at 25°C for 1 h. The resulting mixture was diluted with water and extracted with dichloromethane (20 mL x 3). The combined organic layers were washed with saturated sodium bicarbonate aqueous solution and sodium chloride aqueous solution, and dried over anhydrous sodium sulphate. After filtration, the filtrate was concentrated under reduced pressure. The residue was purified by flash silica gel column chromatography (petroleum ether : tetrahydrofuran = 88:12) to afford compound **26b:** tert-butyl ((tert-butoxycarbonyl)oxy)(3-(4-(2,3-dichloro-6-((2-(trimethylsilyl)ethoxy)methoxy)phenyl)-2-thiopyrrolidin-1-yl)propyl)carbamate (210.0 mg, 68.6%).
¹H NMR (400 MHz, CDCl₃) *δ* 7.35 (d, 1H), 7.11 (d, 1H), 5.31-5.19 (m, 2H), 4.53-4.42 (m, 1H), 4.20-3.70 (m, 8H), 3.48-3.27 (m, 2H), 2.05-2.00 (m, 2H), 1.56 (s, 9H), 1.52 (s, 9H), 0.99-0.94 (m, 2H), 0.05 (s, 9H);
MS m/z (ESI): 665.1 [M+H]⁺.

### Step c:

### 4-(2,3-Dichloro-6-hydroxyphenyl)-1-(3-(hydroxyamino)propyl)pyrrolidine-2-thione

To a stirred solution of tert-butyl ((tert-butoxycarbonyl)oxy)(3-(4-(2,3-dichloro-6-((2-(trimethylsilyl)ethoxy)methoxy)phenyl)-2-thiopyrrolidin-1-yl)propyl)carbamate (210.0 mg, 0.32 mmol) in methanol (3 mL) was added oxalyl chloride (200.2 mg, 1.58 mmol) at 0°C. The reaction solution was stirred at 25°C for 2 h. The reaction solution was concentrated under reduced pressure. The concentrate was purified by high performance liquid chromatography (Welchrom C18, 20*150 mm, 5 µm; mobile phase A: formic acid in water, mobile phase B: acetonitrile, gradient ratio: phase B 5%-95%), adjusted to pH = 7 with saturated sodium bicarbonate solution, extracted, and concentrated under reduced pressure to afford compound **26:** 4-(2,3-dichloro-6-hydroxyphenyl)-1-(3-(hydroxyamino)propyl) pyrrolidine-2-thione (38.2 mg, 36.1%).
¹H NMR (400 MHz, DMSO*_d₆*) *δ* 7.34 (d, 1H), 6.83 (d, 1H), 4.32-4.22 (m, 1H), 4.02-3.84 (m, 3H), 3.69-3.62 (m, 1H), 3.19-3.16 (m, 2H), 2.75 (t, 2H), 1.84-1.76 (m, 2H);
MS m/z (ESI): 334.9 [M+H]⁺.

### Example 7

### Synthesis of compound 34

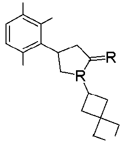

### 4-(2,3-Dichloro-6-hydroxyphenyl)-1-(2-azaspiro[3.3]heptan-6-yl)pyrrolidine-2-thione

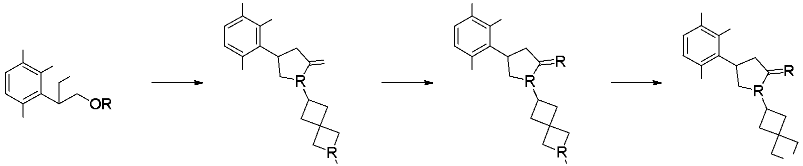

### Step a:

### tert-Butyl 6-(4-(2,3-dichloro-6-((2-(trimethylsilyl)ethoxy)methoxy)phenyl)-2-oxopyrrolidin-1-yl)-2-azaspiro[3.3]heptane-2-carboxylate

To a stirred solution of intermediate **1-e** (500.0 mg, 1.18 mmol) in dichloroethane (5 mL) were added tert-butyl 6-oxo-2-azaspiro[3.3]heptane-2-carboxylate (500.1 mg, 2.37 mmol), sodium triacetoxyborohydride (749.0 mg, 3.53 mmol) and triethylamine (0.49 mL, 3.55 mmol) at 20°C, and the reaction solution was stirred at 80°C for 16 h. Water was added, and the mixture was extracted with dichloromethane. The combined organic layers were washed with sodium chloride aqueous solution and dried over anhydrous sodium sulphate. After filtration, the filtrate was concentrated under reduced pressure, and the residue was purified by flash silica gel column chromatography (petroleum ether : ethyl acetate = 1:1) to afford compound **34a:** tert-butyl 6-(4-(2,3-dichloro-6-((2-(trimethylsilyl)ethoxy)methoxy)phenyl)-2-oxopyrrolidin-1-yl)-2-azaspiro[3.3]heptane-2-carboxylate (100.0 mg, 14.8%).

MS m/z (ESI): 571.3 [M+H]⁺.

### Step b:

### tert-Butyl 6-(4-(2,3-dichloro-6-((2-(trimethylsilyl)ethoxy)methoxy)phenyl)-2-thiopyrrolidin-1-yl)-2-azaspiro[3.3]heptane-2-carboxylate

To a stirred solution of tert-butyl 6-(4-(2,3-dichloro-6-((2-(trimethylsilyl)ethoxy)methoxy)phenyl)-2-oxopyrrolidin-1-yl)-2-azaspiro[3.3]heptane-2-carboxylate (120.0 mg, 0.21 mmol) in toluene (6 mL) was added Lawesson's reagent (84.9 mg, 0.21 mmol) at 20°C. The reaction solution was stirred at 110°C for 1 h. The reaction solution was concentrated under reduced pressure. The residue was purified by flash silica gel column chromatography (petroleum ether : ethyl acetate = 7:3) to afford compound **34b:** tert-butyl 6-(4-(2,3-dichloro-6-((2-(trimethylsilyl)ethoxy)methoxy)phenyl)-2-thiopyrrolidin-1-yl)-2-azaspiro[3.3]heptane-2-carboxylate (80.0 mg, 64.8%).

¹H NMR (400 MHz, DMSO*_d*₆) *δ* 7.54 (d, 1H), 7.15 (d, 1H), 5.29-5.28 (m, 1H), 5.15-5.07 (m, 2H), 4.37-4.33 (m, 1H), 4.19 (t, 1H), 3.95-3.76 (m, 3H), 3.69-3.62 (m, 3H), 3.04-2.98 (m, 1H), 2.48-2.35 (m, 2H), 1.38 (s, 9H), 0.91-0.83 (m, 4H), 0.02 (s, 9H).

### Step c:

### 4-(2,3-Dichloro-6-hydroxyphenyl)-1-(2-azaspiro[3.3]heptan-6-yl)pyrrolidine-2-thione

tert-Butyl 6-(4-(2,3-dichloro-6-((2-(trimethylsilyl)ethoxy)methoxy)phenyl)-2-thiopyrrolidin-1-yl)-2-azaspiro[3.3]heptane-2-carboxylate (55.0 mg, 0.09 mmol) was dissolved in a mixture of trifluoroacetic acid : dichloromethane =1:10 (3 mL) at 25°C, and the mixture was stirred at 25°C for 4 h. The reaction solution was concentrated under reduced pressure, and the residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **34:** 4-(2,3-dichloro-6-hydroxyphenyl)-1-(2-azaspiro[3.3]heptan-6-yl)pyrrolidine-2-thione (3.8 mg, 11.3%).
¹H NMR (400 MHz, DMSO*_d*₆) *δ* 8.40 (s, 1H), 7.32 (d, 1H), 6.94 (d, 1H), 4.44 (d, 1H), 4.27-4.25 (m, 1H), 4.08-3.91 (m, 4H), 3.74 (d, 1H), 3.27-3.22 (m, 1H), 3.12-3.07 (m, 3H), 1.94 (d, 2H), 1.59-1.54 (m, 2H);
MS m/z (ESI): 356.9 [M+H]⁺.

### Example 8

### Synthesis of compound 37

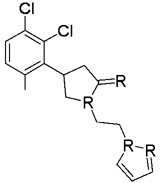

### 1-(2-(1H-Pyrazol-1-yl)ethyl)-4-(2,3-dichloro-6-hydroxyphenyl)pyrrolidine-2-thione

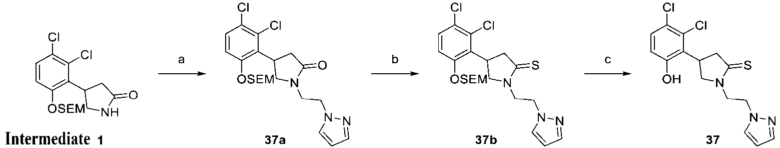

### Step a:

### 1-(2-(1H-Pyrazol-1-yl)ethyl)-4-(2,3-dichloro-6-((2-(trimethylsilyl)ethoxy) methoxy)phenyl)pyrrolidin-2-one

To a stirred solution of intermediate 1 (200.0 mg, 0.53 mmol) in toluene (4 mL) were added 1-(2-bromoethyl)pyrazole (279.0 mg, 1.59 mmol), potassium hydroxide (59.6 mg, 1.06 mmol) and tetrabutylammonium bromide (34.3 mg, 0.11 mmol) at 25°C, and the reaction solution was stirred at 30°C for 24 h. After the reaction was completed, the reaction solution was concentrated under reduced pressure, and the residue was purified by flash silica gel column chromatography (petroleum ether : ethyl acetate = 1:1) to afford compound **37a:** 1-(2-(1H-pyrazol-1-yl)ethyl)-4-(2,3-dichloro-6-((2-(trimethylsilyl)ethoxy)methoxy)phenyl)pyrrolidin-2-one (230.0 mg, 92.0%).
¹H NMR (400 MHz, DMSO*_d₆*) *δ* 7.75 (d, 1H), 7.51 (d, 1H), 7.45 (d, 1H), 7.15-7.10 (m, 1H), 6.24-6.23 (m, 1H), 5.31-5.22 (m, 2H), 4.29-4.17 (m, 3H), 3.74-3.48 (m, 5H), 3.33-3.28 (m, 2H), 2.54-2.51 (m, 1H), 0.91-0.85 (m, 2H), 0.04 (s, 9H);
MS m/z (ESI): 470.1 [M+H]⁺.

### Step b:

### 1-(2-(1H-Pyrazol-1-yl)ethyl)-4-(2,3-dichloro-6-((2-(trimethylsilyl)ethoxy) methoxy)phenyl)pyrrolidine-2-thione

To a stirred solution of 1-(2-(1H-pyrazol-1-yl)ethyl)-4-(2,3-dichloro-6-((2-(trimethylsilyl)ethoxy)methoxy)phenyl)pyrrolidin-2-one (70.0 mg, 0.15 mmol) in tetrahydrofuran (2 mL) was added Lawesson's reagent (72.2 mg, 0.18 mmol) at 20°C. The reaction solution was stirred at 20°C for 2 h. The resulting mixture was diluted with water and extracted with ethyl acetate (50 mL x 3). The combined organic layers were washed with sodium chloride aqueous solution and dried over anhydrous sodium sulphate. After filtration, the filtrate was concentrated under reduced pressure, and the residue was purified by flash silica gel column chromatography (dichloromethane : tetrahydrofuran = 50:1) to afford compound **37b:** 1-(2-(1H-pyrazol-1-yl)ethyl)-4-(2,3-dichloro-6-((2-(trimethylsilyl)ethoxy)methoxy) phenyl)pyrrolidine-2-thione (59.0 mg, 81.5%).
¹H NMR (400 MHz, DMSO*_d₆*) 7.77 (d, 1H), 7.53-7.47 (m, 2H), 7.12 (d, 1H), 6.27-6.26 (m, 1H), 5.31-5.18 (m, 2H), 4.50-4.44 (m, 2H), 4.42-4.16 (m, 3H), 4.09-4.00 (m, 4H), 3.30-3.20 (m, 1H), 3.12-3.04 (m, 1H), 0.90-0.84 (m, 2H), 0.04 (s, 9H);
MS m/z (ESI): 486.2 [M+H]⁺.

### Step c:

### 1-(2-(1H-Pyrazol-1-yl)ethyl)-4-(2,3-dichloro-6-hydroxyphenyl)pyrrolidine-2-thione

To a stirred solution of 1-(2-(1H-pyrazol-1-yl)ethyl)-4-(2,3-dichloro-6-((2-(trimethylsilyl)ethoxy)methoxy)phenyl)pyrrolidine-2-thione (170.0 mg, 0.35 mmol) in dichloromethane (2 mL) was added hydrogen chloride dioxane solution (0.2 mL, 4 mol/L) at 0°C, and the mixture was stirred at 20°C for 2 h. The reaction solution was concentrated under reduced pressure, and the residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **37:** 1-(2-(1H-pyrazol-1-yl)ethyl)-4-(2,3-dichloro-6-hydroxyphenyl)pyrrolidine-2-thione (54.3 mg, 43.6%).
¹H NMR (400 MHz, DMSO*_d₆*) *δ* 10.52 (s, 1H), 7.74 (d, 1H), 7.48 (d, 1H), 7.34 (d, 1H), 6.82 (d, 1H), 6.25 (t, 1H), 4.48-4.40 (m, 2H), 4.21-4.12 (m, 2H), 4.08-4.01 (m, 1H), 3.73-3.68 (m, 1H), 3.52-3.47 (m, 1H), 3.26-3.09 (m, 2H);
MS m/z (ESI): 355.9 [M+H]⁺.

### Example 9

### Synthesis of compound 40 and compound 40-1

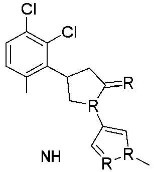

### 4-(2,3-Dichloro-6-hydroxyphenyl)-1-(1-methyl-1H-pyrazol-4-yl)pyrrolidine-2-thione

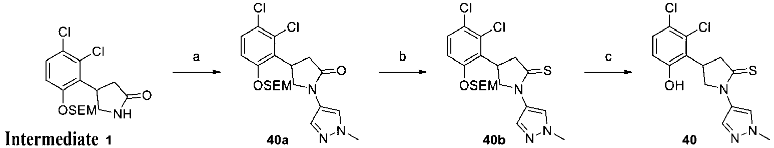

### Step a:

### 4-(2,3-Dichloro-6-((2-(trimethylsilyl)ethoxy)methoxy)phenyl)-1-(1-methyl-1H-pyrazol-4-yl)pyrrolidin-2-one

Under nitrogen protection at 20°C, 4-bromo-1-methylpyrazole (160.4 mg, 1.0 mmol) and caesium carbonate (432.9 mg, 1.33 mmol) were added to a stirred solution of intermediate 1 (250.0 mg, 0.66 mmol) in a mixed solution of *N,N-*dimethylformamide/acetonitrile (4 mL, v/v = 1:1), and the reaction solution was stirred at 20°C for 20 min, followed by the addition of cuprous iodide (126.5 mg, 0.66 mmol) and *N,N*-dimethylethylenediamine (58.6 mg, 0.66 mmol). The reaction solution was stirred at 85°C for 2 h. After the reaction was completed, the resulting mixture was diluted with water and extracted with ethyl acetate (25 mL x 3). The combined organic layers were washed with sodium chloride aqueous solution and dried over anhydrous sodium sulphate. After filtration, the filtrate was concentrated under reduced pressure, and the residue was purified by flash silica gel column chromatography (petroleum ether : ethyl acetate = 1:1) to afford compound **40a:** 4-(2,3-dichloro-6-((2-(trimethylsilyl)ethoxy)methoxy)phenyl)-1-(1-methyl-1H-pyrazol-4-yl)pyrrolidin-2-one (280.0 mg, 92.3%).
¹H NMR (400 MHz, CDCl₃) *δ* 8.05 (s, 1H), 7.41 (s, 1H), 7.33 (d, 1H), 7.07 (d, 1H), 5.16 (s, 2H), 4.62-4.51 (m, 1H), 4.05-3.99 (m, 1H), 3.90 (s, 3H), 3.84-3.79 (m, 1H), 3.62-3.55 (m, 2H), 2.88-2.81 (m, 2H), 0.89-0.83 (m, 2H), 0.05 (s, 9H);
MS m/z (ESI): 456.1 [M+H]⁺.

### Step b:

### 4-(2,3-Dichloro-6-((2-(trimethylsilyl)ethoxy)methoxy)phenyl)-1-(1-methyl-1H-pyrazol-4-yl)pyrrolidine-2-thione

To a stirred solution of 4-(2,3-dichloro-6-((2-(trimethylsilyl)ethoxy) methoxy)phenyl)-1-(1-methyl-1H-pyrazol-4-yl)pyrrolidin-2-one (280.0 mg, 0.61 mmol) in tetrahydrofuran (5 mL) was added Lawesson's reagent (272.9 mg, 0.67 mmol) at 20°C. The reaction solution was stirred at 20°C for 16 h. The resulting mixture was diluted with water and extracted with ethyl acetate (50 mL x 3). The combined organic layers were washed with sodium chloride aqueous solution and dried over anhydrous sodium sulphate. After filtration, the filtrate was concentrated under reduced pressure, and the residue was purified by flash silica gel column chromatography (petroleum ether : ethyl acetate = 3:1) to afford compound **40b:** 4-(2,3-dichloro-6-((2-(trimethylsilyl)ethoxy)methoxy)phenyl)-1-(1-methyl-1H-pyrazol-4-yl)pyrrolidine-2-thione (240.0 mg, 82.8%).
¹H NMR (400 MHz, CDCl₃) *δ* 9.06 (s, 1H), 7.59 (s, 1H), 7.33 (d, 1H), 7.07 (d, 1H), 5.18-5.11 (m, 2H), 4.65-4.54 (m, 1H), 4.46-4.39 (m, 1H), 4.26-4.21 (m, 1H), 3.93 (s, 3H), 3.61-3.52 (m, 3H), 3.42-3.34 (m, 1H), 0.88-0.83 (m, 2H), 0.04 (s, 9H);
MS m/z (ESI): 472.0 [M+H]⁺.

### Step c:

### 4-(2,3-Dichloro-6-hydroxyphenyl)-1-(1-methyl-1H-pyrazol-4-yl)pyrrolidine-2-thione

To a stirred solution of 4-(2,3-dichloro-6-((2-(trimethylsilyl)ethoxy) methoxy)phenyl)-1-(1-methyl-1H-pyrazol-4-yl)pyrrolidine-2-thione (200.0 mg, 0.44 mmol) in dichloromethane (4 mL) was added hydrogen chloride dioxane solution (0.4 mL, 4 mol/L) at 0°C, and the mixture was stirred at 20°C for 1 h. The reaction solution was concentrated under reduced pressure, and the residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **40:** 4-(2,3-dichloro-6-hydroxyphenyl)-1-(1-methyl-1H-pyrazol-4-yl)pyrrolidine-2-thione (71.4 mg, 49.3%).
¹H NMR (400 MHz, DMSO*_d₆*) δ 10.58 (s, 1H), 8.78 (s, 1H), 8.02 (s, 1H), 7.36 (d, 1H), 7.84 (d, 1H), 4.49-4.39 (m, 2H), 4.29-4.25 (m, 1H), 3.86 (s, 3H), 3.42-3.25 (m, 2H);
MS m/z (ESI): 341.9 [M+H]⁺.

Compound **40-1** was synthesised using intermediate **3** as the starting material and following the same synthetic route as compound **40.**

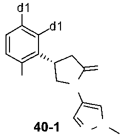

### Example 10

### Synthesis of compound 42

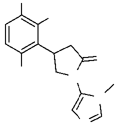

### 4-(2,3-Dichloro-6-hydroxyphenyl)-1-(1-methyl-1H-imidazol-5-yl)pyrrolidine-2-thione

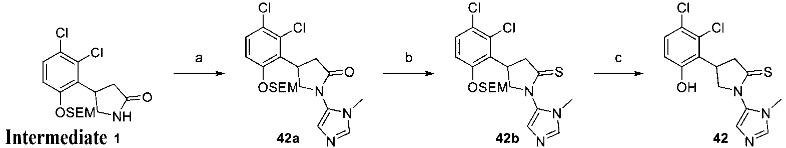

### Step a:

### 4-(2,3-Dichloro-6-((2-(trimethylsilyl)ethoxy)methoxy)phenyl)-1-(1-methyl-1H-imidazol-5-yl)pyrrolidin-2-one

Under nitrogen protection at 20°C, 5-bromo-1-methylimidazole (94.1 mg, 0.58 mmol) and caesium carbonate (519.4 mg, 1.59 mmol) were added to a stirred solution of intermediate 1 (200.0 mg, 0.53 mmol) in a mixed solution of *N,N-*dimethylformamide/acetonitrile (5 mL, v/v = 1:1), and the reaction solution was stirred at 20°C for 20 min, followed by the addition of cuprous iodide (101.2 mg, 0.53 mmol) and N,N-dimethylethylenediamine (46.8 mg, 0.53 mmol). The reaction solution was stirred at 85°C for 2 h. After the reaction was completed, the resulting mixture was diluted with saturated ammonium chloride solution and extracted with ethyl acetate (25 mL x 3). The combined organic layers were washed with sodium chloride aqueous solution and dried over anhydrous sodium sulphate. After filtration, the filtrate was concentrated under reduced pressure, and the residue was purified by flash silica gel column chromatography (petroleum ether : ethyl acetate = 4:1) to afford compound **42a:** 4-(2,3-dichloro-6-((2-(trimethylsilyl)ethoxy) methoxy)phenyl)-1-(1-methyl-1H-imidazol-5-yl)pyrrolidin-2-one (216.0 mg, 89.0%).
¹H NMR (400 MHz, DMSO*_d₆*) *δ* 7.64 (s, 1H), 7.55 (d, 1H), 7.19 (d, 1H), 6.88 (s, 1H), 5.39-5.32 (m, 2H), 4.55-4.47 (m, 1H), 3.95 (t, 1H), 3.78-3.68 (m, 3H), 3.32 (s, 3H), 2.87-2.79 (m, 1H), 2.71-2.65 (m, 1H), 0.93-0.85 (m, 2H), 0.05 (s, 9H);
MS m/z (ESI): 456.1 [M+H]⁺.

### Step b:

### 4-(2,3-Dichloro-6-((2-(trimethylsilyl)ethoxy)methoxy)phenyl)-1-(1-methyl-1H-imidazol-5-yl)pyrrolidine-2-thione

To a stirred solution of 4-(2,3-dichloro-6-((2-(trimethylsilyl)ethoxy) methoxy)phenyl)-1-(1-methyl-1H-imidazol-5-yl)pyrrolidin-2-one (130.0 mg, 0.28 mmol) in toluene (5 mL) was added Lawesson's reagent (115.2 mg, 0.28 mmol) at 20°C. The reaction solution was stirred at 100°C for 1 h. The resulting mixture was cooled to room temperature, diluted with water and extracted with ethyl acetate (25 mL x 3). The combined organic layers were washed with sodium chloride aqueous solution and dried over anhydrous sodium sulphate. After filtration, the filtrate was concentrated under reduced pressure, and the residue was purified by flash silica gel column chromatography (dichloromethane : methanol = 20:1) to afford compound **42b:** 4-(2,3-dichloro-6-((2-(trimethylsilyl)ethoxy)methoxy)phenyl)-1-(1-methyl-1H-imidazol-5-yl)pyrrolidine-2-thione (100.0 mg, 74.3%).
¹H NMR (400 MHz, DMSO*_d₆*) *δ* 7.72 (s, 1H), 9.32 (s, 1H), 7.58 (d, 1H), 7.22 (d, 1H), 6.98 (s, 1H), 5.46-5.32 (m, 2H), 4.66-4.61 (m, 1H), 4.36 (t, 1H), 4.12-4.08 (m, 1H), 3.77-3.73 (m, 2H), 3.59 (s, 3H), 3.51 (d, 1H), 3.28-3.23 (m, 1H), 0.95-0.90 (m, 2H), 0.01 (s, 9H);
MS m/z (ESI): 472.0 [M+H]⁺.

### Step c:

### 4-(2,3-Dichloro-6-hydroxyphenyl)-1-(2-(2-methyl-1H-imidazol-1-yl)ethyl)pyrrolidine-2-thione

To a stirred solution of 4-(2,3-dichloro-6-((2-(trimethylsilyl)ethoxy) methoxy)phenyl)-1-(1-methyl-1H-imidazol-5-yl)pyrrolidine-2-thione (100.0 mg, 0.21 mmol) in tetrahydrofuran (10 mL) was added tetrabutylammonium fluoride (553.3 mg, 2.12 mol/L) at 0°C, and the mixture was stirred at 20°C for 4 h. The resulting mixture was diluted with water and extracted with ethyl acetate (25 mL x 3). The combined organic layers were washed with sodium chloride aqueous solution and dried over anhydrous sodium sulphate. After filtration, the filtrate was concentrated under reduced pressure, and the residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **42:** 4-(2,3-dichloro-6-hydroxyphenyl)-1-(1-methyl-1H-imidazol-5-yl)pyrrolidine-2-thione (8.6 mg, 11.9%).
¹H NMR (400 MHz, DMSO*_d₆*) *δ* 10.72 (s, 1H), 7.66 (s, 1H), 7.37 (d, 1H), 6.91 (s, 1H), 6.88 (d, 1H), 4.55-4.49 (m, 1H), 4.26 (t, 1H), 4.15-4.11 (m, 1H), 3.55 (s, 3H), 3.47-3.39 (m, 1H), 3.29-3.24 (m, 1H);
MS m/z (ESI): 341.9 [M+H]⁺.

### Example 11

### Synthesis of compound 43

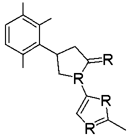

### 4-(2,3-Dichloro-6-hydroxyphenyl)-1-(2-methylthiazol-5-yl)pyrrolidine-2-thione

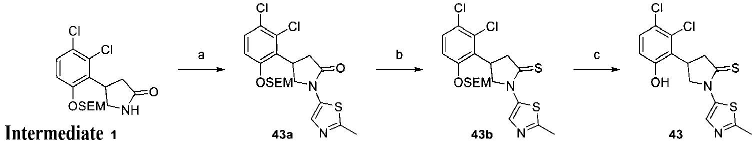

### Step a:

### 4-(2,3-Dichloro-6-((2-(trimethylsilyl)ethoxy)methoxy)phenyl)-1-(2-methylthiazol-5-yl)pyrrolidin-2-one

Under nitrogen protection at 20°C, 5-bromo-2-methylthiazole (57.3 mg, 0.32 mmol) and caesium carbonate (190.5 mg, 0.58 mmol) were added to a stirred solution of intermediate 1 (110.0 mg, 0.29 mmol) in a mixed solution of *N,N-*dimethylformamide/acetonitrile (4 mL, v/v = 1:1), and the reaction solution was stirred at 20°C for 20 min, followed by the addition of cuprous iodide (66.8 mg, 0.35 mmol) and *N,N*-dimethylethylenediamine (77.3 mg, 0.88 mmol). The reaction solution was stirred at 85°C for 2 h. After the reaction was completed, the reaction solution was concentrated under reduced pressure, and the residue was purified by flash silica gel column chromatography (petroleum ether : tetrahydrofuran = 7:3) to afford compound **43a:** 4-(2,3-dichloro-6-((2-(trimethylsilyl)ethoxy)methoxy)phenyl)-1-(2-methylthiazol-5-yl)pyrrolidin-2-one (110.0 mg, 79.5%).

¹H NMR (400 MHz, DMSO*_d*₆) *δ* 7.55 (d, 1H), 7.25 (s, 1H), 7.11 (d, 1H), 5.19-5.11 (m, 2H), 4.57-4.49 (m, 1H), 4.22 (t, 1H), 3.76-3.73 (m, 1H), 3.39-3.52 (m, 2H), 3.03-2.96 (m, 1H), 2.66-2.60 (m, 1H), 2.56 (s, 3H), 0.78-0.73 (m, 2H), - 0.09 (s, 9H).

### Step b:

### 4-(2,3-Dichloro-6-((2-(trimethylsilyl)ethoxy)methoxy)phenyl)-1-(2-methylthiazol-5-yl)pyrrolidine-2-thione

To a stirred solution of 4-(2,3-dichloro-6-((2-(trimethylsilyl)ethoxy) methoxy)phenyl)-1-(2-methylthiazol-5-yl)pyrrolidin-2-one (170.0 mg, 0.36 mmol) in toluene (5 mL) was added Lawesson's reagent (101.6 mg, 0.25 mmol) at 20°C. The reaction solution was stirred at 100°C for 1 h. The resulting mixture was diluted with water and extracted with ethyl acetate (50 mL x 3). The combined organic layers were washed with saturated sodium bicarbonate solution and dried over anhydrous sodium sulphate. After filtration, the filtrate was concentrated under reduced pressure, and the residue was purified by flash silica gel column chromatography (petroleum ether : tetrahydrofuran = 4:1) to afford compound **43b:** 4-(2,3-dichloro-6-((2-(trimethylsilyl)ethoxy)methoxy)phenyl)-1-(2-methylthiazol-5-yl)pyrrolidine-2-thione (120.0 mg, 68.3%).

MS m/z (ESI): 489.0 [M+H]⁺.

### Step c:

### 4-(2,3-Dichloro-6-hydroxyphenyl)-1-(2-methylthiazol-5-yl)pyrrolidine-2-thione

To a stirred solution of 4-(2,3-dichloro-6-((2-(trimethylsilyl)ethoxy) methoxy)phenyl)-1-(2-methylthiazol-5-yl)pyrrolidine-2-thione (200.0 mg, 0.44 mmol) in dichloromethane (2 mL) was added trifluoroacetic acid (0.4 mL) at 20°C, and the mixture was stirred at 20°C for 1 h. The reaction solution was concentrated under reduced pressure, and the residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **43:** 4-(2,3-dichloro-6-hydroxyphenyl)-1-(2-methylthiazol-5-yl)pyrrolidine-2-thione (40.0 mg, 45.4%).
¹H NMR (400 MHz, DMSO*_d₆*) *δ* 10.59 (s, 1H), 7.74 (s, 1H), 7.38 (d, 1H), 6.83 (d, 1H), 4.65-4.51 (m, 2H), 4.39-4.35 (m, 1H), 3.56-3.34 (m, 1H), 3.31-3.28 (m, 1H), 2.59 (s, 3H);
MS m/z (ESI): 358.9 [M+H]⁺.

### Example 12

### Synthesis of compound 61-1

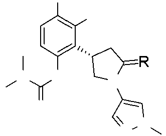

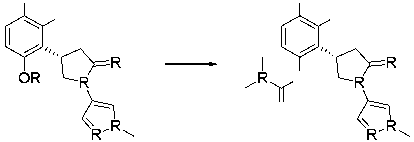

### Step a:

### (S)-3,4-Dichloro-2-(1-(1-methyl-1H-pyrazol-4-yl)-5-thiopyrrolidin-3-yl)phenyl dimethylcarbamate

To a stirred solution of compound **40-1** (150.0 mg, 0.44 mmol) in dichloromethane (8 mL) were added triethylamine (130.0 mg, 1.28 mmol), 4-dimethylaminopyridine (160.0 mg, 1.31 mmol) and *N,N*-dimethylcarbamoyl chloride (94.3 mg, 0.88 mmol) at 20°C, and the mixture was stirred at 30°C for 1 h. The resulting mixture was diluted with water and extracted with ethyl acetate (50 mL x 3). The combined organic layers were washed with saturated sodium bicarbonate solution and dried over anhydrous sodium sulphate. After filtration, the filtrate was concentrated under reduced pressure, and the residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **61-1:** (S)-3,4-dichloro-2-(1-(1-methyl-1H-pyrazol-4-yl)-5-thiopyrrolidin-3-yl)phenyl dimethylcarbamate (51.6 mg, 28.5%).
¹H NMR (400 MHz, DMSO*_d*₆) *δ* 8.79 (s, 1H), 8.01 (s, 1H), 7.61 (d, 1H), 7.17 (d, 1H), 4.58-4.47 (m, 2H), 4.18-4.15 (m, 1H), 3.87 (s, 3H), 3.60-3.52 (m, 1H), 3.13-3.07 (m, 1H), 2.79 (s, 3H), 2.71 (s, 3H);
MS m/z (ESI): 413.0 [M+H]⁺.

### Example 13

### Synthesis of compound 66 and compound 66-1

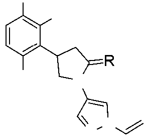

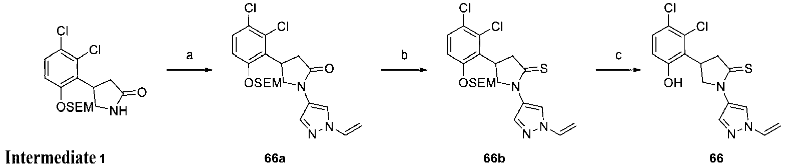

### Step a:

### rac-4-(2,3-Dichloro-6-((2-(trimethylsilyl)ethoxy)methoxy)phenyl)-1-(1-vinyl-1H-pyrazol-4-yl)pyrrolidin-2-one

Under nitrogen protection at 20°C, 1-vinyl-4-iodopyrazole (87.7 mg, 0.40 mmol) and caesium carbonate (173.1 mg, 0.53 mmol) were added to a stirred solution of intermediate 1 (100.0 mg, 0.27 mmol) in a mixed solution of *N,N-*dimethylformamide/acetonitrile (2 mL, v/v = 1:1), and the reaction solution was stirred at 20°C for 20 min, followed by the addition of cuprous iodide (50.6 mg, 0.27 mmol) and *N,N*-dimethylethylenediamine (23.4 mg, 0.27 mmol). The reaction solution was stirred at 85°C for 2 h. After the reaction was completed, the resulting mixture was diluted with water and extracted with ethyl acetate (50 mL x 3). The combined organic layers were washed with sodium chloride aqueous solution and dried over anhydrous sodium sulphate. After filtration, the filtrate was concentrated under reduced pressure, and the residue was purified by flash silica gel column chromatography (petroleum ether : ethyl acetate = 3:1) to afford compound **66a:** rac-4-(2,3-dichloro-6-((2-(trimethylsilyl)ethoxy)methoxy)phenyl)-1-(1-vinyl-1H-pyrazol-4-yl)pyrrolidin-2-one (84.0 mg, 67.4%).
¹H NMR (400 MHz, DMSO*_d₆*) *δ* 8.31 (s, 1H), 7.89 (s, 1H), 7.54 (d, 1H), 7.23-7.11 (m, 2H), 5.50 (d, 1H), 5.24-5.16 (m, 2H), 4.81 (d, 1H), 4.53-4.46 (m, 1H), 4.11-4.05 (m, 1H), 3.71-3.66 (m, 1H), 3.58-3.43 (m, 2H), 2.92-2.82 (m, 1H), 2.66-2.58 (m, 1H), 0.79-0.74 (m, 2H), -0.11 (m, 9H);
MS m/z (ESI): 468.1 [M+H]⁺.

### Step b:

### rac-4-(2,3-Dichloro-6-((2-(trimethylsilyl)ethoxy)methoxy)phenyl)-1-(1-vinyl-1H-pyrazol-4-yl)pyrrolidine-2-thione

To a stirred solution of compound **66a** (50.0 mg, 0.10 mmol) in tetrahydrofuran (2 mL) was added Lawesson's reagent (86.3 mg, 0.21 mmol) at 20°C. The reaction solution was stirred at 85°C for 5 min. The reaction solution was cooled to room temperature, diluted with 10 mL of saturated sodium bicarbonate solution, and extracted with ethyl acetate (15 mL x 3). The combined organic layers were washed with saturated sodium chloride aqueous solution and dried over anhydrous sodium sulphate. After filtration, the filtrate was concentrated under reduced pressure, and the residue was purified by flash silica gel column chromatography (petroleum ether : tetrahydrofuran = 20:1) to afford compound **66b:** rac-4-(2,3-dichloro-6-((2-(trimethylsilyl)ethoxy)methoxy)phenyl)-1-(1-vinyl-1H-pyrazol-4-yl)pyrrolidine-2-thione (40.0 mg, 77.3%).
¹H NMR (300 MHz, CDCl₃) *δ* 9.31 (s, 1H), 7.77 (s, 1H), 7.33 (d, 1H), 7.08-6.99 (m, 2H), 5.58-5.53 (m, 1H), 5.18-5.11 (m, 2H), 4.94-4.91 (m, 1H), 4.67-4.56 (m, 1H), 4.49-4.42 (m, 1H), 4.28-4.23 (m, 1H), 3.63-3.53 (m, 3H), 3.43-3.35 (m, 1H), 0.87-0.82 (m, 2H), -0.05 (s, 9H);
MS m/z (ESI): 484.1 [M+H]⁺.

### Step c:

### rac-4-(2,3-Dichloro-6-hydroxyphenyl)-1-(1-vinyl-1H-pyrazol-4-yl)pyrrolidine-2-thione

To a stirred solution of compound **66b** (110.0 mg, 0.23 mmol) in dichloromethane (2 mL) was added hydrogen chloride dioxane solution (2 mL, 4 M) at 0°C, and the mixture was stirred at 0°C for 1 h. The reaction solution was diluted with 10 mL of water and extracted with dichloromethane (15 mL x 3). The combined organic layers were washed with saturated sodium bicarbonate aqueous solution and dried over anhydrous sodium sulphate. After filtration, the filtrate was concentrated under reduced pressure, and the residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **66:** rac-4-(2,3-dichloro-6-hydroxyphenyl)-1-(1-vinyl-1H-pyrazol-4-yl)pyrrolidine-2-thione (20.1 mg, 25.0%).
¹H NMR (400 MHz, DMSO*_d₆*) *δ* 10.59 (s, 1H), 9.06 (s, 1H), 8.33 (s, 1H), 7.37 (d, 1H), 7.30-7.24 (m, 1H), 6.84 (d, 1H), 5.61-5.57 (m, 1H), 4.90 (d, 1H), 4.51-4.44 (m, 2H), 4.33-4.30 (m, 1H), 3.45-3.28 (m, 2H);
MS m/z (ESI): 354.0 [M+H]⁺.

Compound **66-1** was synthesised using intermediate **3** as the starting material and following the same synthetic route as compound **66.**

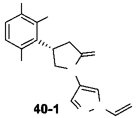

### Example 14

### Synthesis of compound 68 and compound 68-1

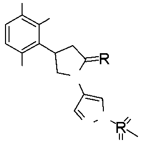

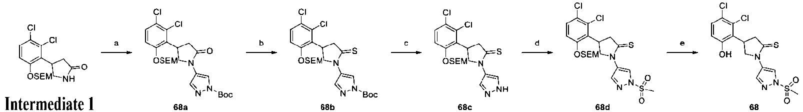

### Step a:

### rac-tert-Butyl 4-(4-(2,3-dichloro-6-((2-(trimethylsilyl)ethoxy)methoxy)phenyl) -2-oxopyrrolidin-1-yl)-1H-pyrazole-1-carboxylate

To a stirred solution of intermediate 1 (300.0 mg, 0.80 mmol) in a mixed solution of N,N-dimethylformamide/acetonitrile (12 mL, v/v = 1:1) were added tert-butyl 4-iodopyrazole-1-carboxylate (703.3 mg, 2.39 mmol) and potassium phosphate (507.6 mg, 2.39 mmol) at 25°C. The reaction solution was stirred at 25°C for 15 min, followed by the addition of cuprous iodide (151.8 mg, 0.80 mmol) and *N,N*-dimethylethylenediamine (70.3 mg, 0.80 mmol). The reaction solution was stirred at 65°C for 2 h. After the reaction was completed, the resulting mixture was diluted with water and extracted with ethyl acetate (50 mL x 3). The combined organic layers were washed with sodium chloride aqueous solution and dried over anhydrous sodium sulphate. After filtration, the filtrate was concentrated under reduced pressure, and the residue was purified by flash silica gel column chromatography (petroleum ether : ethyl acetate = 3:1) to afford compound **68a:** rac-tert-butyl 4-(4-(2,3-dichloro-6-((2-(trimethylsilyl)ethoxy)methoxy)phenyl)-2-oxopyrrolidin-1-yl)-1H-pyrazole-1-carboxylate (180.0 mg, 41.6%).

MS m/z (ESI): 542.1 [M+H]⁺.

### Step b:

### rac-tert-Butyl 4-(4-(2,3-dichloro-6-((2-(trimethylsilyl)ethoxy)methoxy)phenyl) -2-thiopyrrolidin-1-yl)-1H-pyrazole-1-carboxylate

To a stirred solution of compound **68a** (200.0 mg, 0.37 mmol) in toluene (8 mL) was added Lawesson's reagent (149.1 mg, 0.37 mmol) at 20°C. The reaction solution was stirred at 90°C for 20 min. The reaction solution was cooled to room temperature, diluted with 15 mL of saturated sodium bicarbonate solution, and extracted with ethyl acetate (20 mL x 3). The combined organic layers were washed with saturated sodium chloride aqueous solution and dried over anhydrous sodium sulphate. After filtration, the filtrate was concentrated under reduced pressure, and the residue was purified by flash silica gel column chromatography (petroleum ether : tetrahydrofuran = 10:1) to afford compound **68b:** rac-tert-butyl 4-(4-(2,3-dichloro-6-((2-(trimethylsilyl)ethoxy)methoxy)phenyl)-2-thiopyrrolidin-1-yl)-1H-pyrazole-1-carboxylate (50.0 mg, 24.3%).

MS m/z (ESI): 558.1 [M+H]⁺.

### Step c:

### rac-4-(2,3-Dichloro-6-((2-(trimethylsilyl)ethoxy)methoxy)phenyl)-1-(1H-pyrazol-4-yl)pyrrolidine-2-thione

To a stirred solution of compound **68b** (70.0 mg, 0.13 mmol) in methanol (2 mL) was added potassium carbonate (52.0 mg, 0.38 mmol) at 25°C, and the reaction solution was stirred at 30°C for 1 h. After the reaction was completed, the resulting mixture was diluted with water and extracted with ethyl acetate (50 mL x 3). The combined organic layers were washed with sodium chloride aqueous solution and dried over anhydrous sodium sulphate. After filtration, the filtrate was concentrated under reduced pressure, and the residue was purified by flash silica gel column chromatography (petroleum ether : tetrahydrofuran = 4:1) to afford compound **68c:** rac-4-(2,3-dichloro-6-((2-(trimethylsilyl)ethoxy)methoxy)phenyl)-1-(1H-pyrazol-4-yl)pyrrolidine-2-thione (40.0 mg, 69.6%).

MS m/z (ESI): 458.0 [M+H]⁺.

### Step d:

### rac-4-(2,3-Dichloro-6-((2-(trimethylsilyl)ethoxy)methoxy)phenyl)-1-(1-(methylsulfonyl)-1H-pyrazol-4-yl)pyrrolidine-2-thione

To a stirred solution of compound **68c** (40.0 mg, 0.09 mmol) in dichloromethane (1 mL) were added triethylamine (13.2 mg, 0.13 mmol) and methylsulfonyl chloride (11.9 mg, 0.10 mmol) at 0°C, and the reaction solution was stirred at 0°C for 1 h. After the reaction was completed, the resulting mixture was diluted with water and extracted with ethyl acetate (10 mL x 3). The combined organic layers were washed with sodium chloride aqueous solution and dried over anhydrous sodium sulphate. After filtration, the filtrate was concentrated under reduced pressure, and the residue was purified by flash silica gel column chromatography (petroleum ether : tetrahydrofuran = 4:1) to afford compound **68d:** rac-4-(2,3-dichloro-6-((2-(trimethylsilyl)ethoxy)methoxy)phenyl)-1-(1-(methylsulfonyl)-1H-pyrazol-4-yl)pyrrolidine-2-thione (40.0 mg, 85.4%).

MS m/z (ESI): 536.0 [M+H]⁺.

### Step e:

### rac-4-(2,3-Dichloro-6-hydroxyphenyl)-1-(1-(methylsulfonyl)-1H-pyrazol-4-yl)pyrrolidine-2-thione

To a stirred solution of compound **68d** (40.0 mg, 0.07 mmol) in dichloromethane (3 mL) was added hydrogen chloride dioxane solution (0.5 mL, 4N) at 20°C, and the mixture was stirred at 30°C for 1 h. The resulting mixture was diluted with saturated sodium bicarbonate aqueous solution and extracted with dichloromethane (10 mL x 3). The combined organic layers were washed with sodium chloride aqueous solution and dried over anhydrous sodium sulphate. After filtration, the filtrate was concentrated under reduced pressure, and the residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **68:** rac-4-(2,3-dichloro-6-hydroxyphenyl)-1-(1-(methylsulfonyl)-1H-pyrazol-4-yl)pyrrolidine-2-thione (8.2 mg, 27.1%).
¹H NMR (400 MHz, DMSO*_d₆*) *δ* 10.59 (s, 1H), 9.37 (s, 1H), 8.62 (s, 1H), 7.38 (d, 1H), 6.84 (d, 1H), 4.56-4.46 (m, 2H), 4.37-4.35 (m, 1H), 3.60 (s, 3H), 3.48-3.41 (m, 1H), 3.36-3.27 (m, 1H);
MS m/z (ESI): 405.9 [M+H]⁺.

Compound **68-1** was synthesised using intermediate **3** as the starting material and following the same synthetic route as compound **68.**

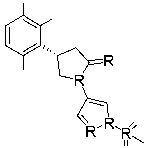

### Example 15

### Synthesis of compound 76

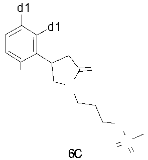

### N-(3-(4-(2,3-Dichloro-6-hydroxyphenyl)-2-oxopyrrolidin-1-yl)propyl)methanesulfonamide

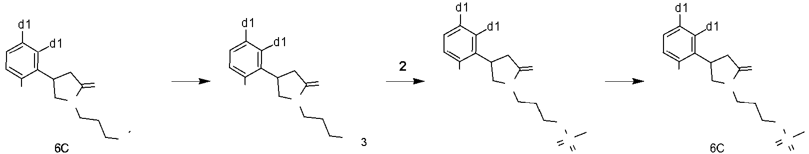

### Step a:

### 1-(3-Aminopropyl)-4-(2,3-dichloro-6-((2-(trimethylsilyl)ethoxy)methoxy) phenyl)pyrrolidin-2-one

To a stirred solution of compound **2a** (108.0 mg, 0.25 mmol) in dichloromethane (2 mL) was added zinc bromide (227.9 mg, 1.01 mmol) at 25°C. The reaction solution was stirred at 25°C for 2 h. The reaction solution was filtered to afford compound **76a:** 1-(3-aminopropyl)-4-(2,3-dichloro-6-((2-(trimethylsilyl)ethoxy)methoxy)phenyl)pyrrolidin-2-one (87.0 mg, 99.2%).

MS m/z (ESI): 433.0 [M+H]⁺.

### Step b:

### N-(3-(4-(2,3-Dichloro-6-(2-(trimethylsilyl)ethoxy)methoxy)phenyl)-2-oxopyrrolidin-1-yl)propyl)methanesulfonamide

To a stirred solution of 1-(3-aminopropyl)-4-(2,3-dichloro-6-((2-(trimethylsilyl)ethoxy)methoxy)phenyl)pyrrolidin-2-one (80.0 mg, 0.18 mmol) in dichloromethane (2 mL) were added methylsulfonyl chloride (0.03 mL, 0.23 mmol) and triethylamine (0.08 mL, 0.55 mmol) at 25°C, and the reaction solution was stirred at 25°C for 1 h. The reaction solution was concentrated, and the residue was purified by flash silica gel column chromatography (petroleum ether : ethyl acetate = 10:1) to afford compound **76b:** *N*-(3-(4-(2,3-dichloro-6-(2-(trimethylsilyl)ethoxy)methoxy)phenyl)-2-oxopyrrolidin-1-yl)propyl)methanesulfonamide (55.0 mg, 58.3%).

MS m/z (ESI): 511.0 [M+H]⁺.

### Step c:

### N-(3-(4-(2,3-Dichloro-6-hydroxyphenyl)-2-oxopyrrolidin-1-yl)propyl)methanesulfonamide

To a stirred solution of *N*-(3-(4-(2,3-dichloro-6-(2-(trimethylsilyl)ethoxy)methoxy)phenyl)-2-oxopyrrolidin-1-yl)propyl)methanesulfonamide (100.0 mg, 0.20 mmol) in dichloromethane (5 mL) was added trifluoroacetic acid (0.5 mL) at 0°C, and the reaction solution was stirred at 0°C for 1 h. The reaction solution was concentrated. The residue was purified by flash reverse-phase C18 column chromatography (water : acetonitrile = 6:4, with 10 mM ammonium bicarbonate) to afford compound **76:** *N*-(3-(4-(2,3-dichloro-6-hydroxyphenyl)-2-oxopyrrolidin-1-yl)propyl)methanesulfonamide (20.0 mg, 26.8%).

¹H NMR (400 MHz, CD₃OD): *δ* 7.27 (d, 1H), 6.79 (d, 1H), 4.50-4.37 (m, 1H), 3.79-3.68 (m, 2H), 3.58-3.48 (m, 1H), 3.41-3.36 (m, 1H), 3.16-3.11 (m, 2H), 2.96 (s, 3H), 2.85 (dd, 1H), 2.70 (dd, 1H), 1.91-1.80 (m, 2H).

MS m/z (ESI): 381.2 [M+H]⁺.

### Example 16

### Synthesis of compound 77

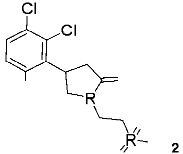

### 2-(4-(2,3-Dichloro-6-hydroxyphenyl)-2-oxopyrrolidin-1-yl)ethane-1-sulfonamide

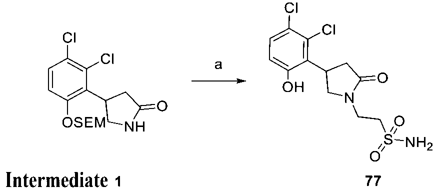

### Step a:

### 2-(4-(2,3-Dichloro-6-hydroxyphenyl)-2-oxopyrrolidin-1-yl)ethane-1-sulfonamide

To a stirred solution of intermediate 1 (100.0 mg, 0.27 mmol) in *N,N-*dimethylformamide (1 mL) was added sodium hydride (21.2 mg, 0.53 mmol) at 20°C, and the reaction solution was stirred at 20°C for 0.5 h, followed by the addition of ethylenesulfonamide (28.4 mg, 0.27 mmol). The reaction solution was stirred at 80°C for 18 h. The reaction solution was diluted with methanol and water, and concentrated. The residue was purified by flash reverse-phase C18 column chromatography (water : acetonitrile = 6:4, with 10 mM ammonium bicarbonate) to afford compound 77: 2-(4-(2,3-dichloro-6-hydroxyphenyl)-2-oxopyrrolidin-1-yl)ethane-1-sulfonamide (20.0 mg, 21.3%).

¹H NMR (400 MHz, CD₃OD): *δ* 7.26 (d, 1H), 6.78 (dd, 1H), 4.48-4.39 (m, 1H), 4.21-3.96 (m, 1H), 3.86-3.60 (m, 3H), 3.29 (s, 1H), 2.95-2.74 (m, 2H), 2.71-2.60 (m, 1H).

MS m/z (ESI): 353.0 [M+H]⁺.

### Example 17

### Synthesis of compound 78

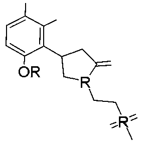

### 4-(2,3-Dichloro-6-hydroxyphenyl)-1-(2-(S-methylsulfonylimino)ethyl) pyrrolidin-2-one

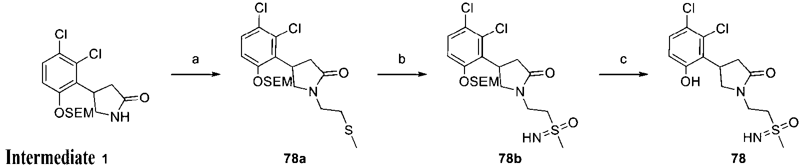

### Step a:

### 4-(2,3-Dichloro-6-((2-(trimethylsilyl)ethoxy)methoxy)phenyl)-1-(2-(methylthio)ethyl)pyrrolidin-2-one

To a stirred solution of intermediate 1 (100.0 mg, 0.27 mmol) in tetrahydrofuran (5 mL) was added sodium hydride (6.4 mg, 0.16 mmol) at 20°C. The reaction solution was stirred at 20°C for 0.5 h, and then (2-bromoethyl)(methyl)sulfane (82.4 mg, 0.53 mmol) was added. The reaction solution was stirred at 60°C for 18 h, and diluted with water and ethyl acetate. The aqueous layer was extracted with ethyl acetate (25 mL x 3), and the combined organic layers were washed with sodium chloride aqueous solution and dried over anhydrous magnesium sulphate. After filtration, the filtrate was concentrated under reduced pressure, and the residue was purified by flash silica gel column chromatography (petroleum ether : ethyl acetate = 1:1) to afford compound **78a:** 4-(2,3-dichloro-6-((2-(trimethylsilyl)ethoxy)methoxy)phenyl)-1-(2-(methylthio)ethyl)pyrrolidin-2-one (20.0 mg, 16.7%).

MS m/z (ESI): 450.3 [M+H]⁺.

### Step b:

### 4-(2,3-Dichloro-6-((2-(trimethylsilyl)ethoxy)methoxy)phenyl)-1-(2-(S-methylsulfonylimino)ethyl)pyrrolidin-2-one

To a stirred solution of 4-(2,3-dichloro-6-((2-(trimethylsilyl)ethoxy)methoxy)phenyl)-1-(2-(methylthio)ethyl)pyrrolidin-2-one (180.0 mg, 0.40 mmol) in methanol (1 mL) were added iodobenzene acetate (0.2 mL, 0.80 mmol) and ammonium carbamate (124.8 mg, 1.60 mmol) at 20°C. The reaction solution was stirred at 20°C for 18 h. The filtrate was concentrated under reduced pressure, and the residue was purified by flash silica gel column chromatography (petroleum ether : ethyl acetate = 1:1) to afford compound **78b:** 4-(2,3-dichloro-6-((2-(trimethylsilyl)ethoxy)methoxy)phenyl)-1-(2-(S-methylsulfonylimino)ethyl)pyrrolidin-2-one (100.0 mg, 52.0%).

MS m/z (ESI): 481.2 [M+H]⁺.

### Step c:

### 4-(2,3-Dichloro-6-hydroxyphenyl)-1-(2-(S-methylsulfonylimino)ethyl) pyrrolidin-2-one

To a stirred solution of 4-(2,3-dichloro-6-((2-(trimethylsilyl)ethoxy) methoxy)phenyl)-1-(2-(S-methylsulfonylimino)ethyl)pyrrolidin-2-one (100.0 mg, 0.21 mmol) in dioxane (5 mL) was added hydrochloric acid (2 mL) at 20°C. The reaction solution was stirred at 20°C for 1 h. The resulting mixture was extracted with saturated sodium bicarbonate aqueous solution and dichloromethane, and the combined organic layers were washed with sodium chloride aqueous solution and dried over anhydrous magnesium sulphate. After filtration, the filtrate was concentrated under reduced pressure. The residue was purified by flash reverse-phase C18 column chromatography (water : acetonitrile = 7:3) to afford compound **78:** 4-(2,3-dichloro-6-hydroxyphenyl)-1-(2-(S-methylsulfonylimino)ethyl) pyrrolidin-2-one (20.0 mg, 27.4%).

¹H NMR (400 MHz, CD₃OD) *δ* 7.29 (d, 1H), 6.81 (d, 1H), 4.51-4.42 (m, 1H), 4.08-3.89 (m, 4H), 3.87-3.72 (m, 2H), 3.61 (s, 3H), 2.86-2.83 (m, 1H), 2.71 (dd, 1H).

MS m/z (ESI): 351.0 [M+H]⁺.

### Example 18

### Synthesis of compound 79

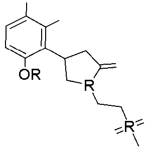

### 4-(2,3-Dichloro-6-hydroxyphenyl)-1-(2-(methylsulfonyl)ethyl)pyrrolidin-2-one

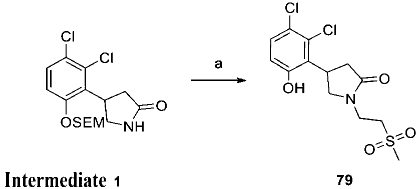

### Step a:

### 4-(2,3-Dichloro-6-hydroxyphenyl)-1-(2-(methylsulfonyl)ethyl)pyrrolidin-2-one

To a stirred solution of intermediate 1 (200.0 mg, 0.53 mmol) in dimethylsulfoxide (5 mL) were added caesium carbonate (519.4 mg, 1.59 mmol) and 1-bromo-2-(methylsulfonyl)ethane (0.05 mL, 0.53 mmol) at 20°C. The reaction solution was reacted under microwave at 130°C for 1 h. The reaction solution was filtered, and the filter cake was purified by flash reverse-phase C18 column chromatography (water : acetonitrile = 6:4, with 10 mM ammonium bicarbonate) to afford compound **79:** 4-(2,3-dichloro-6-hydroxyphenyl)-1-(2-(methylsulfonyl)ethyl) pyrrolidin-2-one (25.0 mg, 13.4%).

¹H NMR (400 MHz, CD₃OD): *δ* 7.27 (d, 1H), 6.79 (d, 1H), 4.50-4.37 (m, 1H), 3.93-3.69 (m, 4H), 3.43 (t, 2H), 3.06 (s, 3H), 2.83 (dd, 1H), 2.74-2.60 (m, 1H).

MS m/z (ESI): 352.0 [M+H]⁺.

### Example 19

### Synthesis of compound 90

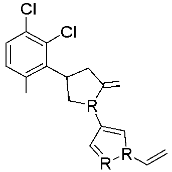

### 4-(2,3-Dichloro-6-hydroxyphenyl)-1-(1-vinyl-1H-pyrazol-4-yl)pyrrolidin-2-one

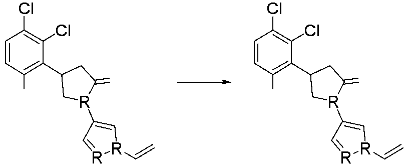

### Step a:

### rac-4-(2,3-Dichloro-6-hydroxyphenyl)-1-(1-vinyl-1H-pyrazol-4-yl)pyrrolidin-2-one

To a stirred solution of compound **66a** (149.0 mg, 0.32 mmol) in tetrahydrofuran (1.5 mL) was added tetrabutylammonium fluoride (831.7 mg, 3.18 mmol) at 20°C, and the mixture was stirred at 60°C for 3 h. The reaction solution was concentrated under reduced pressure, and the residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **90:** rac-4-(2,3-dichloro-6-hydroxyphenyl)-1-(1-vinyl-1H-pyrazol-4-yl)pyrrolidin-2-one (91.4 mg, 85.0%).
¹H NMR (400 MHz, DMSO*_d₆*) *δ* 10.54 (s, 1H), 8.33 (s, 1H), 7.90 (s, 1H), 7.38 (d, 1H), 7.25-7.17 (m, 1H), 6.85 (d, 1H), 5.53 (d, 1H), 4.83 (d, 1H), 4.46-4.41 (m, 1H), 4.02 (t, 1H), 3.82-3.77 (m, 1H), 2.79-2.76 (m, 2H);
MS m/z (ESI): 337.9 [M+H]⁺.

### Example 20

### Synthesis of compound 92

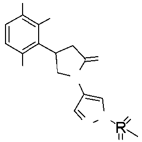

### 4-(2,3-Dichloro-6-hydroxyphenyl)-1-(1-(methylsulfonyl)-1H-pyrazol-4-yl)pyrrolidin-2-one

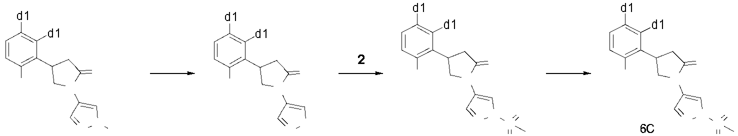

### Step a:

rac-4-(2,3-Dichloro-6-((2-(trimethylsilyl)ethoxy)methoxy)phenyl)-1-(1H-pyrazol-4-yl)pyrrolidin-2-one To a stirred solution of compound **68a** (180.0 mg, 0.33 mmol) in methanol (5 mL) was added potassium carbonate (137.6 mg, 1.0 mmol) at 25°C, and the reaction solution was stirred at 25°C for 1 h. After the reaction was completed, the resulting mixture was diluted with water and extracted with ethyl acetate (50 mL x 3). The combined organic layers were washed with sodium chloride aqueous solution and dried over anhydrous sodium sulphate. After filtration, the filtrate was concentrated under reduced pressure, and the residue was purified by flash silica gel column chromatography (petroleum ether : ethyl acetate = 7:3) to afford compound **92a:** rac-4-(2,3-dichloro-6-((2-(trimethylsilyl)ethoxy)methoxy)phenyl)-1-(1H-pyrazol-4-yl)pyrrolidin-2-one (90.0 mg, 61.3%).

MS m/z (ESI): 442.0 [M+H]⁺.

### Step b:

### rac-4-(2,3-Dichloro-6-((2-(trimethylsilyl)ethoxy)methoxy)phenyl)-1-(1-(methylsulfonyl)-1H-pyrazol-4-yl)pyrrolidin-2-one

To a stirred solution of compound **92a** (90.0 mg, 0.20 mmol) in dichloromethane (2 mL) were added triethylamine (30.9 mg, 0.31 mmol) and methylsulfonyl chloride (27.8 mg, 0.24 mmol) at 0°C, and the reaction solution was stirred at 0°C for 1 h. After the reaction was completed, the resulting mixture was diluted with water and extracted with ethyl acetate (25 mL x 3). The combined organic layers were washed with sodium chloride aqueous solution and dried over anhydrous sodium sulphate. After filtration, the filtrate was concentrated under reduced pressure, and the residue was purified by flash silica gel column chromatography (petroleum ether : ethyl acetate = 3:1) to afford compound **92b:** rac-4-(2,3-dichloro-6-((2-(trimethylsilyl)ethoxy)methoxy)phenyl)-1-(1-(methylsulfonyl)-1H-pyrazol-4-yl)pyrrolidin-2-one (90.0 mg, 85.0%).

MS m/z (ESI): 520.0 [M+H]⁺.

### Step c:

### rac-4-(2,3-Dichloro-6-hydroxyphenyl)-1-(1-(methylsulfonyl)-1H-pyrazol-4-yl)pyrrolidin-2-one

To a stirred solution of compound **92b** (70.0 mg, 0.13 mmol) in dichloromethane (4 mL) was added trifluoroacetic acid (0.2 mL) at 20°C, and the mixture was stirred at 25°C for 2 h. The reaction solution was concentrated under reduced pressure, and the residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **92:** rac-4-(2,3-dichloro-6-hydroxyphenyl)-1-(1-(methylsulfonyl)-1H-pyrazol-4-yl)pyrrolidin-2-one (31.4 mg, 59.8%).
¹H NMR (400 MHz, DMSO*_d₆*) *δ* 10.54 (s, 1H), 8.41 (s, 1H), 8.29 (s, 1H), 7.37 (d, 1H), 6.85 (d, 1H), 4.46-4.41 (m, 1H), 4.05 (t, 1H), 3.84-3.80 (m, 1H), 3.53 (s, 3H), 2.85-2.73 (m, 2H);
MS m/z (ESI): 389.9 [M+H]⁺.

### Example 21

### Synthesis of compound 94 and compound 94-1

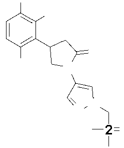

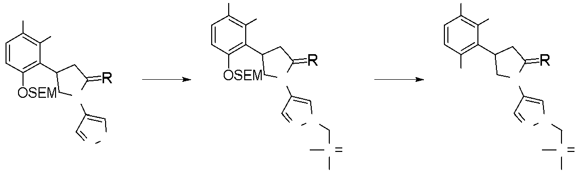

### Step a:

### rac-4-(2,3-Dichloro-6-((2-(trimethylsilyl)ethoxy)methoxy)phenyl)-1-(1-((dimethylphosphoryl)methyl)-1H-pyrazol-4-yl)pyrrolidine-2-thione

To a stirred solution of compound **68c** (450.0 mg, 0.98 mmol) in *N,N-*dimethylformamide (10 mL) were added chloro(dimethylphosphoryl)methane (149.0 mg, 1.18 mmol), 18-crown-6 (51.9 mg, 0.20 mmol) and potassium carbonate (271.3 mg, 1.96 mmol) at 20°C, and the reaction solution was stirred at 80°C for 10 h. After the reaction was completed, the resulting mixture was diluted with water and extracted with dichloromethane (25 mL x 3). The combined organic layers were washed with sodium chloride aqueous solution and dried over anhydrous sodium sulphate. After filtration, the filtrate was concentrated under reduced pressure, and the residue was purified by flash silica gel column chromatography (dichloromethane : methanol = 20:1) to afford compound **94a:** rac-4-(2,3-dichloro-6-((2-(trimethylsilyl)ethoxy)methoxy)phenyl)-1-(1-((dimethylphosphoryl)methyl)-1H-pyrazol-4-yl)pyrrolidine-2-thione (340.0 mg, 63.2%).
¹H NMR (400 MHz, CDCl₃) *δ* 9.01 (s, 1H), 7.78 (s, 1H), 7.32 (d, 1H), 7.06 (d, 1H), 5.18-5.10 (m, 2H), 4.59-4.54 (m, 3H), 4.37 (t, 1H), 4.26-3.42 (m, 1H), 3.60 (t, 2H), 3.55-3.51 (m, 1H), 3.48-3.42 (m, 1H), 1.57-1.52 (m, 6H), 0.88-0.83 (m, 2H), 0.00 (s, 9H);
MS m/z (ESI): 548.0 [M+H]⁺.

### Step b:

### rac-4-(2,3-Dichloro-6-hydroxyphenyl)-1-(1-((dimethylphosphoryl)methyl)-1H-pyrazol-4-yl)pyrrolidine-2-thione

To a stirred solution of compound **94a** (170.0 mg, 0.31 mmol) in dichloromethane (5 mL) was added trifluoroacetic acid (0.5 mL) at 20°C, and the mixture was stirred at 30°C for 1 h. After the reaction was completed, the resulting mixture was diluted with saturated sodium bicarbonate solution and extracted with dichloromethane (20 mL x 3). The combined organic layers were washed with sodium chloride aqueous solution and dried over anhydrous sodium sulphate. After filtration, the filtrate was concentrated under reduced pressure, and the residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **94:** rac-4-(2,3-dichloro-6-hydroxyphenyl)-1-(1-((dimethylphosphoryl)methyl)-1H-pyrazol-4-yl)pyrrolidine-2-thione (74.4 mg, 57.4%).
¹H NMR (400 MHz, DMSO*_d₆*) *δ* 10.60 (brs, 1H), 8.95 (s, 1H), 8.06 (s, 1H), 7.36 (d, 1H), 6.82 (d, 1H), 4.70 (d, 2H), 4.49-4.41 (m, 2H), 4.32-4.27 (m, 1H), 3.42-3.26 (m, 2H), 1.45-1.41 (m, 6H);
MS m/z (ESI): 417.9 [M+H]⁺.

Compound **94-1** was synthesised using intermediate **3** as the starting material and following the same synthetic route as compound **94.**

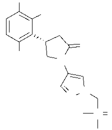

### Example 22

### Synthesis of compound 100

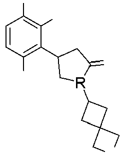

### 4-(2,3-Dichloro-6-hydroxyphenyl)-1-(2-azaspiro[3.3]heptan-6-yl)pyrrolidin-2-one

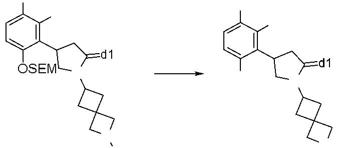

### Step a:

### 4-(2,3-Dichloro-6-hydroxyphenyl)-1-(2-azaspiro[3.3]heptan-6-yl)pyrrolidin-2-one

To a stirred solution of tert-butyl 6-(4-(2,3-dichloro-6-((2-(trimethylsilyl)ethoxy)methoxy)phenyl)-2-oxopyrrolidin-1-yl)-2-azaspiro[3.3]heptane-2-carboxylate (100.0 mg, 0.17 mmol) in dichloromethane (0.2 mL) was added trifluoroacetic acid (0.1 mL) at 25°C, and the reaction solution was stirred at 25°C for 1 h. The reaction solution was concentrated under reduced pressure, and the residue was purified by flash reverse-phase C18 column chromatography (water : acetonitrile = 7:3) to afford compound **100:** 4-(2,3-dichloro-6-hydroxyphenyl)-1-(2-azaspiro[3.3]heptan-6-yl)pyrrolidin-2-one (21.0 mg, 35.2%).

¹H NMR (400 MHz, CD₃OD): *δ* 7.27 (d, 1H), 6.79 (d, 1H), 4.55-4.35 (m, 2H), 4.19 (s, 2H), 4.04 (s, 2H), 3.82-3.67 (m, 2H), 2.84 (dd, 1H), 2.74-2.51 (m, 5H).

MS m/z (ESI): 341.2 [M+H]⁺.

### Example 23

### Synthesis of compound 101

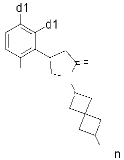

### 1-(6-Aminospiro[3.3]heptan-2-yl)-4-(2,3-dichloro-6-hydroxyphenyl) pyrrolidin-2-one

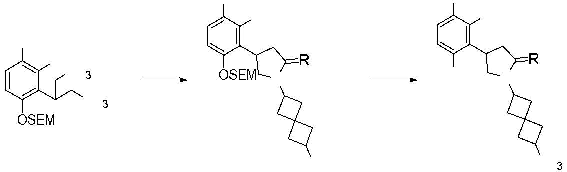

### Step a:

### tert-Butyl (6-(4-(2,3-dichloro-6-((2-(trimethylsilyl)ethoxy)methoxy)phenyl)-2-oxopyrrolidin-1-yl)spiro[3.3]heptan-2-yl)carbamate

To a stirred solution of intermediate **1-e** (200.0 mg, 0.47 mmol) in dichloroethane (5 mL) were added tert-butyl (6-oxospiro[3.3]heptan-2-yl)carbamate (320.0 mg, 1.42 mmol), sodium triacetoxyborohydride (299.6 mg, 1.41 mmol) and triethylamine (0.2 mL, 1.43 mmol) at 20°C, and the reaction solution was stirred at 80°C for 16 h. Water was added, and the mixture was extracted with dichloromethane. The combined organic layers were washed with sodium chloride aqueous solution and dried over anhydrous sodium sulphate. After filtration, the filtrate was concentrated under reduced pressure, and the residue was purified by flash silica gel column chromatography (petroleum ether : ethyl acetate = 1:1) to afford compound **101a:** tert-butyl (6-(4-(2,3-dichloro-6-((2-(trimethylsilyl)ethoxy)methoxy)phenyl)-2-oxopyrrolidin-1-yl)spiro[3.3]heptan-2-yl)carbamate (20.0 mg, 7.2%).

MS m/z (ESI): 585.2 [M+H]⁺.

### Step b:

### 1-(6-Aminospiro[3.3]heptan-2-yl)-4-(2,3-dichloro-6-hydroxyphenyl) pyrrolidin-2-one

To a stirred solution of tert-butyl (6-(4-(2,3-dichloro-6-((2-(trimethylsilyl)ethoxy)methoxy)phenyl)-2-oxopyrrolidin-1-yl)spiro[3.3]heptan-2-yl)carbamate (100.0 mg, 0.17 mmol) in dichloromethane (0.2 mL) was added trifluoroacetic acid (0.1 mL) at 25°C, and the reaction solution was stirred at 25°C for 1 h. The reaction solution was concentrated under reduced pressure, and the residue was purified by flash reverse-phase C18 column chromatography (water : acetonitrile = 7:3) to afford compound **101:** 1-(6-aminospiro[3.3]heptan-2-yl)-4-(2,3-dichloro-6-hydroxyphenyl)pyrrolidin-2-one (21.0 mg, 34.6%).

¹H NMR (400 MHz, CD₃OD): *δ* 7.27 (d, 1H), 6.80 (d, 1H), 4.60-4.36 (m, 2H), 3.88-3.78 (m, 1H), 3.76-3.65 (m, 2H), 2.93-2.70 (m, 2H), 2.60-2.59 (m, 1H), 2.45-2.34 (m, 4H), 2.30-2.17 (m, 3H).

MS m/z (ESI): 355.1 [M+H]⁺.

### Example 24

### Synthesis of compound 103

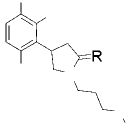

### 4-(2,3-Dichloro-6-hydroxyphenyl)-1-(3-(hydroxyamino)propyl)pyrrolidin-2-one

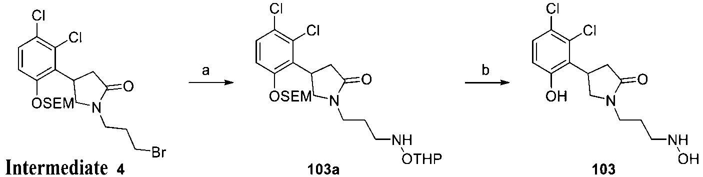

### Step a:

### 4-(2,3-Dichloro-6-((2-(trimethylsilyl)ethoxy)methoxy)phenyl)-1-(3-(((tetrahydro-2H-pyran-2-yl)oxy)amino)propyl)pyrrolidin-2-one

To a stirred solution of intermediate 4 (200.0 mg, 0.40 mmol) in N,N-dimethylformamide (10 mL) were added O-(oxan-2-yl)hydroxylamine (141.0 mg, 1.20 mmol) and K₂CO₃ (167.0 mg, 1.21 mmol) at 20°C, and the reaction solution was stirred at 65°C for 2 h. The reaction solution was concentrated, and the residue was purified by flash silica gel column chromatography (dichloromethane : methanol = 9:1) to afford compound **103a:** 4-(2,3-dichloro-6-((2-(trimethylsilyl)ethoxy)methoxy)phenyl)-1-(3-(((tetrahydro-2H-pyran-2-yl)oxy)amino)propyl)pyrrolidin-2-one (210.0 mg, 97.9%).

MS m/z (ESI): 533.0 [M+H]⁺.

### Step c:

### 4-(2,3-Dichloro-6-hydroxyphenyl)-1-(3-(hydroxyamino)propyl)pyrrolidin-2-one

To a stirred solution of 4-(2,3-dichloro-6-((2-(trimethylsilyl)ethoxy) methoxy)phenyl)-1-(3-(((tetrahydro-2H-pyran-2-yl)oxy)amino)propyl)pyrrolidin-2-one (210.0 mg, 0.39 mmol) in dichloromethane (20 mL) was added hydrogen chloride dioxane solution (2 mL, 4 N) at 15°C, and the reaction solution was stirred at 15°C for 1 h. The resulting mixture was washed with saturated sodium bicarbonate aqueous solution and extracted with dichloromethane (30 mL x 3). The combined organic layers were washed with sodium chloride aqueous solution and dried over anhydrous sodium sulphate. After filtration, the filtrate was concentrated under reduced pressure. The residue was purified by flash silica gel column chromatography (dichloromethane : methanol = 9:1) to afford compound 103: 4-(2,3-dichloro-6-hydroxyphenyl)-1-(3-(hydroxyamino)propyl)pyrrolidin-2-one (46.5 mg, 37.0%).

¹H NMR (400 MHz, DMSO_*d6*): *δ* 10.39 (s, 1H), 8.53 (s, 1H), 7.33 (d, 1H), 6.83 (d, 1H), 4.60 (brs, 1H), 4.15-4.06 (m, 1H), 4.50-3.36 (m, 4H), 3.23-3.09 (m, 2H), 2.98-2.89 (m, 1H), 2.84-2.73 (m, 1H), 1.72-1.59 (m, 2H).

MS m/z (ESI): 319.0 [M+H]⁺.

### Example 25

### Synthesis of compound 104

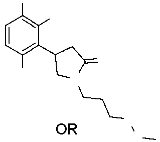

### 4-(2,3-Dichloro-6-hydroxyphenyl)-1-(3-(methoxyamino)propyl)pyrrolidin-2-one

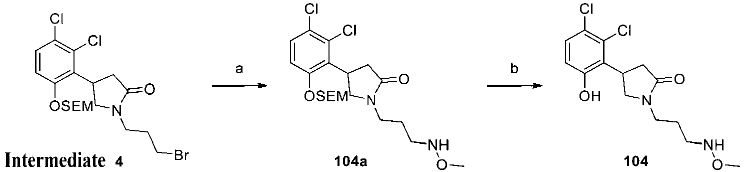

### Step a:

### 4-(2,3-Dichloro-6-((2-(trimethylsilyl)ethoxy)methoxy)phenyl)-1-(3-(methoxyamino)propyl)pyrrolidin-2-one

To a stirred solution of intermediate 4 (300.0 mg, 0.60 mmol) in *N,N-*dimethylformamide (6 mL) were added O-methylhydroxylamine hydrochloride (503.8 mg, 6.03 mmol), tetrabutylammonium iodide (267.4 mg, 0.72 mmol) and N,N-diisopropylethylamine (1169.4 mg, 9.05 mmol) at 20°C, and the reaction solution was stirred at 100°C for 1 h. After the reaction was completed, the resulting mixture was diluted with water and extracted with ethyl acetate (50 mL x 3). The combined organic layers were washed with sodium chloride aqueous solution and dried over anhydrous sodium sulphate. After filtration, the filtrate was concentrated under reduced pressure, and the residue was purified by flash silica gel column chromatography (petroleum ether : tetrahydrofuran = 2:1) to afford compound **104a:** 4-(2,3-dichloro-6-((2-(trimethylsilyl)ethoxy)methoxy)phenyl)-1-(3-(methoxyamino)
propyl)pyrrolidin-2-one (100.0 mg, 35.8%).

MS m/z (ESI): 463.2 [M+H]⁺.

### Step b:

### 4-(2,3-Dichloro-6-hydroxyphenyl)-1-(3-(methoxyamino)propyl)pyrrolidin-2-one

To a stirred solution of 4-(2,3-dichloro-6-((2-(trimethylsilyl)ethoxy)methoxy) phenyl)-1-(3-(methoxyamino)propyl)pyrrolidin-2-one (100.0 mg, 0.22 mmol) in dichloromethane (2 mL) was added hydrogen chloride dioxane solution (0.5 mL, 4 N) at 0°C, and the mixture was stirred at 25°C for 2 h. The reaction solution was concentrated under reduced pressure, and the residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **104:** 4-(2,3-dichloro-6-hydroxyphenyl)-1-(3-(methoxyamino)propyl)pyrrolidin-2-one (9.3 mg, 12.9%).
¹H NMR (400 MHz, DMSO*_d₆*) *δ* 10.40 (s, 1H), 7.33 (d, 1H), 6.82 (d, 1H), 4.50 (t, 1H), 4.13-4.06 (m, 1H), 3.52 (s, 3H), 3.48-3.39 (m, 4H), 3.23-3.08 (m, 2H), 2.96-2.90 (m, 1H), 2.84-2.78 (m, 1H), 1.71-1.62 (m, 2H);
MS m/z (ESI): 333.0 [M+H]⁺.

### Example 26

### Synthesis of compound 105

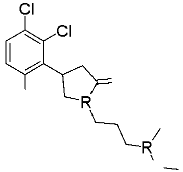

### 4-(2,3-Dichloro-6-hydroxyphenyl)-1-(3-(methoxy(methyl)amino)propyl) pyrrolidin-2-one

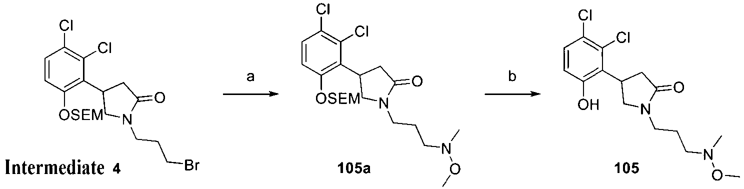

### Step a:

### 4-(2,3-Dichloro-6-((2-(trimethylsilyl)ethoxy)methoxy)phenyl)-1-(3-(methoxy(methyl)amino)propyl)pyrrolidin-2-one

To a stirred solution of intermediate 4 (300.0 mg, 0.60 mmol) in *N,N-*dimethylformamide (5 mL) were added dimethylhydroxylamine hydrochloride (294.2 mg, 3.01 mmol), tetrabutylammonium iodide (267.4 mg, 0.72 mmol) and N,N-diisopropylethylamine (623.7 mg, 4.83 mmol) at 20°C, and the reaction solution was stirred at 100°C for 1 h. After the reaction was completed, the resulting mixture was diluted with water and extracted with ethyl acetate (50 mL x 3). The combined organic layers were washed with sodium chloride aqueous solution and dried over anhydrous sodium sulphate. After filtration, the filtrate was concentrated under reduced pressure, and the residue was purified by flash silica gel column chromatography (petroleum ether : tetrahydrofuran = 3:1) to afford compound **105a:** 4-(2,3-dichloro-6-((2-(trimethylsilyl)ethoxy)methoxy)phenyl)-1-(3-(methoxyamino)propyl)pyrrolidin-2-one (80.0 mg, 27.8%).

MS m/z(ESI): 477.1 [M+H]⁺.

### Step b:

### 4-(2,3-Dichloro-6-hydroxyphenyl)-1-(3-(methoxy(methyl)amino)propyl) pyrrolidin-2-one

To a stirred solution of 4-(2,3-dichloro-6-((2-(trimethylsilyl)ethoxy)methoxy) phenyl)-1-(3-(methoxyamino)propyl)pyrrolidin-2-one (80.0 mg, 0.17 mmol) in dichloromethane (2.5 mL) was added hydrogen chloride dioxane solution (0.5 mL, 4 N) at 25°C, and the mixture was stirred at 25°C for 1 h. The reaction solution was concentrated under reduced pressure, and the residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **105:** 4-(2,3-dichloro-6-hydroxyphenyl)-1-(3-(methoxy(methyl)amino)propyl)pyrrolidin-2-one (7.3 mg, 12.5%).
¹H NMR (400 MHz, DMSO*_d₆*) *δ* 9.99 (brs, 1H), 7.33 (d, 1H), 6.83 (d, 1H), 4.25-4.16 (m, 1H), 3.59-3.50 (m, 2H), 3.41 (s, 3H), 3.38-3.31 (m, 2H), 3.25-3.18 (m, 1H), 2.66-2.60 (m, 1H), 2.57-2.52 (m, 2H), 2.46 (s, 3H), 1.71-1.64 (m, 2H);
MS m/z (ESI): 347.0 [M+H]⁺.

### Example 27

### Synthesis of compound 113

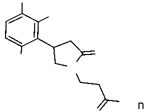

### 3-(4-(2,3-Dichloro-6-hydroxyphenyl)-2-oxopyrrolidin-1-yl)propanethioamide

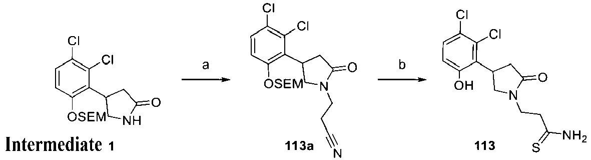

### Step a:

### 3-(4-(2,3-Dichloro-6-(2-(trimethylsilyl)ethoxy)methoxy)phenyl)-2-oxopyrrolidin-1-yl)propanenitrile

To a stirred solution of intermediate **1** (580.0 mg, 1.54 mmol) in tetrahydrofuran (7 mL) were added acrylonitrile (0.1 mL, 1.54 mmol) and sodium hydroxide (184.9 mg, 4.62 mmol) at 20°C, and the reaction solution was stirred at 20°C for 10 h. The resulting mixture was diluted with water (100 mL) and extracted with ethyl acetate (100 mL x 3). The combined organic layers were washed with sodium chloride aqueous solution and dried over anhydrous sodium sulphate. After filtration, the filtrate was concentrated under reduced pressure. The residue was purified by flash silica gel column chromatography (petroleum ether : ethyl acetate = 1:1) to afford compound **113a:** 3-(4-(2,3-dichloro-6-(2-(trimethylsilyl)ethoxy)methoxy)phenyl)-2-oxopyrrolidin-1-yl)propanenitrile (497.0 mg, 75.1%).

¹H NMR (400 MHz, CDCl₃) *δ* 7.32 (d, 1H), 7.09 (d, 1H), 5.27-5.22 (m, 2H), 4.51-4.42 (m, H), 3.80-3.69 (m, 4H), 3.69-3.57 (m, 2H), 2.81 (dd, 1H), 2.75-2.63 (m, 3H), 0.98-0.89 (m, 2H), 0.00 (s, 9H).

### Step b:

### 3-(4-(2,3-Dichloro-6-hydroxyphenyl)-2-oxopyrrolidin-1-yl)propanethioamide

To a stirred solution of **113a** (100.0 mg, 0.23 mmol) in ethyl acetate (1 mL) were added hydrochloric acid (0.1 mL) and diethyl dithiophosphate (9.31 mg, 0.05 mmol) at 0°C, and the reaction solution was stirred at 20°C for 18 h. The reaction solution was concentrated under reduced pressure. The residue was purified by flash reverse-phase C18 column chromatography (water : acetonitrile = 6:4, with 10 mM sodium bicarbonate) to afford compound **113:** 3-(4-(2,3-dichloro-6-hydroxyphenyl)-2-oxopyrrolidin-1-yl)propanethioamide (20.0 mg, 25.8%).

¹H NMR (400 MHz, CD₃OD) *δ* 7.27 (d, 1H), 6.78 (dd, 1H), 4.52-4.34 (m, 1H), 4.19-3.95 (m, 2H), 3.79-3.71 (m, 2H), 3.28 (d, 1H), 2.92-2.59 (m, 3H).

MS m/z (ESI): 333.1 [M+H]⁺.

### Example 28

### Synthesis of compound 114

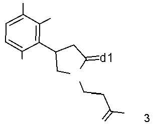

### 3-(4-(2,3-Dichloro-6-hydroxyphenyl)-2-oxopyrrolidin-1-yl)propionimide

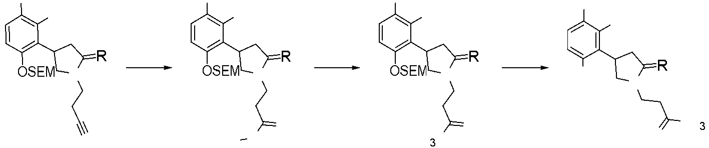

### Step a:

### 3-(4-(2,3-Dichloro-6-((2-(trimethylsilyl)ethoxy)methoxy)phenyl)-2-oxopyrrolidin-1-yl)-N-hydroxypropimidamide

To a stirred solution of 3-(4-(2,3-dichloro-6-(2-(trimethylsilyl)ethoxy)methoxy)phenyl)-2-oxopyrrolidin-1-yl)propanenitrile (500.0 mg, 1.16 mmol) in ethanol (10 mL) were added hydroxylamine hydrochloride (242.7 mg, 3.49 mmol) and potassium acetate (171.4 mg, 1.75 mmol) at 15°C, and the reaction solution was stirred at 80°C for 18 h. The resulting mixture was diluted with water (100 mL) and extracted with ethyl acetate (100 mL x 3). The combined organic layers were washed with sodium chloride aqueous solution and dried over anhydrous sodium sulphate. After filtration, the filtrate was concentrated under reduced pressure. The residue was subjected to flash silica gel column chromatography (petroleum ether : ethyl acetate = 1:1) to afford compound **114a:** 3-(4-(2,3-dichloro-6-((2-(trimethylsilyl)ethoxy)methoxy)phenyl)-2-oxopyrrolidin-1-yl)-*N*-hydroxypropimidamide (300.0 mg, 55.7%).

MS m/z (ESI): 462.1 [M+H]⁺.

### Step b:

### 3-(4-(2,3-Dichloro-6-(2-(trimethylsilyl)ethoxy)methoxy)phenyl)-2-oxopyrrolidin-1-yl)propionimide

To a stirred solution of 3-(4-(2,3-dichloro-6-((2-(trimethylsilyl)ethoxy) methoxy)phenyl)-2-oxopyrrolidin-1-yl)-*N*-hydroxypropimidamide (20.0 mg, 0.04 mmol) in ethanol/water (2 mL, v/v = 1:1) was added iron powder (50.0 mg, 0.43 mmol) at 20°C, and hydrochloric acid (15 µL, 0.04 mmol) was added dropwise to the reaction solution at 105°C. The reaction solution was stirred at 105°C for 1 h and concentrated under reduced pressure, and the residue was purified by flash reverse-phase C18 column chromatography (water : acetonitrile = 1:1) to afford compound **114b:** 3-(4-(2,3-dichloro-6-(2-(trimethylsilyl)ethoxy)methoxy)phenyl)-2-oxopyrrolidin-1-yl)propionimide (5.0 mg, 25.9%).

MS m/z (ESI): 446.2 [M+H]⁺.

### Step c:

### 3-(4-(2,3-Dichloro-6-hydroxyphenyl)-2-oxopyrrolidin-1-yl)propionimide

To a stirred solution of 3-(4-(2,3-dichloro-6-(2-(trimethylsilyl)ethoxy)methoxy)phenyl)-2-oxopyrrolidin-1-yl)propionimide (50.0 mg, 0.11 mmol) in dichloromethane (5 mL) was added hydrochloric acid (1.5 mL, 6.00 mmol) at 20°C, and the reaction solution was stirred at 20°C for 1 h. The reaction solution was dissolved in saturated sodium bicarbonate and adjusted to pH = 7, and purified by flash reverse-phase C18 column chromatography (water : acetonitrile = 6:4) to afford compound **114:** 3-(4-(2,3-dichloro-6-hydroxyphenyl)-2-oxopyrrolidin-1-yl)propionimide (9.0 mg, 25.4%).
¹H NMR (400 MHz, CD₃OD) *δ* 7.18 (d, 1H), 6.69 (d, 1H), 4.43 (d, 1H), 3.91-3.51 (m, 5H), 2.91 (dd, 1H), 2.77-2.68 (m, 2H);
MS m/z (ESI): 316.0 [M+H]⁺.

### Example 29

### Synthesis of compound 115

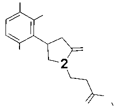

### 3-(4-(2,3-Dichloro-6-hydroxyphenyl)-2-oxopyrrolidin-1-yl)-N-hydroxypropionamide

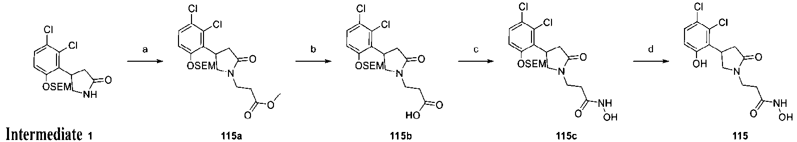

### Step a:

### Methyl 3-(4-(2,3-dichloro-6-((2-(trimethylsilyl)ethoxy)methoxy)phenyl)-2-oxopyrrolidin-1-yl)propanoate

To a stirred solution of intermediate **1** (400.0 mg, 1.06 mmol) in toluene (10 mL) were added tetrabutylammonium bromide (68.5 mg, 0.21 mmol), potassium hydroxide (71.6 mg, 1.28 mmol) and methyl acrylate (137.2 mg, 1.59 mmol) at 20°C, and the reaction solution was stirred at 20°C for 3 h. The resulting mixture was diluted with water and extracted with ethyl acetate (100 mL x 3). The combined organic layers were washed with sodium chloride aqueous solution and dried over anhydrous sodium sulphate. After filtration, the filtrate was concentrated under reduced pressure. The residue was purified by flash silica gel column chromatography (petroleum ether : tetrahydrofuran = 7:3) to afford compound **115a:** methyl 3-(4-(2,3-dichloro-6-((2-(trimethylsilyl)ethoxy)methoxy)phenyl)-2-oxopyrrolidin-1-yl)propanoate (370.0 mg, 75.3%).

MS m/z (ESI): 462.1 [M+H]⁺.

### Step b:

### 3-(4-(2,3-Dichloro-6-((2-(trimethylsilyl)ethoxy)methoxy)phenyl)-2-oxopyrrolidin-1-yl)propanoic acid

To a stirred solution of methyl 3-(4-(2,3-dichloro-6-((2-(trimethylsilyl)ethoxy)methoxy)phenyl)-2-oxopyrrolidin-1-yl)propanoate (400.0 mg, 0.87 mmol) in methanol/water (6 mL/1.5 mL) was added lithium hydroxide monohydrate (145.2 mg, 3.46 mmol) at 15°C, and the reaction solution was stirred at 15°C for 2 h. The reaction solution was adjusted to pH 3 with 1N hydrochloric acid, and extracted with ethyl acetate (50 mL x 3). The combined organic layers were washed with sodium chloride aqueous solution and dried over anhydrous sodium sulphate. After filtration, the filtrate was concentrated under reduced pressure to afford compound **115b:** 3-(4-(2,3-dichloro-6-((2-(trimethylsilyl)ethoxy)methoxy)phenyl)-2-oxopyrrolidin-1-yl)propanoic acid (351.0 mg, 90.5%).

MS m/z (ESI): 448.2 [M+H]⁺.

### Step c:

### 3-(4-(2,3-Dichloro-6-((2-(trimethylsilyl)ethoxy)methoxy)phenyl)-2-oxopyrrolidin-1-yl)-N-hydroxypropionamide

To a stirred solution of 3-(4-(2,3-dichloro-6-((2-(trimethylsilyl)ethoxy)methoxy)phenyl)-2-oxopyrrolidin-1-yl)propanoic acid (150.0 mg, 0.33 mmol) in tetrahydrofuran (6 mL) was added *N,N-*carbonyldiimidazole (65.1 mg, 0.40 mmol) at 20°C, and the reaction solution was stirred at 40°C for 3 h. Hydroxylamine hydrochloride (46.5 mg, 0.67 mmol) was added, and the mixture was stirred at 40°C for another 16 h. The resulting mixture was extracted with ethyl acetate (50 mL x 3). The combined organic layers were washed with sodium chloride aqueous solution and dried over anhydrous sodium sulphate. After filtration, the reaction solution was concentrated under reduced pressure, and the residue was purified by flash silica gel column chromatography (dichloromethane : methanol = 93:7) to afford compound **115c:** 3-(4-(2,3-dichloro-*6-((2-(trimethylsilyl)ethoxy)methoxy)phenyl)-2-oxopyrrolidin-1-yl)-N-*hydroxypropionamide (130.0 mg, 83.9%).

MS m/z (ESI): 463.0 [M+H]⁺.

### Step d:

### 3-(4-(2,3-Dichloro-6-hydroxyphenyl)-2-oxopyrrolidin-1-yl)-N-hydroxypropionamide

To a stirred solution of 3-(4-(2,3-dichloro-6-((2-(trimethylsilyl)ethoxy)methoxy)phenyl)-2-oxopyrrolidin-1-yl)-N-hydroxypropionamide (130.0 mg, 0.28 mmol) in dichloromethane (10 mL) was added hydrogen chloride dioxane solution (1 mL, 4 N) at 15°C, and the reaction solution was stirred at 15°C for 1 h. The reaction solution was concentrated under reduced pressure, and the residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **115:** 3-(4-(2,3-dichloro-6-hydroxyphenyl)-2-oxopyrrolidin-1-yl)-*N*-hydroxypropionamide (75.0 mg, 80.2%).
¹H NMR (400 MHz, DMSO*_d₆*) *δ* 9.53 (brs, 3H), 7.32 (d, 1H), 6.82 (d, 1H), 4.21-4.14 (m, 1H), 3.60-3.34 (m, 4H), 2.66-2.60 (m, 1H), 2.50-2.47 (m, 1H), 2.20 (t, 2H);
MS m/z(ESI): 332.9 [M+H]⁺.

### Example 30

### Synthesis of compound 116

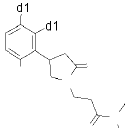

### 3-(4-(2,3-Dichloro-6-hydroxyphenyl)-2-oxopyrrolidin-1-yl)-N-hydroxy-N-methylpropionamide

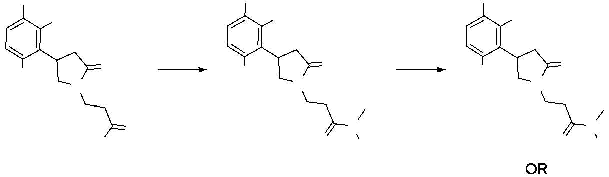

### Step a:

### 3-(4-(2,3-Dichloro-6-((2-(trimethylsilyl)ethoxy)methoxy)phenyl)-2-oxopyrrolidin-1-yl)-N-hydroxy-N-methylpropionamide

To a stirred solution of **115b** (180.0 mg, 0.40 mmol) in tetrahydrofuran (4 mL) was added *N,N'*-carbonyldiimidazole (78.1 mg, 0.48 mmol) at 20°C, and the reaction solution was stirred at 40°C for 3 h. *N*-Methylhydroxylamine hydrochloride (67.1 mg, 0.80 mmol) was added, and the mixture was stirred at 40°C for another 16 h. The resulting mixture was extracted with ethyl acetate (50 mL x 3). The combined organic layers were washed with sodium chloride aqueous solution and dried over anhydrous sodium sulphate. After filtration, the reaction solution was concentrated under reduced pressure, and the residue was purified by flash silica gel column chromatography (dichloromethane : methanol = 93:7) to afford compound **116a:** 3-(4-(2,3-dichloro-6-((2-(trimethylsilyl)ethoxy)methoxy)phenyl)-2-oxopyrrolidin-1-yl)-N-hydroxy-*N-*methylpropionamide (170.0 mg, 88.7%).

MS m/z (ESI): 476.9 [M+H]⁺.

### Step b:

### 3-(4-(2,3-Dichloro-6-hydroxyphenyl)-2-oxopyrrolidin-1-yl)-N-hydroxy-N-methylpropionamide

To a stirred solution of 3-(4-(2,3-dichloro-6-((2-(trimethylsilyl)ethoxy) methoxy)phenyl)-2-oxopyrrolidin-1-yl)-*N*-hydroxy-*N*-methylpropionamide (170.0 mg, 0.36 mmol) in tetrahydrofuran (2 mL) was added tetrabutylammonium fluoride (931.1 mg, 3.56 mmol) at 15°C, and the reaction solution was stirred at 70°C for 1 h and then at 15°C for 2 h. The resulting mixture was extracted with ethyl acetate (100 mL x 3). The combined organic layers were washed with sodium chloride aqueous solution and dried over anhydrous sodium sulphate. After filtration, the reaction solution was concentrated under reduced pressure, and the residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **116:** 3-(4-(2,3-dichloro-6-hydroxyphenyl)-2-oxopyrrolidin-1-yl)-N-hydroxy-*N-*methylpropionamide (30.0 mg, 24.3%).
¹H NMR (400 MHz, DMSO*_d₆*) *δ* 9.86 (brs, 2H), 7.32 (d, 1H), 6.81 (d, 1H), 4.22-4.13 (m, 1H), 3.58 (t, 1H), 3.52-3.48 (m, 1H), 3.44-3.40 (m, 2H), 3.08 (s, 3H), 2.63-2.56 (m, 3H), 2.50-2.47 (m, 1H);
MS m/z (ESI): 347.0 [M+H]⁺.

### Example 31

### Synthesis of compound 117

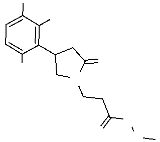

### 3-(4-(2,3-Dichloro-6-hydroxyphenyl)-2-oxopyrrolidin-1-yl)-N-methoxypropionamide

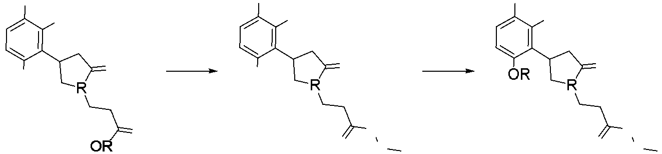

### Step a:

### 3-(4-(2,3-Dichloro-6-((2-(trimethylsilyl)ethoxy)methoxy)phenyl)-2-oxopyrrolidin-1-yl)-N-methoxypropionamide

To a stirred solution of **115b** (140.0 mg, 0.31 mmol) in tetrahydrofuran (4 mL) was added *N,N'*-carbonyldiimidazole (60.7 mg, 0.37 mmol) at 20°C, and the reaction solution was stirred at 40°C for 3 h. Methoxyamine hydrochloride (52.2 mg, 0.62 mmol) was added, and the mixture was stirred at 40°C for another 16 h. The resulting mixture was extracted with ethyl acetate (50 mL x 3). The combined organic layers were washed with sodium chloride aqueous solution and dried over anhydrous sodium sulphate. After filtration, the reaction solution was concentrated under reduced pressure, and the residue was purified by flash silica gel column chromatography (petroleum ether : tetrahydrofuran = 1:1) to afford compound **117a:** 3-(4-(2,3-dichloro-6-((2-(trimethylsilyl)ethoxy)methoxy)phenyl)-2-oxopyrrolidin-1-yl)-N-methoxypropionamide (140.0 mg, 93.9%).

MS m/z (ESI): 477.0 [M+H]⁺.

### Step b:

### 3-(4-(2,3-Dichloro-6-hydroxyphenyl)-2-oxopyrrolidin-1-yl)-N-methoxypropionamide

To a stirred solution of 3-(4-(2,3-dichloro-6-((2-(trimethylsilyl)ethoxy) methoxy)phenyl)-2-oxopyrrolidin-1-yl)-*N*-methoxypropionamide (140.0 mg, 0.29 mmol) in dichloromethane (10 mL) was added hydrogen chloride dioxane solution (1 mL, 4 N) at 15°C, and the reaction solution was stirred at 15°C for 0.5 h. The reaction solution was concentrated under reduced pressure, and the residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **117:** 3-(4-(2,3-dichloro-6-hydroxyphenyl)-2-oxopyrrolidin-1-yl)-*N*-methoxypropionamide (45.0 mg, 44.2%).
¹H NMR (400 MHz, DMSO_*d*₆) *δ* 11.07 (brs, 1H), 10.36 (brs, 1H), 7.33 (d, 1H), 6.83 (d, 1H), 4.23-4.14 (m, 1H), 3.57 (s, 3H), 3.57-3.38 (m, 4H), 2.66-2.60 (m, 1H), 2.50-2.44 (m, 1H), 2.19 (t, 2H);
MS m/z (ESI): 347.0 [M+H]⁺.

### Example 32

### Synthesis of compound 118

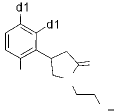

### 4-(2,3-Dichloro-6-hydroxyphenyl)-1-(2-(hydroxyamino)ethyl)pyrrolidin-2-one

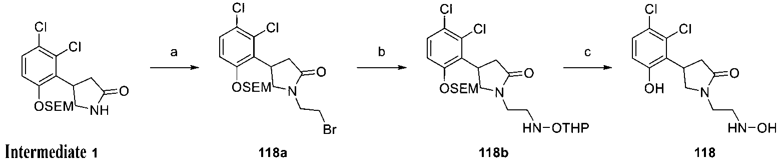

### Step a:

### 1-(2-Bromoethyl)-4-(2,3-dichloro-6-(2-(trimethylsilyl)ethoxy)methoxy) phenyl)pyrrolidin-2-one

To a stirred solution of intermediate 1 (600.0 mg, 1.59 mmol) in toluene (10 mL) were added potassium hydroxide (178.9 mg, 3.19 mmol), tetrabutylammonium bromide (514.0 mg, 1.59 mmol) and 1,2-dibromoethane (2995.0 mg, 15.94 mmol) at 20°C, and the reaction solution was stirred at 40°C for 18 h. The resulting mixture was diluted with water (100 mL) and extracted with ethyl acetate (100 mL x 3). The combined organic layers were washed with sodium chloride aqueous solution and dried over anhydrous sodium sulphate. After filtration, the filtrate was concentrated under reduced pressure. The residue was purified by flash silica gel column chromatography (petroleum ether : tetrahydrofuran = 7:3) to afford compound **118a:** 1-(2-bromoethyl)-4-(2,3-dichloro-6-(2-(trimethylsilyl)ethoxy)methoxy)phenyl) pyrrolidin-2-one (375.0 mg, 48.7%).
¹H NMR (400 MHz, CDCl₃): δ 7.32 (d, 1H), 7.09 (d, 1H), 5.26-5.22 (m, 2H), 4.47-4.40 (m, 1H), 3.82-3.67 (m, 6H), 3.55-3.52 (m, 2H), 2.84-2.78 (m, 1H), 2.72-2.65 (m, 1H), 0.94 (t, 2H), 0.01 (s, 9H);
MS m/z (ESI): 481.8 [M+H]⁺.

### Step b:

### 4-(2,3-Dichloro-6-((2-(trimethylsilyl)ethoxy)methoxy)phenyl)-1-(2-(((tetrahydro-2H-pyran-2-yl)oxy)amino)ethyl)pyrrolidin-2-one

To a stirred solution of 1-(2-bromoethyl)-4-(2,3-dichloro-6-(2-(trimethylsilyl)ethoxy)methoxy)phenyl)pyrrolidin-2-one (175.0 mg, 0.36 mmol) in N,N-dimethylformamide (5 mL) were added O-(oxan-2-yl)hydroxylamine (127.3 mg, 1.08 mmol) and K₂CO₃ (150.1 mg, 1.09 mmol) at 20°C, and the reaction solution was stirred at 65°C for 2 h. The reaction solution was concentrated, and the residue was purified by flash silica gel column chromatography (dichloromethane : methanol = 15:1) to afford compound **118b:** 4-(2,3-dichloro-6-((2-(trimethylsilyl)ethoxy)methoxy)phenyl)-1-(2-(((tetrahydro-2H-pyran-2-yl)oxy)amino)ethyl)pyrrolidin-2-one (180.0 mg, 95.7%).

MS m/z (ESI): 519.0 [M+H]⁺.

### Step c:

### 4-(2,3-Dichloro-6-hydroxyphenyl)-1-(2-(hydroxyamino)ethyl)pyrrolidin-2-one

To a stirred solution of 4-(2,3-dichloro-6-((2-(trimethylsilyl)ethoxy)methoxy) phenyl)-1-(2-(((tetrahydro-2H-pyran-2-yl)oxy)amino)ethyl)pyrrolidin-2-one (320.0 mg, 0.62 mmol) in dichloromethane (10 mL) was added hydrogen chloride dioxane solution (2 mL, 4 N) at 15°C, and the reaction solution was stirred at 15°C for 2 h. The reaction solution was concentrated under reduced pressure. The concentrate was purified by high performance liquid chromatography (Welchrom C18, 20*150 mm, 5 µm; mobile phase A: formic acid in water, mobile phase B: acetonitrile, gradient ratio: phase B 5%-95%), adjusted to pH = 7 with saturated sodium bicarbonate solution, extracted, concentrated under reduced pressure, and purified to afford compound **118:** 4-(2,3-dichloro-6-hydroxyphenyl)-1-(2-(hydroxyamino)ethyl) pyrrolidin-2-one (80.0 mg, 42.6%).

¹H NMR (400 MHz, DMSO_*d*₆) *δ* 10.35 (s, 1H), 8.43 (s, 1H), 7.33 (d, 1H), 6.82 (d, 1H), 4.66 (s, 1H), 4.13-4.04 (m, 1H), 3.54-3.42 (m, 4H), 3.29-3.24 (m, 1H), 3.06-2.91 (m, 2H), 2.78-2.72 (m, 1H).

MS m/z (ESI): 304.9 [M+H]⁺.

### Example 33

### Synthesis of compound 119

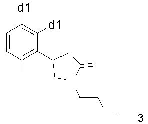

### 1-(2-(Aminooxy)ethyl)-4-(2,3-dichloro-6-hydroxyphenyl)pyrrolidin-2-one

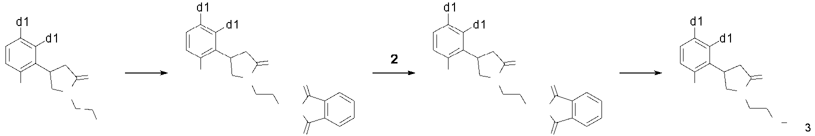

### Step a:

### 2-(2-(4-(2,3-Dichloro-6-(2-(trimethylsilyl)ethoxy)methoxy)phenyl)-2-oxopyrrolidin-1-yl)ethoxyisoindoline-1,3-dione

To a stirred solution of 1-(2-bromoethyl)-4-(2,3-dichloro-6-(2-(trimethylsilyl)ethoxy)methoxy)phenyl)pyrrolidin-2-one (200.0 mg, 0.41 mmol) in N,N-dimethylformamide (5 mL) were added 2-hydroxyisoindoline-1,3-dione (81.0 mg, 0.50 mmol) and potassium carbonate (171.6 mg, 1.24 mmol) at 20°C, and the reaction solution was stirred at 80°C for 0.5 h. The reaction solution was extracted with ethyl acetate (50 mL x 3), and the organic phase was concentrated under reduced pressure. The residue was purified by flash silica gel column chromatography (dichloromethane : methanol = 15:1) to afford compound **119a:** 2-(2-(4-(2,3-dichloro-6-(2-(trimethylsilyl)ethoxy)methoxy)phenyl)-2-oxopyrrolidin-1-yl)ethoxyisoindoline-1,3-dione (200.0 mg, 66.6%).

MS m/z (ESI): 565.1 [M+H]⁺.

### Step b:

### 2-(2-(4-(2,3-Dichloro-6-hydroxyphenyl)-2-oxopyrrolidin-1-yl)ethoxy)isoindoline-1,3-dione

To a stirred solution of 2-(2-(4-(2,3-dichloro-6-(2-(trimethylsilyl)ethoxy)methoxy)phenyl)-2-oxopyrrolidin-1-yl)ethoxyisoindoline-1,3-dione (200.0 mg, 0.35 mmol) in dichloromethane (10 mL) was added hydrogen chloride dioxane solution (1 mL, 4 N) at 15°C, and the reaction solution was stirred at 15°C for 1 h. The reaction solution was concentrated under reduced pressure, and the residue was purified by flash silica gel column chromatography (dichloromethane : methanol = 15:1) to afford compound **119b:** 2-(2-(4-(2,3-dichloro-6-hydroxyphenyl)-2-oxopyrrolidin-1-yl)ethoxy)isoindoline-1,3-dione (150.0 mg, 97.4%).

MS m/z (ESI): 435.0 [M+H]⁺.

### Step c:

### 1-(2-(Aminooxy)ethyl)-4-(2,3-dichloro-6-hydroxyphenyl)pyrrolidin-2-one

To a stirred solution of 2-(2-(4-(2,3-dichloro-6-hydroxyphenyl)-2-oxopyrrolidin-1-yl)ethoxy)isoindoline-1,3-dione (90.0 mg, 0.21 mmol) in methanol (2 mL) was added hydrazine hydrate (20.7 mg, 0.41 mmol) at 15°C, and the reaction solution was stirred at 15°C for 0.5 h. The reaction solution was extracted with dichloromethane (50 mL x 3), and the organic phase was concentrated under reduced pressure. The residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **119:** 1-(2-(aminooxy)ethyl)-4-(2,3-dichloro-6-hydroxyphenyl) pyrrolidin-2-one (25.0 mg, 39.6%).

¹H NMR (400 MHz, DMSO_*d*₆): δ 7.31 (d, 1H), 6.81 (d, 1H), 6.01 (brs, 2H), 4.23-4.14 (m, 1H), 3.64-3.56 (m, 4H), 3.48-3.30 (m, 2H), 2.72-2.65 (m, 1H), 2.50-2.45 (m, 1H).

MS m/z (ESI): 304.9 [M+H]⁺.

### Example 34

### Synthesis of compound 120

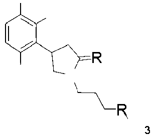

### 1-(3-(Aminooxy)propyl)-4-(2,3-dichloro-6-hydroxyphenyl)pyrrolidin-2-one

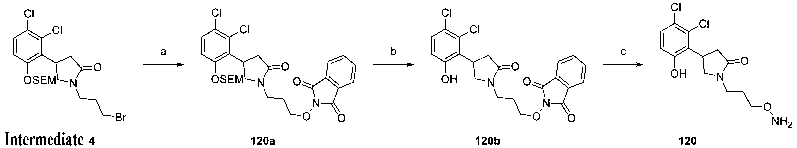

### Step a:

### 2-(3-(4-(2,3-Dichloro-6-(2-(trimethylsilyl)ethoxy)methoxy)phenyl)-2-oxopyrrolidin-1-yl)propoxy)isoindoline-1,3-dione

To a stirred solution of intermediate 4 (230.0 mg, 0.46 mmol) in *N,N-*dimethylformamide (10 mL) were added 2-hydroxyisoindoline-1,3-dione (91.0 mg, 0.56 mmol) and potassium carbonate (192.0 mg, 1.39 mmol) at 20°C, and the reaction solution was stirred at 80°C for 0.5 h. Water was added, and the reaction solution was extracted with ethyl acetate (100 mL x 3). The organic layers were combined, dried over anhydrous sodium sulphate, and filtered, and the filtrate was concentrated under reduced pressure. The residue was purified by flash silica gel column chromatography (petroleum ether : tetrahydrofuran = 85:15) to afford compound **120a:** 2-(3-(4-(2,3-dichloro-6-(2-(trimethylsilyl)ethoxy)methoxy)phenyl)-2-oxopyrrolidin-1-yl)propoxy)isoindoline-1,3-dione (200.0 mg, 74.6%).

¹H NMR (400 MHz, DMSO_*d6*): *δ* 7.90-7.88 (m, 4H), 7.53 (d, 1H), 7.16 (d, 1H), 7.34-7.27 (m, 2H), 4.36-4.30 (m, 1H), 4.21 (d, 2H), 3.75-3.60 (m, 4H), 3.54-3.43 (m, 2H), 2.67-2.48 (m, 2H), 1.97-1.90 (m, 2H), 0.88 (t, 2H), 0.00 (s, 9H).

MS m/z (ESI): 579.0 [M+H]⁺.

### Step b:

### 2-(3-(4-(2,3-Dichloro-6-hydroxyphenyl)-2-oxopyrrolidin-1-yl)propoxy)isoindoline-1,3-dione

To a stirred solution of 2-(3-(4-(2,3-dichloro-6-(2-(trimethylsilyl)ethoxy)methoxy)phenyl)-2-oxopyrrolidin-1-yl)propoxy)isoindoline-1,3-dione (180.0 mg, 0.31 mmol) in dichloromethane (20 mL) was added hydrogen chloride dioxane solution (2.5 mL, 4 N) at 15°C, and the reaction solution was stirred at 15°C for 3 h. The resulting mixture was diluted with dichloromethane and extracted with saturated sodium bicarbonate aqueous solution, and washed. The organic phases were combined, dried over anhydrous sodium sulphate, and filtered. The filtrate was concentrated under reduced pressure, and the residue was purified by flash silica gel column chromatography (petroleum ether : ethyl acetate = 4:1) to afford compound **120b:** 2-(3-(4-(2,3-dichloro-6-hydroxyphenyl)-2-oxopyrrolidin-1-yl)propoxy)isoindoline-1,3-dione (130.0 mg, 93.2%).

¹H NMR (400 MHz, DMSO_*d6*): δ 10.44 (s, 1H), 7.88-7.84 (m, 4H), 7.34 (d, 2H), 6.83 (d, 2H), 4.27-4.19 (m, 1H), 4.20-4.16 (m, 2H), 3.64 (t, 1H), 3.56-3.51 (m, 1H), 3.48-3.34 (m, 2H), 2.62-2.51 (m, 2H), 1.95-1.87 (m, 2H).

MS m/z (ESI): 449.0 [M+H]⁺.

### Step c:

### 1-(3-(Aminooxy)propyl)-4-(2,3-dichloro-6-hydroxyphenyl)pyrrolidin-2-one

To a stirred solution of 2-(3-(4-(2,3-dichloro-6-hydroxyphenyl)-2-oxopyrrolidin-1-yl)propoxy)isoindoline-1,3-dione (130.0 mg, 0.29 mmol) in methanol (5 mL) was added hydrazine hydrate (146.0 mg, 2.89 mmol) at 15°C, and the reaction solution was stirred at 15°C for 1 h. The reaction solution was extracted with dichloromethane and saturated sodium chloride aqueous solution, dried over anhydrous sodium sulphate, and filtered, and the filtrate was concentrated under reduced pressure. The residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **120:** 1-(3-(aminooxy)propyl)-4-(2,3-dichloro-6-hydroxyphenyl)pyrrolidin-2-one (36.9 mg, 40.0%).

¹H NMR (400 MHz, DMSO_*d6*): *δ* 7.32 (d, 1H), 6.82 (d, 1H), 5.92 (brs, 2H), 4.24-4.15 (m, 1H), 3.58-3.48 (m, 4H), 3.33-3.26 (m, 1H), 3.23-3.16 (m, 1H), 2.64 (dd, 1H), 2.49-2.45 (m, 1H), 1.74-1.66 (m, 2H).

MS m/z (ESI): 319.0 [M+H]⁺.

### Example 35

### Synthesis of compound 121 and compound 121-1

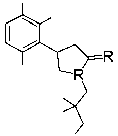

### 4-(2,3-Dichloro-6-hydroxyphenyl)-1-(2,2-difluoro-3-hydroxypropyl)pyrrolidine-2-thione

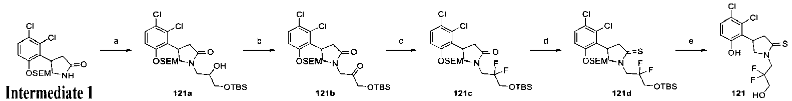

### Step a:

### 1-(3-((tert-Butyldimethylsilyl)oxy)-2-hydroxypropyl)-4-(2,3-dichloro-6-((2-(trimethylsilyl)ethoxy)

### methoxy)phenyl)pyrrolidin-2-one

To a stirred solution of intermediate 1 (100.0 mg, 0.27 mmol) in toluene (2 mL) were added tert-butyldimethylsilyl glycidyl ether (200.2 mg, 1.06 mmol), potassium hydroxide (59.6 mg, 1.06 mmol) and tetrabutylammonium iodide (19.6 mg, 0.05 mmol) at 20°C, and the reaction solution was stirred at 30°C for 16 h. After the reaction was completed, the reaction solution was concentrated under reduced pressure, and the residue was purified by flash silica gel column chromatography (petroleum ether : tetrahydrofuran = 2:1) to afford compound **121a:** 1-(3-((tert-butyldimethylsilyl)oxy)-2-hydroxypropyl)-4-(2,3-dichloro-6-((2-(trimethylsilyl)ethoxy)methoxy)phenyl)pyrrolidin-2-one (104.0 mg, 69.3%).
¹H NMR (400 MHz, CDCl₃) δ 7.24 (d, 1H), 7.03-7.00 (m, 1H), 5.17-5.10 (m, 2H), 4.40-4.32 (m, 1H), 3.99-3.86 (m, 1H), 3.83-3.72 (m, 1H), 3.69-3.56 (m, 4H), 3.52-3.51 (m, 1H), 3.47-3.27 (m, 2H), 2.74-2.60 (m, 2H), 0.88-0.81 (m, 2H), 0.82 (s, 9H), 0.00 (s, 6H), -0.07 (s, 9H);
MS m/z (ESI): 564.0 [M+H]⁺.

### Step b:

### 1-(3-((tert-Butyldimethylsilyl)oxy)-2-oxopropyl)-4-(2,3-dichloro-6-((2-(trimethylsilyl)ethoxy)methoxy)phenyl)pyrrolidin-2-one

To a stirred solution of 1-(3-((tert-butyldimethylsilyl)oxy)-2-hydroxypropyl)-4-(2,3-dichloro-6-((2-(trimethylsilyl)ethoxy)methoxy)phenyl)pyrrolidin-2-one (60.0 mg, 0.11 mmol) in acetonitrile (1 mL) was added 2-iodoxybenzoic acid (119.1 mg, 0.43 mmol) at 20°C. The reaction solution was stirred at 80°C for 2 h. The resulting mixture was diluted with water and extracted with ethyl acetate (50 mL x 3). The combined organic layers were washed with sodium chloride aqueous solution and dried over anhydrous sodium sulphate. After filtration, the filtrate was concentrated under reduced pressure, and the residue was purified by flash silica gel column chromatography (petroleum ether : tetrahydrofuran = 4:1) to afford compound **121b:** 1-(3-((tert-butyldimethylsilyl)oxy)-2-oxopropyl)-4-(2,3-dichloro-6-((2-(trimethylsilyl)ethoxy)methoxy)phenyl)pyrrolidin-2-one (25.0 mg, 41.8%).
¹H NMR (400 MHz, CDCl₃) δ 7.24 (d, 1H), 7.04 (d, 1H), 5.25-5.23 (m, 2H), 4.40-4.18 (m, 5H), 3.72-3.52 (m, 4H), 2.80-2.78 (m, 1H), 2.67-2.63 (m, 1H), 0.89-0.79 (m, 2H), 0.87 (s, 9H), 0.04 (s, 6H), -0.07 (s, 9H);
MS m/z (ESI): 562.1 [M+H]⁺.

### Step c:

### 1-(3-((tert-Butyldimethylsilyl)oxy)-2,2-difluoropropyl)-4-(2,3-dichloro-6-((2-(trimethylsilyl)ethoxy)methoxy)phenyl)pyrrolidin-2-one

To a stirred solution of 1-(3-((tert-butyldimethylsilyl)oxy)-2-oxopropyl)-4-(2,3-dichloro-6-((2-(trimethylsilyl)ethoxy)methoxy)phenyl)pyrrolidin-2-one (60.0 mg, 0.11 mmol) in dichloromethane (2 mL) was added diethylaminosulphur trifluoride (171.8 mg, 1.07 mmol) at 0°C under nitrogen protection. The reaction solution was stirred at 0°C for 3 h. The resulting mixture was diluted with water and extracted with ethyl acetate (50 mL x 3). The combined organic layers were washed with sodium chloride aqueous solution and dried over anhydrous sodium sulphate. After filtration, the filtrate was concentrated under reduced pressure, and the residue was purified by flash silica gel column chromatography (petroleum ether : tetrahydrofuran = 10:1) to afford compound **121c:** 1-(3-((tert-butyldimethylsilyl)oxy)-2,2-difluoropropyl)-4-(2,3-dichloro-6-((2-(trimethylsilyl)ethoxy)methoxy)phenyl)pyrrolidin-2-one (30.0 mg, 48.1%).
¹H NMR (400 MHz, CDCl₃) δ 7.26-7.20 (m, 1H), 7.05-7.02 (m, 1H), 5.16-5.14 (m, 2H), 4.39-4.31 (m, 1H), 3.88-3.59 (m, 8H), 2.75-2.60 (m, 2H), 0.88-0.85 (m, 2H), 0.84 (s, 9H), 0.03 (s, 6H), -0.07 (s, 9H);
MS m/z (ESI): 584.1 [M+H]⁺.

### Step d:

### 1-(3-((tert-Butyldimethylsilyl)oxy)-2,2-difluoropropyl)-4-(2,3-dichloro-6-((2-(trimethylsilyl)ethoxy)methoxy)phenyl)pyrrolidine-2-thione

To a stirred solution of 1-(3-((tert-butyldimethylsilyl)oxy)-2,2-difluoropropyl)-4-(2,3-dichloro-6-((2-(trimethylsilyl)ethoxy)methoxy)phenyl)pyrrolidin-2-one (30.0 mg, 0.05 mmol) in tetrahydrofuran (1 mL) was added Lawesson's reagent (23.0 mg, 0.06 mmol) at 20°C. The reaction solution was stirred at 20°C for 0.5 h. The resulting mixture was diluted with water and extracted with ethyl acetate (50 mL x 3). The combined organic layers were washed with sodium chloride aqueous solution and dried over anhydrous sodium sulphate. After filtration, the filtrate was concentrated under reduced pressure, and the residue was purified by flash silica gel column chromatography (petroleum ether : tetrahydrofuran = 20:1) to afford compound **121d:** 1-(3-((tert-butyldimethylsilyl)oxy)-2,2-difluoropropyl)-4-(2,3-dichloro-6-((2-(trimethylsilyl)ethoxy)methoxy)phenyl)pyrrolidine-2-thione (25.0 mg, 81.1%).
¹H NMR (400 MHz, CDCl₃) δ 7.31 (d, 1H), 7.09 (d, 1H), 5.23-5.18 (m, 2H), 4.60-4.41 (m, 2H), 4.29-4.17 (m, 1H), 4.10 (d, 2H), 3.91-3.84 (m, 2H), 3.73-3.67 (m, 2H), 3.44-3.28 (m, 2H), 0.94-0.89 (m, 2H), 0.91 (s, 9H), 0.11 (s, 6H), 0.00 (s, 9H);
MS m/z (ESI): 600.0 [M+H]⁺.

### Step e:

### 4-(2,3-Dichloro-6-hydroxyphenyl)-1-(2,2-difluoro-3-hydroxypropyl) pyrrolidine-2-thione

To a stirred solution of 1-(3-((tert-butyldimethylsilyl)oxy)-2,2-difluoropropyl)-4-(2,3-dichloro-6-((2-(trimethylsilyl)ethoxy)methoxy)phenyl)pyrrolidine-2-thione (25.0 mg, 0.04 mmol) in dichloromethane (2 mL) was added trifluoroacetic acid (0.7 mL) at 25°C, and the mixture was stirred at 25°C for 5 h. The resulting mixture was diluted with water and extracted with dichloromethane (25 mL x 3). The combined organic layers were washed with saturated sodium bicarbonate aqueous solution and dried over anhydrous sodium sulphate. After filtration, the filtrate was concentrated under reduced pressure, and the residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **121:** 4-(2,3-dichloro-6-hydroxyphenyl)-1-(2,2-difluoro-3-hydroxypropyl)pyrrolidine-2-thione (7.2 mg, 48.6%).
¹H NMR (400 MHz, CD₃OD) δ 7.27 (d, 1H), 6.79 (d, 1H), 4.59-4.43 (m, 2H), 4.29-4.11 (m, 3H), 3.81-3.71 (m, 2H), 3.45-3.30 (m, 2H);
MS m/z (ESI): 355.8 [M+H]⁺.

Compound **121-1** was synthesised using intermediate **3** as the starting material and following the same synthetic route as compound **121.**

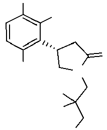

### Example 36

Synthesis of compound 124 and compound 124-1

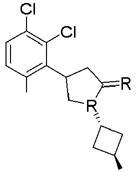

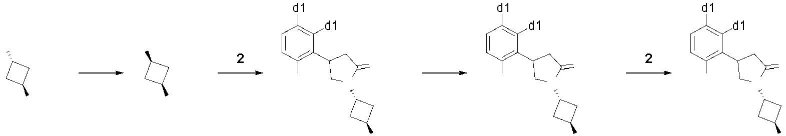

### Step a:

### tert-Butyl ((1s,3s)-3-iodocyclobutoxy)dimethylsilane

To a stirred solution of 3-[(tert-butyldimethylsilyl)oxy]cyclobutan-1-ol (800.0 mg, 3.95 mmol) in tetrahydrofuran (16 mL) were added triphenylphosphine (2.07 g, 2.94 mmol), imidazole (570.0 mg, 8.37 mmol) and iodine (2.11 g, 8.31 mmol) at 0°C under nitrogen protection. The reaction solution was stirred at 30°C for 3 h. The resulting mixture was diluted with saturated sodium thiosulphate aqueous solution and extracted with ethyl acetate (50 mL x 3). The combined organic layers were washed with sodium chloride aqueous solution and dried over anhydrous sodium sulphate. After filtration, the filtrate was concentrated under reduced pressure, and the residue was purified by flash silica gel column chromatography (petroleum ether) to afford compound **124a:** tert-butyl ((1s,3s)-3-iodocyclobutoxy)dimethylsilane (940.0 mg, 76.1%).

¹H NMR (400 MHz, CDCl₃) δ 4.23-4.16 (m, 1H), 3.87-3.79 (m, 1H), 3.02-2.94 (m, 2H), 2.61-2.53 (m, 2H), 0.87 (s, 9H), 0.02 (s, 6H).

### Step b:

### rac-1-((1r,3r)-3-((tert-Butyldimethylsilyl)oxy)cyclobutyl)-4-(2,3-dichloro-6-((2-(trimethylsilyl)ethoxy)methoxy)phenyl)pyrrolidin-2-one

To a stirred solution of intermediate 1 (300.0 mg, 0.80 mmol) in dioxane (2 mL) were added compound **124a** (370.0 mg, 1.18 mmol) and caesium carbonate (520.0 mg, 1.60 mmol) at 20°C, and the reaction solution was stirred at 120°C for 16 h. After the reaction was completed, the resulting mixture was diluted with water and extracted with ethyl acetate (50 mL x 3). The combined organic layers were washed with sodium chloride aqueous solution and dried over anhydrous sodium sulphate. After filtration, the filtrate was concentrated under reduced pressure, and the residue was purified by flash silica gel column chromatography (petroleum ether : tetrahydrofuran = 4:1) to afford compound **124b:** rac-1-((1r,3r)-3-((tert-butyldimethylsilyl)oxy)cyclobutyl)-4-(2,3-dichloro-6-((2-(trimethylsilyl)ethoxy)methoxy)phenyl)pyrrolidin-2-one (200.0 mg, 37.1%).

¹H NMR (400 MHz, CDCl₃) δ 7.31 (d, 1H), 7.09 (d, 1H), 5.22-5.17 (m, 2H), 4.94-4.87 (m, 1H), 4.44-4.35 (m, 2H), 3.76-3.67 (m, 3H), 3.62-3.58 (m, 1H), 2.80-2.67 (m, 2H), 2.50-2.26 (m, 4H), 0.95-0.91 (m, 2H), 0.88 (s, 9H), 0.02 (s, 6H), 0.00 (s, 9H).

### Step c:

### rac-1-((1r,3r)-3-((tert-Butyldimethylsilyl)oxy)cyclobutyl)-4-(2,3-dichloro-6-((2-(trimethylsilyl)ethoxy)methoxy)phenyl)pyrrolidine-2-thione

To a stirred solution of compound **124b** (180.0 mg, 0.32 mmol) in tetrahydrofuran (10 mL) was added Lawesson's reagent (260.0 mg, 0.64 mmol) at 20°C. The reaction solution was stirred at 80°C for 15 min. The resulting mixture was diluted with water and extracted with ethyl acetate (50 mL x 3). The combined organic layers were washed with sodium chloride aqueous solution and dried over anhydrous sodium sulphate. After filtration, the filtrate was concentrated under reduced pressure, and the residue was purified by flash silica gel column chromatography (petroleum ether : tetrahydrofuran = 15:1) to afford compound **124c:** rac-1-((1r,3r)-3-((tert-butyldimethylsilyl)oxy)cyclobutyl)-4-(2,3-dichloro-6-((2-(trimethylsilyl)ethoxy)methoxy)phenyl)pyrrolidine-2-thione (140.0 mg, 75.6%).

¹H NMR (400 MHz, CDCl₃) δ 7.32 (d, 1H), 7.08 (d, 1H), 5.54-5.47 (m, 1H), 5.18 (dd, 2H), 4.67-4.42 (m, 2H), 4.10 (t, 1H), 3.95-3.90 (m, 1H), 3.73-3.68 (m, 2H), 3.44-3.37 (m, 1H), 3.29-3.23 (m, 1H), 2.50-2.42 (m, 4H), 0.95-0.90 (m, 2H), 0.88 (s, 9H), 0.03 (s, 6H), 0.00 (s, 9H).

### Step d:

### rac-4-(2,3-Dichloro-6-hydroxyphenyl)-1-((1r,3r)-3-hydroxycyclobutyl) pyrrolidine-2-thione

To a stirred solution of compound **124c** (140.0 mg, 0.24 mmol) in dichloromethane (3 mL) was added trifluoroacetic acid (1.5 mL) at 0°C, and the mixture was stirred at 30°C for 2 h. The resulting mixture was diluted with water and extracted with dichloromethane (25 mL x 3). The combined organic layers were washed with sodium chloride aqueous solution and dried over anhydrous sodium sulphate. After filtration, the filtrate was concentrated under reduced pressure, and the residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **124:** rac-4-(2,3-dichloro-6-hydroxyphenyl)-1-((1r,3r)-3-hydroxycyclobutyl)pyrrolidine-2-thione (38.0 mg, 47.1%).
¹H NMR (400 MHz, DMSO_*d₆*) δ 10.50 (s, 1H), 7.35 (d, 1H), 6.83 (d, 1H), 5.37-5.30 (m, 1H), 5.14 (d, 1H), 4.32-4.24 (m, 2H), 4.15 (t, 1H), 3.95 (dd, 1H), 3.24-3.08 (m, 2H), 2.54-2.50 (m, 1H), 2.47-2.37 (m, 1H), 2.20-2.14 (m, 2H);
MS m/z (ESI): 332.0 [M+H]⁺.

Compound **124-1** was synthesised using intermediate **3** as the starting material and following the same synthetic route as compound **124.**

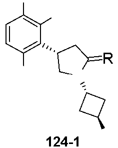

### Example 37

### Synthesis of compound 125

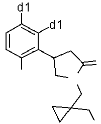

### rac-4-(2,3-Dichloro-6-hydroxyphenyl)-1-((1-(hydroxymethyl)cyclopropyl) methyl)pyrrolidine-2-thione

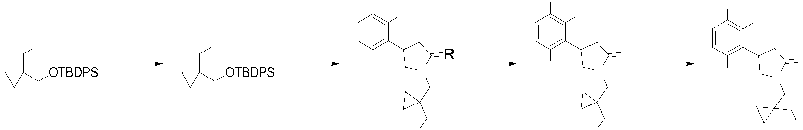

### Step a:

### tert-Butyl ((1-(iodomethyl)cyclopropyl)methoxy)diphenylsilane

To a stirred solution of (1-(((tert-butyldiphenylsilyl)oxy) methyl)cyclopropyl)methanol (500.0 mg, 1.47 mmol) in tetrahydrofuran (20 mL) were added triphenylphosphine (770.2 mg, 2.94 mmol), imidazole (200.0 mg, 2.94 mmol) and iodine (745.3 mg, 2.94 mmol) at 0°C under nitrogen protection. The reaction solution was stirred at 20°C for 2 h. The resulting mixture was diluted with saturated sodium thiosulphate aqueous solution and extracted with petroleum ether (50 mL x 3). The combined organic layers were washed with sodium chloride aqueous solution and dried over anhydrous sodium sulphate. After filtration, the filtrate was concentrated under reduced pressure, and the residue was purified by flash silica gel column chromatography (petroleum ether) to afford compound **125a:** tert-butyl ((1-(iodomethyl)cyclopropyl)methoxy)diphenylsilane (510.0 mg, 77.1%).

¹H NMR (400 MHz, CDCl₃) δ 7.69-7.67 (m, 4H), 7.43-7.36 (m, 6H), 3.58 (s, 2H), 3.41 (s, 2H), 1.07 (s, 9H), 0.78-0.74 (m, 2H), 0.60-0.56 (m, 2H).

### Step b:

### rac-1-((1-(((tert-Butyldiphenylsilyl)oxy)methyl)cyclopropyl)methyl)-4-(2,3-dichloro-6-((2-(trimethylsilyl)ethoxy)methoxy)phenyl)pyrrolidin-2-one

To a stirred solution of intermediate 1 (200.0 mg, 0.53 mmol) in toluene (10 mL) were added tert-butyl ((1-(iodomethyl)cyclopropyl)methoxy)diphenylsilane (478.7 mg, 1.06 mmol), potassium hydroxide (59.6 mg, 1.06 mmol) and tetrabutylammonium iodide (19.6 mg, 0.05 mmol) at 20°C, and the reaction solution was stirred at 40°C for 16 h. After the reaction was completed, the resulting mixture was diluted with water and extracted with ethyl acetate (50 mL x 3). The combined organic layers were washed with sodium chloride aqueous solution and dried over anhydrous sodium sulphate. After filtration, the filtrate was concentrated under reduced pressure, and the residue was purified by flash silica gel column chromatography (petroleum ether : ethyl acetate = 5:1) to afford compound **125b:** 1-((1-(((tert-butyldiphenylsilyl)oxy)methyl)cyclopropyl)methyl)-4-(2,3-dichloro-6-((2-(trimethylsilyl)ethoxy)methoxy)phenyl)pyrrolidin-2-one (247.0 mg, 66.5%).
¹H NMR (400 MHz, DMSO_*d₆*) δ 7.62-7.58 (m, 4H), 7.51 (d, 1H), 7.46-7.37 (m, 6H), 7.10 (d, 1H), 5.14 (d, 1H), 5.04 (d, 1H), 4.25-4.20 (m, 1H), 3.62-3.48 (m, 4H), 3.42-3.23 (m, 4H), 2.57-2.50 (m, 2H), 0.99 (s, 9H), 0.80 (t, 2H), 0.46-0.37 (m, 4H), -0.09 (s, 9H);
MS m/z (ESI): 698.0 [M+H]⁺.

### Step c:

### rac-1-((1-(((tert-Butyldiphenylsilyl)oxy)methyl)cyclopropyl)methyl)-4-(2,3-dichloro-6-((2-(trimethylsilyl)ethoxy)methoxy)phenyl)pyrrolidine-2-thione

To a stirred solution of 1-((1-(((tert-butyldiphenylsilyl)oxy)methyl) cyclopropyl)methyl)-4-(2,3-dichloro-6-((2-(trimethylsilyl)ethoxy)methoxy)phenyl) pyrrolidin-2-one (300.0 mg, 0.43 mmol) in tetrahydrofuran (7 mL) was added Lawesson's reagent (104.2 mg, 0.26 mmol) at 20°C. The reaction solution was stirred at 20°C for 1 h. The resulting mixture was diluted with water and extracted with ethyl acetate (50 mL x 3). The combined organic layers were washed with sodium chloride aqueous solution and dried over anhydrous sodium sulphate. After filtration, the filtrate was concentrated under reduced pressure, and the residue was purified by flash silica gel column chromatography (petroleum ether : ethyl acetate = 8:1) to afford compound **125c:** 1-((1-(((tert-butyldiphenylsilyl)oxy)methyl) cyclopropyl)methyl)-4-(2,3-dichloro-6-((2-(trimethylsilyl)ethoxy)methoxy) phenyl)pyrrolidine-2-thione (270.0 mg, 88.0%).
¹H NMR (400 MHz, DMSO_*d₆*) δ 7.62-7.57 (m, 4H), 7.51 (d, 1H), 7.44-7.36 (m, 6H), 7.09 (d, 1H), 5.11-5.06 (m, 2H), 4.30-4.22 (m, 1H), 4.11-4.04 (m, 2H), 3.76-3.71 (m, 1H), 3.64 (d, 1H), 3.58-3.54 (m, 3H), 3.42 (d, 1H), 3.29-3.19 (m, 1H), 3.10-3.04 (m, 1H), 0.99 (s, 9H), 0.87-0.74 (m, 2H), 0.64-0.44 (m, 4H), -0.07 (s, 9H);
MS m/z (ESI): 714.0 [M+H]⁺.

### Step d:

### rac-4-(2,3-Dichloro-6-hydroxyphenyl)-1-((1-(hydroxymethyl)cyclopropyl) methyl)pyrrolidine-2-thione

To a stirred solution of 1-((1-(((tert-butyldiphenylsilyl)oxy)methyl) cyclopropyl)methyl)-4-(2,3-dichloro-6-((2-(trimethylsilyl)ethoxy)methoxy)phenyl) pyrrolidine-2-thione (170.0 mg, 0.24 mmol) in dichloromethane (16 mL) was added trifluoroacetic acid (3 mL) at 25°C, and the mixture was stirred at 20°C for 1 h. The resulting mixture was diluted with water and extracted with dichloromethane (25 mL x 3). The combined organic layers were washed with sodium chloride aqueous solution and dried over anhydrous sodium sulphate. After filtration, the filtrate was concentrated under reduced pressure, and the concentrate was dissolved in tetrahydrofuran (15 mL). Tetrabutylammonium fluoride (124.4 mg, 0.48 mmol) was added, and the reaction solution was stirred at 20°C for 16 h. The resulting mixture was diluted with water and extracted with ethyl acetate (25 mL x 3). The combined organic layers were washed with sodium chloride aqueous solution and dried over anhydrous sodium sulphate. After filtration, the filtrate was concentrated under reduced pressure, and the residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **125:** rac-4-(2,3-dichloro-6-hydroxyphenyl)-1-((1-(hydroxymethyl)cyclopropyl)methyl)pyrrolidine-2-thione (35.6 mg, 43.0%).
¹H NMR (400 MHz, DMSO_*d₆*) δ 10.55 (brs, 1H), 7.35 (d, 1H), 6.85 (d, 1H), 4.37 (brs, 1H), 4.30-4.23 (m, 1H), 4.09-3.95 (m, 3H), 3.59 (d, 1H), 3.39 (d, 1H), 3.28-3.15 (m, 3H), 0.55-0.48 (m, 4H);
MS m/z (ESI): 345.9 [M+H]⁺.

### Example 38

### Synthesis of compound 126

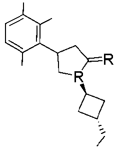

### rac-4-(2,3-Dichloro-6-hydroxyphenyl)-1-((1r,3r)-3-(hydroxymethyl) cyclobutyl)pyrrolidine-2-thione

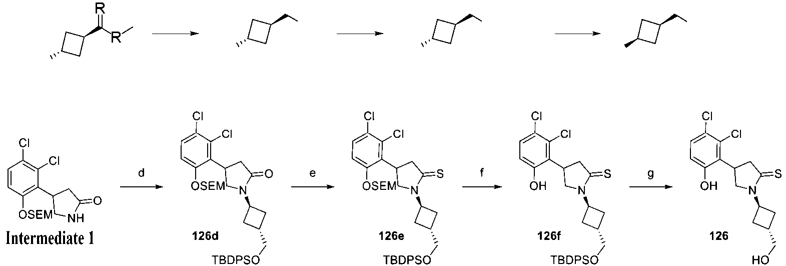

### Step a:

### (1r,3r)-3-(Hydroxymethyl)cyclobutan-1-ol

To a stirred solution of methyl (1r,3r)-3-hydroxycyclobutane-1-carboxylate (2.60 g, 20.0 mmol) in tetrahydrofuran (50 mL) was added lithium aluminium tetrahydride (8.8 mL, 2.5 mol/L) at 0°C. The reaction solution was stirred at 0°C for 1 h. The resulting mixture was diluted with water and extracted with ethyl acetate (100 mL x 3). The combined organic layers were washed with sodium chloride aqueous solution and dried over anhydrous sodium sulphate. After filtration, the filtrate was concentrated under reduced pressure to afford crude **126a:** (1r,3r)-3-(hydroxymethyl)cyclobutan-1-ol (2.00 g, 98.0%), which was directly used in the next reaction.

### Step b:

### (1r,3r)-3-(((tert-Butyldiphenylsilyl)oxy)methyl)cyclobutan-1-ol

To a stirred solution of (1r,3r)-3-(hydroxymethyl)cyclobutan-1-ol (2.00 g, 19.6 mmol) in dichloromethane (40 mL) were added imidazole (1.33 g, 19.6 mmol) and tert-butyldiphenylchlorosilane (4.85 g, 17.6 mmol) at 0°C. The reaction solution was stirred at 0°C for 1 h. The reaction solution was concentrated under reduced pressure, and the residue was purified by flash silica gel column chromatography (petroleum ether : ethyl acetate = 4:1) to afford compound **126b:** (1r,3r)-3-(((tert-butyldiphenylsilyl)oxy)methyl)cyclobutan-1-ol (2.70 g, 40.5%).

¹H NMR (300 MHz, CDCl₃) δ 7.68-7.65 (m, 4H), 7.46-7.35 (m, 6H), 4.42-4.37 (m, 1H), 3.64 (d, 2H), 2.44-2.37 (m, 1H), 2.29-2.18 (m, 2H), 2.06-1.97 (m, 2H), 1.07 (s, 9H).

### Step c:

### tert-Butyl (((1s,3s)-3-iodocyclobutyl)methoxy)diphenylsilane

To a stirred solution of (1r,3r)-3-(((tert-butyldiphenylsilyl)oxy) methyl)cyclobutan-1-ol (2.70 g, 7.9 mmol) in tetrahydrofuran (50 mL) were added triphenylphosphine (2.38 g, 9.1 mmol), imidazole (0.81 g, 11.9 mmol) and iodine (2.41 g, 9.5 mmol) at 25°C. The reaction solution was stirred at 25°C for 18 h. The reaction solution was concentrated under reduced pressure, and the residue was purified by flash silica gel column chromatography (petroleum ether) to afford compound **126c:** tert-butyl (((1s,3s)-3-iodocyclobutyl)methoxy)diphenylsilane (0.52 g, 14.6%).

¹H NMR (300 MHz, CDCl₃) δ 7.68-7.65 (m, 4H), 7.45-7.35 (m, 6H), 4.41-4.38 (m, 1H), 3.62 (d, 2H), 2.73-2.56 (m, 5H), 1.09 (s, 9H).

### Step d:

### rac-1-((1r,3r)-3-(((tert-Butyldiphenylsilyl)oxy)methyl)cyclobutyl)-4-(2,3-dichloro-6-((2-(trimethylsilyl)ethoxy)methoxy)phenyl)pyrrolidin-2-one

To a stirred solution of intermediate 1 (300.0 mg, 0.80 mmol) in dioxane (2 mL) were added tert-butyl (((1s,3s)-3-iodocyclobutyl)methoxy)diphenylsilane (430.8 mg, 0.96 mmol) and caesium carbonate (519.4 mg, 1.59 mmol) at 20°C, and the reaction solution was stirred at 120°C for 16 h. After the reaction was completed, the reaction solution was concentrated under reduced pressure, and the residue was purified by flash silica gel column chromatography (petroleum ether : ethyl acetate = 3:1) to afford compound **126d:** rac-1-((1r,3r)-3-(((tert-butyldiphenylsilyl)oxy)methyl)cyclobutyl)-4-(2,3-dichloro-6-((2-(trimethylsilyl)ethoxy)methoxy)phenyl)pyrrolidin-2-one (210.0 mg, 37.7%).

MS m/z (ESI): 698.2 [M+H]⁺.

### Step e:

### rac-1-((1r,3r)-3-(((tert-Butyldiphenylsilyl)oxy)methyl)cyclobutyl)-4-(2,3-dichloro-6-((2-(trimethylsilyl)ethoxy)methoxy)phenyl)pyrrolidine-2-thione

To a stirred solution of 1-((1r,3r)-3-(((tert-butyldiphenylsilyl)oxy) methyl)cyclobutyl)-4-(2,3-dichloro-6-((2-(trimethylsilyl)ethoxy)methoxy)phenyl) pyrrolidin-2-one (210.0 mg, 0.30 mmol) in toluene (6 mL) was added Lawesson's reagent (121.5 mg, 0.30 mmol) at 20°C. The reaction solution was stirred at 80°C for 10 min. The reaction solution was concentrated under reduced pressure, and the residue was purified by flash silica gel column chromatography (petroleum ether : ethyl acetate = 4:1) to afford compound **126e:** rac-1-((1r,3r)-3-(((tert-butyldiphenylsilyl)oxy)methyl)cyclobutyl)-4-(2,3-dichloro-6-((2-(trimethylsilyl)ethoxy)methoxy)phenyl)pyrrolidine-2-thione (90.0 mg, 41.9%).

¹H NMR (400 MHz, DMSO_*d*₆) δ 7.70-7.68 (m, 4H), 7.56 (d, 1H), 7.51-7.46 (m, 6H), 7.17 (d, 1H), 5.41-5.39 (m, 1H), 5.31-5.18 (m, 2H), 4.39-4.37 (m, 1H), 4.31-4.28 (m, 1H), 3.97-3.93 (m, 1H), 3.76-3.64 (m, 4H), 3.32-3.33 (m, 1H), 3.07-3.01 (m, 1H), 2.48-2.34 (m, 3H), 2.23-2.17 (m, 2H), 1.07 (s, 9H), 0.93-0.84 (m, 2H), 0.00 (s, 9H).

### Step f:

### rac-1-((1r,3r)-3-(((tert-Butyldiphenylsilyl)oxy)methyl)cyclobutyl)-4-(2,3-dichloro-6-hydroxyphenyl)pyrrolidine-2-thione

To a stirred solution of 1-((1r,3r)-3-(((tert-butyldiphenylsilyl)oxy)methyl) cyclobutyl)-4-(2,3-dichloro-6-((2-(trimethylsilyl)ethoxy)methoxy)phenyl) pyrrolidine-2-thione (90.0 mg, 0.13 mmol) in dichloromethane (4 mL) was added trifluoroacetic acid (1 mL) at 25°C, and the mixture was stirred at 25°C for 1 h. The resulting mixture was diluted with water and extracted with dichloromethane (30 mL x 3). The combined organic layers were washed with saturated sodium bicarbonate aqueous solution and dried over anhydrous sodium sulphate. After filtration, the filtrate was concentrated under reduced pressure, and the residue was purified by flash silica gel column chromatography (petroleum ether : ethyl acetate = 6:1) to afford compound **126f:** rac-1-((1r,3r)-3-(((tert-butyldiphenylsilyl)oxy)methyl)cyclobutyl)-4-(2,3-dichloro-6-hydroxyphenyl)pyrrolidine-2-thione (72.6 mg, 98.6%).

MS m/z (ESI): 584.1 [M+H]⁺.

### Step g:

### rac-4-(2,3-Dichloro-6-hydroxyphenyl)-1-((1r,3r)-3-(hydroxymethyl)cyclobutyl)pyrrolidine-2-thione

To a stirred solution of 1-((1r,3r)-3-(((tert-butyldiphenylsilyl)oxy)methyl) cyclobutyl)-4-(2,3-dichloro-6-hydroxyphenyl)pyrrolidine-2-thione (80.0 mg, 0.14 mmol) in tetrahydrofuran (2 mL) was added tetrabutylammonium fluoride (107.3 mg, 0.41 mmol) at 25°C, and the mixture was stirred at 25°C for 2 h. The resulting mixture was diluted with water and extracted with ethyl acetate (30 mL x 3). The combined organic layers were washed with sodium chloride aqueous solution and dried over anhydrous sodium sulphate. After filtration, the filtrate was concentrated under reduced pressure, and the residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **126:** rac-4-(2,3-dichloro-6-hydroxyphenyl)-1-((1r,3r)-3-(hydroxymethyl)cyclobutyl)pyrrolidine-2-thione (19.1 mg, 40.3%).
¹H NMR (400 MHz, DMSO_*d*₆) δ 10.54 (s, 1H), 7.35 (d, 1H), 6.82 (d, 1H), 5.27-5.23 (m, 1H), 4.68 (t, 1H), 4.30-4.25 (m, 1H), 4.18 (t, 1H), 4.02-3.98 (m, 1H), 3.47 (t, 2H), 3.23-3.11 (m, 2H), 2.38-2.07 (m, 3H), 2.05 (t, 2H);
MS m/z (ESI): 345.9 [M+H]⁺.

### Example 39

### Synthesis of compound 127

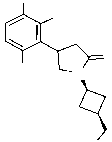

### 4-(2,3-Dichloro-6-hydroxyphenyl)-1-((1s,3s)-3-(hydroxymethyl)cyclobutyl) pyrrolidine-2-thione

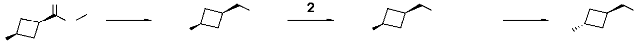

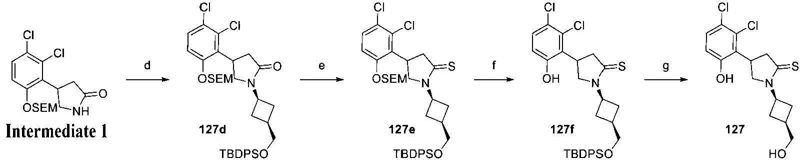

### Step a:

### (1s,3s)-3-(Hydroxymethyl)cyclobutan-1-ol

To a stirred solution of methyl (1s,3s)-3-hydroxycyclobutane-1-carboxylate (4.00 g, 20.0 mmol) in tetrahydrofuran (80 mL) was added lithium aluminium tetrahydride (1.23 g, 31.0 mmol) at 0°C. The reaction solution was stirred at 0°C for 2 h. The resulting mixture was diluted with water and extracted with ethyl acetate (100 mL x 3). The combined organic layers were washed with sodium chloride aqueous solution and dried over anhydrous sodium sulphate. After filtration, the filtrate was concentrated under reduced pressure to afford crude **127a:** (1s,3s)-3-(hydroxymethyl)cyclobutan-1-ol (3.10 g, 98.8%), which was directly used in the next reaction.

### Step b:

### (1s,3s)-3-(((tert-Butyldiphenylsilyl)oxy)methyl)cyclobutan-1-ol

To a stirred solution of (1r,3r)-3-(hydroxymethyl)cyclobutan-1-ol (3.10 g, 30.4 mmol) in dichloromethane (60 mL) were added imidazole (3.10 g, 45.5 mmol) and tert-butyldiphenylchlorosilane (8.36 g, 30.4 mmol) at 0°C. The reaction solution was stirred at 0°C for 1 h. The reaction solution was concentrated under reduced pressure, and the residue was purified by flash silica gel column chromatography (petroleum ether : ethyl acetate = 4:1) to afford compound **127b:** (1s,3s)-3-(((tert-butyldiphenylsilyl)oxy)methyl)cyclobutan-1-ol (2.10 g, 20.3%).

¹H NMR (300 MHz, CDCl₃) δ 7.70-7.68 (m, 4H), 7.47-7.39 (m, 6H), 4.18-4.14 (m, 1H), 3.64 (d, 2H), 2.44-2.37 (m, 2H), 2.07-2.03 (m, 1H), 1.79-1.72 (m, 2H), 1.09 (s, 9H).

### Step c:

### tert-Butyl (((1r,3r)-3-iodocyclobutyl)methoxy)diphenylsilane

To a stirred solution of (1s,3s)-3-(((tert-butyldiphenylsilyl)oxy)methyl) cyclobutan-1-ol (2.10 g, 6.2 mmol) in tetrahydrofuran (60 mL) were added triphenylphosphine (3.25 g, 12.4 mmol), imidazole (0.89 g, 13.1 mmol) and iodine (3.30 g, 13.0 mmol) at 25°C. The reaction solution was stirred at 25°C for 2 h. The reaction solution was concentrated under reduced pressure, and the residue was purified by flash silica gel column chromatography (petroleum ether) to afford compound **127c:** tert-butyl (((1r,3r)-3-iodocyclobutyl)methoxy)diphenylsilane (0.66 g, 23.8%).

¹H NMR (300 MHz, CDCl₃) δ 7.66-7.63 (m, 4H), 7.45-7.37 (m, 6H), 4.62-4.55 (m, 1H), 3.64 (d, 2H), 2.81-2.72 (m, 1H), 2.66-2.62 (m, 4H), 1.06 (s, 9H).

### Step d:

### rac-1-((1s,3s)-3-(((tert-Butyldiphenylsilyl)oxy)methyl)cyclobutyl)-4-(2,3-dichloro-6-((2-(trimethylsilyl)ethoxy)methoxy)phenyl)pyrrolidin-2-one

To a stirred solution of intermediate 1 (300.0 mg, 0.80 mmol) in dioxane (2 mL) were added tert-butyl (((1r,3r)-3-iodocyclobutyl)methoxy)diphenylsilane (430.8 mg, 0.96 mmol) and caesium carbonate (519.4 mg, 1.59 mmol) at 20°C, and the reaction solution was stirred at 120°C for 16 h. After the reaction was completed, the reaction solution was concentrated under reduced pressure, and the residue was purified by flash silica gel column chromatography (petroleum ether : ethyl acetate = 3:1) to afford compound **127d:** rac-1-((1s,3s)-3-(((tert-butyldiphenylsilyl)oxy)methyl) cyclobutyl)-4-(2,3-dichloro-6-((2-(trimethylsilyl)ethoxy)methoxy)phenyl) pyrrolidin-2-one (190.0 mg, 34.1%).

¹H NMR (400 MHz, DMSO_*d*₆) δ 7.64-7.58 (m, 5H), 7.47-7.39 (m, 6H), 7.20 (d, 1H), 5.30-5.26 (m, 2H), 4.56-4.52 (m, 1H), 4.31-4.29 (m, 1H), 3.74-3.64 (m, 5H), 3.50-3.46 (m, 1H), 2.73-2.57 (m, 2H), 2.29-2.07 (m, 5H), 1.02 (s, 9H), 0.93-0.89 (m, 2H), 0.00 (s, 9H).

### Step e:

### rac-1-((1s,3s)-3-(((tert-Butyldiphenylsilyl)oxy)methyl)cyclobutyl)-4-(2,3-dichloro-6-((2-(trimethylsilyl)ethoxy)methoxy)phenyl)pyrrolidine-2-thione

To a stirred solution of 1-((1s,3s)-3-(((tert-butyldiphenylsilyl)oxy) methyl)cyclobutyl)-4-(2,3-dichloro-6-((2-(trimethylsilyl)ethoxy)methoxy)phenyl) pyrrolidin-2-one (190.0 mg, 0.27 mmol) in toluene (6 mL) was added Lawesson's reagent (110.0 mg, 0.27 mmol) at 20°C. The reaction solution was stirred at 80°C for 15 min. The reaction solution was concentrated under reduced pressure, and the residue was purified by flash silica gel column chromatography (petroleum ether : ethyl acetate = 4:1) to afford compound **127e:** rac-1-((1s,3s)-3-(((tert-butyldiphenylsilyl)oxy)methyl)cyclobutyl)-4-(2,3-dichloro-6-((2-(trimethylsilyl)ethoxy)methoxy)phenyl)pyrrolidine-2-thione (130.0 mg, 66.9%).

¹H NMR (400 MHz, DMSO_*d*₆) δ 7.63-7.57 (m, 5H), 7.45-7.38 (m, 6H), 7.18 (d, 1H), 5.28-5.13 (m, 3H), 4.39-4.34 (m, 1H), 4.18-4.13 (m, 1H), 3.83-3.79 (m, 1H), 3.71-3.66 (m, 4H), 3.32-3.11 (m, 1H), 3.08-3.02 (m, 1H), 2.35-2.10 (m, 5H), 1.00 (s, 9H), 0.91-0.88 (m, 2H), 0.00 (s, 9H).

### Step f:

### rac-1-((1s,3s)-3-(((tert-Butyldiphenylsilyl)oxy)methyl)cyclobutyl)-4-(2,3-dichloro-6-hydroxyphenyl)pyrrolidine-2-thione

To a stirred solution of 1-((1s,3s)-3-(((tert-butyldiphenylsilyl)oxy)methyl)cyclobutyl)-4-(2,3-dichloro-6-((2-(trimethylsilyl)ethoxy)methoxy)phenyl)pyrrolidine-2-thione (130.0 mg, 0.18 mmol) in dichloromethane (4 mL) was added trifluoroacetic acid (1 mL) at 25°C, and the mixture was stirred at 25°C for 2 h. The resulting mixture was diluted with water and extracted with dichloromethane (30 mL x 3). The combined organic layers were washed with saturated sodium bicarbonate aqueous solution and dried over anhydrous sodium sulphate. After filtration, the filtrate was concentrated under reduced pressure, and the residue was purified by flash silica gel column chromatography (petroleum ether : ethyl acetate = 6:1) to afford compound 127f: rac-1-((1s,3s)-3-(((tert-butyldiphenylsilyl)oxy)methyl)cyclobutyl)-4-(2,3-dichloro-6-hydroxyphenyl)pyrrolidine-2-thione (90.0 mg, 84.6%).

MS m/z (ESI): 584.0 [M+H]⁺.

### Step g:

### rac-4-(2,3-Dichloro-6-hydroxyphenyl)-1-((1s,3s)-3-(hydroxymethyl)cyclobutyl)pyrrolidine-2-thione

To a stirred solution of 1-((1s,3s)-3-(((tert-butyldiphenylsilyl)oxy)methyl) cyclobutyl)-4-(2,3-dichloro-6-hydroxyphenyl)pyrrolidine-2-thione (90.0 mg, 0.15 mmol) in tetrahydrofuran (3 mL) was added tetrabutylammonium fluoride (120.7 mg, 0.46 mmol) at 25°C, and the mixture was stirred at 25°C for 3 h. The resulting mixture was diluted with water and extracted with ethyl acetate (30 mL x 3). The combined organic layers were washed with sodium chloride aqueous solution and dried over anhydrous sodium sulphate. After filtration, the filtrate was concentrated under reduced pressure, and the residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **127:** rac-4-(2,3-dichloro-6-hydroxyphenyl)-1-((1s,3s)-3-(hydroxymethyl)cyclobutyl)pyrrolidine-2-thione (34.6 mg, 64.9%).
¹H NMR (400 MHz, DMSO_*d*₆) δ 10.55 (s, 1H), 7.34 (d, 1H), 6.82 (d, 1H), 5.08-5.04 (m, 1H), 4.50-4.51 (m, 1H), 4.28-4.24 (m, 1H), 4.09 (t, 1H), 3.96-3.91 (m, 1H),3.36 (s, 2H), 3.23-3.09 (m, 2H), 2.21-1.96 (m, 5H);
MS m/z (ESI): 345.9 [M+H]⁺.

### Example 40

### Synthesis of compound 128

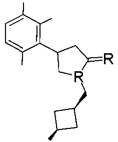

### 4-(2,3-Dichloro-6-hydroxyphenyl)-1-(((1s,3s)-3-hydroxycyclobutyl)methyl) pyrrolidine-2-thione

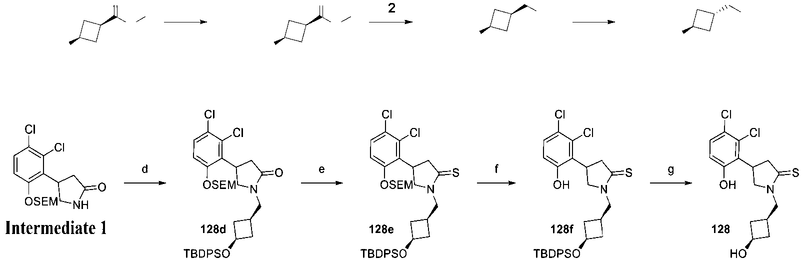

### Step a:

### Methyl (1s,3s)-3-((tert-butyldiphenylsilyl)oxy)cyclobutane-1-carboxylate

To a stirred solution of methyl (1s,3s)-3-hydroxycyclobutane-1-carboxylate (0.50 g, 3.84 mmol) in dichloromethane (10 mL) were added tert-butyldiphenylchlorosilane (1.16 g, 4.22 mmol) and imidazole (523.1 mg, 7.68 mmol) at 25°C. The reaction solution was stirred at 25°C for 4.5 h. The resulting mixture was diluted with water and extracted with ethyl acetate (100 mL x 3). The combined organic layers were washed with hydrochloric acid solution and dried over anhydrous sodium sulphate. After filtration, the filtrate was concentrated under reduced pressure, and the residue was purified by flash silica gel column chromatography (petroleum ether : tetrahydrofuran = 20:1) to afford compound **128a:** methyl (1s,3s)-3-((tert-butyldiphenylsilyl)oxy)cyclobutane-1-carboxylate (1.30 g, 91.8%).

¹H NMR (300 MHz, CDCl₃) δ 7.66-7.63 (m, 4H), 7.45-7.35 (m, 6H), 4.16-4.06 (m, 1H), 3.67 (s, 3H), 2.47-2.17 (m, 5H), 1.03 (s, 9H).

### Step b:

### ((1s,3s)-3-((tert-Butyldiphenylsilyl)oxy)cyclobutyl)methanol

To a stirred solution of methyl (1s,3s)-3-((tert-butyldiphenylsilyl)oxy)cyclobutane-1-carboxylate (1.00 g, 2.71 mmol) in tetrahydrofuran (20 mL) was added a solution of lithium borohydride in tetrahydrofuran (2.70 mL, 5.43 mmol, 2 M) at 0°C. The reaction solution was stirred at 50°C for 3 h. The resulting mixture was diluted with water and extracted with ethyl acetate (100 mL x 3). The combined organic layers were washed with sodium chloride aqueous solution and dried over anhydrous sodium sulphate. After filtration, the filtrate was concentrated under reduced pressure, and the residue was purified by flash silica gel column chromatography (petroleum ether : tetrahydrofuran = 4:1) to afford compound **128b:** ((1s,3s)-3-((tert-butyldiphenylsilyl)oxy)cyclobutyl) methanol (0.84 g, 90.9%).

¹H NMR (400 MHz, CDCl₃) δ 7.67-7.65 (m, 4H), 7.44-7.35 (m, 6H), 4.19-4.11 (m, 1H), 3.59 (d, 2H), 2.27-2.21 (m, 2H), 1.87-1.75 (m, 3H), 1.03 (s, 9H).

### Step c:

### tert-Butyl ((1r,3r)-3-(iodomethyl)cyclobutoxy)diphenylsilane

To a stirred solution of ((1s,3s)-3-((tert-butyldiphenylsilyl)oxy)cyclobutyl) methanol (0.84 g, 2.47 mmol) in tetrahydrofuran (16 mL) were added triphenylphosphine (1.29 g, 4.93 mmol), imidazole (344.3 mg, 5.06 mmol) and iodine (1.28 g, 5.06 mmol) at 0°C under nitrogen protection. The reaction solution was stirred at 25°C for 2 h. The resulting mixture was diluted with saturated sodium thiosulphate solution and extracted with ethyl acetate (50 mL x 3). The combined organic layers were washed with sodium chloride aqueous solution and dried over anhydrous sodium sulphate. After filtration, the filtrate was concentrated under reduced pressure, and the residue was purified by flash silica gel column chromatography (petroleum ether) to afford compound **128c:** tert-butyl ((1r,3r)-3-(iodomethyl)cyclobutoxy)diphenylsilane (0.77 g, 69.3%).

¹H NMR (400 MHz, CDCl₃) δ 7.65-7.63 (m, 4H), 7.44-7.36 (m, 6H), 4.06-3.99 (m, 1H), 3.21 (d, 2H), 2.35-2.28 (m, 2H), 2.02-1.91 (m, 1H), 1.70-1.62 (m, 2H), 1.03 (s, 9H).

### Step d:

### rac-1-(((1s,3s)-3-((tert-Butyldiphenylsilyl)oxy)cyclobutyl)methyl)-4-(2,3-dichloro-6-((2-(trimethylsilyl)ethoxy)methoxy)phenyl)pyrrolidin-2-one

Under nitrogen protection at 20°C, tert-butyl ((1r,3r)-3-(iodomethyl)cyclobutoxy)diphenylsilane (718.1 mg, 1.59 mmol), potassium hydroxide (119.3 mg, 2.13 mmol) and tetrabutylammonium iodide (39.3 mg, 0.11 mmol) were added to a stirred solution of intermediate 1 (200.0 mg, 0.53 mmol) in toluene (10 mL), and the reaction solution was stirred at 80°C for 16 h. After the reaction was completed, the reaction solution was concentrated under reduced pressure, and the residue was purified by flash silica gel column chromatography (petroleum ether : tetrahydrofuran = 7:3) to afford compound **128d:** rac-1-(((1s,3s)-3-((tert-butyldiphenylsilyl)oxy)cyclobutyl)methyl)-4-(2,3-dichloro-6-((2-(trimethylsilyl)ethoxy)methoxy)phenyl)pyrrolidin-2-one (350.0 mg, 94.2%).

¹H NMR (400 MHz, CDCl₃) δ 7.64-7.63 (m, 4H), 7.43-7.35 (m, 6H), 7.30 (d, 1H), 7.06 (d, 1H), 5.20-5.15 (m, 2H), 4.38-4.33 (m, 1H), 4.09-4.04 (m, 1H), 3.70-3.31 (m, 6H), 2.77-2.61 (m, 2H), 2.29-2.20 (m, 2H), 1.85-1.77 (m, 3H), 1.02 (s, 9H), 0.93-0.89 (m, 2H), -0.02 (s, 9H).

### Step e:

### rac-1-(((1s,3s)-3-((tert-Butyldiphenylsilyl)oxy)cyclobutyl)methyl)-4-(2,3-dichloro-6-((2-(trimethylsilyl)ethoxy)methoxy)phenyl)pyrrolidine-2-thione

To a stirred solution of 1-(((1s,3s)-3-((tert-butyldiphenylsilyl)oxy) cyclobutyl)methyl)-4-(2,3-dichloro-6-((2-(trimethylsilyl)ethoxy)methoxy)phenyl) pyrrolidin-2-one (320.0 mg, 0.46 mmol) in tetrahydrofuran (10 mL) was added Lawesson's reagent (185.2 mg, 0.46 mmol) at 20°C. The reaction solution was stirred at 80°C for 15 min. The resulting mixture was diluted with saturated sodium bicarbonate solution and extracted with ethyl acetate (50 mL x 3). The combined organic layers were washed with sodium chloride aqueous solution and dried over anhydrous sodium sulphate. After filtration, the filtrate was concentrated under reduced pressure, and the residue was purified by flash silica gel column chromatography (petroleum ether : tetrahydrofuran = 4:1) to afford compound **128e:** rac-1-(((1s,3s)-3-((tert-butyldiphenylsilyl)oxy)cyclobutyl)methyl)-4-(2,3-dichloro-6-((2-(trimethylsilyl)ethoxy)methoxy)phenyl)pyrrolidine-2-thione (320.0 mg, 97.8%).

¹H NMR (400 MHz, CDCl₃) δ 7.61-7.63 (m, 4H), 7.44-7.35 (m, 6H), 7.31 (d, 1H), 7.05 (d, 1H), 5.21-5.11 (m, 2H), 4.41-4.36 (m, 1H), 4.13-4.05 (m, 1H), 3.95-3.64 (m, 6H), 3.38-3.22 (m, 2H), 2.36-2.27 (m, 2H), 1.90-1.84 (m, 3H), 1.02 (s, 9H), 0.93-0.84 (m, 2H), -0.01 (s, 9H).

### Step f:

### rac-1-(((1s,3s)-3-((tert-Butyldiphenylsilyl)oxy)cyclobutyl)methyl)-4-(2,3-dichloro-6-hydroxyphenyl)pyrrolidine-2-thione

To a stirred solution of 1-(((1s,3s)-3-((tert-butyldiphenylsilyl)oxy)cyclobutyl) methyl)-4-(2,3-dichloro-6-((2-(trimethylsilyl)ethoxy)methoxy)phenyl)pyrrolidine-2-thione (160.0 mg, 0.22 mmol) in dichloromethane (4 mL) was added trifluoroacetic acid (1 mL) at 25°C, and the mixture was stirred at 25°C for 1 h. The resulting mixture was diluted with saturated sodium bicarbonate aqueous solution and extracted with dichloromethane (30 mL x 3). The combined organic layers were washed with sodium chloride aqueous solution and dried over anhydrous sodium sulphate. After filtration, the filtrate was concentrated under reduced pressure to afford crude **128f:** rac-1-(((1s,3s)-3-((tert-butyldiphenylsilyl)oxy)cyclobutyl)methyl)-4-(2,3-dichloro-6-hydroxyphenyl)pyrrolidine-2-thione (130.0 mg, 99.3%).

MS m/z (ESI): 584.1 [M+H]⁺.

### Step g:

### rac-4-(2,3-Dichloro-6-hydroxyphenyl)-1-(((1s,3s)-3-hydroxycyclobutyl)methyl)pyrrolidine-2-thione

To a stirred solution of 1-(((1s,3s)-3-((tert-butyldiphenylsilyl)oxy)cyclobutyl) methyl)-4-(2,3-dichloro-6-hydroxyphenyl)pyrrolidine-2-thione (130.0 mg, 0.22 mmol) in tetrahydrofuran (5 mL) was added tetrabutylammonium fluoride (174.4 mg, 0.67 mmol) at 25°C, and the mixture was stirred at 25°C for 3 h. The resulting mixture was diluted with water and extracted with ethyl acetate (30 mL x 3). The combined organic layers were washed with sodium chloride aqueous solution and dried over anhydrous sodium sulphate. After filtration, the filtrate was concentrated under reduced pressure, and the residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **128:** rac-4-(2,3-dichloro-6-hydroxyphenyl)-1-(((1s,3s)-3-hydroxycyclobutyl)methyl)pyrrolidine-2-thione (42.5 mg, 55.2%).
¹H NMR (400 MHz, DMSO_*d*₆) δ 10.50 (s, 1H), 7.34 (d, 1H), 6.83 (d, 1H), 5.02 (s, 1H), 4.26-4.22 (m, 1H), 3.98-3.87 (m, 4H), 3.61-3.56 (m, 1H), 3.17-3.14 (m, 2H), 2.33-2.27 (m, 2H), 2.10-2.02 (m, 1H), 1.66-1.57 (m, 2H);
MS m/z (ESI): 345.9 [M+H]⁺.

### Example 41

### Synthesis of compound 129 and compound 129-1

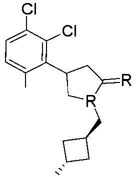

### rac-4-(2,3-Dichloro-6-hydroxyphenyl)-1-(((1r,3r)-3-hydroxycyclobutyl) methyl)pyrrolidine-2-thione

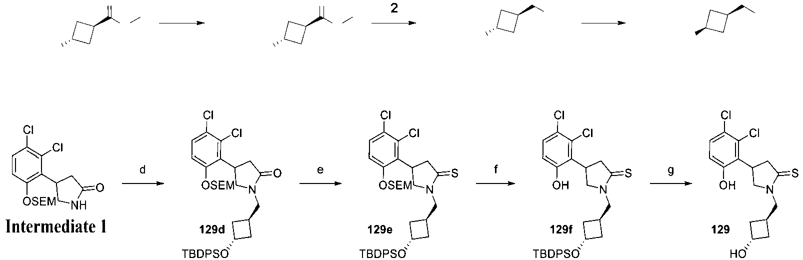

### Step a:

### Methyl (1r,3r)-3-((tert-butyldiphenylsilyl)oxy)cyclobutane-1-carboxylate

To a stirred solution of methyl (1r,3r)-3-hydroxycyclobutane-1-carboxylate (200.0 mg, 1.54 mmol) in N,N-dimethylformamide (10 mL) were added tert-butyldiphenylchlorosilane (464.7 mg, 1.69 mmol) and imidazole (229.9 mg, 3.38 mmol) at 0°C. The reaction solution was stirred at 25°C for 16 h. The resulting mixture was diluted with water and extracted with ethyl acetate (50 mL x 3). The combined organic layers were washed with hydrochloric acid solution and dried over anhydrous sodium sulphate. After filtration, the filtrate was concentrated under reduced pressure, and the residue was purified by flash silica gel column chromatography (petroleum ether : ethyl acetate = 97:3) to afford compound **129a:** methyl (1r,3r)-3-((tert-butyldiphenylsilyl)oxy)cyclobutane-1-carboxylate (420.0 mg, 74.1%).
¹H NMR (400 MHz, CDCl₃) δ 7.64-7.62 (m, 4H), 7.44-7.35 (m, 6H), 4.59-4.52 (m, 1H), 3.62 (s, 3H), 3.02-2.95 (m, 1H), 2.46-2.30 (m, 4H), 1.04 (s, 9H);
MS m/z (ESI): 369.0 [M+H]⁺.

### Step b:

### ((1r,3r)-3-((tert-Butyldiphenylsilyl)oxy)cyclobutyl)methanol

To a stirred solution of methyl (1r,3r)-3-((tert-butyldiphenylsilyl)oxy)cyclobutane-1-carboxylate (200.0 g, 0.54 mmol) in tetrahydrofuran (5 mL) was added lithium aluminium tetrahydride (30.9 mg, 0.81 mmol) at 0°C. The reaction solution was stirred at 0°C for 1 h. The resulting mixture was diluted with water and extracted with ethyl acetate (50 mL x 3). The combined organic layers were washed with sodium chloride aqueous solution and dried over anhydrous sodium sulphate. After filtration, the filtrate was concentrated under reduced pressure, and the residue was purified by flash silica gel column chromatography (petroleum ether : ethyl acetate = 6:1) to afford compound **129b:** ((1r,3r)-3-((tert-butyldiphenylsilyl)oxy)cyclobutyl)methanol (155.0 mg, 83.9%).

MS m/z (ESI): 341.0 [M+H]⁺.

### Step c:

### tert-Butyl ((1s,3s)-3-(iodomethyl)cyclobutoxy)diphenylsilane

To a stirred solution of ((1r,3r)-3-((tert-butyldiphenylsilyl)oxy) cyclobutyl)methanol (3.60 g, 10.60 mmol) in tetrahydrofuran (100 mL) were added triphenylphosphine (4.17 g, 15.90 mmol), imidazole (1.44 g, 21.2 mmol) and iodine (4.04 g, 15.90 mmol) at 0°C under nitrogen protection. The reaction solution was stirred at 25°C for 16 h. The reaction solution was concentrated under reduced pressure, and the residue was purified by flash silica gel column chromatography (petroleum ether : ethyl acetate = 100:1) to afford compound **129c:** tert-butyl ((1s,3s)-3-(iodomethyl)cyclobutoxy)diphenylsilane (4.30 g, 90.3%).

¹H NMR (400 MHz, DMSO*_d*₆) δ 7.60-7.58 (m, 4H), 7.46-7.43 (m, 6H), 4.48-4.43 (m, 1H), 3.32 (d, 2H), 2.61-2.51 (m, 1H), 2.19-2.12 (m, 2H), 1.97-1.91 (m, 2H), 0.98 (s, 9H).

### Step d:

### rac-1-(((1r,3r)-3-((tert-Butyldiphenylsilyl)oxy)cyclobutyl)methyl)-4-(2,3-dichloro-6-((2-(trimethylsilyl)ethoxy)methoxy)phenyl)pyrrolidin-2-one

Under nitrogen protection at 20°C, tert-butyl ((1s,3s)-3-(iodomethyl)cyclobutoxy)diphenylsilane (143.6 mg, 0.32 mmol) and potassium hydroxide (29.8 mg, 0.53 mmol) were added to a stirred solution of intermediate 1 (100.0 mg, 0.27 mmol) in toluene (5 mL), and the reaction solution was stirred at 100°C for 16 h. After the reaction was completed, the reaction solution was concentrated under reduced pressure, and the residue was purified by flash silica gel column chromatography (petroleum ether : ethyl acetate = 87:13) to afford compound **129d:** rac-1-(((1r,3r)-3-((tert-butyldiphenylsilyl)oxy)cyclobutyl)methyl) -4-(2,3-dichloro-6-((2-(trimethylsilyl)ethoxy)methoxy)phenyl)pyrrolidin-2-one (100.0 mg, 53.8%).
¹H NMR (400 MHz, DMSO_*d*₆) δ 7.64-7.63 (m, 4H), 7.56-7.45 (m, 7H), 7.17 (d, 1H), 5.26 (s, 2H), 4.50-4.47 (m, 1H), 4.26-4.25 (m, 1H), 3.71-3.65 (m, 2H), 3.60-3.56 (m, 1H), 3.32-3.27 (m, 1H), 3.21-3.15 (m, 3H), 2.42-2.40 (m, 1H), 2.19-2.03 (m, 4H), 1.02 (s, 9H), 0.91 (t, 3H), 0.00 (s, 9H);
MS m/z (ESI): 699.0 [M+H]⁺.

### Step e:

### rac-1-(((1r,3r)-3-((tert-Butyldiphenylsilyl)oxy)cyclobutyl)methyl)-4-(2,3-dichloro-6-((2-(trimethylsilyl)ethoxy)methoxy)phenyl)pyrrolidine-2-thione

To a stirred solution of 1-(((1r,3r)-3-((tert-butyldiphenylsilyl)oxy)cyclobutyl) methyl)-4-(2,3-dichloro-6-((2-(trimethylsilyl)ethoxy)methoxy)phenyl)pyrrolidin-2-one (400.0 mg, 0.57 mmol) in tetrahydrofuran (10 mL) was added Lawesson's reagent (231.5 mg, 0.57 mmol) at 80°C. The reaction solution was stirred at 25°C for 15 min. The reaction solution was concentrated under reduced pressure, and the residue was purified by flash silica gel column chromatography (petroleum ether : ethyl acetate = 93:7) to afford compound **129e:** rac-1-(((1r,3r)-3-((tert-butyldiphenylsilyl)oxy)cyclobutyl)methyl)-4-(2,3-dichloro-6-((2-(trimethylsilyl)ethoxy)methoxy)phenyl)pyrrolidine-2-thione (320.0 mg, 78.2%).

MS m/z (ESI): 715.0 [M+H]⁺.

### Step f:

### rac-1-(((1r,3r)-3-((tert-Butyldiphenylsilyl)oxy)cyclobutyl)methyl)-4-(2,3-dichloro-6-hydroxyphenyl)pyrrolidine-2-thione

To a stirred solution of 1-(((1r,3r)-3-((tert-butyldiphenylsilyl)oxy)cyclobutyl) methyl)-4-(2,3-dichloro-6-((2-(trimethylsilyl)ethoxy)methoxy)phenyl)pyrrolidine-2-thione (400.0 mg, 0.57 mmol) in dichloromethane (5 mL) was added trifluoroacetic acid (1 mL) at 25°C, and the mixture was stirred at 25°C for 1 h. The resulting mixture was diluted with saturated sodium bicarbonate aqueous solution and extracted with dichloromethane (30 mL x 3). The combined organic layers were washed with sodium chloride aqueous solution and dried over anhydrous sodium sulphate. After filtration, the filtrate was concentrated under reduced pressure to afford crude **129f:** rac-1-(((1r,3r)-3-((tert-butyldiphenylsilyl)oxy)cyclobutyl)methyl)-4-(2,3-dichloro-6-hydroxyphenyl)pyrrolidine-2-thione (310.0 mg, 94.3%).

MS m/z (ESI): 584.4 [M+H]⁺.

### Step g:

### rac-4-(2,3-Dichloro-6-hydroxyphenyl)-1-(((1r,3r)-3-hydroxycyclobutyl)methyl)pyrrolidine-2-thione

To a stirred solution of 1-(((1r,3r)-3-((tert-butyldiphenylsilyl)oxy)cyclobutyl) methyl)-4-(2,3-dichloro-6-hydroxyphenyl)pyrrolidine-2-thione (350.0 mg, 0.50 mmol) in tetrahydrofuran (10 mL) was added tetrabutylammonium fluoride (392.8 mg, 1.50 mmol) at 25°C, and the mixture was stirred at 25°C for 3 h. The resulting mixture was diluted with water and extracted with ethyl acetate (30 mL x 3). The combined organic layers were washed with sodium chloride aqueous solution and dried over anhydrous sodium sulphate. After filtration, the filtrate was concentrated under reduced pressure, and the residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **129:** rac-4-(2,3-dichloro-6-hydroxyphenyl)-1-(((1r,3r)-3-hydroxycyclobutyl)methyl)pyrrolidine-2-thione (85.0 mg, 41.0%).
¹H NMR (400 MHz, DMSO*_d*₆) δ10.54 (s, 1H), 7.35 (d, 1H), 6.82 (d, 1H), 5.27-5.23 (m, 1H), 4.68 (t, 1H), 4.30-4.25 (m, 1H), 4.18 (t, 1H), 4.02-3.98 (m, 1H), 3.47 (t, 2H), 3.23-3.11 (m, 2H), 2.38-2.07 (m, 3H), 2.05 (t, 2H);
MS m/z (ESI): 345.9 [M+H]⁺.

Compound **129-1** was synthesised using intermediate 3 as the starting material and following the same synthetic route as compound **129.**

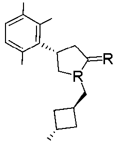

### Example 42

### Synthesis of compound 130 and compound 130-1

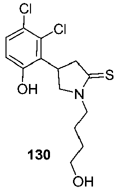

### rac-4-(2,3-Dichloro-6-hydroxyphenyl)-1-(4-hydroxybutyl)pyrrolidine-2-thione

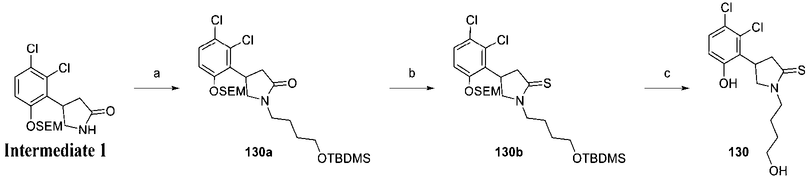

### Step a:

### rac-1-(4-((tert-Butyldimethylsilyl)oxy)butyl)-4-(2,3-dichloro-6-((2-(trimethylsilyl)ethoxy)methoxy)phenyl)pyrrolidin-2-one

To a stirred solution of intermediate 1 (225.0 mg, 0.60 mmol) in toluene (3 mL) were added tert-butyl (4-iodobutoxy)dimethylsilane (375.8 mg, 1.20 mmol), potassium hydroxide (100.6 mg, 1.79 mmol) and tetrabutylammonium iodide (44.2 mg, 0.20 mmol) at 20°C, and the reaction solution was stirred at 50°C for 16 h. After the reaction was completed, the reaction solution was concentrated under reduced pressure, and the residue was purified by flash silica gel column chromatography (petroleum ether : tetrahydrofuran = 4:1) to afford compound **130a:** rac-1-(4-((tert-butyldimethylsilyl)oxy)butyl)-4-(2,3-dichloro-6-((2-(trimethylsilyl)ethoxy)methoxy)phenyl)pyrrolidin-2-one (282.0 mg, 83.8%).
¹H NMR (400 MHz, DMSO_*d₆*) δ 7.52 (d, 1H), 7.14 (d, 1H), 5.30-5.24 (m, 2H), 4.32-4.26 (m, 1H), 3.69-3.57 (m, 5H), 3.48-3.42 (m, 2H), 3.30-3.19 (m, 1H), 2.57-2.54 (m, 2H), 1.52-1.45 (m, 4H), 0.90-0.87 (m, 2H), 0.86 (s, 9H), 0.03 (s, 6H), -0.03 (s, 9H);
MS m/z (ESI): 562.2 [M+H]⁺.

### Step b:

### rac-1-(4-((tert-Butyldimethylsilyl)oxy)butyl)-4-(2,3-dichloro-6-((2-(trimethylsilyl)ethoxy)methoxy)phenyl)pyrrolidine-2-thione

To a stirred solution of 1-(4-((tert-butyldimethylsilyl)oxy)butyl)-4-(2,3-dichloro-6-((2-(trimethylsilyl)ethoxy)methoxy)phenyl)pyrrolidin-2-one (250.0 mg, 0.44 mmol) in tetrahydrofuran (6 mL) was added Lawesson's reagent (107.8 mg, 0.27 mmol) at 80°C. The reaction solution was stirred at 25°C for 15 min. The reaction solution was concentrated under reduced pressure, and the residue was purified by flash silica gel column chromatography (petroleum ether : tetrahydrofuran = 5:1) to afford compound **130b:** rac-1-(4-((tert-butyldimethylsilyl)oxy)butyl)-4-(2,3-dichloro-6-((2-(trimethylsilyl)ethoxy)methoxy)phenyl)pyrrolidine-2-thione (205.0 mg, 79.7%).
¹H NMR (400 MHz, DMSO_*d₆*) 7.52 (d, 1H), 7.13 (d, 1H), 5.30-5.17 (m, 2H), 4.38-4.31 (m, 1H), 4.10-4.03 (m, 1H), 3.87-3.78 (m, 2H), 3.69-3.60 (m, 5H), 3.27-3.24 (m, 1H), 3.08-3.00 (m, 1H), 1.73-1.62 (m, 2H), 1.53-1.44 (m, 2H), 0.91-0.90 (m, 2H), 0.86 (s, 9H), 0.04 (s, 6H), -0.02 (s, 9H);
MS m/z (ESI): 578.2 [M+H]⁺.

### Step c:

### rac-4-(2,3-Dichloro-6-hydroxyphenyl)-1-(4-hydroxybutyl)pyrrolidine-2-thione

To a stirred solution of 1-(4-((tert-butyldimethylsilyl)oxy)butyl)-4-(2,3-dichloro-6-((2-(trimethylsilyl)ethoxy)methoxy)phenyl)pyrrolidine-2-thione (160.0 mg, 0.28 mmol) in dichloromethane (2 mL) was added hydrogen chloride dioxane solution (0.2 mL, 4 M) at 20°C, and the mixture was stirred at 20°C for 2 h. The reaction solution was concentrated under reduced pressure, and the residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **130:** rac-4-(2,3-dichloro-6-hydroxyphenyl)-1-(4-hydroxybutyl)pyrrolidine-2-thione (21.2 mg, 22.9%).
¹H NMR (400 MHz, DMSO_*d₆*) δ 10.52 (s, 1H), 7.35 (d, 1H), 6.83 (d, 1H), 4.42 (s, 1H), 4.31-4.22 (m, 1H), 4.00-3.91 (m, 2H), 3.88-3.81 (m, 1H), 3.64-3.57 (m, 1H), 3.44-3.41 (m, 2H), 3.17 (d, 2H), 1.69-1.62 (m, 2H), 1.48-1.41 (m, 2H);
MS m/z (ESI): 333.9 [M+H]⁺.

Compound **130-1** was synthesised using intermediate **3** as the starting material and following the same synthetic route as compound **130.**

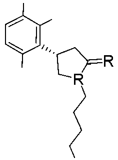

### Example 43

### Synthesis of compound 131

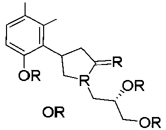

### rac-4-(2,3-Dichloro-6-hydroxyphenyl)-1-((S)-2,3-dihydroxypropyl) pyrrolidine-2-thione

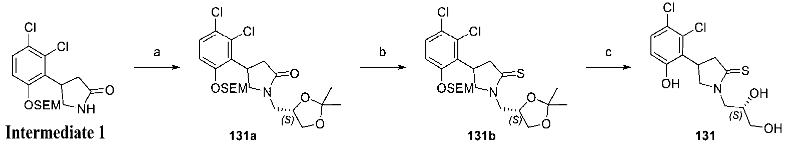

### Step a:

### rac-4-(2,3-Dichloro-6-((2-(trimethylsilyl)ethoxy)methoxy)phenyl)-1-(((S)-2,2-dimethyl-1,3-dioxolan-4-yl)methyl)pyrrolidin-2-one

To a stirred solution of intermediate 1 (50.0 mg, 0.13 mmol) in toluene (1 mL) were added (R)-4-iodomethyl-2,2-dimethyl-1,3-dioxolane (64.3 mg, 0.27 mmol), potassium hydroxide (29.8 mg, 0.53 mmol) and tetrabutylammonium iodide (9.8 mg, 0.03 mmol) at 20°C, and the reaction solution was stirred at 60°C for 16 h. After the reaction was completed, the resulting mixture was diluted with water and extracted with ethyl acetate (50 mL x 3). The combined organic layers were washed with sodium chloride aqueous solution and dried over anhydrous sodium sulphate. After filtration, the filtrate was concentrated under reduced pressure, and the residue was purified by flash silica gel column chromatography (petroleum ether : tetrahydrofuran = 2:1) to afford compound **131a:** rac-4-(2,3-dichloro-6-((2-(trimethylsilyl)ethoxy)methoxy)phenyl)-1-(((*S*)-2,2-dimethyl-1,3-dioxolan-4-yl)methyl)pyrrolidin-2-one (56.0 mg, 85.9%).
¹H NMR (400 MHz, DMSO_*d₆*) δ 7.52 (d, 1H), 7.14 (d, 1H), 5.31-5.27 (m, 2H), 4.29-4.20 (m, 2H), 4.02-3.98 (m, 1H), 3.75-3.61 (m, 4H), 3.59-3.41 (m, 2H), 3.38-3.36 (m, 1H), 2.61-2.55 (m, 2H), 1.25 (s, 6H), 0.91-0.85 (m, 2H), -0.03 (s, 9H);
MS m/z (ESI): 490.1 [M+H]⁺.

### Step b:

### rac-4-(2,3-Dichloro-6-((2-(trimethylsilyl)ethoxy)methoxy)phenyl)-1-(((S)-2,2-dimethyl-1,3-dioxolan-4-yl)methyl)pyrrolidine-2-thione

To a stirred solution of 4-(2,3-dichloro-6-((2-(trimethylsilyl)ethoxy)methoxy) phenyl)-1-(((S)-2,2-dimethyl-1,3-dioxolan-4-yl)methyl)pyrrolidin-2-one (56.0 mg, 0.11 mmol) in tetrahydrofuran (1.5 mL) was added Lawesson's reagent (32.3 mg, 0.08 mmol) at 20°C. The reaction solution was stirred at 80°C for 0.5 h. The resulting mixture was diluted with water and extracted with ethyl acetate (50 mL x 3). The combined organic layers were washed with sodium chloride aqueous solution and dried over anhydrous sodium sulphate. After filtration, the filtrate was concentrated under reduced pressure, and the residue was purified by flash silica gel column chromatography (petroleum ether : tetrahydrofuran = 5:1) to afford compound **131b:** rac-4-(2,3-dichloro-6-((2-(trimethylsilyl)ethoxy)methoxy)phenyl)-1-(((*S*)-2,2-dimethyl-1,3-dioxolan-4-yl)methyl)pyrrolidine-2-thione (30.0 mg, 51.9%).
¹H NMR (400 MHz, DMSO_*d₆*) δ 7.54 (d, 1H), 7.17-7.14 (m, 1H), 5.34-5.32 (m, 1H), 5.23-5.20 (m, 1H), 4.46-4.35 (m, 2H), 4.22-4.16(m, 1H), 4.08-3.68 (m, 7H), 3.34-3.31 (m, 1H), 3.12-3.06 (m, 1H), 1.37 (s, 3H), 1.28 (s, 3H), 0.93-0.85 (m, 2H), 0.00 (s, 9H);
MS m/z (ESI): 506.2 [M+H]⁺.

### Step c:

### rac-4-(2,3-Dichloro-6-hydroxyphenyl)-1-((S)-2,3-dihydroxypropyl) pyrrolidine-2-thione

To a stirred solution of 4-(2,3-dichloro-6-((2-(trimethylsilyl)ethoxy)methoxy) phenyl)-1-(((S)-2,2-dimethyl-1,3-dioxolan-4-yl)methyl)pyrrolidine-2-thione (125.0 mg, 0.25 mmol) in dichloromethane (2 mL) was added hydrogen chloride dioxane solution (0.2 mL) at 25°C, and the mixture was stirred at 25°C for 2 h. The reaction solution was concentrated under reduced pressure, and the residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **131:** rac-4-(2,3-dichloro-6-hydroxyphenyl)-1-((S)-2,3-dihydroxypropyl)pyrrolidine-2-thione (48.1 mg, 58.0%).
¹H NMR (400 MHz, DMSO_*d₆*) δ 10.51 (d, 1H), 7.35 (d, 1H), 6.83 (d, 1H), 4.97-4.86 (m, 1H), 4.64-4.59 (m, 1H), 4.32-4.22 (m, 1H), 4.16-3.86 (m, 4H), 3.66-3.46 (m, 1H), 3.38-3.32 (m, 2H), 3.26-3.12 (m, 2H);
MS m/z (ESI): 335.9 [M+H]⁺.

### Example 44

### Synthesis of compound 148

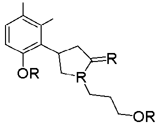

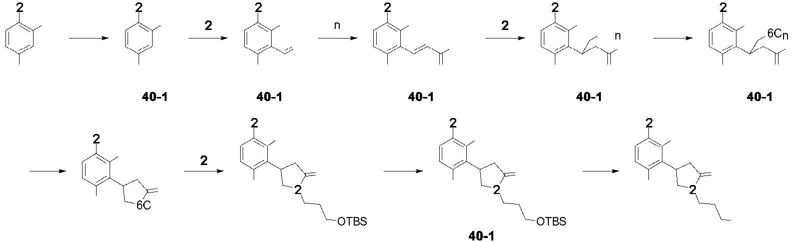

### Step a:

### (2-((3-Chloro-4-fluorophenoxy)methoxy)ethyl)trimethylsilane

3-Chloro-4-fluorophenol (4.00 g, 27.30 mmol) and triethylamine (5.52 g, 54.55 mmol) were added to a round-bottom flask, and dissolved in dichloromethane (40 mL). [2-(Chloromethoxy)ethyl]trimethylsilane (5.46 g, 32.75 mmol) was added portionwise at 0°C. The reaction mixture was stirred at 25°C for 2 hours. The reaction solution was diluted with 80 mL of saturated ammonium chloride solution and extracted with dichloromethane (100 mL x 3). The combined organic layers were washed with sodium chloride aqueous solution and dried over anhydrous sodium sulphate. After filtration, the filtrate was concentrated under reduced pressure. The residue was subjected to flash silica gel column chromatography (petroleum ether : ethyl acetate = 50:1) to afford compound **148a:** (2-((3-chloro-4-fluorophenoxy)methoxy)ethyl)trimethylsilane (4.27 g, 56.5%).

¹H NMR (300 MHz, CDCl₃): *δ* 7.10 (dd, 1H), 7.03 (t, 1H), 6.90-6.86 (m, 1H), 5.15 (s, 2H), 3.76-3.72 (m, 2H), 0.97-0.92 (m, 2H), 0.00 (s, 9H).

### Step b:

### 2-Chloro-3-fluoro-6-((2-(trimethylsilyl)ethoxy)methoxy)benzaldehyde

To a solution of compound **148a** (5.16 g, 18.64 mmol) in tetrahydrofuran (60 mL) was slowly added dropwise n-butyllithium (13.9 mL, 21.76 mmol, 1.6 M) at - 78°C under nitrogen protection. After the dropwise addition was completed, the reaction solution was stirred for another 1 hour, and N,N-dimethylformamide (3.40 g, 46.52 mmol) was added dropwise at -78°C, followed by further stirring for 2 hours. The reaction mixture was quenched with ice water (50 mL) and extracted with ethyl acetate (100 mL x 3). The combined organic layers were washed with sodium chloride aqueous solution and dried over anhydrous sodium sulphate. After filtration, the filtrate was concentrated under reduced pressure. The residue was purified by flash silica gel column chromatography (petroleum ether : ethyl acetate = 20:1) to afford compound **148b:** 2-chloro-3-fluoro-6-((2-(trimethylsilyl)ethoxy)methoxy) benzaldehyde (4.61 g, 81.1%).

¹HNMR (300 MHz, CDCl₃): *δ* 10.42 (s, 1H), 7.23-7.20 (m, 1H), 7.14-7.10 (m, 1H), 5.24 (s, 2H), 3.73 (t, 2H), 0.90 (t, *J =* 8.1 Hz, 2H), 0.045 (s, 9H).

### Step c:

### Ethyl (E)-3-(2-chloro-3-fluoro-6-((2-(trimethylsilyl)ethoxy)methoxy)phenyl) acrylate

Under nitrogen protection at 0°C, sodium hydride (0.72 g, 30.20 mmol, 60%) was added to a stirred solution of triethyl phosphonoacetate (5.08 g, 22.68 mmol) in tetrahydrofuran (160 mL), and the reaction solution was stirred at 0°C for 0.5 h, followed by the addition of compound **148b** (4.61 g, 15.12 mmol). The reaction solution was stirred at 25°C for 16 hours. The reaction mixture was quenched with water (50 mL) and extracted with ethyl acetate (50 mL x 3). The combined organic layers were washed with sodium chloride aqueous solution and dried over anhydrous sodium sulphate. After filtration, the filtrate was concentrated under reduced pressure. The residue was purified by flash silica gel column chromatography (petroleum ether : ethyl acetate = 20:1) to afford compound **148c:** ethyl (*E*)-3-(2-chloro-3-fluoro-6-((2-(trimethylsilyl)ethoxy)methoxy)phenyl)acrylate (4.50 g, 79.4%).

¹HNMR (300 MHz, CDCl₃): *δ* 7.94 (d, 1H), 7.11-7.06 (m, 2H), 6.85 (d, 1H), 5.24 (s, 2H), 4.28 (q, 2H), 3.74 (t, 2H), 1.34 (t, 3H), 0.94 (t, 2H), 0.010 (s, 9H).

### Step d:

### rac-Ethyl 3-(2-chloro-3-fluoro-6-((2-(trimethylsilyl)ethoxy)methoxy)phenyl)-4-nitrobutyrate

To a stirred solution of compound **148c** (4.50 g, 12.00 mmol) in nitromethane (50 mL) was added 1,8-diazabicyclo[5.4.0]undec-7-ene (2.19 g, 14.39 mmol) at 20°C under nitrogen protection. The reaction solution was stirred at 60°C for another 16 h, ice water (150 mL) was added, and the mixture was extracted with ethyl acetate (160 mL x 3). The combined organic layers were washed with sodium chloride aqueous solution and dried over anhydrous sodium sulphate. After filtration, the filtrate was concentrated under reduced pressure. The residue was purified by flash silica gel column chromatography (petroleum ether : ethyl acetate = 20:1-8:1) to afford compound **148d:** rac-ethyl 3-(2-chloro-3-fluoro-6-((2-(trimethylsilyl)ethoxy)methoxy)phenyl)-4-nitrobutyrate (4.20 g, 80.3%).

¹HNMR (400 MHz, CDCl₃): δ 7.13-7.14 (m, 2H), 5.29 (s, 2H), 4.97-4.87 (m, 3H), 4.18-4.11 (m, 2H), 3.85-3.79 (m, 2H), 2.93 (d, 2H), 1.24 (t, 3H), 1.04-0.99 (m, 2H), 0.064 (s, 9H).

### Step e:

### rac-Ethyl 4-amino-3-(2-chloro-3-fluoro-6-((2-(trimethylsilyl)ethoxy)methoxy) phenyl)butyrate

To a stirred solution of compound **148d** (4.20 g, 9.63 mmol) in acetic acid (20 mL) was added portionwise zinc powder (9.42 g, 144.06 mmol) under nitrogen protection. The reaction solution was stirred at 25°C for 5 h. The reaction mixture was filtered, the filter cake was washed with ethyl acetate (50 mL x 3), and the filtrate was concentrated under reduced pressure. The residue was purified by flash silica gel column chromatography (dichloromethane : methanol = 20:1-10:1) to afford compound **148e:** rac-ethyl 4-amino-3-(2-chloro-3-fluoro-6-((2-(trimethylsilyl)ethoxy)methoxy)phenyl)butyrate (3.80 g, 97.2%).

¹HNMR (300 MHz, CDCl₃): *δ* 7.07-7.00 (m, 2H), 5.25-5.21 (m, 2H), 4.91 (s, 4H), 4.08-4.01 (m, 2H), 3.78-3.66 (m, 2H), 3.21-3.19 (m, 2H), 2.86 (d, 1H), 1.26-1.13 (m, 3H), 0.98-0.91 (m, 2H), 0.00 (s, 9H).

### Step f:

### rac-4-(2,3-Dichloro-6-((2-(trimethylsilyl)ethoxy)methoxy)phenyl)pyrrolidin-2-one

Compound **148e** (3.80 g, 9.36 mmol) and potassium carbonate (3.90 g, 28.22 mmol) were dissolved in methanol (35 mL) and stirred. The reaction solution was stirred at 20°C for 2 h. The resulting mixture was diluted with water (30 mL) and extracted with ethyl acetate (50 mL x 3). The combined organic layers were washed with sodium chloride aqueous solution and dried over anhydrous sodium sulphate. After filtration, the filtrate was concentrated under reduced pressure. The residue was purified by flash silica gel column chromatography (dichloromethane : methanol = 20:1) to afford compound **148f:** rac-4-(2,3-dichloro-6-((2-(trimethylsilyl)ethoxy)methoxy)phenyl)pyrrolidin-2-one (2.20 g, 65.3%).

¹HNMR (400 MHz, CDCl₃): *δ* 7.10-6.97 (m, 2H), 6.36 (s, 1H), 5.23 (s, 2H), 4.48-4.42 (m, 1H), 3.75-3.70 (m, 2H), 3.67-3.56 (m, 2H), 2.82-2.74 (m, 1H), 2.62-2.53 (m, 1H), 0.96-0.91 (m, 2H), 0.00 (s, 9H).

### Step g:

### rac-1-(3-((tert-Butyldimethylsilyl)oxy)propyl)-4-(2-chloro-3-fluoro-6-((2-(trimethylsilyl)ethoxy)methoxy)phenyl)pyrrolidin-2-one

Under nitrogen protection at 25°C, potassium hydroxide (187.1 mg, 3.33 mmol), tetrabutylammonium iodide (135.5 mg, 0.37 mmol) and (3-iodopropoxy)(tert-butyl)dimethylsilane (667.4 mg, 2.22 mmol) were added to a stirred solution of compound **148f** (400.0 mg, 1.11 mmol) in toluene (5 mL), and the reaction solution was stirred at 80°C for 16 h. The resulting mixture was diluted with water and extracted with ethyl acetate (25 mL x 3). The combined organic layers were washed with sodium chloride aqueous solution and dried over anhydrous sodium sulphate. After filtration, the filtrate was concentrated under reduced pressure. The residue was purified by flash silica gel column chromatography (petroleum ether : ethyl acetate = 3:1) to afford compound **148g:** rac-1-(3-((tert-butyldimethylsilyl)oxy)propyl)-4-(2-chloro-3-fluoro-6-((2-(trimethylsilyl)ethoxy)methoxy)phenyl)pyrrolidin-2-one (400.0 mg, 67.6%).

¹H NMR (400 MHz, CDCl₃): δ 7.08 (dd, 1H), 6.99 (dd, 1H), 5.23-5.14 (m, 2H), 4.35-4.26 (m, 1H), 3.77-3.62 (m, 5H), 3.56 (dd, 1H), 3.50-3.29 (m,2H), 2.84-2.58 (m, 2H), 1.82-1.75 (m, 2H), 0.95-0.90 (m. 2H), 0.89 (s, 9H), 0.07 (s, 6H), 0.00 (s, 9H).

### Step h:

### rac-1-(3-((tert-Butyldimethylsilyl)oxy)propyl)-4-(2-chloro-3-fluoro-6-hydroxyphenyl)pyrrolidine-2-thione

To a stirred solution of compound **148g** (400.0 mg, 0.75 mmol) in tetrahydrofuran (20 mL) was added Lawesson's reagent (185.1 mg, 0.46 mmol) at 20°C. The reaction solution was stirred at 25°C for 15 min. The reaction solution was concentrated, and the residue was purified by flash silica gel column chromatography (petroleum ether : tetrahydrofuran = 5:1) to afford compound **148h:** rac-1-(3-((tert-butyldimethylsilyl)oxy)propyl)-4-(2-chloro-3-fluoro-6-hydroxyphenyl)pyrrolidine-2-thione (80.0 mg, 25.5%).

¹H NMR (400 MHz, CDCl₃): δ 7.09-7.06 (m, 1H), 7.02-6.95 (m, 1H), 5.21-5.13 (m, 2H), 4.41-4.31 (m, 1H), 3.94 (t, 1H), 3.97-3.80 (m, 3H), 3.72-3.64 (m, 4H), 3.40-3.21 (m, 2H), 2.00-1.82 (m, 2H), 0.95-0.88 (m, 2H), 0.89 (s, 9H), 0.07 (s, 6H), 0.00 (s, 9H).

### Step i:

### rac-4-(2-Chloro-3-fluoro-6-hydroxyphenyl)-1-(3-hydroxypropyl)pyrrolidine-2-thione

To a stirred solution of compound **148h** (50.0 mg, 0.12 mmol) in dichloromethane (3 mL) was added hydrogen chloride dioxane solution (1.0 mL, 6N) at 0°C. The reaction solution was stirred at 20°C for 3 h. The reaction solution was concentrated under reduced pressure, and the residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **148:** rac-4-(2-chloro-3-fluoro-6-hydroxyphenyl)-1-(3-hydroxypropyl)pyrrolidine-2-thione (11.6 mg, 31.9%).
¹H NMR (400 MHz, CD₃OD): δ 7.00-6.95 (m, 1H), 6.77-6.71(m, 1H), 4.40-4.30(m, 1H), 4.17-3.97 (m, 3H), 3.80-3.70 (m, 1H), 3.63 (t, 2H), 3.38-3.22 (m, 2H), 1.98-1.79 (m, 2H);
MS m/z (ESI): 304.0 [M+H]⁺.

### Example 45

### Synthesis of compound 149

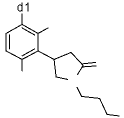

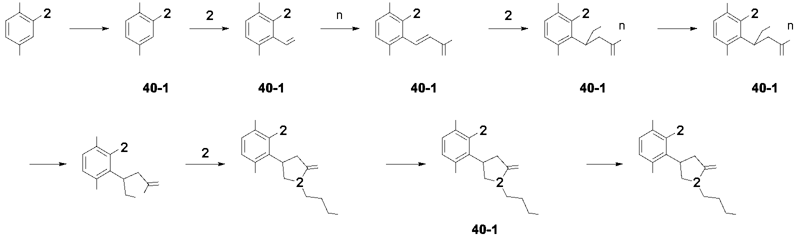

### Step a:

### (2-((4-Chloro-3-fluorophenoxy)methoxy)ethyl)trimethylsilane

4-Chloro-3-fluorophenol (5.00 g, 34.12 mmol) and triethylamine (10.33 g, 103.10 mmol) were added to a round-bottom flask, and dissolved in dichloromethane (100 mL). [2-(Chloromethoxy)ethyl]trimethylsilane (6.82 g, 40.91 mmol) was added portionwise at 0°C. The reaction mixture was stirred at 25°C for 5 hours. The reaction solution was diluted with 80 mL of saturated ammonium chloride solution and extracted with dichloromethane (100 mL x 3). The combined organic layers were washed with sodium chloride aqueous solution and dried over anhydrous sodium sulphate. After filtration, the filtrate was concentrated under reduced pressure. The residue was subjected to flash silica gel column chromatography (petroleum ether : ethyl acetate = 50:1) to afford compound **149a:** (2-((4-chloro-3-fluorophenoxy)methoxy)ethyl)trimethylsilane (8.40 g, 88.9%).

¹H NMR (400 MHz, CDCl₃): *δ* 7.28 (t, 1H), 6.90 (dd, 1H), 6.82-6.79 (m, 1H), 5.20 (s, 2H), 3.76 (t, 2H), 0.97 (t, 2H), 0.03 (s, 9H).

### Step b:

### 3-Chloro-2-fluoro-6-((2-(trimethylsilyl)ethoxy)methoxy)benzaldehyde

To a solution of compound **149a** (8.00 g, 28.90 mmol) in tetrahydrofuran (100 mL) was slowly added dropwise n-butyllithium (13.9 mL, 34.75 mmol, 2.5M) at -78°C under nitrogen protection. After the dropwise addition was completed, the reaction solution was stirred for another 1 hour, and N,N-dimethylformamide (5.28 g, 72.24 mmol) was added dropwise at -78°C, followed by further stirring for 4 hours. The reaction mixture was quenched with ice water (100 mL) and extracted with ethyl acetate (150 mL x 3). The combined organic layers were washed with sodium chloride aqueous solution and dried over anhydrous sodium sulphate. After filtration, the filtrate was concentrated under reduced pressure. The residue was purified by flash silica gel column chromatography (petroleum ether : ethyl acetate = 25:1) to afford compound **149b:** 3-chloro-2-fluoro-6-((2-(trimethylsilyl)ethoxy)methoxy) benzaldehyde (8.30 g, 94.2%).

¹HNMR (400 MHz, DMSO_*d*₆): *δ* 10.28 (d, 1H), 7.83 (d, 1H), 7.17 (dd, 1H), 5.41 (s, 2H), 3.75 (t, 2H), 0.87 (t, 2H), 0.00 (s, 9H).

### Step c:

### Ethyl (E)-3-(3-chloro-2-fluoro-6-((2-(trimethylsilyl)ethoxy)methoxy)phenyl) acrylate

Under nitrogen protection at 0°C, sodium hydride (1.97 g, 49.25 mmol, 60%) was added to a stirred solution of triethyl phosphonoacetate (8.27 g, 36.89 mmol) in tetrahydrofuran (150 mL), and the reaction solution was stirred at 0°C for 0.5 h, followed by the addition of compound **149b** (7.50 g, 24.60 mmol). The reaction solution was stirred at 25°C for 16 hours. The reaction mixture was quenched with water (100 mL) and extracted with ethyl acetate (200 mL x 3). The combined organic layers were washed with sodium chloride aqueous solution and dried over anhydrous sodium sulphate. After filtration, the filtrate was concentrated under reduced pressure. The residue was purified by flash silica gel column chromatography (petroleum ether : ethyl acetate = 20:1) to afford compound **149c:** ethyl (*E*)*-*3-(3-chloro-2-fluoro-6-((2-(trimethylsilyl)ethoxy)methoxy)phenyl)acrylate (4.40 g, 47.7%).

MS m/z (ESI): 375.0 [M+H]⁺.

### Step d:

### rac-Ethyl 3-(3-chloro-2-fluoro-6-((2-(trimethylsilyl)ethoxy)methoxy)phenyl)-4-nitrobutyrate

To a stirred solution of compound **149c** (4.40 g, 11.74 mmol) in nitromethane (30 mL) was added 1,8-diazabicyclo[5.4.0]undec-7-ene (1.96 g, 12.87 mmol) at 20°C under nitrogen protection. The reaction solution was stirred at 60°C for another 16 h, ice water (150 mL) was added, and the mixture was extracted with ethyl acetate (100 mL x 3). The combined organic layers were washed with sodium chloride aqueous solution and dried over anhydrous sodium sulphate. After filtration, the filtrate was concentrated under reduced pressure. The residue was purified by flash silica gel column chromatography (petroleum ether : ethyl acetate = 20:1) to afford compound **149d:** rac-ethyl 3-(3-chloro-2-fluoro-6-((2-(trimethylsilyl)ethoxy) methoxy)phenyl)-4-nitrobutyrate (2.40 g, 46.9%).

MS m/z (ESI): 436.0 [M+H]⁺.

### Step e:

### rac-Ethyl 4-amino-3-(3-chloro-2-fluoro-6-((2-(trimethylsilyl)ethoxy)methoxy) phenyl)butyrate

To a stirred solution of compound **149d** (2.40 g, 5.51 mmol) in acetic acid (40 mL) was added portionwise zinc powder (5.40 g, 82.58 mmol) under nitrogen protection. The reaction solution was stirred at 25°C for 6 h. The reaction mixture was filtered, the filter cake was washed with ethyl acetate (50 mL x 3), and the filtrate was concentrated under reduced pressure. The residue was purified by flash silica gel column chromatography (dichloromethane : methanol = 10:1) to afford compound **149e:** rac-ethyl 4-amino-3-(3-chloro-2-fluoro-6-((2-(trimethylsilyl)ethoxy)methoxy)phenyl)butyrate (1.70 g, 76.1%).

MS m/z (ESI): 406.3 [M+H]⁺.

### Step f:

### rac-4-(3-chloro-2-fluoro-6-((2-(trimethylsilyl)ethoxy)methoxy)phenyl) pyrrolidin-2-one

Compound **149e** (1.60 g, 3.94 mmol) and potassium carbonate (1.62 g, 11.72 mmol) were dissolved in methanol (30 mL) and stirred. The reaction solution was stirred at 20°C for 2 h. The resulting mixture was diluted with water (50 mL) and extracted with ethyl acetate (50 mL x 3). The combined organic layers were washed with sodium chloride aqueous solution and dried over anhydrous sodium sulphate. After filtration, the filtrate was concentrated under reduced pressure. The residue was purified by flash silica gel column chromatography (dichloromethane : methanol = 50:1) to afford compound **149f:** rac-4-(3-chloro-2-fluoro-6-((2-(trimethylsilyl)ethoxy)methoxy)phenyl)pyrrolidin-2-one (1.20 g, 84.6%).
¹H NMR (400 MHz, DMSO_*d*₆): δ 7.75 (s, 1H), 7.42 (t, 1H), 6.98 (d, 1H), 5.30 (s, 2H), 4.11-4.02 (m, 1H), 3.70 (t, 2H), 3.50 (t, 1H), 3.17 (d, 1H), 2.42 (d, 2H), 0.88 (t, 2H), 0.00 (s, 9H);
MS m/z (ESI): 360.0 [M+H]⁺.

### Step g:

### rac-1-(3-((tert-Butyldimethylsilyl)oxy)propyl)-4-(3-chloro-2-fluoro-6-((2-(trimethylsilyl)ethoxy)methoxy)phenyl)pyrrolidin-2-one

Under nitrogen protection at 25°C, potassium hydroxide (46.8 mg, 0.83 mmol), tetrabutylammonium iodide (33.9 mg, 0.092 mmol) and (3-iodopropoxy)(tert-butyl)dimethylsilane (166.9 mg, 0.56 mmol) were added to a stirred solution of compound **149f** (100.0 mg, 0.27 mmol) in toluene (5 mL), and the reaction solution was stirred at 40°C for 16 h. The resulting mixture was diluted with water and extracted with ethyl acetate (25 mL x 3). The combined organic layers were washed with sodium chloride aqueous solution and dried over anhydrous sodium sulphate. After filtration, the filtrate was concentrated under reduced pressure. The residue was purified by flash silica gel column chromatography (petroleum ether : ethyl acetate = 4:1) to afford compound **149g:** rac-1-(3-((tert-butyldimethylsilyl)oxy)propyl)-4-(3-chloro-2-fluoro-6-((2-(trimethylsilyl)ethoxy)methoxy)phenyl)pyrrolidin-2-one (113.3 mg, 80.1%).

MS m/z (ESI): 532.5 [M+H]⁺.

### Step h:

### rac-1-(3-((tert-Butyldimethylsilyl)oxy)propyl)-4-(3-chloro-2-fluoro-6-((2-(trimethylsilyl)ethoxy)methoxy)phenyl)pyrrolidine-2-thione

To a stirred solution of compound **149g** (90.0 mg, 0.17 mmol) in tetrahydrofuran (5 mL) was added Lawesson's reagent (136.8 mg, 0.34 mmol) at 20°C. The reaction solution was stirred at 25°C for 15 min. The reaction solution was concentrated, and the residue was purified by flash silica gel column chromatography (petroleum ether : ethyl acetate = 20:1) to afford compound **149h:** rac-1-(3-((tert-butyldimethylsilyl)oxy)propyl)-4-(3-chloro-2-fluoro-6-((2-(trimethylsilyl)ethoxy)methoxy)phenyl)pyrrolidine-2-thione (74.2 mg, 80.0%).

MS m/z (ESI): 548.5 [M+H]⁺.

### Step i:

### rac-4-(3-Chloro-2-fluoro-6-hydroxyphenyl)-1-(3-hydroxypropyl)pyrrolidine-2-thione

To a stirred solution of compound **149h** (37.0 mg, 0.067 mmol) in dichloromethane (3 mL) was added hydrogen chloride dioxane solution (2.0 mL, 6N) at 0°C. The reaction solution was stirred at 20°C for 3 h. The reaction solution was concentrated under reduced pressure, and the residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **149:** rac-4-(3-chloro-2-fluoro-6-hydroxyphenyl)-1-(3-hydroxypropyl)pyrrolidine-2-thione (5.6 mg, 27.3%).
¹H NMR (400 MHz, CD₃OD): δ 7.15 (t, 1H), 6.62 (dd, 1H), 4.14-4.08 (m, 1H), 4.04-3.96 (m, 3H), 3.82-3.77 (m, 1H), 3.63 (t, 2H), 3.30-3.27 (m, 2H), 1.96-1.89 (m, 2H);
MS m/z (ESI): 304.0 [M+H]⁺.

### Example 46

### Synthesis of compound 150 and compound 150-1

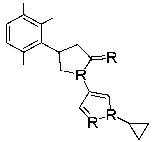

### rac-1-(1-Cyclopropyl-1H-pyrazol-4-yl)-4-(2,3-dichloro-6-hydroxyphenyl) pyrrolidine-2-thione

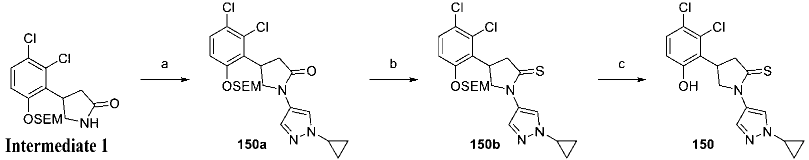

### Step a:

### 1-(1-Cyclopropyl-1H-pyrazol-4-yl)-4-(2,3-dichloro-6-((2-(trimethylsilyl)ethoxy)methoxy)phenyl)pyrrolidin-2-one

Under nitrogen protection at 20°C, 4-bromo-1-cyclopropylpyrazole (186.4 mg, 1.00 mmol) and caesium carbonate (432.9 mg, 1.33 mmol) were added to a stirred solution of intermediate 1 (250.0 mg, 0.66 mmol) in acetonitrile/*N,N-*dimethylformamide (4 mL, v/v = 1:1), and the reaction solution was stirred at 20°C for 20 min. Cuprous iodide (126.5 mg, 0.66 mmol) and *N,N-*dimethylethylenediamine (58.6 mg, 0.66 mmol) were added to the reaction solution, and the reaction solution was stirred at 85°C for 2 h. After the reaction was completed, the reaction solution was concentrated under reduced pressure, and the residue was purified by flash silica gel column chromatography (petroleum ether : ethyl acetate = 3:1) to afford compound **150a:** rac-1-(1-cyclopropyl-1H-pyrazol-4-yl)-4-(2,3-dichloro-6-((2-(trimethylsilyl)ethoxy)methoxy)phenyl)pyrrolidin-2-one (290.0 mg, 90.5%).
¹H NMR (400 MHz, DMSO_*d₆*) δ 8.00 (s, 1H), 7.62 (s, 1H), 7.53 (d, 1H), 7.12 (d, 1H), 5.23-5.17 (m, 2H), 4.49-4.42 (m, 1H), 4.03-3.99 (m, 1H), 3.72-3.63 (m, 2H), 3.55-3.46 (m, 2H), 2.84-2.78 (m, 1H), 2.67-2.59 (m, 1H), 0.99-0.93 (m, 4H), 0.79-0.75 (m, 2H), -0.10 (s, 9H);
MS m/z (ESI): 482.0 [M+H]⁺.

### Step b:

### rac-1-(1-Cyclopropyl-1H-pyrazol-4-yl)-4-(2,3-dichloro-6-((2-(trimethylsilyl)ethoxy)methoxy)phenyl)pyrrolidine-2-thione

To a stirred solution of 1-(4-((tert-butyldimethylsilyl)oxy)butyl)-4-(2,3-dichloro-6-((2-(trimethylsilyl)ethoxy)methoxy)phenyl)pyrrolidin-2-one (50.0 mg, 0.10 mmol) in tetrahydrofuran (1.5 mL) was added Lawesson's reagent (50.3 mg, 0.12 mmol) at 20°C. The reaction solution was stirred at 20°C for 16 h. The reaction solution was concentrated under reduced pressure, and the residue was purified by flash silica gel column chromatography (petroleum ether : ethyl acetate = 4:1) to afford compound **150b:** rac-1-(1-cyclopropyl-1H-pyrazol-4-yl)-4-(2,3-dichloro-6-((2-(trimethylsilyl)ethoxy)methoxy)phenyl)pyrrolidine-2-thione (50.0 mg, 96.8%).
¹H NMR (400 MHz, DMSO_*d₆*) *δ* 8.79 (s, 1H), 8.07 (s, 1H), 7.54 (d, 1H), 7.11 (d, 1H), 5.20-5.14 (m, 2H), 4.54-4.49 (m, 2H), 4.15-4.11 (m, 1H), 3.79-3.74 (m, 1H), 3.54-3.45 (m, 3H), 3.18-3.12 (m, 1H), 1.02-0.96 (m, 4H), 0.80-075 (m, 2H), -0.09 (s, 9H);
MS m/z (ESI): 498.0 [M+H]⁺.

### Step c:

### rac-1-(1-Cyclopropyl-1H-pyrazol-4-yl)-4-(2,3-dichloro-6-hydroxyphenyl)pyrrolidine-2-thione

To a stirred solution of 1-(4-((tert-butyldimethylsilyl)oxy)butyl)-4-(2,3-dichloro-6-((2-(trimethylsilyl)ethoxy)methoxy)phenyl)pyrrolidine-2-thione (100.0 mg, 0.20 mmol) in dichloromethane (3 mL) was added hydrogen chloride dioxane solution (0.3 mL, 4 M) at 20°C, and the mixture was stirred at 20°C for 1 h. The reaction solution was concentrated under reduced pressure, and the residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **150:** rac-1-(1-cyclopropyl-1H-pyrazol-4-yl)-4-(2,3-dichloro-6-hydroxyphenyl)pyrrolidine-2-thione (29.0 mg, 39.3%).
¹H NMR (400 MHz, DMSO_*d₆*) δ 10.58 (s, 1H), 8.79 (s, 1H), 8.11 (s, 1H), 7.37 (d, 1H), 6.83 (d, 1H), 4.52-4.41 (m, 2H), 4.38-4.24 (m, 1H), 3.82-3.74 (m, 1H), 3.43-3.25 (m, 2H), 1.04-0.94 (m, 4H);
MS m/z (ESI): 367.9 [M+H]⁺.

Compound **150-1** was synthesised using intermediate **3** as the starting material and following the same synthetic route as compound **150.**

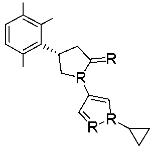

### Example 47

### Synthesis of compound 159 and compound 159-1

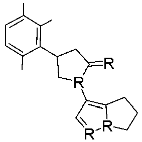

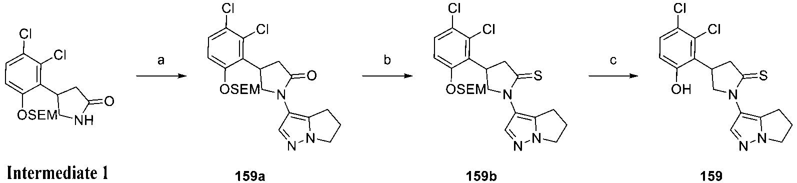

### Step a:

### rac-4-(2,3-Dichloro-6-((2-(trimethylsilyl)ethoxy)methoxy)phenyl)-1-(5,6-dihydro-4H-pyrrolo[1,2-b]pyrazol-3-yl)pyrrolidin-2-one

Under nitrogen protection at 20°C, 3-bromo-5,6-dihydro-4H-pyrrolo[1,2-b]pyrazole (372.8 mg, 1.99 mmol) and caesium carbonate (865.8 mg, 2.66 mmol) were added to a stirred solution of intermediate 1 (500.0 mg, 1.33 mmol) in acetonitrile/N,N-dimethylformamide (20 mL, v/v = 1:1), and the reaction solution was stirred at 30°C for 15 min. Cuprous iodide (375.6 mg, 1.99 mmol) and *N,N-*dimethylethylenediamine (585.6 mg, 6.64 mmol) were added to the reaction solution, and the reaction solution was stirred at 85°C for 2 h. After the reaction was completed, the reaction solution was concentrated under reduced pressure, and the residue was purified by flash silica gel column chromatography (petroleum ether : tetrahydrofuran = 10:3) to afford compound **159a:** rac-4-(2,3-dichloro-6-((2-(trimethylsilyl)ethoxy)methoxy)phenyl)-1-(5,6-dihydro-4H-pyrrolo[1,2-b]pyrazol-3-yl)pyrrolidin-2-one (620.0 mg, 96.7%).
¹H NMR (400 MHz, CDCl₃) δ 7.58 (s, 1H), 7.32 (d, 1H), 7.08 (d, 1H), 5.21-5.18 (m, 2H), 4.56-4.51 (m, 1H), 4.13 (t, 2H), 3.99 (t, 1H), 3.88-3.84 (m, 1H), 3.67-3.62 (m, 2H), 3.19-3.04 (m, 2H), 2.86 (d, 2H), 2.66-2.58 (m, 2H), 0.95-0.89 (m, 2H), 0.00 (s, 9H);
MS m/z (ESI): 482.1 [M+H]⁺.

### Step b:

### rac-4-(2,3-Dichloro-6-((2-(trimethylsilyl)ethoxy)methoxy)phenyl)-1-(5,6-dihydro-4H-pyrrolo[1,2-b]pyrazol-3-yl) 2-pyrrolidinethione

To a stirred solution of compound **159a** (620.0 mg, 1.24 mmol) in tetrahydrofuran (20 mL) was added Lawesson's reagent (779.6 mg, 1.93 mmol) at 20°C. The reaction solution was stirred at 80°C for 30 min. The reaction solution was cooled to room temperature, diluted with 30 mL of water, and extracted with ethyl acetate (30 mL x 3). The combined organic layers were washed with sodium chloride aqueous solution and dried over anhydrous sodium sulphate. After filtration, the reaction solution was concentrated under reduced pressure, and the residue was purified by flash silica gel column chromatography (petroleum ether : tetrahydrofuran = 4:1) to afford compound **159b:** rac-4-(2,3-dichloro-6-((2-(trimethylsilyl)ethoxy)methoxy)phenyl)-1-(5,6-dihydro-4H-pyrrolo[1,2-b]pyrazol-3-yl) 2-pyrrolidinethione (390.0 mg, 60.9%).
¹H NMR (400 MHz, CDCl₃) δ 7.89 (s, 1H), 7.33 (d, 1H), 7.09 (d, 1H), 5.27-5.18 (m, 2H), 4.59-4.52 (m, 1H), 4.29 (t, 1H), 4.23-4.14 (m, 3H), 3.72-3.68 (m, 2H), 3.56-3.48 (m, 1H), 3.39-3.33 (m, 1H), 3.17-2.99 (m, 2H), 2.66-2.59 (m, 2H), 0.93-0.88 (m, 2H), 0.00 (s, 9H);
MS m/z (ESI): 498.1 [M+H]⁺.

### Step c:

### rac-4-(2,3-Dichloro-6-hydroxyphenyl)-1-(5,6-dihydro-4H-pyrrolo[1,2-b]pyrazol-3-yl)pyrrolidine-2-thione

To a stirred solution of compound **159b** (200.0 mg, 0.40 mmol) in dichloromethane (3 mL) was added dioxane/hydrogen chloride solution (3 mL, 6 N) at 20°C, and the mixture was stirred at 30°C for 1 h. The reaction solution was adjusted to pH = 7 with saturated sodium bicarbonate and extracted with ethyl acetate (10 mL x 3). The combined organic layers were washed with sodium chloride aqueous solution and dried over anhydrous sodium sulphate. After filtration, the reaction solution was concentrated under reduced pressure, and the residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **159:** rac-4-(2,3-dichloro-6-hydroxyphenyl)-1-(5,6-dihydro-4H-pyrrolo[1,2-b]pyrazol-3-yl)pyrrolidine-2-thione (117.7 mg, 79.7%).
¹H NMR (400 MHz, DMSO_*d₆*) δ 10.57(s, 1H), 8.09 (s, 1H), 7.35(d, 1H), 6.84 (d, 1H), 4.45-4.36 (m, 2H), 4.24-4.20 (m, 1H), 4.09 (t, 2H), 3.39-3.20 (m, 2H), 3.00-2.96 (m, 2H), 2.57-2.53 (m, 2H);
MS m/z (ESI): 368.0 [M+H]⁺.

Compound **159-1** was synthesised using intermediate **3** as the starting material and following the same synthetic route as compound **159.**

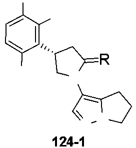

### Example 48

### Synthesis of compound 160

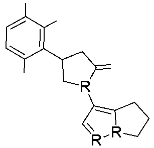

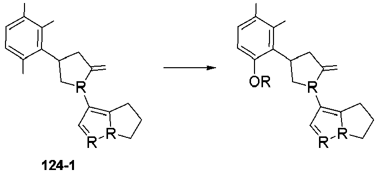

### Step a:

### rac-4-(2,3-Dichloro-6-hydroxyphenyl)-1-(5,6-dihydro-4H-pyrrolo[1,2-b]pyrazol-3-yl)pyrrolidin-2-one

To a stirred solution of compound **159a** (80.0 mg, 0.17 mmol) in dichloromethane (2 mL) was added dioxane/hydrogen chloride solution (0.5 mL, 6 N) at 0°C, and the mixture was stirred at 25°C for 1 h. The reaction solution was adjusted to pH = 7 with saturated sodium bicarbonate and extracted with ethyl acetate (10 mL x 3). The combined organic layers were washed with sodium chloride aqueous solution and dried over anhydrous sodium sulphate. After filtration, the reaction solution was concentrated under reduced pressure, and the residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **160:** rac-4-(2,3-dichloro-6-hydroxyphenyl)-1-(5,6-dihydro-4H-pyrrolo[1,2-b]pyrazol-3-yl)pyrrolidin-2-one (27.0 mg, 46.2%).
¹H NMR (400 MHz, DMSO_*d₆*) δ 10.47(brs, 1H), 7.67 (s, 1H), 7.35(d, 1H), 6.84 (d, 1H), 4.41-4.32 (m, 1H), 4.05-3.95 (m, 3H), 3.83-3.80 (m, 1H), 3.07-2.92 (m, 2H), 2.73-2.64 (m, 2H), 2.50-2.45 (m, 2H);
MS m/z (ESI): 352.0 [M+H]⁺.

### Example 49

### Synthesis of compound 161 and compound 161-1

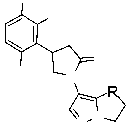

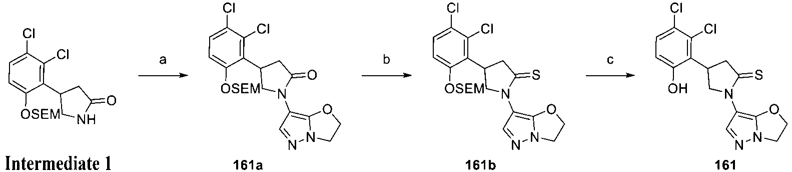

### Step a:

### rac-4-(2,3-Dichloro-6-((2-(trimethylsilyl)ethoxy)methoxy)phenyl)-1-(2,3-dihydropyrazolo[5,1-b]oxazol-7-yl)pyrrolidin-2-one

Under nitrogen protection at 20°C, 7-iodo-2,3-dihydropyrazolo[5,1-b]oxazole (600.0 mg, 2.54 mmol) and caesium carbonate (1656.7 mg, 5.08 mmol) were added to a stirred solution of intermediate 1 (956.8 mg, 2.54 mmol) in acetonitrile/*N,N*-dimethylformamide (20 mL, v/v = 1:1), and the reaction solution was stirred at 85°C for 20 min. Cuprous iodide (484.2 mg, 2.54 mmol) and *N,N-*dimethylethylenediamine (224.1 mg, 2.54 mmol) were added to the reaction solution, and the reaction solution was stirred at 85°C for 1.5 h. After the reaction was completed, the reaction solution was concentrated under reduced pressure, and the residue was purified by flash silica gel column chromatography (petroleum ether : tetrahydrofuran = 2:1) to afford compound **161a:** rac-4-(2,3-dichloro-6-((2-(trimethylsilyl)ethoxy)methoxy)phenyl)-1-(2,3-dihydropyrazolo[5,1-b]oxazol-7-yl)pyrrolidin-2-one (900.0 mg, 73.1%).
¹H NMR (400 MHz, DMSO_*d₆*) δ 7.57 (s, 1H), 7.53 (d, 1H), 7.14 (d, 1H), 5.27 (s, 2H), 5.09-5.04 (m, 2H), 4.44-4.39 (m, 1H), 4.28-4.24 (m, 2H), 3.98-3.93 (m, 1H), 3.67-3.56 (m, 3H), 2.78-2.71 (m, 1H), 2.56-2.54 (m, 1H), 0.86-0.82 (m, 2H), -0.07 (s, 9H);
MS m/z (ESI): 484.1 [M+H]⁺.

### Step b:

### rac-4-(2,3-Dichloro-6-((2-(trimethylsilyl)ethoxy)methoxy)phenyl)-1-(2,3-dihydropyrazolo[5,1-b]oxazol-7-yl)pyrrolidine-2-thione

To a stirred solution of compound **161a** (200.0 mg, 0.41 mmol) in tetrahydrofuran (8 mL) was added Lawesson's reagent (167.0 mg, 0.41 mmol) at 20°C. The reaction solution was stirred at 25°C for 1 h. The reaction solution was diluted with 20 mL of water and extracted with ethyl acetate (20 mL x 3). The combined organic layers were washed with sodium chloride aqueous solution and dried over anhydrous sodium sulphate. After filtration, the reaction solution was concentrated under reduced pressure, and the residue was purified by flash silica gel column chromatography (petroleum ether : tetrahydrofuran = 2:1) to afford compound **161b:** rac-4-(2,3-dichloro-6-((2-(trimethylsilyl)ethoxy)methoxy) phenyl)-1-(2,3-dihydropyrazolo[5,1-b]oxazol-7-yl)pyrrolidine-2-thione (88.0 mg, 42.6%).
¹H NMR (400 MHz, CDCl₃) δ 8.05 (s, 1H), 7.25 (d, 1H), 7.02 (d, 1H), 5.19 (d, 1H), 5.13 (d, 1H), 5.06 (t, 2H), 4.50-4.43 (m, 1H), 4.31-4.23 (m, 3H), 4.09-4.05 (m, 1H), 3.64 (t, 2H), 3.46-3.39 (m, 1H), 3.29-3.22 (m, 1H), 0.86-0.81 (m, 2H), -0.08 (s, 9H);
MS m/z (ESI): 500.1 [M+H]⁺.

### Step c:

### rac-4-(2,3-Dichloro-6-hydroxyphenyl)-1-(2,3-dihydropyrazolo[5,1-b]oxazol-7-yl)pyrrolidine-2-thione

To a stirred solution of compound **161b** (260.0 mg, 0.52 mmol) in dichloromethane (6 mL) was added dioxane/hydrogen chloride solution (3 mL, 6 N) at 0°C, and the mixture was stirred at 0°C for 1 h. The reaction solution was adjusted to pH = 7 with saturated sodium bicarbonate and extracted with dichloromethane (15 mL x 3). The combined organic layers were washed with sodium chloride aqueous solution and dried over anhydrous sodium sulphate. After filtration, the reaction solution was concentrated under reduced pressure, and the residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **161:** rac-4-(2,3-dichloro-6-hydroxyphenyl)-1-(2,3-dihydropyrazolo[5,1-b]oxazol-7-yl)pyrrolidine-2-thione (62.3 mg, 32.4%).
¹H NMR (400 MHz, DMSO_*d₆*) δ 10.58 (s, 1H), 7.97 (s, 1H), 7.36 (d, 1H), 6.84 (d, 1H), 5.14 (t, 2H), 4.46-4.37 (m, 1H), 4.33-4.24 (m, 3H), 4.21-4.16 (m, 1H), 3.34-3.18 (m, 2H);
MS m/z (ESI): 370.0 [M+H]⁺.

Compound **161-1** was synthesised using intermediate **3** as the starting material and following the same synthetic route as compound **161.**

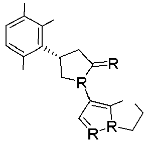

### Example 50

### Synthesis of compound 162 and compound 162-1

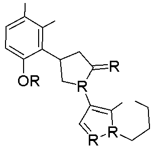

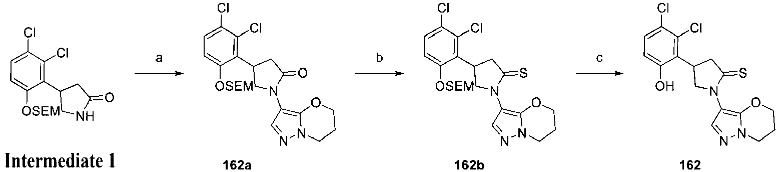

### Step a:

### rac-4-(2,3-Dichloro-6-((2-(trimethylsilyl)ethoxy)methoxy)phenyl)-1-(6,7-dihydro-5H-pyrazolo[5,1-b][1,3]oxazin-3-yl)pyrrolidin-2-one

Under nitrogen protection at 20°C, 3-iodo-6,7-dihydro-5H-pyrazolo[5,1-b][1,3]oxazine (100.0 mg, 0.40 mmol) and caesium carbonate (260.6 mg, 0.80 mmol) were added to a stirred solution of intermediate 1 (180.6 mg, 0.48 mmol) in acetonitrile/*N,N*-dimethylformamide (4 mL, v/v = 1:1), and the reaction solution was stirred at 25°C for 20 min. Cuprous iodide (76.2 mg, 0.40 mmol) and *N,N-*dimethylethylenediamine (35.3 mg, 0.40 mmol) were added to the reaction solution, and the reaction solution was stirred at 85°C for 1 h. After the reaction was completed, the reaction solution was concentrated under reduced pressure, and the residue was purified by flash silica gel column chromatography (petroleum ether : tetrahydrofuran = 2:1) to afford compound **162a:** rac-4-(2,3-dichloro-6-((2-(trimethylsilyl)ethoxy)methoxy)phenyl)-1-(6,7-dihydro-5H-pyrazolo[5,1-b][1,3]oxazin-3-yl)pyrrolidin-2-one (165.0 mg, 82.8%).
¹H NMR (400 MHz, CDCl₃) δ 7.65 (s, 1H), 7.33 (d, 1H), 7.12 (d, 1H), 5.29-5.24 (m, 2H), 4.56-4.47 (m, 1H), 4.32 (t, 2H), 4.18 (t, 2H), 4.02 (t, 1H), 3.83 (dd, *J* = 9.2, 6.8 Hz, 1H), 3.75-3.70 (m, 2H), 2.85-2.82 (m, 2H), 2.31-2.25 (m, 2H), 0.95-0.91 (m, 2H), 0.00 (s, 9H);
MS m/z (ESI): 498.1 [M+H]⁺.

### Step b:

### rac-4-(2,3-Dichloro-6-((2-(trimethylsilyl)ethoxy)methoxy)phenyl)-1-(6,7-dihydro-5H-pyrazolo[5,1-b][1,3]oxazin-3-yl)pyrrolidine-2-thione

To a stirred solution of compound **162a** (50.0 mg, 0.10 mmol) in tetrahydrofuran (2 mL) was added Lawesson's reagent (40.6 mg, 0.10 mmol) at 20°C. The reaction solution was stirred at 75°C for 5 min. The reaction solution was diluted with 10 mL of water and extracted with ethyl acetate (10 mL x 3). The combined organic layers were washed with sodium chloride aqueous solution and dried over anhydrous sodium sulphate. After filtration, the reaction solution was concentrated under reduced pressure, and the residue was purified by flash silica gel column chromatography (petroleum ether : tetrahydrofuran = 2:1) to afford compound **162b:** rac-4-(2,3-dichloro-6-((2-(trimethylsilyl)ethoxy)methoxy)phenyl)-1-(6,7-dihydro-5H-pyrazolo[5,1-b][1,3]oxazin-3-yl)pyrrolidine-2-thione (47.0 mg, 91.1%).
¹H NMR (400 MHz, CDCl₃) δ 7.89 (s, 1H), 7.31 (d, 1H), 7.09 (d, 1H), 5.27-5.20 (m, 2H), 4.55-4.48 (m, 1H), 4.36-4.31 (m, 3H), 4.18 (t, 2H), 4.10 (dd, 1H), 3.74-3.69 (m, 2H), 3.49 (dd, 1H), 3.34 (dd, 1H), 2.31-2.25 (m, 2H), 0.93-0.88 (m, 2H), -0.01 (s, 9H);
MS m/z (ESI): 514.1 [M+H]⁺.

### Step c:

### rac-4-(2,3-Dichloro-6-hydroxyphenyl)-1-(6,7-dihydro-5H-pyrazolo[5,1-b][1,3]oxazin-3-yl)pyrrolidine-2-thione

To a stirred solution of compound **162b** (220.0 mg, 0.43 mmol) in dichloromethane (8 mL) was added dioxane/hydrogen chloride solution (1 mL, 6 N) at 0°C, and the mixture was stirred at 0°C for 2 h. The reaction solution was adjusted to pH = 7 with saturated sodium bicarbonate and extracted with dichloromethane (15 mL x 3). The combined organic layers were washed with sodium chloride aqueous solution and dried over anhydrous sodium sulphate. After filtration, the reaction solution was concentrated under reduced pressure, and the residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **162:** rac-4-(2,3-dichloro-6-hydroxyphenyl)-1-(6,7-dihydro-5H-pyrazolo[5,1-b][1,3]oxazin-3-yl)pyrrolidine-2-thione (40.9 mg, 24.9%).
¹H NMR (400 MHz, DMSO*_d₆*) δ 10.60 (s, 1H), 7.73 (s, 1H), 7.36 (d, 1H), 6.84 (d, 1H), 4.44-4.37 (m, 1H), 4.33 (t, 2H), 4.20 (d, 2H), 4.09 (t, 2H), 3.27 (d, 2H), 2.22-2.16 (m, 2H);
MS m/z (ESI): 384.0 [M+H]⁺.

Compound **162-1** was synthesised using intermediate 3 as the starting material and following the same synthetic route as compound **162.**

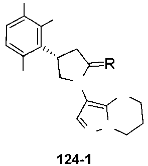

### Example 51

### Synthesis of compound 163 and compound 163-1

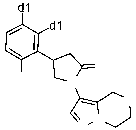

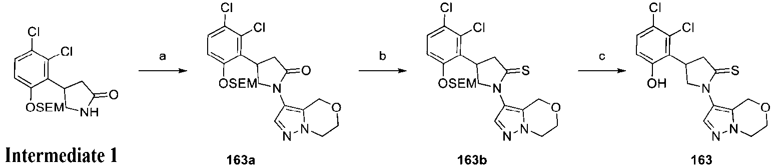

### Step a:

### rac-4-(2,3-Dichloro-6-((2-(trimethylsilyl)ethoxy)methoxy)phenyl)-1-(6,7-dihydro-4H-pyrazolo[5,1-c][1,4]oxazin-3-yl)pyrrolidin-2-one

Under nitrogen protection at 20°C, 3-bromo-6,7-dihydro-4H-pyrazolo[5,1-c][1,4]oxazine (404.6 mg, 1.99 mmol) and caesium carbonate (865.8 mg, 2.66 mmol) were added to a stirred solution of intermediate 1 (500.0 mg, 1.33 mmol) in acetonitrile/N,N-dimethylformamide (10 mL, v/v = 1:1), and the reaction solution was stirred at 25°C for 20 min. Cuprous iodide (253.0 mg, 1.33 mmol) and *N,N-*dimethylethylenediamine (117.1 mg, 1.33 mmol) were added to the reaction solution, and the reaction solution was stirred at 85°C for 4 h. After the reaction was completed, the reaction solution was concentrated under reduced pressure, and the residue was purified by flash silica gel column chromatography (petroleum ether : ethyl acetate = 3:2) to afford compound **163a:** rac-4-(2,3-dichloro-6-((2-(trimethylsilyl)ethoxy)methoxy)phenyl)-1-(6,7-dihydro-4H-pyrazolo[5,1-c][1,4]oxazin-3-yl)pyrrolidin-2-one (590.0 mg, 89.1%).
¹H NMR (400 MHz, CDCl₃) δ 7.43 (s, 1H), 7.34 (d, 1H), 7.09 (d, 1H), 5.24-5.20 (m, 2H), 4.97-4.87 (m, 2H), 4.58-4.53 (m, 1H), 4.21-4.09 (m, 4H), 3.99-3.89 (m, 2H), 3.70-3.48 (m, 2H), 2.83 (d, 2H), 0.93-0.88 (m, 2H), 0.00 (s, 9H);
MS m/z (ESI): 498.1 [M+H]⁺.

### Step b:

### rac-4-(2,3-Dichloro-6-((2-(trimethylsilyl)ethoxy)methoxy)phenyl)-1-(6,7-dihydro-4H-pyrazolo[5,1-c][1,4]oxazin-3-yl)pyrrolidine-2-thione

To a stirred solution of compound **163a** (560.0 mg, 1.12 mmol) in tetrahydrofuran (20 mL) was added Lawesson's reagent (363.5 mg, 0.90 mmol) at 20°C. The reaction solution was stirred at 70°C for 5 min. The reaction solution was diluted with 30 mL of water and extracted with ethyl acetate (30 mL x 3). The combined organic layers were washed with sodium chloride aqueous solution and dried over anhydrous sodium sulphate. After filtration, the reaction solution was concentrated under reduced pressure, and the residue was purified by flash silica gel column chromatography (petroleum ether : ethyl acetate = 2:1) to afford compound **163b:** rac-4-(2,3-dichloro-6-((2-(trimethylsilyl)ethoxy)methoxy)phenyl)-1-(6,7-dihydro-4H-pyrazolo[5,1-c][1,4]oxazin-3-yl)pyrrolidine-2-thione (533.0 mg, 92.2%).
¹H NMR (400 MHz, CDCl₃) δ 7.60 (s, 1H), 7.35 (d, 1H), 7.11 (d, 1H), 5.29 (d, 1H), 5.22 (d, 1H), 4.94-4.83 (m, 2H), 4.63-4.58 (m, 1H), 4.30-4.12 (m, 6H), 3.75-3.71 (m, 2H), 3.55-3.48 (m, 1H), 3.42-3.36 (m, 1H), 0.95-0.91 (m, 2H), 0.00 (s, 9H);
MS m/z (ESI): 514.3 [M+H]⁺.

### Step c:

### rac-4-(2,3-Dichloro-6-hydroxyphenyl)-1-(6,7-dihydro-4H-pyrazolo[5,1-c][1,4]oxazin-3-yl)pyrrolidine-2-thione

To a stirred solution of compound **163b** (231.0 mg, 0.45 mmol) in dichloromethane (6 mL) was added trifluoroacetic acid (2 mL) at 0°C, and the mixture was stirred at 25°C for 0.5 h. The reaction solution was adjusted to pH = 8-9 with saturated sodium bicarbonate and extracted with dichloromethane (15 mL x 3). The combined organic layers were washed with sodium chloride aqueous solution and dried over anhydrous sodium sulphate. After filtration, the reaction solution was concentrated under reduced pressure, and the residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **163:** rac-4-(2,3-dichloro-6-hydroxyphenyl)-1-(6,7-dihydro-4H-pyrazolo[5,1-c][1,4]oxazin-3-yl)pyrrolidine-2-thione (121.2 mg, 70.2%).
¹H NMR (400 MHz, DMSO*_d₆*) δ 10.57 (s, 1H), 7.71(s, 1H), 7.36 (d, 1H), 6.86 (d, 1H), 4.82-4.72 (m, 2H), 4.45-4.32 (m, 2H), 4.14-4.05 (m, 5H), 3.42-3.35 (m, 1H), 3.20-3.13 (m, 1H);
MS m/z (ESI): 384.0 [M+H]⁺.

Compound **163-1** was synthesised using intermediate **3** as the starting material and following the same synthetic route as compound **163.**

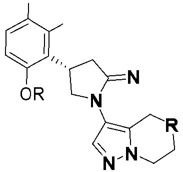

### Example 52

### Synthesis of compound 164 and compound 164-1

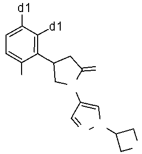

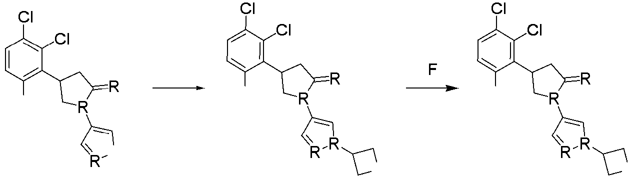

### Step a:

### rac-4-(2,3-Dichloro-6-((2-(trimethylsilyl)ethoxy)methoxy)phenyl)-1-(1-(oxetan-3-yl)-1H-pyrazol-4-yl)pyrrolidine-2-thione

To a stirred solution of compound **68c** (210.0 mg, 0.46 mmol) in acetonitrile (10 mL) were added 3-iodooxetane (170.0 mg, 0.92 mmol), 18-crown-6 (24.2 mg, 0.09 mmol) and caesium carbonate (450.0 mg, 1.38 mmol) at 20°C, and the reaction solution was stirred at 85°C for 4 h. After the reaction was completed, the resulting mixture was diluted with water and extracted with ethyl acetate (25 mL x 3). The combined organic layers were washed with sodium chloride aqueous solution and dried over anhydrous sodium sulphate. After filtration, the filtrate was concentrated under reduced pressure, and the residue was purified by flash silica gel column chromatography (petroleum ether : tetrahydrofuran = 1:1) to afford compound **164a:** rac-4-(2,3-dichloro-6-((2-(trimethylsilyl)ethoxy)methoxy)phenyl)-1-(1-(oxetan-3-yl)-1H-pyrazol-4-yl)pyrrolidine-2-thione (125.0 mg, 53.0%).

MS m/z (ESI): 514.1 [M+H]⁺.

### Step b:

### rac-4-(2,3-Dichloro-6-hydroxyphenyl)-1-(1-(oxetan-3-yl)-1H-pyrazol-4-yl)pyrrolidine-2-thione

To a stirred solution of compound **164a** (55.0 mg, 0.11 mmol) in dichloromethane (4 mL) was added trifluoroacetic acid (1 mL) at 0°C, and the mixture was stirred at 25°C for 0.5 h. The reaction solution was adjusted to pH = 8-9 with saturated sodium bicarbonate and extracted with dichloromethane (15 mL x 3). The combined organic layers were washed with sodium chloride aqueous solution and dried over anhydrous sodium sulphate. After filtration, the reaction solution was concentrated under reduced pressure, and the residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **164:** rac-4-(2,3-dichloro-6-hydroxyphenyl)-1-(1-(oxetan-3-yl)-1H-pyrazol-4-yl)pyrrolidine-2-thione (12.4 mg, 30.2%).
¹H NMR (400 MHz, DMSO*_d₆*) δ 10.55 (s, 1H), 8.94 (s, 1H), 8.24 (s, 1H), 7.37 (d, 1H), 6.83 (d, 1H), 5.66-5.59 (m, 1H), 4.94-4.85 (m, 4H), 4.52-4.42 (m, 2H), 4.34-4.26 (m, 1H), 3.43-3.35 (m, 1H), 3.33-3.26 (m, 1H);
MS m/z (ESI): 383.9 [M+H]⁺.

Compound **164-1** was synthesised using intermediate **3** as the starting material and following the same synthetic route as compound **164.**

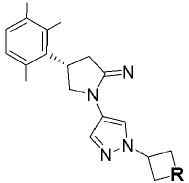

### Example 53

### Synthesis of compound 165

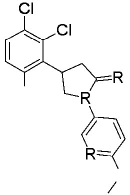

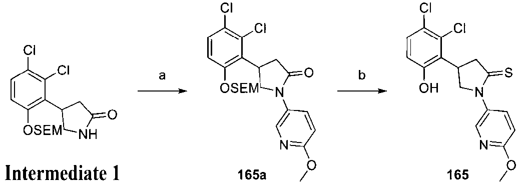

### Step a:

### rac-4-(2,3-Dichloro-6-((2-(trimethylsilyl)ethoxy)methoxy)phenyl)-1-(6-methoxypyridin-3-yl)pyrrolidin-2-one

Under nitrogen protection at 20°C, 5-bromo-2-methoxypyridine (150.0 mg, 0.80 mmol) and caesium carbonate (350.0 mg, 1.07 mmol) were added to a stirred solution of intermediate 1 (200.0 mg, 0.53 mmol) in acetonitrile/N,N-dimethylformamide (8 mL, v/v = 1:1), and the reaction solution was stirred at 30°C for 20 min. Cuprous iodide (100.0 mg, 1.99 mmol) and N,N-dimethylethylenediamine (585.6 mg, 6.64 mmol) were added to the reaction solution, and the reaction solution was stirred at 85°C for 3 h. After the reaction was completed, the reaction solution was concentrated under reduced pressure, and the residue was purified by flash silica gel column chromatography (petroleum ether : tetrahydrofuran = 6:1) to afford compound **165a:** rac-4-(2,3-dichloro-6-((2-(trimethylsilyl)ethoxy)methoxy)phenyl)-1-(6-methoxypyridin-3-yl)pyrrolidin-2-one (90.0 mg, 35.0%).

MS m/z (ESI): 483.1 [M+H]⁺.

### Step b:

### rac-4-(2,3-Dichloro-6-hydroxyphenyl)-1-(6-methoxypyridin-3-yl)pyrrolidine-2-thione

To a stirred solution of compound **165a** (140.0 mg, 0.29 mmol) in dioxane (6 mL) was added phosphorus pentasulfide (130.0 mg, 0.58 mmol) at 20°C. The reaction solution was stirred at 100°C for 5 min. The reaction solution was cooled to room temperature, diluted with 30 mL of saturated sodium bicarbonate solution, and extracted with ethyl acetate (30 mL x 3). The combined organic layers were washed with sodium chloride aqueous solution and dried over anhydrous sodium sulphate. After filtration, the reaction solution was concentrated under reduced pressure, and the residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **165:** rac-4-(2,3-dichloro-6-hydroxyphenyl)-1-(6-methoxypyridin-3-yl)pyrrolidine-2-thione (6.6 mg, 6.1%).
¹H NMR (400 MHz, DMSO_*d6*) δ 10.66 (brs, 1H), 8.33 (d, 1H), 7.91 (dd, 1H), 7.37 (d, 1H), 6.94 (d, 1H), 6.87 (d, 1H), 4.50-4.39 (m, 2H), 4.27-4.23 (m, 1H), 3.88 (s, 3H), 3.49-3.41 (m, 1H), 3.31-3.26 (m, 1H);
MS m/z (ESI): 369.0 [M+H]⁺.

### Example 54

### Synthesis of compound 166

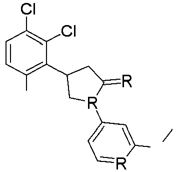

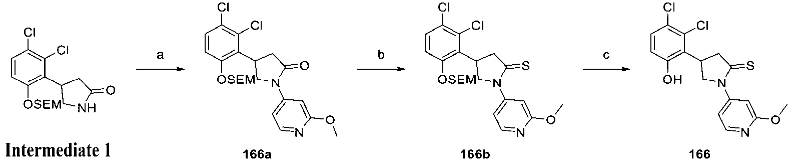

### Step a:

### rac-4-(2,3-Dichloro-6-((2-(trimethylsilyl)ethoxy)methoxy)phenyl)-1-(2-methoxypyridin-4-yl)pyrrolidin-2-one

Under nitrogen protection at 20°C, 4-bromo-2-methoxypyridine (99.9 mg, 0.53 mmol), tris(dibenzylideneacetone)dipalladium (48.7 mg, 0.053 mmol), 4,5-bis(diphenylphosphino)-9,9-dimethylxanthene (61.5 mg, 0.11 mmol) and caesium carbonate (519.4 mg, 1.59 mmol) were added to a stirred solution of intermediate 1 (200.0 mg, 0.53 mmol) in dioxane (50 mL), and the reaction solution was stirred at 105°C for 3 h. After the reaction was completed, the reaction solution was cooled to room temperature, diluted with 50 mL of water, and extracted with ethyl acetate (50 mL x 3). The combined organic layers were washed with sodium chloride aqueous solution and dried over anhydrous sodium sulphate. After filtration, the reaction solution was concentrated under reduced pressure, and the residue was purified by flash silica gel column chromatography (petroleum ether : ethyl acetate = 10:1) to afford compound **166a:** rac-4-(2,3-dichloro-6-((2-(trimethylsilyl)ethoxy)methoxy) phenyl)-1-(2-methoxypyridin-4-yl)pyrrolidin-2-one (200.0 mg, 77.8%).

MS m/z (ESI): 483.6 [M+H]⁺.

### Step b:

### rac-4-(2,3-Dichloro-6-((2-(trimethylsilyl)ethoxy)methoxy)phenyl)-1-(2-methoxypyridin-4-yl)pyrrolidine-2-thione

To a stirred solution of compound **166a** (160.0 mg, 0.33 mmol) in toluene (10 mL) was added Lawesson's reagent (267.7 mg, 0.66 mmol) at 20°C. The reaction solution was stirred at 120°C for 15 min. The reaction solution was cooled to room temperature, diluted with 30 mL of water, and extracted with ethyl acetate (30 mL x 3). The combined organic layers were washed with sodium chloride aqueous solution and dried over anhydrous sodium sulphate. After filtration, the reaction solution was concentrated under reduced pressure, and the residue was purified by flash silica gel column chromatography (petroleum ether : ethyl acetate = 10:1) to afford compound **166b:** rac-4-(2,3-dichloro-6-((2-(trimethylsilyl)ethoxy)methoxy) phenyl)-1-(2-methoxypyridin-4-yl)pyrrolidine-2-thione (75.0 mg, 45.4%).

MS m/z (ESI): 499.3 [M+H]⁺.

### Step c:

### rac-4-(2,3-Dichloro-6-hydroxyphenyl)-1-(2-methoxypyridin-4-yl)pyrrolidine-2-thione

To a stirred solution of compound **166b** (75.0 mg, 0.15 mmol) in dichloromethane (50 mL) was added trifluoroacetic acid (1 mL) at 20°C, and the mixture was stirred at 30°C for 3 h. The reaction solution was concentrated under reduced pressure, and the residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **166:** rac-4-(2,3-dichloro-6-hydroxyphenyl)-1-(2-methoxypyridin-4-yl)pyrrolidine-2-thione (2.3 mg, 4.1%).
¹H NMR (400 MHz, DMSO*_d₆*) δ 10.60 (brs, 1H), 8.24 (d, 1H), 7.43 (dd, 5.6 Hz, 1H), 7.38-7.35 (m, 2H), 6.85 (d, 1H), 4.51-4.42 (m, 2H), 4.36-4.33 (m, 1H), 3.88 (s, 3H), 3.51-3.44 (m, 1H), 3.40-3.35 (m, 1H);
MS m/z (ESI): 369.0 [M+H]⁺.

### Example 55

### Synthesis of compound 167

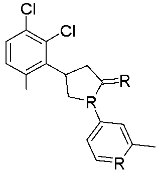

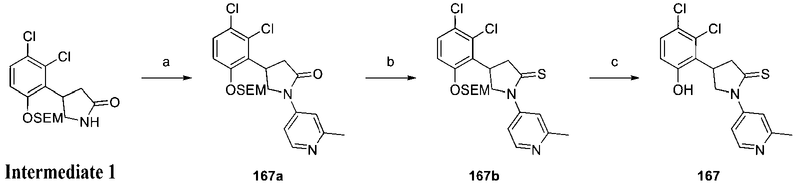

### Step a:

### rac-4-(2,3-Dichloro-6-((2-(trimethylsilyl)ethoxy)methoxy)phenyl)-1-(2-methylpyridin-4-yl)pyrrolidin-2-one

Under nitrogen protection at 20°C, 4-bromo-2-methylpyridine (45.7 mg, 0.27 mmol), tris(dibenzylideneacetone)dipalladium (24.3 mg, 0.027 mmol), 4,5-bis(diphenylphosphino)-9,9-dimethylxanthene (30.8 mg, 0.055 mmol) and caesium carbonate (259.7 mg, 0.80 mmol) were added to a stirred solution of intermediate 1 (100.0 mg, 0.27 mmol) in dioxane (20 mL), and the reaction solution was stirred at 105°C for 3 h. After the reaction was completed, the reaction solution was cooled to room temperature, 10 mL of saturated ammonium chloride solution was added, and the mixture was extracted with ethyl acetate (25 mL x 3). The combined organic layers were washed with sodium chloride aqueous solution and dried over anhydrous sodium sulphate. After filtration, the reaction solution was concentrated under reduced pressure, and the residue was purified by flash silica gel column chromatography (petroleum ether : ethyl acetate = 3:1) to afford compound **167a:** rac-4-(2,3-dichloro-6-((2-(trimethylsilyl)ethoxy)methoxy)phenyl)-1-(2-methylpyridin-4-yl)pyrrolidin-2-one (46.0 mg, 37.0%).
¹H NMR (400 MHz, DMSO*_d₆*) δ 8.46 (d, 1H), 7.75-7.73 (m, 1H), 7.69-7.66 (m, 2H), 7.25 (d, 1H), 5.35-5.29 (m, 2H), 4.60-4.55 (m, 1H), 4.34 (t, 1H), 3.96-3.92 (m, 1H), 3.66-3.54 (m, 2H), 3.16-3.09 (m, 1H), 2.89-2.83 (m, 1H), 2.20 (s, 3H), 0.87 (t, 2H), 0.00 (s, 9H);
MS m/z (ESI): 467.1 [M+H]⁺.

### Step b:

### rac-4-(2,3-Dichloro-6-((2-(trimethylsilyl)ethoxy)methoxy)phenyl)-1-(2-methylpyridin-4-yl)pyrrolidine-2-thione

To a stirred solution of compound **167a** (500.0 mg, 1.07 mmol) in toluene (25 mL) was added Lawesson's reagent (865.2 mg, 2.14 mmol) at 20°C. The reaction solution was stirred at 120°C for 5 min. The reaction solution was cooled to room temperature, diluted with 30 mL of water, and extracted with ethyl acetate (30 mL x 3). The combined organic layers were washed with sodium chloride aqueous solution and dried over anhydrous sodium sulphate. After filtration, the reaction solution was concentrated under reduced pressure, and the residue was purified by flash silica gel column chromatography (petroleum ether : ethyl acetate = 5:1) to afford compound **167b:** rac-4-(2,3-dichloro-6-((2-(trimethylsilyl)ethoxy)methoxy) phenyl)-1-(2-methylpyridin-4-yl)pyrrolidine-2-thione (60.0 mg, 11.6%).

MS m/z (ESI): 483.3 [M+H]⁺.

### Step c:

### rac-4-(2,3-Dichloro-6-hydroxyphenyl)-1-(2-methylpyridin-4-yl)pyrrolidine-2-thione

To a stirred solution of compound **167b** (60.0 mg, 0.12 mmol) in dichloromethane (3 mL) was added trifluoroacetic acid (0.5 mL) at 20°C, and the mixture was stirred at 0°C for 2 h. The reaction solution was adjusted to pH = 7 with saturated sodium bicarbonate and extracted with ethyl acetate (10 mL x 3). The combined organic layers were washed with sodium chloride aqueous solution and dried over anhydrous sodium sulphate. After filtration, the reaction solution was concentrated under reduced pressure, and the residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **167:** rac-4-(2,3-dichloro-6-hydroxyphenyl)-1-(2-methylpyridin-4-yl)pyrrolidine-2-thione (7.3 mg, 16.7%).
¹H NMR (400 MHz, DMSO*_d₆*) δ 8.67-8.47 (m, 1H), 8.30 (s, 1H), 7.69 (d, 2H), 7.36 (d, 1H), 6.88 (d, 1H), 4.78-4.15 (m, 3H), 3.77-3.15 (m, 5H);
MS m/z (ESI): 353.0 [M+H]⁺.

### Example 56

### Synthesis of compound 168

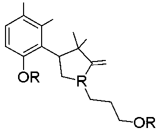

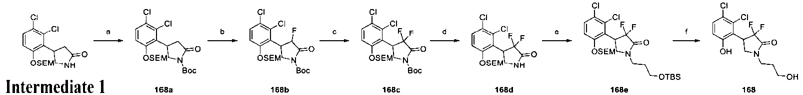

### Step a:

### rac-tert-Butyl 4-(2,3-dichloro-6-((2-(trimethylsilyl)ethoxy)methoxy)phenyl)-2-oxopyrrolidine-1-carboxylate

To a stirred solution of intermediate 1 (1100.0 mg, 2.92 mmol) in acetonitrile (40 mL) were added 4-dimethylaminopyridine (35.7 mg, 0.29 mmol) and di-tert-butyl dicarbonate (1275.8 mg, 5.85 mmol) at 25°C, and the reaction solution was stirred at 25°C for 2 h. After the reaction was completed, the reaction solution was concentrated under reduced pressure, and the residue was purified by flash silica gel column chromatography (petroleum ether : ethyl acetate = 3:1) to afford compound **168a:** rac-tert-butyl 4-(2,3-dichloro-6-((2-(trimethylsilyl)ethoxy)methoxy)phenyl)-2-oxopyrrolidine-1-carboxylate (1.20 g, 86.2%).

¹H NMR (400 MHz, DMSO*_d₆*) δ 7.52 (d, 1H), 7.13 (d, 1H), 5.29-5.22 (m, 2H), 4.30-4.21 (m, 1H), 3.99 (t, 1H), 3.70-3.57 (m, 3H), 2.84 (dd, 1H), 2.64 (dd, 1H), 1.11 (s, 9H), 0.92-0.79 (m, 2H), -0.04 (s, 9H).

### Step b:

### rac-tert-Butyl 4-(2,3-dichloro-6-((2-(trimethylsilyl)ethoxy)methoxy)phenyl)-3-fluoro-2-oxopyrrolidine-1-carboxylate

Under nitrogen protection at -78°C, n-butyllithium (2.2 mL, 5.37 mmol, 2.5M) was added to a stirred solution of diisopropylamine (543.7 mg, 5.37 mmol) in tetrahydrofuran (10 mL), and the reaction solution was stirred at 0°C for 30 min. Then, compound **168a** (1600.0 mg, 3.36 mmol) dissolved in tetrahydrofuran (10 mL) was added to the reaction solution at -78°C, and the reaction solution was stirred at -78°C for 1 h. *N*-Fluorobisbenzenesulfonamide (1270.7 mg, 4.03 mmol) was added, and the reaction solution was stirred at -78°C for 1.5 h. After the reaction was completed, saturated ammonium chloride solution (20 mL) was added to quench the reaction solution, and the mixture was extracted with ethyl acetate (25 mL x 3). The combined organic layers were washed with sodium chloride aqueous solution and dried over anhydrous sodium sulphate. After filtration, the filtrate was concentrated under reduced pressure. The residue was purified by flash silica gel column chromatography (petroleum ether : ethyl acetate = 9:1) to afford compound **168b:** rac-tert-butyl 4-(2,3-dichloro-6-((2-(trimethylsilyl)ethoxy)methoxy)phenyl)-3-fluoro-2-oxopyrrolidine-1-carboxylate (1.01 g, 60.8%).

¹H NMR (400 MHz, DMSO*_d₆*): δ 7.61 (d, 1H), 7.18 (d, 1H), 5.72 (dd, 1H), 5.36-5.29 (m, 2H), 4.46-4.32 (m, 1H), 4.00 (t, 1H), 3.72-3.61 (m, 3H), 1.47 (s, 9H), 0.89-0.84 (m, 2H), -0.03 (s, 9H).

### Step c:

### rac-tert-Butyl 4-(2,3-dichloro-6-((2-(trimethylsilyl)ethoxy)methoxy)phenyl)-3.3-difluoro-2-oxopyrrolidine-1-carboxylate

Under nitrogen protection at -78°C, n-butyllithium (0.5 mL, 1.36 mmol, 2.5M) was added to a stirred solution of diisopropylamine (137.4 mg, 1.36 mmol) in tetrahydrofuran (4 mL), and the reaction solution was stirred at 0°C for 30 min. Then, compound **168b** (480.0 mg, 0.97 mmol) dissolved in tetrahydrofuran (4 mL) was added to the reaction solution at -78°C, and the reaction solution was stirred at -78°C for 1 h. N-Fluorobisbenzenesulfonamide (367.4 mg, 1.17 mmol) was added, and the reaction solution was stirred at -78°C for 1.5 h. After the reaction was completed, saturated ammonium chloride solution (10 mL) was added to quench the reaction solution, and the mixture was extracted with ethyl acetate (15 mL x 3). The combined organic layers were washed with sodium chloride aqueous solution and dried over anhydrous sodium sulphate. After filtration, the filtrate was concentrated under reduced pressure. The residue was purified by flash silica gel column chromatography (petroleum ether : ethyl acetate = 10:1) to afford compound **168c:** rac-tert-butyl 4-(2,3-dichloro-6-((2-(trimethylsilyl)ethoxy)methoxy)phenyl)-3.3-difluoro-2-oxopyrrolidine-1-carboxylate (120.0 mg, 24.1%).

¹H NMR (400 MHz, DMSO*_d₆*): δ 7.40 (d, 1H), 7.12 (d, 1H), 5.11 (dd, 2H), 4.68-4.58 (m, 1H), 4.17-4.11 (m, 1H), 3.74-3.65 (m, 3H), 1.56 (s, 9H), 0.97-0.83 (m, 2H), -0.01 (s, 9H).

### Step d:

### rac-4-(2,3-Dichloro-6-((2-(trimethylsilyl)ethoxy)methoxy)phenyl)-3,3-difluoropyrrolidin-2-one

To a stirred solution of compound **168c** (50.0 mg, 0.096 mmol) in acetonitrile (4 mL) was added ceric ammonium nitrate (10.7 mg, 0.02 mmol) at 20°C. The reaction solution was stirred at 80°C for 2 h. After the reaction was completed, the resulting mixture was cooled to room temperature, diluted with water and extracted with ethyl acetate (10 mL x 3). The combined organic layers were washed with sodium chloride aqueous solution and dried over anhydrous sodium sulphate. After filtration, the filtrate was concentrated under reduced pressure. The residue was purified by flash silica gel column chromatography (petroleum ether : ethyl acetate = 1:1) to afford compound **168d:** rac-4-(2,3-dichloro-6-((2-(trimethylsilyl)ethoxy)methoxy)phenyl)-3,3-difluoropyrrolidin-2-one (15.0 mg, 37.3%).
¹H NMR (400 MHz, CDCl₃): δ 7.40 (d, 1H), 7.17 (s, 1H), 7.14 (d, 1H), 5.20-5.15 (m, 2H), 4.83-4.71 (m, 1H), 3.88-3.84 (m, 1H), 3.73-3.69 (m, 2H), 3.63-3.58 (m, 1H), 0.95-0.88 (m, 2H), 0.01 (s, 9H);
MS m/z (ESI): 429.0 [M+H]⁺.

### Step e:

### rac-1-(3-((tert-Butyldimethylsilyl)oxy)propyl)-4-(2,3-dichloro-6-((2-(trimethylsilyl)ethoxy)methoxy)phenyl)-3,3-difluoropyrrolidin-2-one

To a stirred solution of compound **168d** (86.0 mg, 0.21 mmol) in toluene (5 mL) were added potassium hydroxide (23.4 mg, 0.42 mmol), tetrabutylammonium iodide (7.7 mg, 0.021 mmol) and tert-butyl (3-iodopropoxy)dimethylsilane (125.3 mg, 0.42 mmol) at 25°C, and the reaction solution was stirred at 40°C for 2 h. The resulting mixture was diluted with water and extracted with ethyl acetate (25 mL x 3). The combined organic layers were washed with sodium chloride aqueous solution and dried over anhydrous sodium sulphate. After filtration, the filtrate was concentrated under reduced pressure. The residue was purified by flash silica gel column chromatography (petroleum ether : ethyl acetate = 8:1) to afford compound **168e:** rac-1-(3-((tert-butyldimethylsilyl)oxy)propyl)-4-(2,3-dichloro-6-((2-(trimethylsilyl)ethoxy)methoxy)phenyl)-3,3-difluoropyrrolidin-2-one (95.0 mg, 77.9%).

¹H NMR (400 MHz, CDCl₃): δ 7.39 (d, 1H), 7.14 (d, 1H), 5.12 (dd, 2H), 4.74-4.63 (m, 1H), 3.88-3.83 (m, 1H), 3.75-3.65 (m, 4H), 3.65-3.58 (m, 1H), 3.53-3.47 (m, 1H), 3.43-3.36 (m, 1H), 1.89-1.77 (m, 2H), 0.96-0.87 (m, 2H), 0.90 (s, 9H), 0.61 (s, 6H), 0.01 (s, 9H).

### Step f:

### rac-4-(2,3-Dichloro-6-hydroxyphenyl)-3,3-difluoro-1-(3-hydroxypropyl) pyrrolidin-2-one

To a stirred solution of compound **168e** (95.0 mg, 0.16 mmol) in tetrahydrofuran (5 mL) was added tetrabutylammonium fluoride (212.4 mL, 0.81 mmol) at 25°C. The reaction solution was stirred at 25°C for 1 h. The resulting mixture was diluted with water and extracted with ethyl acetate (25 mL x 3). The combined organic layers were washed with sodium chloride aqueous solution and dried over anhydrous sodium sulphate. After filtration, the filtrate was concentrated under reduced pressure, and the concentrate was diluted with dichloromethane (3 mL). Hydrogen chloride dioxane solution (2.0 mL, 2N) was added at 0°C, and the reaction solution was stirred at 0°C for 1 h. The reaction solution was diluted with saturated sodium bicarbonate and extracted with dichloromethane (10 mL x 3). The combined organic layers were washed with sodium chloride aqueous solution and dried over anhydrous sodium sulphate. After filtration, the filtrate was concentrated under reduced pressure, and the residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **168:** rac-4-(2,3-dichloro-6-hydroxyphenyl)-3,3-difluoro-1-(3-hydroxypropyl)pyrrolidin-2-one (14.0 mg, 25.3%).
¹H NMR (400 MHz, DMSO*_d₆*): δ 10.71 (s, 1H), 7.44 (d, 1H), 6.84 (d, 1H), 4.63-4.52 (m, 1H), 4.55-4.51 (m, 1H), 3.88-3.84 (m, 1H), 3.68-3.63 (m, 1H), 3.46-3.38 (m, 3H), 3.36-3.29 (m, 1H), 1.73-1.66 (m, 2H);
MS m/z (ESI): 340.0 [M+H]⁺.

### Example 57

### Synthesis of compound 169

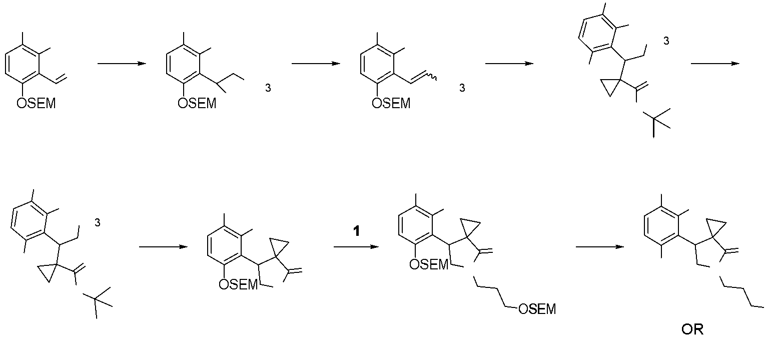

### Step a:

### rac-1-(2,3-Dichloro-6-((2-(trimethylsilyl)ethoxy)methoxy)phenyl)-2-nitroethanol-1-ol

To a stirred solution of compound **int-b** (5.0 g, 15.56 mmol) in nitromethane (60 mL) was added potassium carbonate (5.39 g, 39.00 mmol) at 25°C, and the reaction solution was stirred at 25°C for 0.5 h. After the reaction was completed, the reaction solution was diluted with water (100 mL) and extracted with ethyl acetate (100 mL x 3). The combined organic layers were washed with sodium chloride aqueous solution and dried over anhydrous sodium sulphate. After filtration, the reaction solution was concentrated under reduced pressure, and the residue was purified by flash silica gel column chromatography (petroleum ether : tetrahydrofuran = 10:1) to afford compound **169a:** rac-1-(2,3-dichloro-6-((2-(trimethylsilyl)ethoxy)methoxy)phenyl)-2-nitroethanol-1-ol (5.45 g, 91.6%).

¹H NMR (400 MHz, CDCl₃) δ 7.41 (d, 1H), 7.12 (d, 1H), 6.05-6.01 (m, 1H), 5.35-5.31 (m, 2H), 4.90-4.85 (m, 1H), 4.58-4.54 (m, 1H), 4.05 (d, 1H), 3.81-3.75 (m, 2H), 0.99-0.94 (m, 2H), 0.00 (s, 9H).

### Step b:

### (2-((3,4-Dichloro-2-(2-nitrovinyl)phenoxy)methoxy)ethyl)trimethylsilane

To a stirred solution of compound **169a** (5.45 g, 14.26 mmol) in toluene (60 mL) was added Burgess reagent (10.22 g, 42.89 mmol) at 25°C, and the reaction solution was stirred at 60°C for 2 h. After the reaction was completed, the reaction solution was concentrated under reduced pressure, and the residue was purified by flash silica gel column chromatography (petroleum ether : tetrahydrofuran = 20:1) to afford compound **169b:** rac-1-(2,3-dichloro-6-((2-(trimethylsilyl)ethoxy) methoxy)phenyl)-2-nitroethanol-1-ol (4.90 g, 94.4%).

¹H NMR (400 MHz, CDCl₃): δ 8.46 (d, 1H), 8.02 (d, 1H), 7.48 (d, 1H), 7.16 (d, 1H), 5.36 (s, 2H), 3.78-3.74 (m, 2H), 0.95 (t, 2H), 0.00 (s, 9H).

### Step c:

### rac-tert-Butyl 1-(1-(2,3-dichloro-6-((2-(trimethylsilyl)ethoxy)methoxy)phenyl) -2-nitroethyl)cyclopropane-1-carboxylate

Under nitrogen protection at -78°C, n-butyllithium (5.8 mL, 14.43 mmol, 2.5M) was added to a stirred solution of diisopropylamine (1.46 g, 14.43 mmol) in tetrahydrofuran (15 mL), and the reaction solution was stirred at 0°C for 30 min. Then, tert-butyl cyclopropanecarboxylate (2.05 g, 14.42 mmol) dissolved in tetrahydrofuran (20 mL) was added to the reaction solution at -78°C, and the reaction solution was stirred at -78°C for 1 h. Compound **169b** (3.50 g, 8.37 mmol) dissolved in tetrahydrofuran (30 mL) was added, and the reaction solution was stirred at -78°C for 0.5 h. After the reaction was completed, saturated ammonium chloride solution (30 mL) was added to quench the reaction solution, and the mixture was extracted with ethyl acetate (50 mL x 3). The combined organic layers were washed with sodium chloride aqueous solution and dried over anhydrous sodium sulphate. After filtration, the filtrate was concentrated under reduced pressure. The residue was purified by flash silica gel column chromatography (petroleum ether : tetrahydrofuran = 20:1) to afford compound **169c:** rac-tert-butyl 1-(1-(2,3-dichloro-6-((2-(trimethylsilyl)ethoxy)methoxy)phenyl)-2-nitroethyl)cyclopropane-1-carboxylate (1.40 g, 28.8%).

¹H NMR (400 MHz, CDCl₃): δ 7.28 (d, 1H), 7.04 (d, 1H), 5.31-5.21 (m, 2H), 5.16-5.11 (m, 2H), 4.99-4.95 (m, 1H), 3.74-3.68 (m, 2H), 1.44 (s, 9H), 1.17-1.13 (m, 1H), 1.08-1.02 (m, 1H), 0.95-0.90 (m, 3H), 0.24-0.19 (m, 1H), 0.00 (s, 9H).

### Step d:

### rac-tert-Butyl 1-(2-amino-1-(2,3-dichloro-6-((2-(trimethylsilyl)ethoxy) methoxy)phenyl)ethyl)cyclopropane-1-carboxylate

To a stirred solution of compound **169c** (1.40 g, 2.76 mmol) in acetic acid (15 mL) was added zinc powder (3.66 g, 56.3 mmol) at 25°C. The reaction solution was stirred at 25°C for 16 h. After the reaction was completed, the reaction solution was filtered, and the filtrate was concentrated under reduced pressure to afford compound **169d:** rac-tert-butyl 1-(2-amino-1-(2,3-dichloro-6-((2-(trimethylsilyl)ethoxy)methoxy)phenyl)ethyl)cyclopropane-1-carboxylate (1.20 g, 91.1%).
¹H NMR (400 MHz, CDCl₃): δ 7.27 (d, 1H), 7.05 (d, 1H), 5.17-5.12 (m, 2H), 4.37-4.33 (m, 1H), 3.72 (t, 2H), 3.17-2.91 (m, 2H), 1.31 (s, 9H), 1.14-1.10 (m, 1H), 1.05-1.02 (m, 1H), 1.00-0.87 (m, 3H), 0.56-0.52 (m, 1H), 0.00 (s, 9H);
MS m/z (ESI): 476.2 [M+H]⁺.

### Step e:

### rac-7-(2,3-Dichloro-6-((2-(trimethylsilyl)ethoxy)methoxy)phenyl)-5-azaspiro[2.4]heptan-4-one

To a stirred solution of compound **169d** (1.15 g, 2.41 mmol) in methanol (10 mL) was added 30% sodium methoxide solution (5 mL) at 25°C. The reaction solution was stirred at 25°C for 16 h. After the reaction was completed, the reaction solution was concentrated under reduced pressure, quenched with aqueous solution (20 mL), and extracted with ethyl acetate (20 mL x 3). The combined organic layers were washed with sodium chloride aqueous solution and dried over anhydrous sodium sulphate. After filtration, the filtrate was concentrated under reduced pressure. The residue was purified by flash silica gel column chromatography (petroleum ether : tetrahydrofuran = 4:1) to afford compound **169e:** rac-7-(2,3-dichloro-6-((2-(trimethylsilyl)ethoxy)methoxy)phenyl)-5-azaspiro[2.4]heptan-4-one (0.76 g, 78.3%).
¹H NMR (400 MHz, CDCl₃): δ 7.30 (d, 1H), 7.11 (d, 1H), 5.88 (s, 1H), 5.20-5.17 (m, 2H), 4.54-3.66 (m, 1H), 3.75-3.66 (m, 3H), 3.56-3.52 (m, 1H), 1.22-1.17 (m, 1H), 0.98-0.89 (m, 3H), 0.76-0.71 (m, 1H), 0.31-0.26 (m, 1H), 0.00 (s, 9H);
MS m/z (ESI): 402.0 [M+H]⁺.

### Step f:

### rac-5-(3-((tert-Butyldimethylsilyl)oxy)propyl)-7-(2,3-dichloro-6-((2-(trimethylsilyl)ethoxy)methoxy)phenyl)-5-azaspiro[2.4]heptan-4-one

To a stirred solution of compound **168e** (150.0 mg, 0.37 mmol) in toluene (2 mL) were added potassium hydroxide (41.8 mg, 0.75 mmol), tetrabutylammonium iodide (27.5 mg, 0.075 mmol) and tert-butyl (3-iodopropoxy)dimethylsilane (335.8 mg, 1.12 mmol) at 25°C, and the reaction solution was stirred at 40°C for 16 h. The resulting mixture was diluted with water and extracted with ethyl acetate (25 mL x 3). The combined organic layers were washed with sodium chloride aqueous solution and dried over anhydrous sodium sulphate. After filtration, the filtrate was concentrated under reduced pressure. The residue was purified by flash silica gel column chromatography (petroleum ether : tetrahydrofuran = 10:1) to afford compound **168f:** rac-5-(3-((tert-butyldimethylsilyl)oxy)propyl)-7-(2,3-dichloro-6-((2-(trimethylsilyl)ethoxy)methoxy)phenyl)-5-azaspiro[2.4]heptan-4-one (100.0 mg, 46.7%).

MS m/z (ESI): 574.2 [M+H]⁺.

### Step g:

### rac-7-(2,3-Dichloro-6-hydroxyphenyl)-5-(3-hydroxypropyl)-5-azaspiro[2.4]heptan-4-one

To a solution of compound **168f** (100.0 mg, 0.17 mmol) in dichloromethane (2 mL) was added hydrogen chloride dioxane solution (0.4 mL, 4N) at 25°C, and the reaction solution was stirred at 25°C for 2 h. The reaction solution was diluted with saturated sodium bicarbonate and extracted with dichloromethane (10 mL x 3). The combined organic layers were washed with sodium chloride aqueous solution and dried over anhydrous sodium sulphate. After filtration, the filtrate was concentrated under reduced pressure, and the residue was purified by high performance liquid chromatography (Welchrom C18, 20*150 mm, 5 µm; mobile phase A: ammonium bicarbonate in water, mobile phase B: acetonitrile, gradient ratio: phase B 5%-95%) to afford compound **169:** rac-7-(2,3-dichloro-6-hydroxyphenyl)-5-(3-hydroxypropyl)-5-azaspiro[2.4]heptan-4-one (13.0 mg, 22.6%).
¹H NMR (400 MHz, CDCl₃): δ 9.37 (brs, 1H), 7.18 (d, 1H), 6.75 (d, 1H), 4.48 (brs, 1H), 4.35-4.31 (m, 1H), 3.82-3.75 (m, 2H), 3.68-3.54 (m, 3H), 3.25-3.18 (m, 1H), 1.80-1.77 (m, 2H), 1.22-1.16 (m, 1H), 0.98-0.93 (m, 1H), 0.79-0.74 (m, 1H), 0.40-0.35 (m, 1H);
MS m/z (ESI): 330.0 [M+H]⁺.

**Table 2**

| Compound No. | Structure | Chemical name | MS: (M + H)⁺ |
|---|---|---|---|
| 1 | 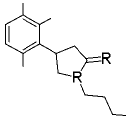 | rac-4-(2,3-Dichloro-6-hydroxyphenyl)-1-(3-hydroxypropyl)pyrrolidine-2-thione | 320.0 |
| 2 | 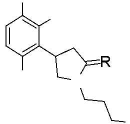 | rac-1-(3-Aminopropyl)-4-(2,3-dichloro-6-hydroxyphenyl)pyrrolidine-2-thione | 319.1 |
| 3 | 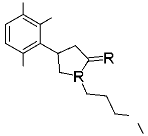 | rac-4-(2,3-Dichloro-6-hydroxyphenyl)-1-(3-(methylamino)propyl)pyrrolidine-2-thione | 333.1 |
| 4 | 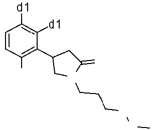 | rac-4-(2,3-Dichloro-6-hydroxyphenyl)-1-(3-(methoxyamino)propyl)pyrrolidine-2-thione | 349.0 |
| 5 | 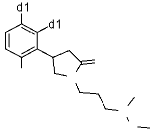 | rac-4-(2,3-Dichloro-6-hydroxyphenyl)-1-(3-(methoxy(methyl)amino)propyl)pyrrolidine -2-thione | 363.1 |
| 6 | 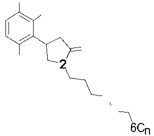 | rac-4-(2,3-Dichloro-6-hydroxyphenyl)-1-(3-((2,2,2-trifluoroethoxy)amino)propyl)pyrrolidine-2-thione | 417.0 |
| 7 | 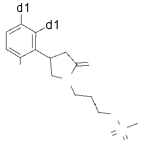 | rac-N-(3-(4-(2,3-Dichloro-6-hydroxyphenyl)-2-thiopyrrolidin-1-yl)propyl)methanesulfonamide | 397.0 |
| 8 | 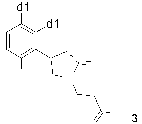 | rac-3-(4-(2,3-Dichloro-6-hydroxyphenyl)-2-thiopyrrolidin-1-yl)propionamide | 333.0 |
| 9 | 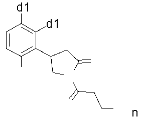 | rac-3-Amino-1-(4-(2,3-dichloro-6-hydroxyphenyl)-2-thiopyrrolidin-1-yl)propan-1-one | 333.0 |
| 10 | 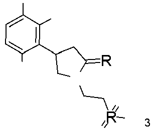 | rac-2-(4-(2,3-Dichloro-6-hydroxyphenyl)-2-thiopyrrolidin-1-yl)ethane-1-sulfonamide | 369.0 |
| 11 | 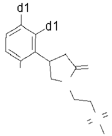 | rac- (2-(4-(2,3-Dichloro-6-hydroxyphenyl)-2-thiopyrrolidin-1-yl)ethyl)(imino)(methyl)-λ⁶-sulfanone | 367.0 |
| 12 | 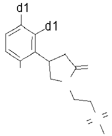 | rac-4-(2,3-Dichloro-6-hydroxyphenyl)-1-(2-(methylsulfonyl)ethyl)pyrrolidine-2-thione | 368.0 |
| 13 | 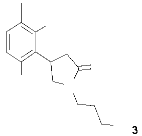 | rac-1-(3-Aminopropyl)-4-(2-chloro-6-hydroxy-3-methylphenyl)pyrrolidine-2-thione | 299.1 |
| 14 | 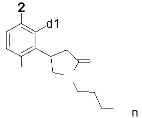 | rac-1-(3-Aminopropyl)-4-(2-chloro-3-fluoro-6-hydroxyphenyl)pyrrolidine-2-thione | 303.1 |
| 15 | 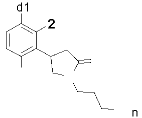 | rac-1-(3-Aminopropyl)-4-(3-chloro-2-fluoro-6-hydroxyphenyl)pyrrolidine-2-thione | 303.1 |
| 16 | 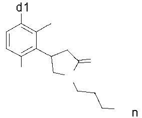 | rac-1-(3-Aminopropyl)-4-(3-chloro-6-hydroxy-2-methylphenyl)pyrrolidine-2-thione | 299.1 |
| 17 | 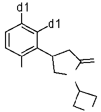 | rac-1-(Azetidin-3-yl)-4-(2,3-dichloro-6-hydroxyphenyl)pyrrolidine-2-thione | 317.0 |
| 18 | 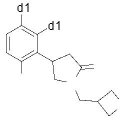 | rac-1-(Azetidin-3-ylmethyl)-4-(2,3-dichloro-6-hydroxyphenyl)pyrrolidine-2-thione | 331.0 |
| 19 | 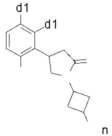 | rac-1-(3-Aminocyclobutyl)-4-(2,3-dichloro-6-hydroxyphenyl)pyrrolidine-2-thione | 331.0 |
| 20 | 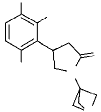 | rac-1-(1-Azabicyclo[1.1.1]pentan-3-yl)-4-(2,3-dichloro-6-hydroxyphenyl)pyrrolidine-2-thione | 329.0 |
| 21 | 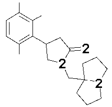 | rac-4-(2,3-Dichloro-6-hydroxyphenyl)- 1-((tetrahydro-1H-pyrrolizin-7a(5H)-yl)methyl)pyrrolidine-2-thione | 385.1 |
| 22 | 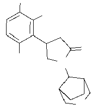 | rac-1-(3-Azabicyclo[3.2.1]octan-8-yl)-4-(2,3-dichloro-6-hydroxyphenyl)pyrrolidine-2-thione | 371.1 |
| 23 | 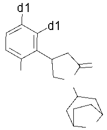 | rac-1-(8-Azabicyclo[3.2.1]octan-3-yl)-4-(2,3-dichloro-6-hydroxyphenyl)pyrrolidine-2-thione | 371.1 |
| 24 | 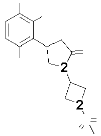 | rac-4-(2,3-Dichloro-6-hydroxyphenyl)-1-(1-(methylsulfonyl)azetidin-3-yl)pyrrolidine-2-thione | 395.0 |
| 25 | 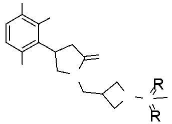 | rac-4-(2,3-Dichloro-6-hydroxyphenyl)-1-((1-(methylsulfonyl)azetidin-3-yl)methyl)pyrrolidine-2-thione | 409.0 |
| 26 | 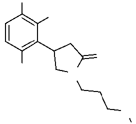 | rac-4-(2,3-Dichloro-6-hydroxyphenyl)-1-(3-(hydroxyamino)propyl)pyrrolidine-2-thione | 335.0 |
| 27 | 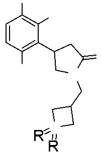 | rac-4-(2,3-Dichloro-6-hydroxyphenyl)-1-((1,1-dioxothietan-3-yl)methyl)pyrrolidine-2-thione | 380.0 |
| 28 | 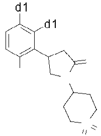 | rac-4-(2,3-Dichloro-6-hydroxyphenyl)-1-(1,1-dioxotetrahydro-2H-thiopyran-4-yl)pyrrolidine-2-thione | 394.0 |
| 29 | 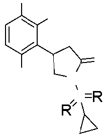 | rac-1-(Cyclopropylsulfonyl)-4-(2,3-dichloro-6-hydroxyphenyl)pyrrolidine-2-thione | 366.0 |
| 30 | 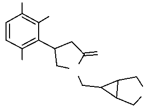 | rac-1-((3-Oxabicyclo[3.1.0]hexan-6-yl)methyl)-4-(2,3-dichloro-6-hydroxyphenyl)pyrrolidine-2-thione | 358.0 |
| 31 | 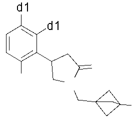 | rac-4-(2,3-Dichloro-6-hydroxyphenyl)-1-((3-hydroxybicyclo[1.1.1]pentan-1-yl)methyl)pyrrolidine-2-thione | 358.0 |
| 32 | 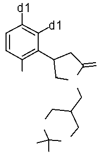 | rac-4-(2,3-Dichloro-6-hydroxyphenyl)-1-((2,2-difluoro-1,3-dioxan-5-yl)methyl)pyrrolidine-2-thione | 398.0 |
| 33 | 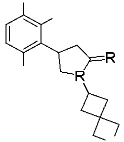 | rac-4-(2,3-Dichloro-6-hydroxyphenyl)- 1-(2-oxaspiro[3.3]heptan-6-yl)pyrrolidine-2-thione | 358.0 |
| 34 | 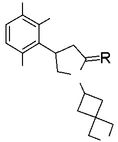 | rac-4-(2,3-Dichloro-6-hydroxyphenyl)-1-(2-azaspiro[3.3]heptan-6-yl)pyrrolidine-2-thione | 357.1 |
| 35 | 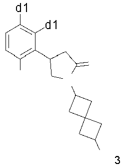 | rac-1-(6-Aminospiro[3.3]heptan-2-yl)-4-(2,3-dichloro-6-hydroxyphenyl)pyrrolidine-2-thione | 371.1 |
| 36 | 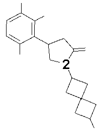 | rac-4-(2,3-Dichloro-6-hydroxyphenyl)-1-(6-hydroxyspiro[3.3]heptan-2-yl)pyrrolidine-2-thione | 372.1 |
| 37 | 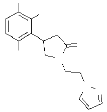 | rac-1-(2-(1H-Pyrazol-1-yl)ethyl)-4-(2,3-dichloro-6-hydroxyphenyl)pyrrolidine-2-thione | 356.0 |
| 38 | 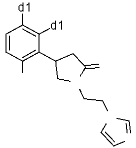 | rac-1-(2-(1H-Imidazol-1-yl)ethyl)-4-(2,3-dichloro-6-hydroxyphenyl)pyrrolidine-2-thione | 356.0 |
| 39 | 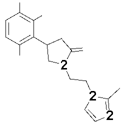 | rac-4-(2,3-Dichloro-6-hydroxyphenyl)-1-(2-(2-methyl-1H-imidazol-1-yl)ethyl)pyrrolidine-2-thione | 370.0 |
| 40 | 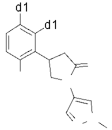 | rac-4-(2,3-Dichloro-6-hydroxyphenyl)-1-(1-methyl-1H-pyrazol-4-yl)pyrrolidine-2-thione | 342.0 |
| 41 | 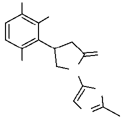 | rac-4-(2,3-Dichloro-6-hydroxyphenyl)-1-(2-methyl-1H-imidazol-5-yl)pyrrolidine-2-thione | 342.0 |
| 42 | 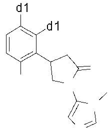 | rac-4-(2,3-Dichloro-6-hydroxyphenyl)-1-(1-methyl-1H-imidazol-5-yl)pyrrolidine-2-thione | 342.0 |
| 43 | 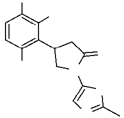 | rac-4-(2,3-Dichloro-6-hydroxyphenyl)-1-(2-methylthiazol-5-yl)pyrrolidine-2-thione | 359.0 |
| 44 | 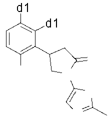 | rac-4-(2,3-Dichloro-6-hydroxyphenyl)-1-(2-methyloxazol-5-yl)pyrrolidine-2-thione | 343.0 |
| 45 | 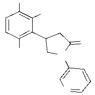 | rac-4-(2,3-Dichloro-6-hydroxyphenyl)-1-(pyridin-3-yl)pyrrolidine-2-thione | 339.0 |
| 46 | 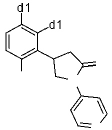 | rac-4-(2,3-Dichloro-6-hydroxyphenyl)-1-(pyridin-4-yl)pyrrolidine-2-thione | 339.0 |
| 47 | 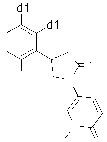 | rac-5-(4-(2,3-Dichloro-6-hydroxyphenyl)-2-thiopyrrolidin-1-yl)-1-methylpyridin-2(1H)-one | 369.0 |
| 48 | 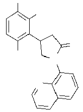 | rac-4-(2,3-Dichloro-6-hydroxyphenyl)-1-(quinolin-8-yl)pyrrolidine-2-thione | 389.0 |
| 49 | 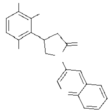 | rac-4-(2,3-Dichloro-6-hydroxyphenyl)-1-(quinolin-3-yl)pyrrolidine-2-thione | 389.0 |
| 50 | 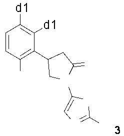 | rac-4-(2,3-Dichloro-6-hydroxyphenyl)-1-(2-(trifluoromethyl)thiazol-5-yl)pyrrolidine-2-thione | 412.9 |
| 51 | 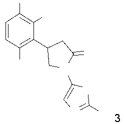 | rac-4-(2,3-Dichloro-6-hydroxyphenyl)-1-(2-(trifluoromethyl)-1H-imidazol-5-yl)pyrrolidine-2-thione | 396.0 |
| 52 | 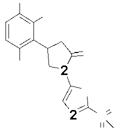 | rac-4-(2,3-Dichloro-6-hydroxyphenyl)-1-(2-(methylsulfonyl)thiazol-5-yl)pyrrolidine-2-thione | 422.9 |
| 53 | 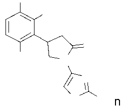 | rac-1-(2-Aminooxazol-4-yl)-4-(2,3-dichloro-6-hydroxyphenyl)pyrrolidine-2-thione | 344.0 |
| 54 | 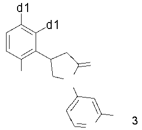 | rac-1-(2-Aminopyridin-4-yl)-4-(2,3-dichloro-6-hydroxyphenyl)pyrrolidine-2-thione | 354.0 |
| 55 | 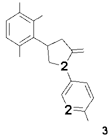 | rac-1-(6-Aminopyridin-3-yl)-4-(2,3-dichloro-6-hydroxyphenyl)pyrrolidine-2-thione | 354.0 |
| 56 | 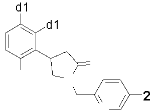 | rac-4-(2,3-Dichloro-6-hydroxyphenyl)-1-((5-fluoropyridin-2-yl)methyl)pyrrolidine-2-thione | 371.0 |
| 57 | 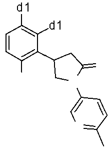 | rac-4-(2,3-Dichloro-6-hydroxyphenyl)-1-(6-methylpyridin-3-yl)pyrrolidine-2-thione | 353.0 |
| 58 | 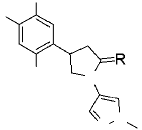 | rac-4-(4,5-Dichloro-2-hydroxyphenyl)-1-(1-methyl-1H-pyrazol-4-yl)pyrrolidine-2-thione | 342.0 |
| 59 | 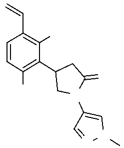 | rac-4-(2-Chloro-6-hydroxy-3-vinylphenyl)-1-(1-methyl-1H-pyrazol-4-yl)pyrrolidine-2-thione | 334.1 |
| 60 | 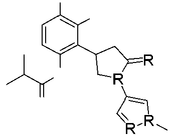 | rac-3,4-Dichloro-2-(1-(1-methyl-1H-pyrazol-4-yl)-5-thiopyrrolidin-3-yl)phenyl isobutyrate | 412.1 |
| 61 | 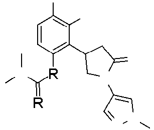 | rac-3,4-Dichloro-2-(1-(1-methyl-1H-pyrazol-4-yl)-5-thiopyrrolidin-3-yl)phenyl dimethylcarbamate | 413.1 |
| 62 | 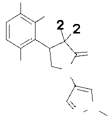 | rac-4-(2,3-Dichloro-6-hydroxyphenyl)-3,3-difluoro-1-(1-methyl-1H-pyrazol-4-yl)pyrrolidine-2-thione | 378.0 |
| 63 | 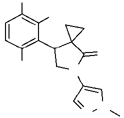 | rac-7-(2,3-Dichloro-6-hydroxyphenyl)-5-(1-methyl-1H-pyrazol-4-yl)-5-azaspiro[2.4]heptane-4-thione | 368.0 |
| 64 | 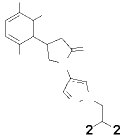 | rac-4-(2,3-Dichloro-6-hydroxyphenyl)-1-(1-(2,2-difluoroethyl)-1H-pyrazol-4-yl)pyrrolidine-2-thione | 392.0 |
| 65 | 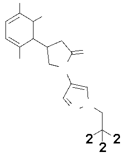 | rac-4-(2,3-Dichloro-6-hydroxyphenyl)-1-(1-(2,2,2-trifluoroethyl)- 1H-pyrazol-4-yl)pyrrolidine-2-thione | 410.0 |
| 66 | 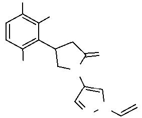 | rac-4-(2,3-Dichloro-6-hydroxyphenyl)-1-(1-vinyl-1H-pyrazol-4-yl)pyrrolidine-2-thione | 354.0 |
| 67 | 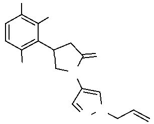 | rac-1-(1-Allyl-1H-pyrazol-4-yl)-4-(2,3-dichloro-6-hydroxyphenyl)pyrrolidine-2-thione | 368.0 |
| 68 | 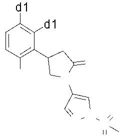 | rac-4-(2,3-Dichloro-6-hydroxyphenyl)-1-(1-(methylsulfonyl)-1H-pyrazol-4-yl)pyrrolidine-2-thione | 406.0 |
| 69 | 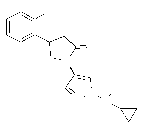 | rac-1-(1-(Cyclopropylsulfonyl)-1H-pyrazol-4-yl)-4-(2,3-dichloro-6-hydroxyphenyl)pyrrolidine-2-thione | 432.0 |
| 70 | 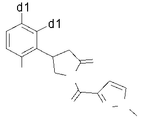 | rac-(4-(2,3-Dichloro-6-hydroxyphenyl)-2-thiopyrrolidin-1-yl)(1-methyl-1H-pyrazol-3-yl)methanone | 370.0 |
| 71 | 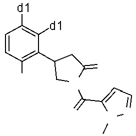 | rac-(4-(2,3-Dichloro-6-hydroxyphenyl)-2-thiopyrrolidin-1-yl)(1-methyl-1H-pyrazol-5-yl)methanone | 370.0 |
| 72 | 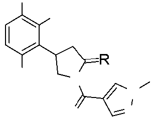 | rac-(4-(2,3-Dichloro-6-hydroxyphenyl)-2-thiopyrrolidin-1-yl)(1-methyl-1H-pyrazol-4-yl)methanone | 370.0 |
| 73 | 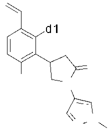 | rac-4-(2-Chloro-6-hydroxy-3-vinylphenyl)-1-(1-methyl-1H-pyrazol-4-yl)pyrrolidin-2-one | 318.1 |
| 74 | 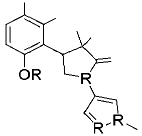 | rac-4-(2,3-Dichloro-6-hydroxyphenyl)-3,3-difluoro-1-(1-methyl-1H-pyrazol-4-yl)pyrrolidin-2-one | 362.0 |
| 75 | 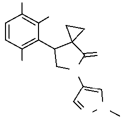 | rac-7-(2,3-Dichloro-6-hydroxyphenyl)-5-(1-methyl-1H-pyrazol-4-yl)-5-azaspiro[2.4]heptan-4-one | 352.1 |
| 76 | 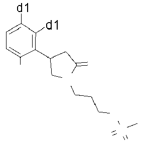 | rac-N-(3-(4-(2,3-Dichloro-6-hydroxyphenyl)-2-oxopyrrolidin-1-yl)propyl)methanesulfonamide | 381.2 |
| 77 | 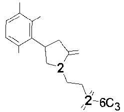 | rac-2-(4-(2,3-Dichloro-6-hydroxyphenyl)-2-oxopyrrolidin-1-yl)ethane-1-sulfonamide | 353.0 |
| 78 | 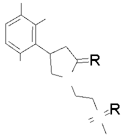 | rac-4-(2,3-Dichloro-6-hydroxyphenyl)-1-(2-(S-methylsulfonylimino)ethyl)pyrrolidin-2-one | 351.0 |
| 79 | 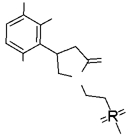 | rac-4-(2,3-Dichloro-6-hydroxyphenyl)-1-(2-(methylsulfonyl)ethyl)pyrrolidin-2-one | 352.0 |
| 80 | 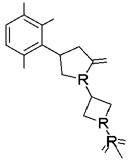 | rac-4-(2,3-Dichloro-6-hydroxyphenyl)-1-(1-(methylsulfonyl)azetidin-3-yl)pyrrolidin-2-one | 379.0 |
| 81 | 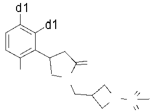 | rac-4-(2,3-Dichloro-6-hydroxyphenyl)-1-((1-(methylsulfonyl)azetidin-3-yl)methyl)pyrrolidin-2-one | 393.0 |
| 82 | 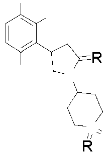 | rac-4-(2,3-Dichloro-6-hydroxyphenyl)-1-(1-imino-1-oxohexahydro-1l6-thiopyran-4-yl)pyrrolidin-2-one | 377.0 |
| 83 | 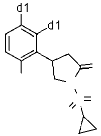 | rac-1-(Cyclopropylsulfonyl)-4-(2,3-dichloro-6-hydroxyphenyl)pyrrolidin-2-one | 350.0 |
| 84 | 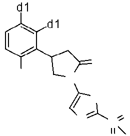 | rac-4-(2,3-Dichloro-6-hydroxyphenyl)-1-(2-(methylsulfonyl)thiazol-5-yl)pyrrolidin-2-one | 407.0 |
| 85 | 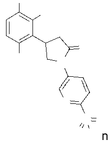 | rac-5-(4-(2,3-Dichloro-6-hydroxyphenyl)-2-oxopyrrolidin-1-yl)pyridine-2-sulfonamide | 402.0 |
| 86 | 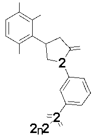 | rac-3-(4-(2,3-Dichloro-6-hydroxyphenyl)-2-oxopyrrolidin-1-yl)benzenesulfonamide | 401.0 |
| 87 | 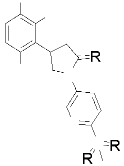 | rac-4-(2,3-Dichloro-6-hydroxyphenyl)-1-(6-(methylsulfonyl)pyridin-3-yl)pyrrolidin-2-one | 401.0 |
| 88 | 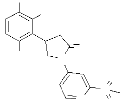 | rac-4-(2,3-Dichloro-6-hydroxyphenyl)-1-(2-(methylsulfonyl)pyridin-4-yl)pyrrolidin-2-one | 401.0 |
| 89 | 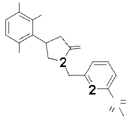 | rac-4-(2,3-Dichloro-6-hydroxyphenyl)-1-((6-(methylsulfonyl)pyridin-2-yl)methyl)pyrrolidin-2-one | 415.0 |
| 90 | 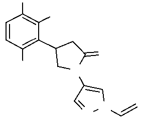 | rac-4-(2,3-Dichloro-6-hydroxyphenyl)-1-(1-vinyl-1H-pyrazol-4-yl)pyrrolidin-2-one | 338.0 |
| 91 | 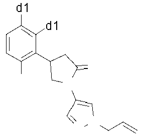 | rac-1-(1-Allyl-1H-pyrazol-4-yl)-4-(2,3-dichloro-6-hydroxyphenyl)pyrrolidin-2-one | 352.1 |
| 92 | 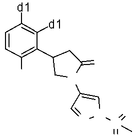 | rac-4-(2,3-Dichloro-6-hydroxyphenyl)-1-(1-(methylsulfonyl)-1H-pyrazol-4-yl)pyrrolidin-2-one | 390.0 |
| 93 | 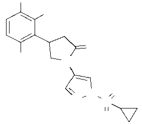 | rac-1-(1-(Cyclopropylsulfonyl)-1H-pyrazol-4-yl)-4-(2,3-dichloro-6-hydroxyphenyl)pyrrolidin-2-one | 416.0 |
| 94 | 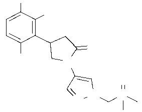 | rac-4-(2,3-Dichloro-6-hydroxyphenyl)-1-(1-((dimethylphosphoryl)methyl)-1H-pyrazol-4-yl)pyrrolidin-2-one | 402.0 |
| 95 | 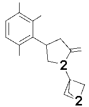 | rac-1-(1-Azabicyclo[1.1.1]pentan-3-yl)-4-(2,3-dichloro-6-hydroxyphenyl)pyrrolidin-2-one | 313.0 |
| 96 | 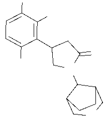 | rac-1-(3-Azabicyclo[3.2.1]octan-8-yl)-4-(2,3-dichloro-6-hydroxyphenyl)pyrrolidin-2-one | 355.1 |
| 97 | 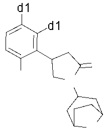 | rac-1-(8-Azabicyclo[3.2.1]octan-3-yl)-4-(2,3-dichloro-6-hydroxyphenyl)pyrrolidin-2-one | 355.1 |
| 98 | 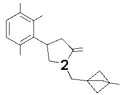 | rac-4-(2,3-Dichloro-6-hydroxyphenyl)-1-(3-hydroxybicyclo[1.1.1]pentan-1-yl)methyl)pyrrolidin-2-one | 342.1 |
| 99 | 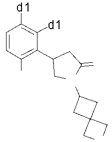 | rac-4-(2,3-Dichloro-6-hydroxyphenyl)-1-(2-oxaspiro[3.3]heptan-6-yl)pyrrolidin-2-one | 342.1 |
| 100 | 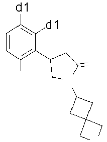 | rac-4-(2,3-Dichloro-6-hydroxyphenyl)-1-(2-azaspiro[3.3]heptan-6-yl)pyrrolidin-2-one | 341.2 |
| 101 | 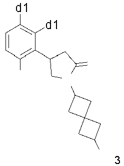 | rac-1-(6-Aminospiro[3.3]heptan-2-yl)-4-(2,3-dichloro-6-hydroxyphenyl)pyrrolidin-2-one | 355.1 |
| 102 | 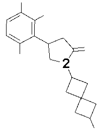 | rac-4-(2,3-Dichloro-6-hydroxyphenyl)-1-(6-hydroxyspiro[3.3]heptan-2-yl)pyrrolidin-2-one | 356.1 |
| 103 | 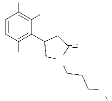 | rac-4-(2,3-Dichloro-6-hydroxyphenyl)-1-(3-(hydroxyamino)propyl)pyrrolidin-2-one | 319.0 |
| 104 | 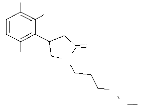 | rac-4-(2,3-Dichloro-6-hydroxyphenyl)-1-(3-(methoxyamino)propyl)pyrrolidin-2-one | 333.1 |
| 105 | 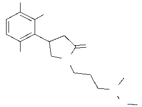 | rac-4-(2,3-Dichloro-6-hydroxyphenyl)-1-(3-(methoxy(methyl)amino)propyl)pyrrolidin-2-one | 347.1 |
| 106 | 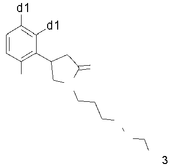 | rac-4-(2,3-Dichloro-6-hydroxyphenyl)-1-(3-((2,2,2-trifluoroethoxy)amino)propyl)pyrrolidin-2-one | 401.1 |
| 107 | 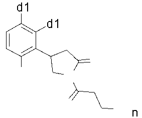 | rac-1-(3-Aminopropanoyl)-4-(2,3-dichloro-6-hydroxyphenyl)pyrrolidin-2-one | 317.0 |
| 108 | 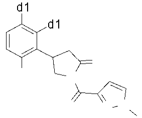 | rac-4-(2,3-Dichloro-6-hydroxyphenyl)-1-(1-methyl-1H-pyrazole-3-carbonyl)pyrrolidin-2-one | 354.0 |
| 109 | 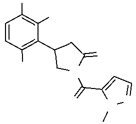 | rac-4-(2,3-Dichloro-6-hydroxyphenyl)-1-(1-methyl-1H-pyrazole-5-carbonyl)pyrrolidin-2-one | 354.0 |
| 110 | 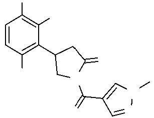 | rac-4-(2,3-Dichloro-6-hydroxyphenyl)-1-(1-methyl-1H-pyrazole-4-carbonyl)pyrrolidin-2-one | 354.0 |
| 111 | 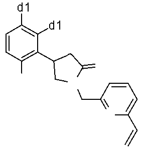 | rac-4-(2,3-Dichloro-6-hydroxyphenyl)-1-((6-vinylpyridin-2-yl)methyl)pyrrolidin-2-one | 363.1 |
| 112 | 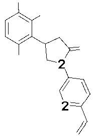 | rac-4-(2,3-Dichloro-6-hydroxyphenyl)-1-(6-vinylpyridin-3-yl)pyrrolidin-2-one | 349.0 |
| 113 | 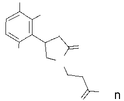 | rac-3-(4-(2,3-Dichloro-6-hydroxyphenyl)-2-oxopyrrolidin-1-yl)propanethioamide | 333.1 |
| 114 | 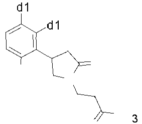 | rac-3-(4-(2,3-Dichloro-6-hydroxyphenyl)-2-oxopyrrolidin-1-yl)propionimide | 316.0 |
| 115 | 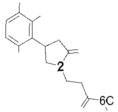 | rac-3-(4-(2,3-Dichloro-6-hydroxyphenyl)-2-oxopyrrolidin-1-yl)-N-hydroxypropionamide | 332.9 |
| 116 | 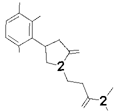 | rac-3-(4-(2,3-Dichloro-6-hydroxyphenyl)-2-oxopyrrolidin-1-yl)-N-hydroxy-N-methylpropionamide | 347.0 |
| 117 | 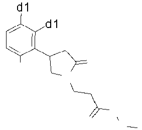 | rac-3-(4-(2,3-Dichloro-6-hydroxyphenyl)-2-oxopyrrolidin-1-yl)-N-methoxypropionamide | 347.0 |
| 118 | 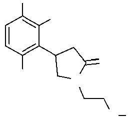 | rac-4-(2,3-Dichloro-6-hydroxyphenyl)-1-(2-(hydroxyamino)ethyl)pyrrolidin-2-one | 304.9 |
| 119 | 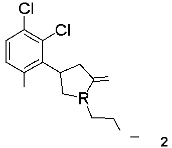 | rac-1-(2-(Aminooxy)ethyl)-4-(2,3-dichloro-6-hydroxyphenyl)pyrrolidin-2-one | 304.9 |
| 120 | 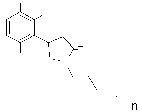 | rac-1-(3-(Aminooxy)propyl)-4-(2,3-dichloro-6-hydroxyphenyl)pyrrolidin-2-one | 319.0 |
| 121 | 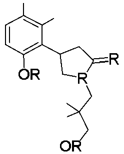 | rac-4-(2,3-Dichloro-6-hydroxyphenyl)-1-(2,2-difluoro-3-hydroxypropyl)pyrrolidine-2-thione | 355.8 |
| 122 | 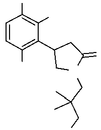 | rac-4-(2,3-Dichloro-6-hydroxyphenyl)-1-(3-hydroxy-2,2-dimethylpropyl)pyrrolidine-2-thione | 348.1 |
| 123 | 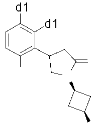 | rac-4-(2,3-Dichloro-6-hydroxyphenyl)-1-((1s,3s)-3-hydroxycyclobutyl)pyrrolidine-2-thione | 332.0 |
| 124 | 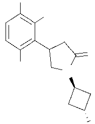 | rac-4-(2,3-Dichloro-6-hydroxyphenyl)-1-((1r,3r)-3-hydroxycyclobutyl)pyrrolidine-2-thione | 332.0 |
| 125 | 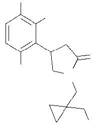 | rac-4-(2,3-Dichloro-6-hydroxyphenyl)-1-((1-(hydroxymethyl)cyclopropyl)methyl)pyrrol idine-2-thione | 345.9 |
| 126 | 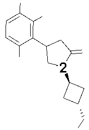 | rac-4-(2,3-Dichloro-6-hydroxyphenyl)-1-((1r,3r)-3-(hydroxymethyl)cyclobutyl)pyrrolidine-2-thione | 345.9 |
| 127 | 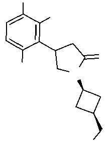 | rac-4-(2,3-Dichloro-6-hydroxyphenyl)-1-((1s,3s)-3-(hydroxymethyl)cyclobutyl)pyrrolidine-2-thione | 345.9 |
| 128 | 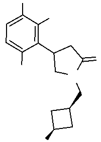 | rac-4-(2,3-Dichloro-6-hydroxyphenyl)-1-(((1s,3s)-3-hydroxycyclobutyl)methyl)pyrrolidine-2-thione | 345.9 |
| 129 | 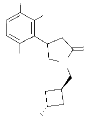 | rac-4-(2,3-Dichloro-6-hydroxyphenyl)-1-(((1r,3r)-3-hydroxycyclobutyl)methyl)pyrrolidine-2-thione | 345.9 |
| 130 | 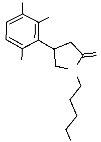 | rac-4-(2,3-Dichloro-6-hydroxyphenyl)-1-(4-hydroxybutyl)pyrrolidine-2-thione | 333.9 |
| 131 | 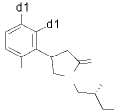 | rac-4-(2,3-Dichloro-6-hydroxyphenyl)-1-((S)-2,3-dihydroxypropyl)pyrrolidine-2-thione | 335.9 |
| 132 | 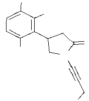 | rac-4-(2,3-Dichloro-6-hydroxyphenyl)-1-(3-hydroxyprop-1-yn-1-yl)pyrrolidine-2-thione | 316.0 |
| 133 | 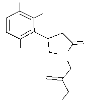 | rac-4-(2,3-Dichloro-6-hydroxyphenyl)-1-(2-(hydroxymethyl)allyl)pyrrolidine-2-thione | 332.0 |
| 134 | 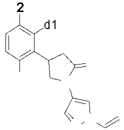 | rac-4-(2-Chloro-3-fluoro-6-hydroxyphenyl)-1-(1-vinyl-1H-pyrazol-4-yl)pyrrolidine-2-thione | 338.0 |
| 135 | 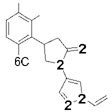 | rac-4-(3-Chloro-2-fluoro-6-hydroxyphenyl)-1-(1-vinyl-1H-pyrazol-4-yl)pyrrolidine-2-thione | 338.0 |
| 136 | 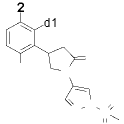 | rac-4-(2-Chloro-3-fluoro-6-hydroxyphenyl)-1-(1-(methylsulfonyl)-1H-pyrazol-4-yl)pyrrolidine-2-thione | 390.0 |
| 137 | 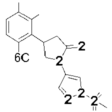 | rac-4-(3-Chloro-2-fluoro-6-hydroxyphenyl)-1-(1-(methylsulfonyl)-1H-pyrazol-4-yl)pyrrolidine-2-thione | 390.0 |
| 138 | 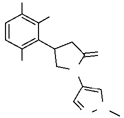 | rac-4-(2-Chloro-3-fluoro-6-hydroxyphenyl)-1-(1-methyl-1H-pyrazol-4-yl)pyrrolidine-2-thione | 326.0 |
| 139 | 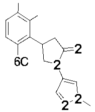 | rac-4-(3-Chloro-2-fluoro-6-hydroxyphenyl)-1-(1-methyl-1H-pyrazol-4-yl)pyrrolidine-2-thione | 326.0 |
| 140 | 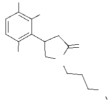 | rac-4-(2-Chloro-3-fluoro-6-hydroxyphenyl)-1-(3-(hydroxyamino)propyl)pyrrolidine-2-thione | 319.1 |
| 141 | 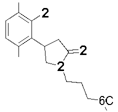 | rac-4-(3-Chloro-2-fluoro-6-hydroxyphenyl)-1-(3-(hydroxyamino)propyl)pyrrolidine-2-thione | 319.1 |
| 142 | 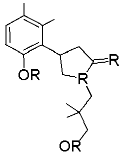 | rac-4-(2-Chloro-3-fluoro-6-hydroxyphenyl)-1-(2,2-difluoro-3-hydroxypropyl)pyrrolidine-2-thione | 340.0 |
| 143 | 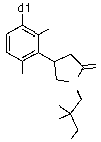 | rac-4-(3-Chloro-2-fluoro-6-hydroxyphenyl)-1-(2,2-difluoro-3-hydroxypropyl)pyrrolidine-2-thione | 340.0 |
| 144 | 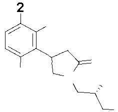 | rac-4-(2-Chloro-3-fluoro-6-hydroxyphenyl)-1-((S)-2,3-dihydroxypropyl)pyrrolidine-2-thione | 320.0 |
| 145 | 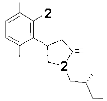 | rac-4-(3-Chloro-2-fluoro-6-hydroxyphenyl)-1-((S)-2,3-dihydroxypropyl)pyrrolidine-2-thione | 320.0 |
| 146 | 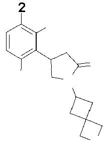 | rac-4-(2-Chloro-3-fluoro-6-hydroxyphenyl)-1-(2-azaspiro[3.3]heptan-6-yl)pyrrolidine-2-thione | 341.1 |
| 147 | 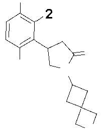 | rac-4-(3-Chloro-2-fluoro-6-hydroxyphenyl)-1-(2-azaspiro[3.3]heptan-6-yl)pyrrolidine-2-thione | 341.1 |
| 148 | 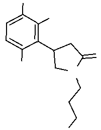 | rac-4-(2-Chloro-3-fluoro-6-hydroxyphenyl)-1-(3-hydroxypropyl)pyrrolidine-2-thione | 304.0 |
| 149 | 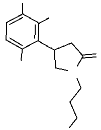 | rac-4-(3-Chloro-2-fluoro-6-hydroxyphenyl)-1-(3-hydroxypropyl)pyrrolidine-2-thione | 304.0 |
| 150 | 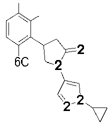 | rac-1-(1-Cyclopropyl-1H-pyrazol-4-yl)-4-(2,3-dichloro-6-hydroxyphenyl)pyrrolidine-2-thione | 367.9 |
| 151 | 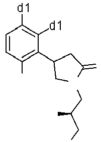 | rac-4-(2,3-Dichloro-6-hydroxyphenyl)-1-((S)-2-fluoro-3-hydroxypropyl)pyrrolidine-2-thione | 338.0 |
| 152 | 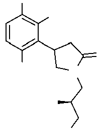 | rac-4-(2-Chloro-3-fluoro-6-hydroxyphenyl)-1-((S)-2-fluoro-3-hydroxypropyl)pyrrolidine-2-thione | 322.0 |
| 153 | 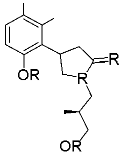 | rac-4-(3-Chloro-2-fluoro-6-hydroxyphenyl)-1-((S)-2-fluoro-3-hydroxypropyl)pyrrolidine-2-thione | 322.0 |
| 154 | 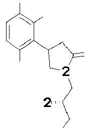 | rac-4-(2,3-Dichloro-6-hydroxyphenyl)-1-((R)-2-fluoro-3-hydroxypropyl)pyrrolidine-2-thione | 338.0 |
| 155 | 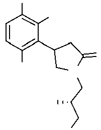 | rac-4-(2-Chloro-3-fluoro-6-hydroxyphenyl)-1-((R)-2-fluoro-3-hydroxypropyl)pyrrolidine-2-thione | 322.0 |
| 156 | 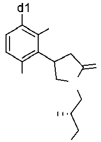 | rac-4-(3-Chloro-2-fluoro-6-hydroxyphenyl)-1-((R)-2-fluoro-3-hydroxypropyl)pyrrolidine-2-thione | 322.0 |
| 157 | 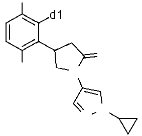 | rac-4-(2-Chloro-3-fluoro-6-hydroxyphenyl)-1-(1-cyclopropyl-1H-pyrazol-4-yl)pyrrolidine-2-thione | 352.1 |
| 158 | 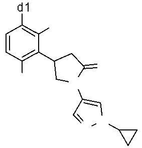 | rac-4-(3-Chloro-2-fluoro-6-hydroxyphenyl)-1-(1-cyclopropyl-1H-pyrazol-4-yl)pyrrolidine-2-thione | 352.1 |
| 159 | 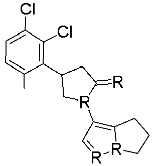 | rac-4-(2,3-Dichloro-6-hydroxyphenyl)-1-(5,6-dihydro-4H-pyrrolo[1,2-b]pyrazol-3-yl)pyrrolidine-2-thione | 368.0 |
| 160 | 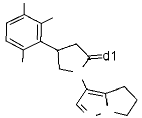 | rac-4-(2,3-Dichloro-6-hydroxyphenyl)-1-(5,6-dihydro-4H-pyrrolo[1,2-b]pyrazol-3-yl)pyrrolidin-2-one | 352.0 |
| 161 | 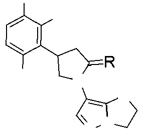 | rac-4-(2,3-Dichloro-6-hydroxyphenyl)-1-(2,3-dihydropyrazolo[5,1-b]oxazol-7-yl)pyrrolidine-2-thione | 370.0 |
| 162 | 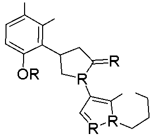 | rac-4-(2,3-Dichloro-6-hydroxyphenyl)-1-(6,7-dihydro-5H-pyrazolo[5,1-b][1,3]oxazin-3-yl)pyrrolidine-2-thione | 384.0 |
| 163 | 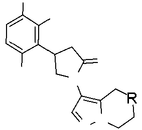 | rac-4-(2,3-Dichloro-6-hydroxyphenyl)-1-(6,7-dihydro-4H-pyrazolo[5,1-c][1,4]oxazin-3-yl)pyrrolidine-2-thione | 384.0 |
| 164 | 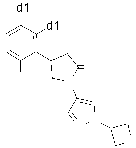 | rac-4-(2,3-Dichloro-6-hydroxyphenyl)-1-(1-(oxetan-3-yl)-1H-pyrazol-4-yl)pyrrolidine-2-thione | 383.9 |
| 165 | 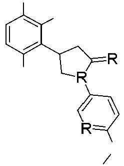 | rac-4-(2,3-Dichloro-6-hydroxyphenyl)-1-(6-methoxypyridin-3-yl)pyrrolidine-2-thione | 369.0 |
| 166 | 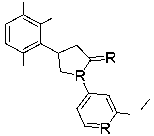 | rac-4-(2,3-Dichloro-6-hydroxyphenyl)-1-(2-methoxypyridin-4-yl)pyrrolidine-2-thione | 369.0 |
| 167 | 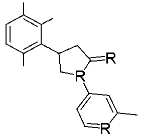 | rac-4-(2,3-Dichloro-6-hydroxyphenyl)-1-(2-methylpyridin-4-yl)pyrrolidine-2-thione | 353.0 |
| 168 | 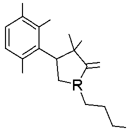 | rac-4-(2,3-Dichloro-6-hydroxyphenyl)-3,3-difluoro-1-(3-hydroxypropyl)pyrrolidin-2-one | 340.0 |
| 169 | 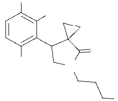 | rac-7-(2,3-Dichloro-6-hydroxyphenyl)-5-(3-hydroxypropyl)-5-azaspiro[2.4]heptan-4-one | 330.0 |
| 1-1 | 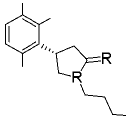 | (*S*)-4-(2,3-Dichloro-6-hydroxyphenyl)-1-(3-hydroxypropyl)pyrrolidine-2-thione | 320.0 |
| 2-1 | 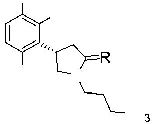 | (*S*)-1-(3-Aminopropyl)-4-(2,3-dichloro-6-hydroxyphenyl)pyrrolidine-2-thione | 319.1 |
| 3-1 | 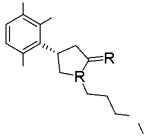 | (*S*)-4-(2,3-Dichloro-6-hydroxyphenyl)-1-(3-(methylamino)propyl)pyrrolidine-2-thione | 333.1 |
| 4-1 | 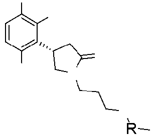 | (*S*)-4-(2,3-Dichloro-6-hydroxyphenyl)-1-(3-(methoxyamino)propyl)pyrrolidine-2-thione | 349.0 |
| 5-1 | 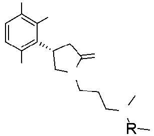 | (*S*)-4-(2,3-Dichloro-6-hydroxyphenyl)-1-(3-(methoxy(methyl)amino)propyl)pyrrolidine -2-thione | 363.1 |
| 6-1 | 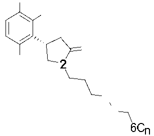 | (*S*)-4-(2,3-Dichloro-6-hydroxyphenyl)-1-(3-((2,2,2-trifluoroethoxy)amino)propyl)pyrrolidine-2-thione | 417.0 |
| 7-1 | 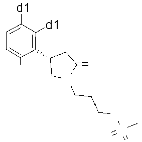 | (*S*)-*N*-(3-(4-(2,3-Dichloro-6-hydroxyphenyl)-2-thiopyrrolidin-1-yl)propyl)methanesulfonamide | 397.0 |
| 8-1 | 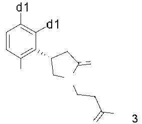 | (*S*)-3-(4-(2,3-Dichloro-6-hydroxyphenyl)-2-thiopyrrolidin-1-yl)propionamide | 333.0 |
| 9-1 | 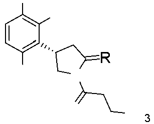 | (*S*)-3-Amino-1-(4-(2,3-dichloro-6-hydroxyphenyl)-2-thiopyrrolidin-1-yl)propan-1-one | 333.0 |
| 10-1 | 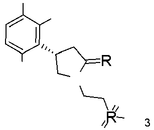 | (*S*)-2-(4-(2,3-Dichloro-6-hydroxyphenyl)-2-thiopyrrolidin-1-yl)ethane-1-sulfonamide | 369.0 |
| 11-1 | 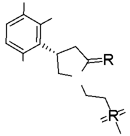 | (*S*)- (2-(4-(2,3-Dichloro-6-hydroxyphenyl)-2-thiopyrrolidin-1-yl)ethyl)(imino)(methyl)-λ⁶-sulfanone | 367.0 |
| 12-1 | 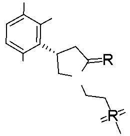 | (*S*)-4-(2,3-Dichloro-6-hydroxyphenyl)-1-(2-(methylsulfonyl)ethyl)pyrrolidine-2-thione | 368.0 |
| 13-1 | 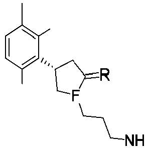 | (*S*)-1-(3-Aminopropyl)-4-(2-chloro-6-hydroxy-3-methylphenyl)pyrrolidine-2-thione | 299.1 |
| 14-1 | 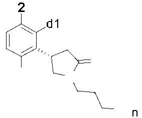 | (*S*)-1-(3-Aminopropyl)-4-(2-chloro-3-fluoro-6-hydroxyphenyl)pyrrolidine-2-thione | 303.1 |
| 15-1 | 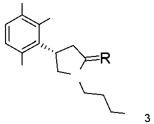 | (*S*)-1-(3-Aminopropyl)-4-(3-chloro-2-fluoro-6-hydroxyphenyl)pyrrolidine-2-thione | 303.1 |
| 16-1 | 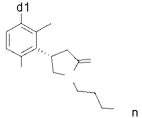 | (*S*)-1-(3-Aminopropyl)-4-(3-chloro-6-hydroxy-2-methylphenyl)pyrrolidine-2-thione | 299.1 |
| 17-1 | 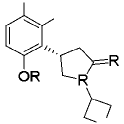 | (*S*)-1-(Azetidin-3-yl)-4-(2,3-dichloro-6-hydroxyphenyl)pyrrolidine-2-thione | 317.0 |
| 18-1 | 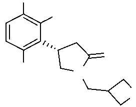 | (*S*)-1-(Azetidin-3-ylmethyl)-4-(2,3-dichloro-6-hydroxyphenyl)pyrrolidine-2-thione | 331.0 |
| 19-1 | 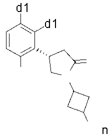 | (*S*)-1-(3-Aminocyclobutyl)-4-(2,3-dichloro-6-hydroxyphenyl)pyrrolidine-2-thione | 331.0 |
| 20-1 | 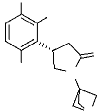 | (*S*)-1-(1-Azabicyclo[1.1.1]pentan-3-yl)-4-(2,3-dichloro-6-hydroxyphenyl)pyrrolidine-2-thione | 329.0 |
| 21-1 | 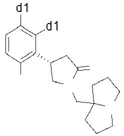 | (*S*)-4-(2,3-Dichloro-6-hydroxyphenyl)-1-((tetrahydro-1H-pyrrolizin-7a(5H)-yl)methyl)pyrrolidine-2-thione | 385.1 |
| 22-1 | 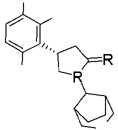 | (*S*)-1-(3-Azabicyclo[3.2.1]octan-8-yl)-4-(2,3-dichloro-6-hydroxyphenyl)pyrrolidine-2-thione | 371.1 |
| 23-1 | 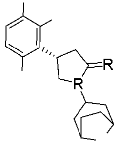 | (*S*)-1-(8-Azabicyclo[3.2.1]octan-3-yl)-4-(2,3-dichloro-6-hydroxyphenyl)pyrrolidine-2-thione | 371.1 |
| 24-1 | 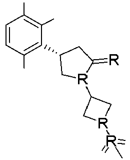 | (*S*)-4-(2,3-Dichloro-6-hydroxyphenyl)-1-(1-(methylsulfonyl)azetidin-3-yl)pyrrolidine-2-thione | 395.0 |
| 25-1 | 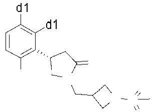 | (*S*)-4-(2,3-Dichloro-6-hydroxyphenyl)-1-((1-(methylsulfonyl)azetidin-3-yl)methyl)pyrrolidine-2-thione | 409.0 |
| 26-1 | 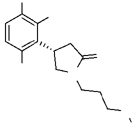 | (*S*)- 4-(2,3-Dichloro-6-hydroxyphenyl)-1-(3-(hydroxyamino)propyl)pyrrolidine-2-thione | 335.0 |
| 27-1 | 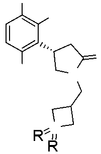 | (*S*)-4-(2,3-Dichloro-6-hydroxyphenyl)-1-((1,1-dioxothietan-3-yl)methyl)pyrrolidine-2-thione | 380.0 |
| 28-1 | 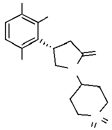 | (*S*)-4-(2,3-Dichloro-6-hydroxyphenyl)-1-(1,1-dioxotetrahydro-2H-thiapyran-4-yl)pyrrolidine-2-thione | 394.0 |
| 29-1 | 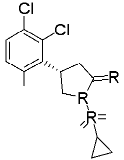 | (*S*)-1-(Cyclopropylsulfonyl)-4-(2,3-dichloro-6-hydroxyphenyl)pyrrolidine-2-thione | 366.0 |
| 30-1 | 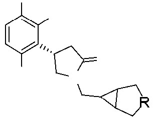 | (*S*)-1-((3-Oxabicyclo[3.1.0]hexan-6-yl)methyl)-4-(2,3-dichloro-6-hydroxyphenyl)pyrrolidine-2-thione | 358.0 |
| 31-1 | 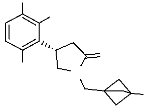 | (*S*)-4-(2,3-Dichloro-6-hydroxyphenyl)-1-((3-hydroxybicyclo[1.1.1]pentan-1-yl)methyl)pyrrolidine-2-thione | 358.0 |
| 32-1 | 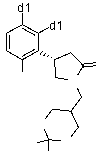 | (*S*)-4-(2,3-Dichloro-6-hydroxyphenyl)-1-((2,2-difluoro-1,3-dioxan-5-yl)methyl)pyrrolidine-2-thione | 398.0 |
| 33-1 | 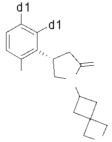 | (*S*)-4-(2,3-Dichloro-6-hydroxyphenyl)-1-(2-oxaspiro[3.3]heptan-6-yl)pyrrolidine-2-thione | 358.0 |
| 34-1 | 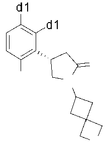 | (*S*)-4-(2,3-Dichloro-6-hydroxyphenyl)-1-(2-azaspiro[3.3]heptan-6-yl)pyrrolidine-2-thione | 357.1 |
| 35-1 | 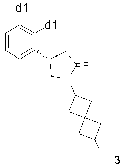 | (*S*)-1-(6-Aminospiro[3.3]heptan-2-yl)-4-(2,3-dichloro-6-hydroxyphenyl)pyrrolidine-2-thione | 371.1 |
| 36-1 | 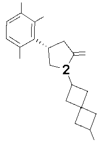 | (*S*)-4-(2,3-Dichloro-6-hydroxyphenyl)-1-(6-hydroxyspiro[3.3]heptan-2-yl)pyrrolidine-2-thione | 372.1 |
| 37-1 | 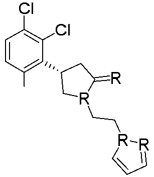 | (*S*)-1-(2-(1H-Pyrazol-1-yl)ethyl)-4-(2,3-dichloro-6-hydroxyphenyl)pyrrolidine-2-thione | 356.0 |
| 38-1 | 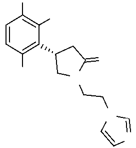 | (*S*)-1-(2-(1H-Imidazol-1-yl)ethyl)-4-(2,3-dichloro-6-hydroxyphenyl)pyrrolidine-2-thione | 356.0 |
| 39-1 | 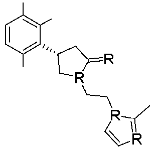 | (*S*)-4-(2,3-Dichloro-6-hydroxyphenyl)-1-(2-(2-methyl-1H-imidazol-1-yl)ethyl)pyrrolidine-2-thione | 370.0 |
| 40-1 | 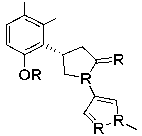 | (*S*)-4-(2,3-Dichloro-6-hydroxyphenyl)-1-(1-methyl-1H-pyrazol-4-yl)pyrrolidine-2-thione | 342.0 |
| 41-1 | 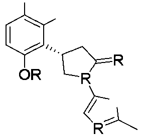 | (*S*)-4-(2,3-Dichloro-6-hydroxyphenyl)-1-(2-methyl-1H-imidazol-5-yl)pyrrolidine-2-thione | 342.0 |
| 42-1 | 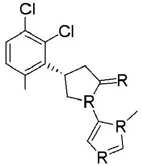 | (*S*)-4-(2,3-Dichloro-6-hydroxyphenyl)-1-(1-methyl-1H-imidazol-5-yl)pyrrolidine-2-thione | 342.0 |
| 43-1 | 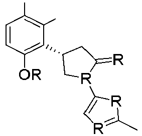 | (*S*)-4-(2,3-Dichloro-6-hydroxyphenyl)-1-(2-methylthiazol-5-yl)pyrrolidine-2-thione | 359.0 |
| 44-1 | 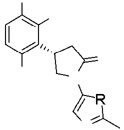 | (*S*)-4-(2,3-Dichloro-6-hydroxyphenyl)-1-(2-methyloxazol-5-yl)pyrrolidine-2-thione | 343.0 |
| 45-1 | 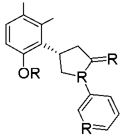 | (*S*)-4-(2,3-Dichloro-6-hydroxyphenyl)-1-(pyridin-3-yl)pyrrolidine-2-thione | 339.0 |
| 46-1 | 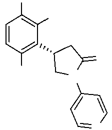 | (*S*)-4-(2,3-Dichloro-6-hydroxyphenyl)-1-(pyridin-4-yl)pyrrolidine-2-thione | 339.0 |
| 47-1 | 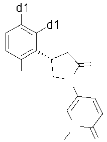 | (*S*)-5-(4-(2,3-Dichloro-6-hydroxyphenyl)-2-thiopyrrolidin-1-yl)-1-methylpyridin-2(1H)-one | 369.0 |
| 48-1 | 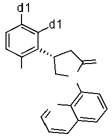 | (*S*)-4-(2,3-Dichloro-6-hydroxyphenyl)-1-(quinolin-8-yl)pyrrolidine-2-thione | 389.0 |
| 49-1 | 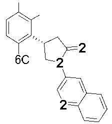 | (*S*)-4-(2,3-Dichloro-6-hydroxyphenyl)-1-(quinolin-3-yl)pyrrolidine-2-thione | 389.0 |
| 50-1 | 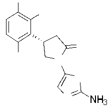 | (*S*)-4-(2,3-Dichloro-6-hydroxyphenyl)-1-(2-(trifluoromethyl)thiazol-5-yl)pyrrolidine-2-thione | 412.9 |
| 51-1 | 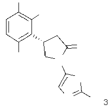 | (*S*)-4-(2,3-Dichloro-6-hydroxyphenyl)-1-(2-(trifluoromethyl)-1H-imidazol-5-yl)pyrrolidine-2-thione | 396.0 |
| 52-1 | 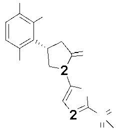 | (*S*)-4-(2,3-Dichloro-6-hydroxyphenyl)-1-(2-(methylsulfonyl)thiazol-5-yl)pyrrolidine-2-thione | 422.9 |
| 53-1 | 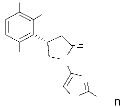 | (*S*)-1-(2-Aminooxazol-4-yl)-4-(2,3-dichloro-6-hydroxyphenyl)pyrrolidine-2-thione | 344.0 |
| 54-1 | 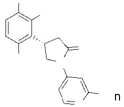 | (*S*)-1-(2-Aminopyridin-4-yl)-4-(2,3-dichloro-6-hydroxyphenyl)pyrrolidine-2-thione | 354.0 |
| 55-1 | 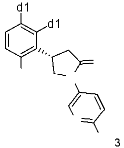 | (*S*)-1-(6-Aminopyridin-3-yl)-4-(2,3-dichloro-6-hydroxyphenyl)pyrrolidine-2-thione | 354.0 |
| 56-1 | 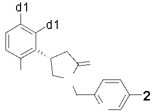 | (*S*)-4-(2,3-Dichloro-6-hydroxyphenyl)-1-((5-fluoropyridin-2-yl)methyl)pyrrolidine-2-thione | 371.0 |
| 57-1 | 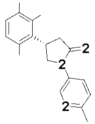 | (*S*)-4-(2,3-Dichloro-6-hydroxyphenyl)-1-(6-methylpyridin-3-yl)pyrrolidine-2-thione | 353.0 |
| 58-1 | 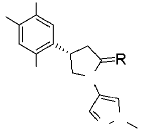 | (*S*)-4-(4,5-Dichloro-2-hydroxyphenyl)-1-(1-methyl-1H-pyrazol-4-yl)pyrrolidine-2-thione | 342.0 |
| 59-1 | 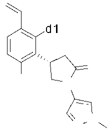 | (*S*)-4-(2-Chloro-6-hydroxy-3-vinylphenyl)-1-(1-methyl-1H-pyrazol-4-yl)pyrrolidine-2-thione | 334.1 |
| 60-1 | 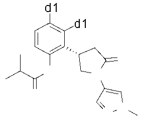 | (*S*)-3,4-Dichloro-2-(1-(1-methyl-1H-pyrazol-4-yl)-5-thiopyrrolidin-3-yl)phenyl isobutyrate | 412.1 |
| 61-1 | 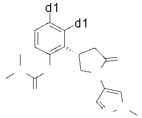 | (*S*)-3,4-Dichloro-2-(1-(1-methyl-1H-pyrazol-4-yl)-5-thiopyrrolidin-3-yl)phenyl dimethylcarbamate | 413.1 |
| 62-1 | 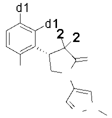 | (*S*)-4-(2,3-Dichloro-6-hydroxyphenyl)-3,3-difluoro-1-(1-methyl-1H-pyrazol-4-yl)pyrrolidine-2-thione | 378.0 |
| 63-1 | 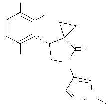 | (*S*)-7-(2,3-Dichloro-6-hydroxyphenyl)-5-(1-methyl-1H-pyrazol-4-yl)-5-azaspiro[2.4]heptane-4-thione | 368.0 |
| 64-1 | 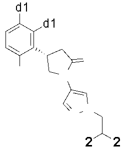 | (*S*)-4-(2,3-Dichloro-6-hydroxyphenyl)-1-(1-(2,2-difluoroethyl)-1H-pyrazol-4-yl)pyrrolidine-2-thione | 392.0 |
| 65-1 | 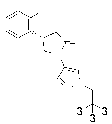 | (*S*)-4-(2,3-Dichloro-6-hydroxyphenyl)-1-(1-(2,2,2-trifluoroethyl)-1H-pyrazol-4-yl)pyrrolidine-2-thione | 410.0 |
| 66-1 | 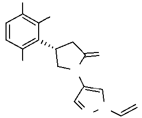 | (*S*)-4-(2,3-Dichloro-6-hydroxyphenyl)-1-(1-vinyl-1H-pyrazol-4-yl)pyrrolidine-2-thione | 354.0 |
| 67-1 | 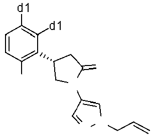 | (*S*)-1-(1-Allyl-1H-pyrazol-4-yl)-4-(2,3-dichloro-6-hydroxyphenyl)pyrrolidine-2-thione | 368.0 |
| 68-1 | 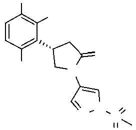 | (*S*)-4-(2,3-Dichloro-6-hydroxyphenyl)-1-(1-(methylsulfonyl)-1H-pyrazol-4-yl)pyrrolidine-2-thione | 406.0 |
| 69-1 | 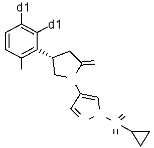 | (*S*)-1-(1-(Cyclopropylsulfonyl)-1H-pyrazol-4-yl)-4-(2,3-dichloro-6-hydroxyphenyl)pyrrolidine-2-thione | 432.0 |
| 70-1 | 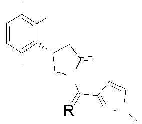 | (*S*)- (4-(2,3-Dichloro-6-hydroxyphenyl)-2-thiopyrrolidin-1-yl)(1-methyl-1H-pyrazol-3-yl)methanone | 370.0 |
| 71-1 | 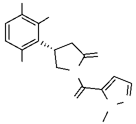 | (*S*)- (4-(2,3-Dichloro-6-hydroxyphenyl)-2-thiopyrrolidin-1-yl)(1-methyl-1H-pyrazol-5-yl)methanone | 370.0 |
| 72-1 | 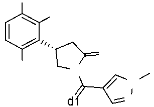 | (*S*)- (4-(2,3-Dichloro-6-hydroxyphenyl)-2-thiopyrrolidin-1-yl)(1-methyl-1H-pyrazol-4-yl)methanone | 370.0 |
| 73-1 | 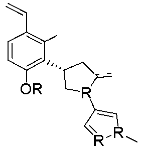 | (*S*)-4-(2-Chloro-6-hydroxy-3-vinylphenyl)-1-(1-methyl-1H-pyrazol-4-yl)pyrrolidin-2-one | 318.1 |
| 74-1 | 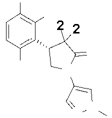 | (*S*)-4-(2,3-Dichloro-6-hydroxyphenyl)-3,3-difluoro-1-(1-methyl-1H-pyrazol-4-yl)pyrrolidin-2-one | 362.0 |
| 75-1 | 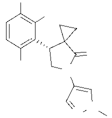 | (*S*)-7-(2,3-Dichloro-6-hydroxyphenyl)-5-(1-methyl-1H-pyrazol-4-yl)-5-azaspiro[2.4]heptan-4-one | 352.1 |
| 76-1 | 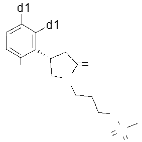 | (*S*)-*N*-(3-(4-(2,3-Dichloro-6-hydroxyphenyl)-2-oxopyrrolidin-1-yl)propyl)methanesulfonamide | 381.2 |
| 77-1 | 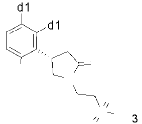 | (*S*)-2-(4-(2,3-Dichloro-6-hydroxyphenyl)-2-oxopyrrolidin-1-yl)ethane-1-sulfonamide | 353.0 |
| 78-1 | 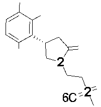 | (*S*)-4-(2,3-Dichloro-6-hydroxyphenyl)-1-(2-(S-methylsulfonylimino)ethyl)pyrrolidin-2-one | 351.0 |
| 79-1 | 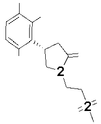 | (*S*)-4-(2,3-Dichloro-6-hydroxyphenyl)-1-(2-(methylsulfonyl)ethyl)pyrrolidin-2-one | 352.0 |
| 80-1 | 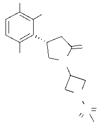 | (*S*)-4-(2,3-Dichloro-6-hydroxyphenyl)-1-(1-(methylsulfonyl)azetidin-3-yl)pyrrolidin-2-one | 379.0 |
| 81-1 | 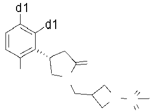 | (*S*)-4-(2,3-Dichloro-6-hydroxyphenyl)-1-((1-(methylsulfonyl)azetidin-3-yl)methyl)pyrrolidin-2-one | 393.0 |
| 82-1 | 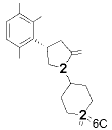 | (*S*)-4-(2,3-Dichloro-6-hydroxyphenyl)-1-(1-imino-1-oxohexahydro-116-thiopyran-4-yl)pyrrolidin-2-one | 377.0 |
| 83-1 | 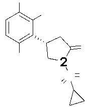 | (*S*)-1-(Cyclopropylsulfonyl)-4-(2,3-dichloro-6-hydroxyphenyl)pyrrolidin-2-one | 350.0 |
| 84-1 | 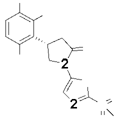 | (*S*)-4-(2,3-Dichloro-6-hydroxyphenyl)-1-(2-(methylsulfonyl)thiazol-5-yl)pyrrolidin-2-one | 407.0 |
| 85-1 | 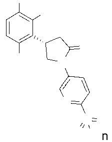 | (*S*)-5-(4-(2,3-Dichloro-6-hydroxyphenyl)-2-oxopyrrolidin-1-yl)pyridine-2-sulfonamide | 402.0 |
| 86-1 | 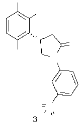 | (*S*)-3-(4-(2,3-Dichloro-6-hydroxyphenyl)-2-oxopyrrolidin-1-yl)benzenesulfonamide | 401.0 |
| 87-1 | 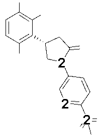 | (*S*)-4-(2,3-Dichloro-6-hydroxyphenyl)-1-(6-(methylsulfonyl)pyridin-3-yl)pyrrolidin-2-one | 401.0 |
| 88-1 | 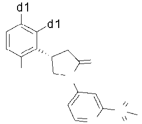 | (*S*)-4-(2,3-Dichloro-6-hydroxyphenyl)-1-(2-(methylsulfonyl)pyridin-4-yl)pyrrolidin-2-one | 401.0 |
| 89-1 | 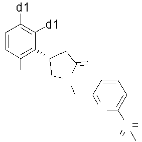 | (*S*)-4-(2,3-Dichloro-6-hydroxyphenyl)-1-((6-(methylsulfonyl)pyridin-2-yl)methyl)pyrrolidin-2-one | 415.0 |
| 90-1 | 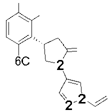 | (*S*)-4-(2,3-Dichloro-6-hydroxyphenyl)-1-(1-vinyl-1H-pyrazol-4-yl)pyrrolidin-2-one | 338.0 |
| 91-1 | 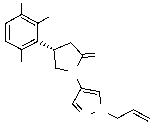 | (*S*)-1-(1-Allyl-1H-pyrazol-4-yl)-4-(2,3-dichloro-6-hydroxyphenyl)pyrrolidin-2-one | 352.1 |
| 92-1 | 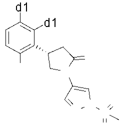 | (*S*)-4-(2,3-Dichloro-6-hydroxyphenyl)-1-(1-(methylsulfonyl)-1H-pyrazol-4-yl)pyrrolidin-2-one | 390.0 |
| 93-1 | 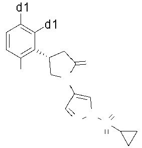 | (*S*)-1-(1-(Cyclopropylsulfonyl)-1H-pyrazol-4-yl)-4-(2,3-dichloro-6-hydroxyphenyl)pyrrolidin-2-one | 416.0 |
| 94-1 | 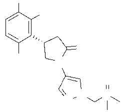 | (*S*)-4-(2,3-Dichloro-6-hydroxyphenyl)-1-(1-((dimethylphosphoryl)methyl)-1H-pyrazol-4-yl)pyrrolidin-2-one | 402.0 |
| 95-1 | 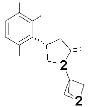 | (*S*)-1-(1-Azabicyclo[1.1.1]pentan-3-yl)-4-(2,3-dichloro-6-hydroxyphenyl)pyrrolidin-2-one | 313.0 |
| 96-1 | 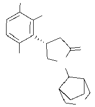 | (*S*)-1-(3-Azabicyclo[3.2.1]octan-8-yl)-4-(2,3-dichloro-6-hydroxyphenyl)pyrrolidin-2-one | 355.1 |
| 97-1 | 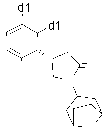 | (*S*)-1-(8-Azabicyclo[3.2.1]octan-3-yl)-4-(2,3-dichloro-6-hydroxyphenyl)pyrrolidin-2-one | 355.1 |
| 98-1 | 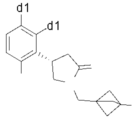 | (*S*)-4-(2,3-Dichloro-6-hydroxyphenyl)-1-(3-hydroxybicyclo[1.1.1]pentan-1-yl)methyl)pyrrolidin-2-one | 342.1 |
| 99-1 | 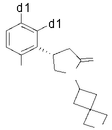 | (*S*)-4-(2,3-Dichloro-6-hydroxyphenyl)-1-(2-oxaspiro[3.3]heptan-6-yl)pyrrolidin-2-one | 342.1 |
| 100-1 | 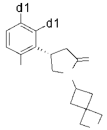 | (*S*)-4-(2,3-Dichloro-6-hydroxyphenyl)-1-(2-azaspiro[3.3]heptan-6-yl)pyrrolidin-2-one | 341.2 |
| 101-1 | 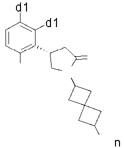 | (*S*)-1-(6-Aminospiro[3.3]heptan-2-yl)-4-(2,3-dichloro-6-hydroxyphenyl)pyrrolidin-2-one | 355.1 |
| 102-1 | 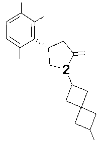 | (*S*)-4-(2,3-Dichloro-6-hydroxyphenyl)-1-(6-hydroxyspiro[3.3]heptan-2-yl)pyrrolidin-2-one | 356.1 |
| 103-1 | 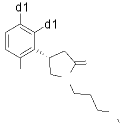 | (*S*)-4-(2,3-Dichloro-6-hydroxyphenyl)-1-(3-(hydroxyamino)propyl)pyrrolidin-2-one | 319.0 |
| 104-1 | 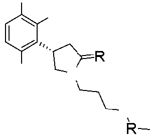 | (*S*)-4-(2,3-Dichloro-6-hydroxyphenyl)-1-(3-(methoxyamino)propyl)pyrrolidin-2-one | 333.1 |
| 105-1 | 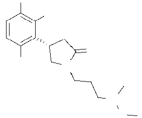 | (*S*)-4-(2,3-Dichloro-6-hydroxyphenyl)-1-(3-(methoxy(methyl)amino)propyl)pyrrolidin-2-one | 347.1 |
| 106-1 | 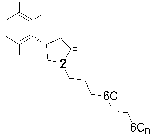 | (*S*)-4-(2,3-Dichloro-6-hydroxyphenyl)-1-(3-((2,2,2-trifluoroethoxy)amino)propyl)pyrrolidin-2-one | 401.1 |
| 107-1 | 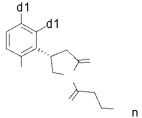 | (*S*)-1-(3-Aminopropanoyl)-4-(2,3-dichloro-6-hydroxyphenyl)pyrrolidin-2-one | 317.0 |
| 108-1 | 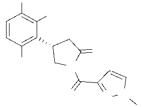 | (*S*)-4-(2,3-Dichloro-6-hydroxyphenyl)-1-(1-methyl-1H-pyrazole-3-carbonyl)pyrrolidin-2-one | 354.0 |
| 109-1 | 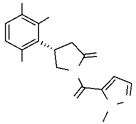 | (*S*)-4-(2,3-Dichloro-6-hydroxyphenyl)-1-(1-methyl-1H-pyrazole-5-carbonyl)pyrrolidin-2-one | 354.0 |
| 110-1 | 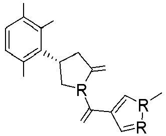 | (*S*)-4-(2,3-Dichloro-6-hydroxyphenyl)-1-(1-methyl-1H-pyrazole-4-carbonyl)pyrrolidin-2-one | 354.0 |
| 111-1 | 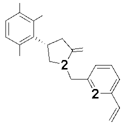 | (*S*)-4-(2,3-Dichloro-6-hydroxyphenyl)-1-((6-vinylpyridin-2-yl)methyl)pyrrolidin-2-one | 363.1 |
| 112-1 | 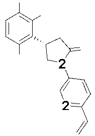 | (*S*)-4-(2,3-Dichloro-6-hydroxyphenyl)-1-(6-vinylpyridin-3-yl)pyrrolidin-2-one | 349.0 |
| 113-1 | 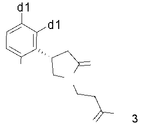 | (*S*)-3-(4-(2,3-Dichloro-6-hydroxyphenyl)-2-oxopyrrolidin-1-yl)propanethioamide | 333.1 |
| 114-1 | 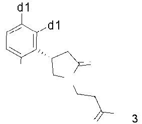 | (*S*)-3-(4-(2,3-Dichloro-6-hydroxyphenyl)-2-oxopyrrolidin-1-yl)propionimide | 316.0 |
| 115-1 | 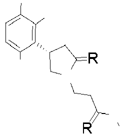 | (*S*)-3-(4-(2,3-Dichloro-6-hydroxyphenyl)-2-oxopyrrolidin-1-yl)-N-hydroxypropionamide | 332.9 |
| 116-1 | 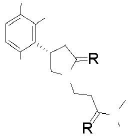 | (*S*)-3-(4-(2,3-Dichloro-6-hydroxyphenyl)-2-oxopyrrolidin-1-yl)-N-hydroxy-N-methylpropionamide | 347.0 |
| 117-1 | 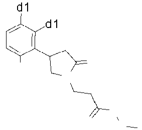 | (*S*)-3-(4-(2,3-Dichloro-6-hydroxyphenyl)-2-oxopyrrolidin-1-yl)-N-methoxypropionamide | 347.0 |
| 118-1 | 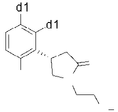 | (*S*)-4-(2,3-Dichloro-6-hydroxyphenyl)-1-(2-(hydroxyamino)ethyl)pyrrolidin-2-one | 304.9 |
| 119-1 | 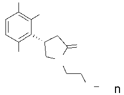 | (*S*)-1-(2-(Aminooxy)ethyl)-4-(2,3-dichloro-6-hydroxyphenyl)pyrrolidin-2-one | 304.9 |
| 120-1 | 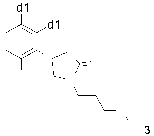 | (*S*)-1-(3-(Aminooxy)propyl)-4-(2,3-dichloro-6-hydroxyphenyl)pyrrolidin-2-one | 319.0 |
| 121-1 | 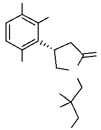 | (S)-4-(2,3-Dichloro-6-hydroxyphenyl)-1-(2,2-difluoro-3-hydroxypropyl)pyrrolidine-2-thione | 355.8 |
| 122-1 | 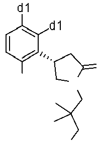 | (S)-4-(2,3-Dichloro-6-hydroxyphenyl)-1-(3-hydroxy-2,2-dimethylpropyl)pyrrolidine-2-thione | 348.1 |
| 123-1 | 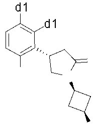 | (S)-4-(2,3-Dichloro-6-hydroxyphenyl)-1-((1s,3s)-3-hydroxycyclobutyl)pyrrolidine-2-thione | 332.0 |
| 124-1 | 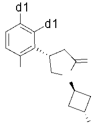 | (S)-4-(2,3-Dichloro-6-hydroxyphenyl)-1-((1r,3r)-3-hydroxycyclobutyl)pyrrolidine-2-thione | 332.0 |
| 125-1 | 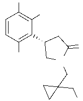 | (S)-4-(2,3-Dichloro-6-hydroxyphenyl)-1-((1-(hydroxymethyl)cyclopropyl)methyl)pyrrol idine-2-thione | 345.9 |
| 126-1 | 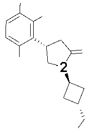 | (S)-4-(2,3-Dichloro-6-hydroxyphenyl)-1-((1r,3r)-3-(hydroxymethyl)cyclobutyl)pyrrolidine-2-thione | 345.9 |
| 127-1 | 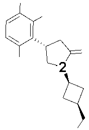 | (S)-4-(2,3-Dichloro-6-hydroxyphenyl)-1-((1s,3s)-3-(hydroxymethyl)cyclobutyl)pyrrolidine-2-thione | 345.9 |
| 128-1 | 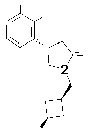 | (S)-4-(2,3-Dichloro-6-hydroxyphenyl)-1-(((1s,3s)-3-hydroxycyclobutyl)methyl)pyrrolidine-2-thione | 345.9 |
| 129-1 | 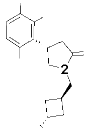 | (S)-4-(2,3-Dichloro-6-hydroxyphenyl)-1-(((1r,3r)-3-hydroxycyclobutyl)methyl)pyrrolidine-2-thione | 345.9 |
| 130-1 | 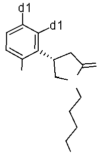 | (S)-4-(2,3-Dichloro-6-hydroxyphenyl)-1-(4-hydroxybutyl)pyrrolidine-2-thione | 333.9 |
| 131-1 | 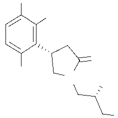 | (S)-4-(2,3-Dichloro-6-hydroxyphenyl)-1-((S)-2,3-dihydroxypropyl)pyrrolidine-2-thione | 335.9 |
| 132-1 | 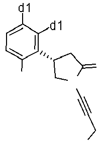 | (S)-4-(2,3-Dichloro-6-hydroxyphenyl)-1-(3-hydroxyprop-1-yn-1-yl)pyrrolidine-2-thione | 316.0 |
| 133-1 | 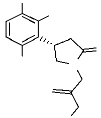 | (S)-4-(2,3-Dichloro-6-hydroxyphenyl)-1-(2-(hydroxymethyl)allyl)pyrrolidine-2-thione | 332.0 |
| 134-1 | 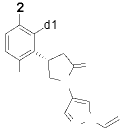 | (S)-4-(2-Chloro-3-fluoro-6-hydroxyphenyl)-1-(1-vinyl-1H-pyrazol-4-yl)pyrrolidine-2-thione | 338.0 |
| 135-1 | 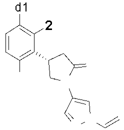 | (S)-4-(3-Chloro-2-fluoro-6-hydroxyphenyl)-1-(1-vinyl-1H-pyrazol-4-yl)pyrrolidine-2-thione | 338.0 |
| 136-1 | 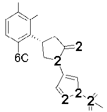 | (S)-4-(2-Chloro-3-fluoro-6-hydroxyphenyl)-1-(1-(methylsulfonyl)-1H-pyrazol-4-yl)pyrrolidine-2-thione | 390.0 |
| 137-1 | 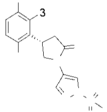 | (S)-4-(3-Chloro-2-fluoro-6-hydroxyphenyl)-1-(1-(methylsulfonyl)-1H-pyrazol-4-yl)pyrrolidine-2-thione | 390.0 |
| 138-1 | 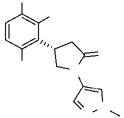 | (S)-4-(2-Chloro-3-fluoro-6-hydroxyphenyl)-1-(1-methyl-1H-pyrazol-4-yl)pyrrolidine-2-thione | 326.0 |
| 139-1 | 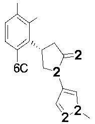 | (S)-4-(3-Chloro-2-fluoro-6-hydroxyphenyl)-1-(1-methyl-1H-pyrazol-4-yl)pyrrolidine-2-thione | 326.0 |
| 140-1 | 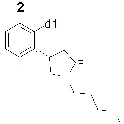 | (S)-4-(2-Chloro-3-fluoro-6-hydroxyphenyl)-1-(3-(hydroxyamino)propyl)pyrrolidine-2-thione | 319.1 |
| 141-1 | 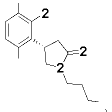 | (S)-4-(3-Chloro-2-fluoro-6-hydroxyphenyl)-1-(3-(hydroxyamino)propyl)pyrrolidine-2-thione | 319.1 |
| 142-1 | 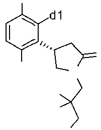 | (S)-4-(2-Chloro-3-fluoro-6-hydroxyphenyl)-1-(2,2-difluoro-3-hydroxypropyl)pyrrolidine-2-thione | 340.0 |
| 143-1 | 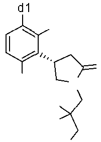 | (S)-4-(3-Chloro-2-fluoro-6-hydroxyphenyl)-1-(2,2-difluoro-3-hydroxypropyl)pyrrolidine-2-thione | 340.0 |
| 144-1 | 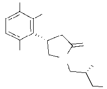 | (S)-4-(2-Chloro-3-fluoro-6-hydroxyphenyl)-1-((S)-2,3-dihydroxypropyl)pyrrolidine-2-thione | 320.0 |
| 145-1 | 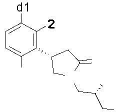 | (S)-4-(3-Chloro-2-fluoro-6-hydroxyphenyl)-1-((S)-2,3-dihydroxypropyl)pyrrolidine-2-thione | 320.0 |
| 146-1 | 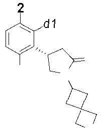 | (S)-4-(2-Chloro-3-fluoro-6-hydroxyphenyl)-1-(2-azaspiro[3.3]heptan-6-yl)pyrrolidine-2-thione | 341.1 |
| 147-1 | 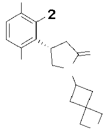 | (S)-4-(3-Chloro-2-fluoro-6-hydroxyphenyl)-1-(2-azaspiro[3.3]heptan-6-yl)pyrrolidine-2-thione | 341.1 |
| 148-1 | 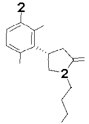 | (S)-4-(2-Chloro-3-fluoro-6-hydroxyphenyl)-1-(3-hydroxypropyl)pyrrolidine-2-thione | 304.0 |
| 149-1 | 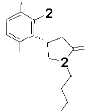 | (S)-4-(3-Chloro-2-fluoro-6-hydroxyphenyl)-1-(3-hydroxypropyl)pyrrolidine-2-thione | 304.0 |
| 150-1 | 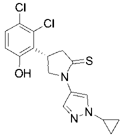 | (S)-1-(1-Cyclopropyl-1H-pyrazol-4-yl)-4-(2,3-dichloro-6-hydroxyphenyl)pyrrolidine-2-thione | 367.9 |
| 151-1 | 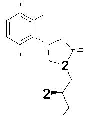 | (S)-4-(2,3-Dichloro-6-hydroxyphenyl)-1-((S)-2-fluoro-3-hydroxypropyl)pyrrolidine-2-thione | 338.0 |
| 152-1 | 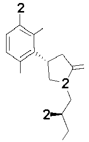 | (S)-4-(2-Chloro-3-fluoro-6-hydroxyphenyl)-1-((S)-2-fluoro-3-hydroxypropyl)pyrrolidine-2-thione | 322.0 |
| 153-1 | 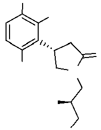 | (S)-4-(3-Chloro-2-fluoro-6-hydroxyphenyl)-1-((S)-2-fluoro-3-hydroxypropyl)pyrrolidine-2-thione | 322.0 |
| 154-1 | 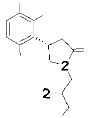 | (*S*)-4-(2,3-Dichloro-6-hydroxyphenyl)-1-((R)-2-fluoro-3-hydroxypropyl)pyrrolidine-2-thione | 338.0 |
| 155-1 | 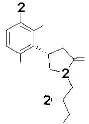 | (*S*)-4-(2-Chloro-3-fluoro-6-hydroxyphenyl)-1-((R)-2-fluoro-3-hydroxypropyl)pyrrolidine-2-thione | 322.0 |
| 156-1 | 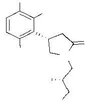 | (*S*)-4-(3-Chloro-2-fluoro-6-hydroxyphenyl)-1-((R)-2-fluoro-3-hydroxypropyl)pyrrolidine-2-thione | 322.0 |
| 157-1 | 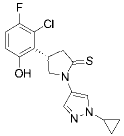 | (*S*)-4-(2-Chloro-3-fluoro-6-hydroxyphenyl)-1-(1-cyclopropyl-1H-pyrazol-4-yl)pyrrolidine-2-thione | 352.1 |
| 158-1 | 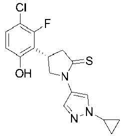 | (S)-4-(3-Chloro-2-fluoro-6-hydroxyphenyl)-1-(1-cyclopropyl-1H-pyrazol-4-yl)pyrrolidine-2-thione | 352.1 |
| 159-1 | 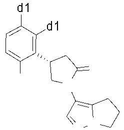 | (*S*)-4-(2,3-Dichloro-6-hydroxyphenyl)-1-(5,6-dihydro-4H-pyrrolo[1,2-b]pyrazol-3-yl)pyrrolidine-2-thione | 368.0 |
| 160-1 | 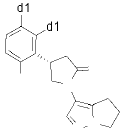 | (*S*)-4-(2,3-Dichloro-6-hydroxyphenyl)-1-(5,6-dihydro-4H-pyrrolo[1,2-b]pyrazol-3-yl)pyrrolidin-2-one | 352.0 |
| 161-1 | 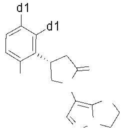 | (*S*)-4-(2,3-Dichloro-6-hydroxyphenyl)-1-(2,3-dihydropyrazolo[5,1-b]oxazol-7-yl)pyrrolidine-2-thione | 370.0 |
| 162-1 | 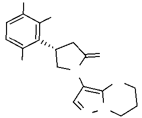 | (*S*)-4-(2,3-Dichloro-6-hydroxyphenyl)-1-(6,7-dihydro-5H-pyrazolo[5,1-b][1,3]oxazin-3-yl)pyrrolidine-2-thione | 384.0 |
| 163-1 | 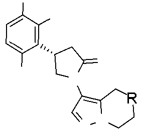 | (*S*)-4-(2,3-Dichloro-6-hydroxyphenyl)-1-(6,7-dihydro-4H-pyrazolo[5,1-c][1,4]oxazin-3-yl)pyrrolidine-2-thione | 384.0 |
| 164-1 | 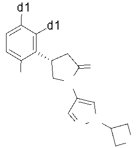 | (*S*)-4-(2,3-Dichloro-6-hydroxyphenyl)-1-(1-(oxetan-3-yl)-1H-pyrazol-4-yl)pyrrolidine-2-thione | 383.9 |
| 165-1 | 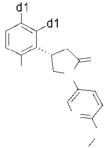 | (*S*)-4-(2,3-Dichloro-6-hydroxyphenyl)-1-(6-methoxypyridin-3-yl)pyrrolidine-2-thione | 369.0 |
| 166-1 | 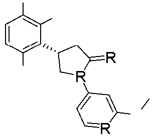 | (*S*)-4-(2,3-Dichloro-6-hydroxyphenyl)-1-(2-methoxypyridin-4-yl)pyrrolidine-2-thione | 369.0 |
| 167-1 | 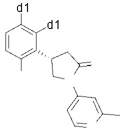 | (*S*)-4-(2,3-Dichloro-6-hydroxyphenyl)-1-(2-methylpyridin-4-yl)pyrrolidine-2-thione | 353.0 |
| 168-1 | 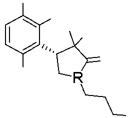 | (*S*)-4-(2,3-Dichloro-6-hydroxyphenyl)-3,3-difluoro-1-(3-hydroxypropyl)pyrrolidin-2-one | 340.0 |
| 169-1 | 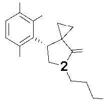 | (*S*)-7-(2,3-Dichloro-6-hydroxyphenyl)-5-(3-hydroxypropyl)-5-azaspiro[2.4]heptan-4-one | 330.0 |

| | | | |
|---|---|---|---|
| Note: the compounds other than Examples 1-57 in Table 2 were prepared in a similar manner to Examples 1-57. | | | |

### Biological Evaluation

**Test example 1:** Determination of inhibitory activity of compounds on Kv1.3 (hKv1.3) channels:

### Method:

### Electrophysiological testing

Potassium currents were measured using HEK293T cells expressing human Kv1.3 (hKv1.3) channels. In HEK293T cells, hKv1.3 had a current amplitude between 300 pA and 30 nA when stimulated at +40 mV.

### Cell preparation

Prior to the start of electrophysiological experiments, Kv1.3 cell lines should be maintained within 70% of the maximum density in the logarithmic growth phase. All reagents should be pre-warmed to 37°C before use.
1) Aspirate the spent medium from the 6 cm culture dish.
2) Add 1 mL of PBS, gently rock the dish to rinse the bottom, and then remove the PBS.
3) Add 1 mL of trypsin, and gently rock the dish to ensure the trypsin covers all the cells.
4) Incubate at 37°C for 2-3 minutes, and then gently pipette the cells to suspend the adherent cells.
5) Transfer the cell suspension to a new centrifuge tube and centrifuge at 1000 rpm for 5 minutes. Adjust the cell concentration to 2 × 10³ cells/mL, and then seed 300 µL of the cell suspension into each well of a 24-well plate containing cell climbing slides. Perform patch clamp experiments after the cells adhered properly.

The intracellular and extracellular solutions used were designed to mimic the cell physiological ionic composition.

Kv1.3 extracellular solution: 137 mM NaCl, 1 mM MgCl₂•6H₂O, 4 mM KCl, 1.8 mM CaCl₂•2H₂O, 10 mM D-Glucose, 10 mM HEPES, pH adjusted to 7.4 with NaOH.

Kv1.3 intracellular solution: 140 mM KCl, 10 mM EGTA, 5 mM MgCl₂•6H₂O, 10 mM HEPES, 5g-ATP, pH adjusted to 7.2 with KOH.

### Test compound dissolution

1) The test compound was dissolved in DMSO to prepare a 30 mM stock solution.
2) The stock solution of the test compound was serially diluted with DMSO to obtain secondary stock solutions at concentrations of 300 µM, 100 µM, 30 µM, 10 µM, and 3 µM.
3) 10 µL of each secondary stock solution was diluted into 10 mL of extracellular solution to achieve final concentrations of 300 nM, 100 nM, 30 nM, 10 nM, and 3 nM for electrophysiological testing.

### Data recording

Kv1.3 potassium current data were collected and stored on a computer using Patchmaster software via an EPC-10 amplifier.
1) The cell climbing slide was removed from the cell culture dish using forceps, immersed with extracellular solution, and placed into the bath chamber on the stage of an inverted microscope.
2) Glass micropipettes were pulled using a P-1000 micropipette puller, filled one-third of their volume with intracellular solution, and then mounted onto an electrode holder.
3) Using a motorized micromanipulator (Scientifica-Double 1U), the recording electrode was brought into contact with the cell surface. At this point, due to the sudden increase in electrode resistance, the current value represented by the seal test pulse displayed in the membrane test window would decrease. The positive pressure was removed, and a negative pressure of 0.5 cm H₂O was applied. It was observed that the sealing resistance increased rapidly until it reached the gigaseal. When the sealing resistance between the recording electrode and the cell membrane was > 1 GΩ, negative pressure was applied to rupture the membrane to form the whole-cell recording mode. After the ruptured membrane stabilized, the membrane capacitance (Cs) and series resistance (Rs) were compensated.
4) Stimulation procedure: the cell was voltage-clamped at -80 mV, depolarized to 40 mV for a duration of 200 ms, and then repolarized to -80 mV. Currents were recorded every 10 s.
5) Kv1.3 potassium channel currents were recorded at room temperature prior to drug application. After the control current value reached a steady state (indicated by overlapping of the four most consecutive current traces), cumulative drug application was performed. The negative control (0.1% DMSO) was applied first, followed by six increasing concentrations of the drugs.

### Data analysis

Raw Kv1.3 current peak data were extracted from PatchMaster software. The current inhibition rate was calculated as follows: Peak current inhibition rate = ((1-(Peak current compound)/(Peak current vehicle))). The mean and standard error were calculated for each concentration, and the concentration-effect relationship was fitted by the Hill equation: I = Imax·{1/[l+(C1/2/[C])h]}, where [C] represents the drug concentration, C1/2 is the half-maximal inhibitory concentration (IC50), and h is the Hill coefficient. Statistical analysis was performed using Graphpad Prism5.0 software.

The selected compounds of the present invention were screened in the above assay procedure to determine the percentage inhibition of Kv1.3 activity at a concentration of 10 nM, and the results are summarized in Table 3 below. In addition, the selected compounds of the present invention were screened in the above assay procedure to determine IC₅₀ values, and the results are summarized in Table 4 below.

**Table 3. Inhibition of Kv1.3 potassium channel activity by exemplary compounds at concentration of 10 nM**

| 10 nM inhibition range: A = inhibition ≥ 75%; B = inhibition ≥ 50% and < 75%; C = inhibition ≥ 25% and < 50%; D = inhibition ≥ 10% and < 25%; E = inhibition < 10%. | | |
|---|---|---|
| Compound No. | Structure | 10 nM inhibition rate (Kv1.3) |
| W1 | 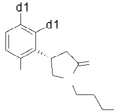 | C |
| 1 | 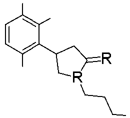 | C |
| 2 | 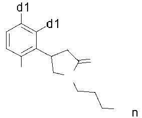 | B |
| 26 | 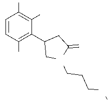 | D |
| 34 | 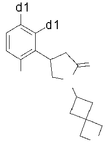 | C |
| 37 | 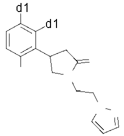 | E |
| 40 | 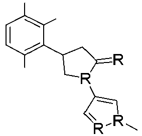 | B |
| 42 | 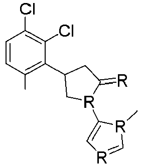 | E |
| 43 | 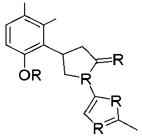 | D |
| 66 | 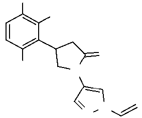 | C |
| 68 | 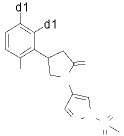 | C |
| 76 | 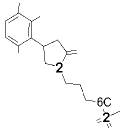 | D |
| 77 | 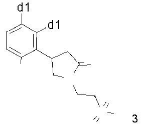 | D |
| 78 | 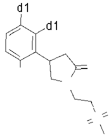 | D |
| 79 | 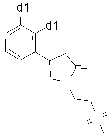 | E |
| 90 | 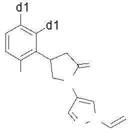 | B |
| 92 | 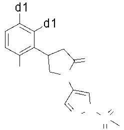 | B |
| 94 | 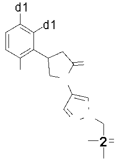 | C |
| 100 | 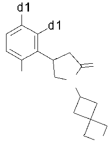 | A |
| 101 | 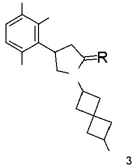 | B |
| 103 | 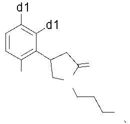 | A |
| 104 | 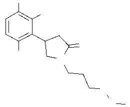 | D |
| 105 | 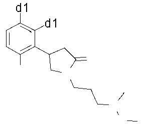 | E |
| 113 | 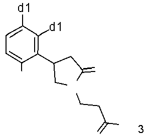 | E |
| 114 | 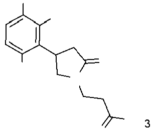 | D |
| 115 | 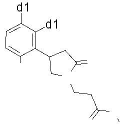 | C |
| 116 | 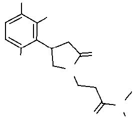 | E |
| 117 | 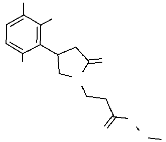 | D |
| 118 | 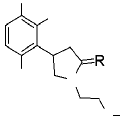 | C |
| 119 | 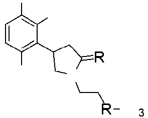 | D |
| 120 | 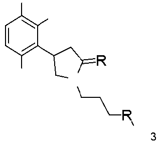 | E |
| 121 | 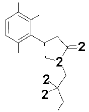 | A |
| 124 | 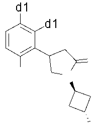 | C |
| 125 | 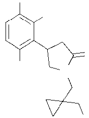 | D |
| 126 | 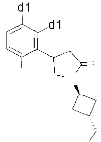 | D |
| 127 | 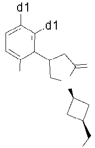 | D |
| 128 | 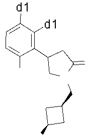 | D |
| 129 | 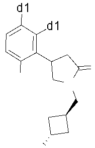 | D |
| 130 | 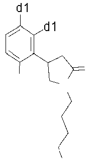 | C |
| 131 | 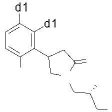 | B |
| 148 | 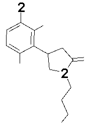 | D |
| 149 | 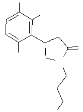 | E |
| 150 | 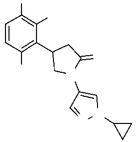 | B |
| 159 | 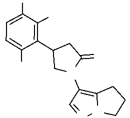 | C |
| 160 | 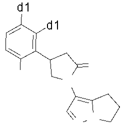 | C |
| 161 | 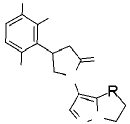 | B |
| 162 | 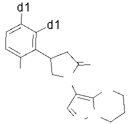 | D |
| 163 | 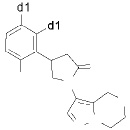 | C |
| 164 | 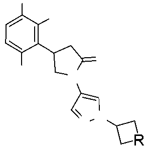 | B |
| 165 | 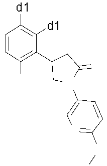 | D |
| 166 | 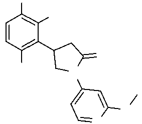 | D |
| 167 | 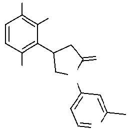 | D |
| 168 | 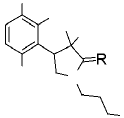 | E |
| 169 | 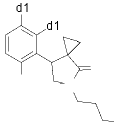 | E |

**Table 4. Inhibitory activity of exemplary compounds against Kv1.3 (hKv1.3) channels**

| IC₅₀ range: S = IC₅₀ ≤ 5 nM; A = IC₅₀ > 5 nM and ≤ 10 nM; B = IC₅₀ > 10 nM and ≤ 30 nM; C = IC₅₀ > 30 nM and ≤ 100 nM; D = IC₅₀ > 100 nM. | | |
|---|---|---|
| Compound No. | Structure | IC₅₀ (Kv1.3) |
| W1 | 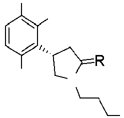 | B |
| W2 | 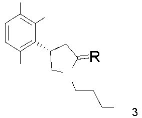 | B |
| 1-1 | 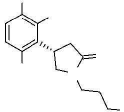 | A |
| 40-1 | 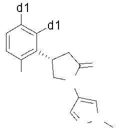 | S |
| 121-1 | 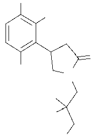 | B |
| 124-1 | 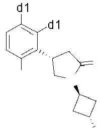 | A |
| 129-1 | 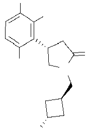 | S |
| 130-1 | 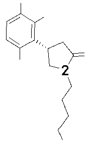 | S |
| 131 | 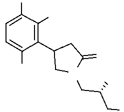 | C |
| 159-1 | 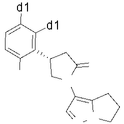 | A |

Under the present experimental conditions, the compounds of the present disclosure are more potent inhibitors of Kv1.3 than reference compounds W1 and W2. Reference compounds W1 and W2 are disclosed in patent WO 2022/076285 A1.

It should be understood that after reading the above content of the present invention, those skilled in the art can make various changes or modifications to the present invention, and these equivalent forms also fall within the scope defined by the appended claims of the present application.

## Claims

1. A compound of formula I or a pharmaceutically acceptable salt, an ester, a stereoisomer, a tautomer, a polymorph, a hydrate, a solvate, an N-oxide, an isotopically labelled compound, a metabolite or a prodrug thereof: 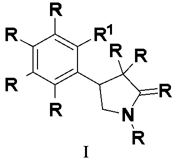 **characterised in that**:
R¹, R², R³ and R⁴ are the same or different, and are each independently selected from a hydrogen atom, halogen, C₁₋₆ alkyl, C₁₋₆ haloalkyl, C₁₋₆ alkoxy, C₁₋₆ haloalkoxy and C₂₋₆ alkenyl;
R⁵ is OR^{a}, wherein the R^{a} is selected from a hydrogen atom, -C(=O)R^{5A} and - C(=O)-NR^{5A}R^{5B}; R^{5A} and R^{5B} are the same or different, and are each independently selected from a hydrogen atom, alkyl, haloalkyl, cycloalkyl, halocycloalkyl and alkenyl;
R⁶ is -(CR^{b}R^{c})ₙR^{d} or -CH₂CR^{b}R^{c}CH₂R^{d}; R^{b} and R^{c} are the same or different, and are each independently selected from a hydrogen atom, halogen, amino, hydroxyl, C₁₋₆ alkyl, C₁₋₆ haloalkyl, 3- to 8-membered cycloalkyl, 3- to 8-membered halocycloalkyl, C₁₋₆ alkoxy, C₁₋₆ haloalkoxy, -NH(C₁₋₆ alkyl), -N(C₁₋₆ alkyl)₂ and C₂₋₆ alkenyl;
R^{d} is selected from hydroxyl, amino, hydroxyamino, -OR^{6A}, -O-NHR^{6A}, - NHR^{6A}, -NR^{6A}-OR^{6B}, -C(=O)R^{6A}, -C(=O)NR^{6A}OR^{6B}, -C(=NH)R^{6A}, - C(=NH)NR^{6A}R^{6B}, -NH-C(=O)R^{6A}, -S(=O)(=NH)R^{6A}, -S(=O)₂R^{6A}, -NH-S(=O)₂R^{6A}, -C(=O)-NR^{6A}R^{6B}, -C(=S)-NR^{6A}R^{6B}, -P(=O)R^{6A}R^{6B}, 3- to 8-membered cycloalkyl, 3- to 8-membered heterocyclyl, 6- to 12-membered spirocyclyl, 6- to 12-membered spiro heterocyclyl, a 5- to 12-membered bridged ring group, 5- to 12-membered bridged heterocyclyl, 6- to 10-membered aryl, 5- to 10-membered heteroaryl containing at least one nitrogen atom and 6- to 12-membered fused heterocyclyl, wherein the 3- to 8-membered cycloalkyl, 3- to 8-membered heterocyclyl, 6- to 12-membered spirocyclyl, 6- to 12-membered spiro heterocyclyl, 5- to 12-membered bridged ring group, 5- to 12-membered bridged heterocyclyl, 6- to 10-membered aryl, 5- to 10-membered heteroaryl containing at least one nitrogen atom and 6- to 12-membered fused heterocyclyl are optionally substituted with one or more R^{6C};
R^{6A} and R^{6B} are the same or different, and are each independently selected from a hydrogen atom, amino, C₁₋₆ alkyl, C₁₋₆ haloalkyl, C₂₋₆ alkenyl, 3- to 8-membered cycloalkyl, 3- to 8-membered heterocyclyl, 6- to 10-membered aryl and 5- to 10-membered heteroaryl, wherein the amino, C₁₋₆ alkyl, C₁₋₆ haloalkyl, C₂₋₆ alkenyl, 3- to 8-membered cycloalkyl, 3- to 8-membered heterocyclyl, 6- to 10-membered aryl and 5- to 10-membered heteroaryl are optionally substituted with one or more substituents selected from: halogen, hydroxyl, amino, cyano, C₁₋₆ alkyl, C₁₋₆ haloalkyl, 3- to 8-membered cycloalkyl, 3- to 8-membered halocycloalkyl, C₁₋₆ alkoxy, 3- to 8-membered cycloalkoxy and C₂₋₆ alkenyl;
R^{6C} is selected from halogen, amino, hydroxyl, cyano, C₁₋₆ alkyl, C₁₋₆ haloalkyl, C₁₋₆ alkoxy, 3- to 8-membered cycloalkoxy, C₁₋₆ haloalkoxy, 3- to 8-membered halocycloalkoxy, C₂₋₆ alkenyl, 3- to 8-membered cycloalkyl, 3- to 8-membered heterocyclyl, 6- to 10-membered aryl, 5- to 10-membered heteroaryl, oxo, -C(=O)R^{6C1}, -NH-C(=O)R^{6C1}, -S(=O)(=NH)R^{6C1}, -S(=O)₂R^{6C1}, -NH-S(=O)₂R^{6C1}, -C(=O)-NR^{6C1}R^{6C2}, -P(=O) R^{6C1}R^{6C2} and -C₁₋₆ alkyl- P(=O) R^{6C1}R^{6C2}, wherein the C₁₋₆ alkyl, C₁₋₆ haloalkyl, C₁₋₆ alkoxy, 3- to 8-membered cycloalkoxy, C₁₋₆ haloalkoxy, 3- to 8-membered halocycloalkoxy, C₂₋₆ alkenyl, 3- to 8-membered cycloalkyl, 3- to 8-membered heterocyclyl, 6- to 10-membered aryl and 5- to 10-membered heteroaryl are optionally substituted with one or more substituents selected from: halogen, hydroxyl, amino, cyano, C₁₋₆ alkyl, C₁₋₆ haloalkyl, 3- to 8-membered cycloalkyl, 3- to 8-membered halocycloalkyl, C₁₋₆ alkoxy, 3- to 8-membered cycloalkoxy and C₂₋₆ alkenyl;
R^{6C1} and R^{6C2} are the same or different, and are each independently selected from a hydrogen atom, C₁₋₆ alkyl, C₁₋₆ haloalkyl, 3- to 8-membered cycloalkyl and 3- to 8-membered heterocyclyl;
n is selected from 0, 1, 2, 3 and 4;
R⁷ and R⁸ are the same or different, and are each independently selected from a hydrogen atom, C₁₋₆ alkyl, halogen and C₁₋₆ haloalkyl; alternatively, R⁷ and R⁸, together with the carbon atom to which they are attached, form 3- to 6-membered cycloalkyl or 3- to 6-membered heterocyclyl, wherein the cycloalkyl and heterocyclyl are optionally substituted with one or more substituents selected from: halogen, hydroxyl, amino, C₁₋₆ alkyl, C₁₋₆ haloalkyl, C₁₋₆ alkoxy, C₁₋₆ haloalkoxy, - C(=O)C₁₋₆ alkyl, -C(=O)C₁₋₆ haloalkyl, -S(=O)C₁₋₆ alkyl, or -S(=O)C₁₋₆ haloalkyl;
X is O, S or Se.

2. The compound or the pharmaceutically acceptable salt, the ester, the stereoisomer, the tautomer, the polymorph, the hydrate, the solvate, the N-oxide, the isotopically labelled compound, the metabolite or the prodrug thereof according to claim 1, **characterised in that** the compound is a compound represented by formula (1-1), 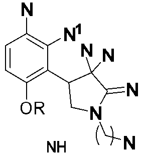 wherein:
n is selected from 0, 1, 2 and 3;
R¹, R², R⁷, R⁸, R^{a} and R^{d} are as defined in claim 1.

3. The compound or the pharmaceutically acceptable salt, the ester, the stereoisomer, the tautomer, the polymorph, the hydrate, the solvate, the N-oxide, the isotopically labelled compound, the metabolite or the prodrug thereof according to claim 2, **characterised in that**:
R¹ and R² are the same or different, and are each independently selected from a hydrogen atom, C₁₋₄ alkyl, C₂₋₆ alkenyl and halogen; preferably, R¹ and R² are the same or different, and are each independently selected from methyl, a chlorine atom and a fluorine atom; more preferably, R¹ and R² are both chlorine atoms;
R^{a} is selected from a hydrogen atom, -C(=O)C₁₋₆ alkyl, -C(=O)-NH(C₁₋₆ alkyl) and -C(=O)-N(C₁₋₆ alkyl)₂; preferably, R^{a} is selected from a hydrogen atom, - C(=O)CH(CH₃)₂ and -C(=O)-N(CH₃)₂; more preferably, R^{a} is a hydrogen atom;
R⁷ and R⁸ are both selected from hydrogen atoms, fluorine atoms and methyl; alternatively, R⁷ and R⁸, together with the carbon atom to which they are attached, form 3- to 6-membered cycloalkyl or 3- to 6-membered heterocyclyl; preferably, R⁷ and R⁸ are both hydrogen atoms or fluorine atoms, or, together with the carbon atom to which they are attached, form cyclopropyl; more preferably, R⁷ and R⁸ are both hydrogen atoms.

4. The compound or the pharmaceutically acceptable salt, the ester, the stereoisomer, the tautomer, the polymorph, the hydrate, the solvate, the N-oxide, the isotopically labelled compound, the metabolite or the prodrug thereof according to claim 2 or 3, **characterised in that**:
when n is 1, 2 or 3, R^{d} is selected from hydroxyl, amino, hydroxyamino, - OR^{6A}, -O-NHR^{6A}, -NHR^{6A}, -NR^{6A}-OR^{6B}, -C(=O)R^{6A}, -C(=O)NR^{6A}OR^{6B}, - C(=NH)R^{6A}, -C(=NH)NR^{6A}R^{6B}, -NH-C(=O)R^{6A}, -S(=O)(=NH)R^{6A}, -S(=O)₂R^{6A}, - NH-S(=O)₂R^{6A} -C(=O)-NR^{6A}R^{6B}, -C(=S)-NR^{6A}R^{6B}, or -P(=O)R^{6A}R^{6B}; R^{6A} and R^{6B} are the same or different, and are each independently selected from a hydrogen atom, amino, C₁₋₆ alkyl, C₁₋₆ haloalkyl, 3- to 8-membered cycloalkyl and 3- to 8-membered heterocyclyl; preferably, R^{6A} and R^{6B} are the same or different, and are each independently selected from a hydrogen atom, amino, C₁₋₆ alkyl and C₁₋₆ haloalkyl; more preferably, R^{6A} and R^{6B} are the same or different, and are each independently selected from a hydrogen atom, amino, methyl and trifluoroethyl.

5. The compound or the pharmaceutically acceptable salt, the ester, the stereoisomer, the tautomer, the polymorph, the hydrate, the solvate, the N-oxide, the isotopically labelled compound, the metabolite or the prodrug thereof according to claim 2 or 3, **characterised in that**:
when n is 0, R^{d} is selected from -C(=O)R^{6A} and -S(=O)₂R^{6A};
R^{6A} is selected from C₁₋₆ alkyl, C₁₋₆ haloalkyl, 3- to 8-membered cycloalkyl and 3- to 8-membered heterocyclyl, wherein the C₁₋₆ alkyl, 3- to 8-membered cycloalkyl, and 3- to 8-membered heterocyclyl are optionally substituted with one or more substituents selected from halogen, hydroxyl, amino, C₁₋₆ alkyl, C₁₋₆ haloalkyl and C₁₋₆ alkoxy; preferably, R^{6A} is selected from - C₂H₄NH₂, 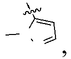 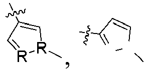 and cyclopropyl.

6. The compound or the pharmaceutically acceptable salt, the ester, the stereoisomer, the tautomer, the polymorph, the hydrate, the solvate, the N-oxide, the isotopically labelled compound, the metabolite or the prodrug thereof according to claim 2, 3 or 5, **characterised in that** R^{d} is selected from - C(=O)C₂H₄NH₂,

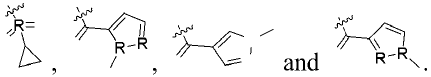

7. The compound or the pharmaceutically acceptable salt, the ester, the stereoisomer, the tautomer, the polymorph, the hydrate, the solvate, the N-oxide, the isotopically labelled compound, the metabolite or the prodrug thereof according to claim 1, **characterised in that** the compound is a compound represented by formula (1-2), 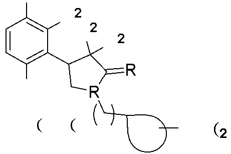 wherein:
ring A is selected from 3- to 8-membered cycloalkyl, 3- to 8-membered heterocyclyl, 6- to 12-membered spirocyclyl, 6- to 12-membered spiro heterocyclyl, a 5- to 12-membered bridged ring group, 5- to 12-membered bridged heterocyclyl, 6- to 10-membered aryl, 5- to 10-membered heteroaryl containing at least one nitrogen atom and 6- to 12-membered fused heterocyclyl;
k is selected from 0, 1, 2, 3 and 4;
n is selected from 0, 1, 2 and 3;
R¹, R², R⁷, R⁸, R^{a} and R^{6C} are as defined in claim 1.

8. The compound or the pharmaceutically acceptable salt, the ester, the stereoisomer, the tautomer, the polymorph, the hydrate, the solvate, the N-oxide, the isotopically labelled compound, the metabolite or the prodrug thereof according to claim 7, **characterised in that**:
R¹ and R² are the same or different, and are each independently selected from a hydrogen atom, C₁₋₄ alkyl, C₂₋₆ alkenyl and halogen; preferably, R¹ and R² are the same or different, and are each independently selected from methyl, a chlorine atom and a fluorine atom;
R^{a} is selected from a hydrogen atom, -C(=O)C₁₋₆ alkyl, -C(=O)-NH(C₁₋₆ alkyl) and -C(=O)-N(C₁₋₆ alkyl)₂; preferably, R^{a} is selected from a hydrogen atom, - C(=O)CH(CH₃)₂ and -C(=O)-N(CH₃)₂; more preferably, R^{a} is a hydrogen atom;
R⁷ and R⁸ are both selected from hydrogen atoms, fluorine atoms and methyl; alternatively, R⁷ and R⁸, together with the carbon atom to which they are attached, form 3- to 6-membered cycloalkyl or 3- to 6-membered heterocyclyl; preferably, R⁷ and R⁸ are both hydrogen atoms or fluorine atoms, or, together with the carbon atom to which they are attached, form cyclopropyl.

9. The compound or the pharmaceutically acceptable salt, the ester, the stereoisomer, the tautomer, the polymorph, the hydrate, the solvate, the N-oxide, the isotopically labelled compound, the metabolite or the prodrug thereof according to claim 7 or 8, **characterised in that**:
n is 0 or 1, and ring A is selected from 3- to 8-membered cycloalkyl, 3- to 8-membered heterocyclyl, 6- to 12-membered spirocyclyl, 6- to 12-membered spiro heterocyclyl, 5- to 12-membered bridged heterocyclyl, 6- to 10-membered aryl, 5-to 10-membered heteroaryl containing at least one nitrogen atom and 6- to 12-membered fused heterocyclyl; preferably, ring A is selected from 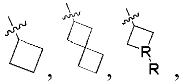 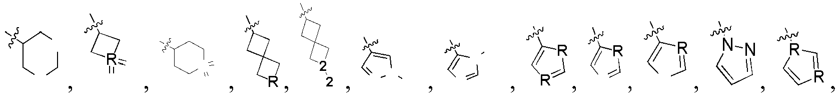 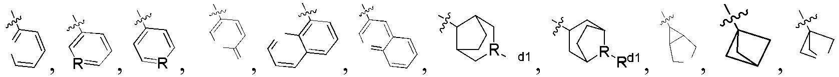 and 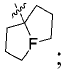
R^{d1} is selected from a hydrogen atom, C₁₋₆ alkyl, C₁₋₆ haloalkyl, 3- to 10-membered cycloalkyl, 3- to 10-membered heterocycloalkyl, -C(=O)C₁₋₆ alkyl, - C(=O)C₁₋₆ haloalkyl, -C(=O)C₃₋₆ cycloalkyl, -S(=O)₂C₁₋₆ alkyl, -S(=O)₂C₃₋₆ cycloalkyl, C₂₋₆ alkenyl and -C₁₋₆ alkyl- P(=O)(C₁₋₆ alkyl)₂; preferably, R^{d1} is selected from a hydrogen atom, C₁₋₆ alkyl, 3- to 6-membered cycloalkyl, 3- to 6-membered heterocycloalkyl, C₁₋₆ haloalkyl, -S(=O)₂C₁₋₆ alkyl, -S(=O)₂C₃₋₆ cycloalkyl and C₂₋₆ alkenyl; more preferably, R^{d1} is selected from a hydrogen atom, methyl, cyclopropyl, oxetanyl, difluoroethyl, trifluoroethyl, vinyl, allyl, - S(=O)₂CH₃ and -S(=O)₂ cyclopropyl.

10. The compound or the pharmaceutically acceptable salt, the ester, the stereoisomer, the tautomer, the polymorph, the hydrate, the solvate, the N-oxide, the isotopically labelled compound, the metabolite or the prodrug thereof according to any one of claims 7-9, **characterised in that**:
R^{6C} is selected from halogen, amino, hydroxyl, cyano, C₁₋₆ alkyl, C₁₋₆ haloalkyl, C₁₋₆ alkoxy, 3- to 8-membered cycloalkoxy, C₁₋₆ haloalkoxy, 3- to 8-membered halocycloalkoxy, C₂₋₆ alkenyl, oxo, -C(=O)R^{6C1}, -NH-C(=O)R^{6C1}, - S(=O)(=NH)R^{6C1}, -S(=O)₂R^{6C1}, -NH-S(=O)₂R^{6C1}, -C(=O)-NR^{6C1}R^{6C2}, -P(=O) R^{6C1}R^{6C2} and -C₁₋₆ alkyl- P(=O) R^{6C1}R^{6C2}; R^{6C1} and R^{6C2} are the same or different, and are each independently selected from a hydrogen atom, C₁₋₆ alkyl, C₁₋₆ haloalkyl, 3- to 8-membered cycloalkyl and 3- to 8-membered heterocyclyl; preferably, R^{6C} is selected from halogen, amino, hydroxyl, C₁₋₆ alkyl, C₁₋₆ haloalkyl and -S(=O)₂ C₁₋₆ alkyl; more preferably, R^{6C} is selected from a fluorine atom, amino, hydroxyl, methyl, trifluoromethyl and -S(=O)₂CH₃;
k is selected from 0, 1, 2, 3 and 4; preferably, k is 0 or 1.

11. The compound or the pharmaceutically acceptable salt, the ester, the stereoisomer, the tautomer, the polymorph, the hydrate, the solvate, the N-oxide, the isotopically labelled compound, the metabolite or the prodrug thereof according to any one of claims 7-10, **characterised in that** structural unit 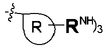 is selected from

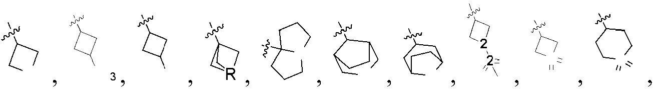

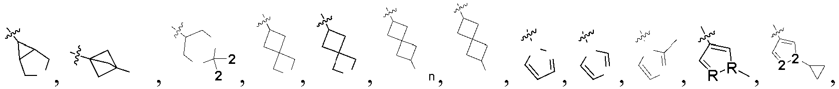

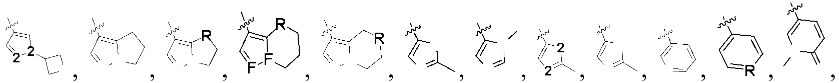

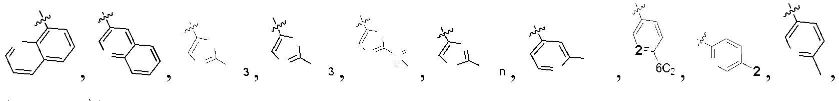

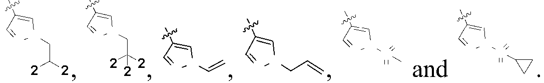

12. The compound or the pharmaceutically acceptable salt, the ester, the stereoisomer, the tautomer, the polymorph, the hydrate, the solvate, the N-oxide, the isotopically labelled compound, the metabolite or the prodrug thereof according to claim 1, **characterised in that** the compound is a compound represented by formula (1-3), 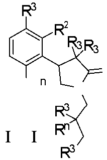 wherein:
R¹ , R², R⁷, R⁸, R^{a}, R^{b}, R^{c} and R^{d} are as defined in claim 1.

13. The compound or the pharmaceutically acceptable salt, the ester, the stereoisomer, the tautomer, the polymorph, the hydrate, the solvate, the N-oxide, the isotopically labelled compound, the metabolite or the prodrug thereof according to claim 12, **characterised in that**:
R¹ and R² are the same or different, and are each independently selected from a hydrogen atom, C₁₋₄ alkyl, C₂₋₆ alkenyl and halogen;
Rₐ is selected from a hydrogen atom, -C(=O)C₁₋₆ alkyl, -C(=O)-NH(C₁₋₆ alkyl) and -C(=O)-N(C₁₋₆ alkyl)₂; preferably, Rₐ is selected from a hydrogen atom, - C(=O)CH(CH₃)₂ and -C(=O)-N(CH₃)₂;
R⁷ and R⁸ are both selected from hydrogen atoms, fluorine atoms and methyl; alternatively, R⁷ and R⁸, together with the carbon atom to which they are attached, form 3- to 6-membered cycloalkyl or 3- to 6-membered heterocyclyl;
R^{b} and R^{c} are the same or different, and are each independently selected from a hydrogen atom, halogen, amino, hydroxyl, C₁₋₄ alkyl, C₁₋₄ haloalkyl, 3- to 6-membered cycloalkyl, 3- to 6-membered halocycloalkyl, C₁₋₄ alkoxy, C₁₋₄ haloalkoxy, -NH(C₁₋₄ alkyl), -N(C₁₋₄ alkyl)₂ and C₂₋₄ alkenyl; alternatively, R^{b} and R^{c}, together with the carbon atom to which they are attached, form C₂₋₄ alkenyl;
R^{d} is selected from hydroxyl, amino, hydroxyamino, -OR^{6A}, -O-NHR^{6A}, - NHR^{6A}, -NR^{6A}-OR^{6B}, -C(=O)R^{6A}, -C(=O)NR^{6A}OR^{6B}, -C(=NH)R^{6A}, - C(=NH)NR^{6A}R^{6B}, -NH-C(=O)R^{6A}, -S(=O)(=NH)R^{6A}, -S(=O)₂R^{6A}, -NH-S(=O)₂R^{6A}, -C(=O)-NR^{6A}R^{6B}, -C(=S)-NR^{6A}R^{6B}, or -P(=O)R^{6A}R^{6B}; preferably, R^{d} is selected from hydroxyl, amino, hydroxyamino, -NHCH₃, -NHOCH₃, -NCH₃OCH₃, - NHOCH₂CF₃, -NHS(=O)₂CH₃, -C(=O)NH₂, -S(=O)₂NH₂, -S(=O)(=NH)CH₃ and - S(=O)₂CH₃;
R^{6A} and R^{6B} are the same or different, and are each independently selected from a hydrogen atom, amino, C₁₋₆ alkyl, C₁₋₆ haloalkyl, 3- to 8-membered cycloalkyl and 3- to 8-membered heterocyclyl; preferably, R^{6A} and R^{6B} are the same or different, and are each independently selected from a hydrogen atom, amino, C₁₋₆ alkyl and C₁₋₆ haloalkyl; more preferably, R^{6A} and R^{6B} are the same or different, and are each independently selected from a hydrogen atom, amino, methyl and trifluoroethyl.

14. The compound or the pharmaceutically acceptable salt, the ester, the stereoisomer, the tautomer, the polymorph, the hydrate, the solvate, the N-oxide, the isotopically labelled compound, the metabolite or the prodrug thereof according to claim 12 or 13, **characterised in that** R¹ and R² are the same or different, and are each independently selected from methyl, a chlorine atom and a fluorine atom.

15. The compound or the pharmaceutically acceptable salt, the ester, the stereoisomer, the tautomer, the polymorph, the hydrate, the solvate, the N-oxide, the isotopically labelled compound, the metabolite or the prodrug thereof according to any one of claims 12-14, **characterised in that** R¹ is a chlorine atom or a fluorine atom, and R² is a chlorine atom or a fluorine atom.

16. The compound or the pharmaceutically acceptable salt, the ester, the stereoisomer, the tautomer, the polymorph, the hydrate, the solvate, the N-oxide, the isotopically labelled compound, the metabolite or the prodrug thereof according to any one of claims 12-15, **characterised in that** R¹ and R² are both chlorine atoms.

17. The compound or the pharmaceutically acceptable salt, the ester, the stereoisomer, the tautomer, the polymorph, the hydrate, the solvate, the N-oxide, the isotopically labelled compound, the metabolite or the prodrug thereof according to any one of claims 12-16, **characterised in that** Rₐ is a hydrogen atom.

18. The compound or the pharmaceutically acceptable salt, the ester, the stereoisomer, the tautomer, the polymorph, the hydrate, the solvate, the N-oxide, the isotopically labelled compound, the metabolite or the prodrug thereof according to any one of claims 12-17, **characterised in that** R⁷ and R⁸ are both hydrogen atoms or fluorine atoms; alternatively, R⁷ and R⁸, together with the carbon atom to which they are attached, form cyclopropyl.

19. The compound or the pharmaceutically acceptable salt, the ester, the stereoisomer, the tautomer, the polymorph, the hydrate, the solvate, the N-oxide, the isotopically labelled compound, the metabolite or the prodrug thereof according to any one of claims 12-18, **characterised in that** R⁷ and R⁸ are both hydrogen atoms.

20. The compound or the pharmaceutically acceptable salt, the ester, the stereoisomer, the tautomer, the polymorph, the hydrate, the solvate, the N-oxide, the isotopically labelled compound, the metabolite or the prodrug thereof according to any one of claims 12-19, **characterised in that** R^{d} is selected from hydroxyl, amino and hydroxyamino.

21. The compound or the pharmaceutically acceptable salt, the ester, the stereoisomer, the tautomer, the polymorph, the hydrate, the solvate, the N-oxide, the isotopically labelled compound, the metabolite or the prodrug thereof according to any one of claims 12-20, **characterised in that** R^{d} is hydroxyl.

22. The compound or the pharmaceutically acceptable salt, the ester, the stereoisomer, the tautomer, the polymorph, the hydrate, the solvate, the N-oxide, the isotopically labelled compound, the metabolite or the prodrug thereof according to any one of claims 12-21, **characterised in that** R^{b} and R^{c} are the same or different, and are each independently selected from a hydrogen atom, a fluorine atom, methyl and hydroxyl; alternatively, R^{b} and R^{c}, together with the carbon atom to which they are attached, form vinyl or cyclopropyl.

23. The compound or the pharmaceutically acceptable salt, the ester, the stereoisomer, the tautomer, the polymorph, the hydrate, the solvate, the N-oxide, the isotopically labelled compound, the metabolite or the prodrug thereof according to any one of claims 12-21, **characterised in that** R^{b} and R^{c} are both fluorine atoms, or one of R^{b} and R^{c} is a fluorine atom or hydroxyl and the other is a hydrogen atom.

24. The compound or the pharmaceutically acceptable salt, the ester, the stereoisomer, the tautomer, the polymorph, the hydrate, the solvate, the N-oxide, the isotopically labelled compound, the metabolite or the prodrug thereof according to any one of claims 12-22, **characterised in that** R^{b} and R^{c} are both fluorine atoms, or one of R^{b} and R^{c} is hydroxyl and the other is a hydrogen atom.

25. The compound or the pharmaceutically acceptable salt, the ester, the stereoisomer, the tautomer, the polymorph, the hydrate, the solvate, the N-oxide, the isotopically labelled compound, the metabolite or the prodrug thereof according to claim 1, **characterised in that** the compound is a compound represented by formula (I-4), 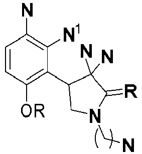 wherein:
R¹ and R² are the same or different, and are each independently selected from a hydrogen atom, C₁₋₄ alkyl, C₂₋₆ alkenyl and halogen; preferably, R¹ and R² are the same or different, and are each independently selected from methyl, a chlorine atom and a fluorine atom;
R^{a} is selected from a hydrogen atom, -C(=O)C₁₋₆ alkyl, -C(=O)-NH(C₁₋₆ alkyl) and -C(=O)-N(C₁₋₆ alkyl)₂; preferably, R^{a} is selected from a hydrogen atom, - C(=O)CH(CH₃)₂ and -C(=O)-N(CH₃)₂; more preferably, R^{a} is a hydrogen atom;
R⁷ and R⁸ are both selected from hydrogen atoms and fluorine atoms; alternatively, R⁷ and R⁸, together with the carbon atom to which they are attached, form 3- to 6-membered cycloalkyl or 3- to 6-membered heterocyclyl; preferably, R⁷ and R⁸ are both hydrogen atoms or fluorine atoms, or, together with the carbon atom to which they are attached, form cyclopropyl;
R^{d} is selected from hydroxyamino, -O-NHR^{6A}, -NR^{6A}-OR^{6B}, - C(=O)NR^{6A}OR^{6B}, -C(=S)-NR^{6A}R^{6B}, -C(=NH)R^{6A}, -C(=NH)NR^{6A}R^{6B}, - S(=O)(=NH)R^{6A}, -S(=O)₂R^{6A}, -NH-S(=O)₂R^{6A} and -P(=O) R^{6A} R^{6B};
R^{6A}, R^{6B} and n are as defined in claim 1.

26. The compound or the pharmaceutically acceptable salt, the ester, the stereoisomer, the tautomer, the polymorph, the hydrate, the solvate, the N-oxide, the isotopically labelled compound, the metabolite or the prodrug thereof according to claim 25, **characterised in that**:
when n is 2 or 3, R^{d} is selected from hydroxyamino, -O-NHR^{6A}, -NR^{6A}-OR^{6B}, -C(=O)NR^{6A}OR^{6B}, -C(=S)-NR^{6A}R^{6B}, -C(=NH)NR^{6A}R^{6B}, -S(=O)(=NH)R^{6A}, - S(=O)₂R^{6A} and -NH-S(=O)₂R^{6A}; R^{6A} and R^{6B} are the same or different, and are each independently selected from a hydrogen atom, amino, C₁₋₆ alkyl, C₁₋₆ haloalkyl and 3- to 8-membered cycloalkyl; preferably, R^{6A} and R^{6B} are the same or different, and are each independently selected from a hydrogen atom, amino, methyl, trifluoroethyl and cyclopropyl.

27. The compound or the pharmaceutically acceptable salt, the ester, the stereoisomer, the tautomer, the polymorph, the hydrate, the solvate, the N-oxide, the isotopically labelled compound, the metabolite or the prodrug thereof according to claim 25 or 26, **characterised in that** R^{d} is selected from -NHOH, - ONH₂, -NHOCH₃, -NCH₃OCH₃, -NHOCH₂CF₃, -C(=S)NH₂, -C(=NH)NH₂, - C(=O)NHOH, -C(=O)NCH₃OH, -C(=O)NHOCH₃, -NHS(=O)₂CH₃, -S(=O)₂NH₂, - S(=O)(=NH)CH₃ and -S(=O)₂CH₃.

28. The compound or the pharmaceutically acceptable salt, the ester, the stereoisomer, the tautomer, the polymorph, the hydrate, the solvate, the N-oxide, the isotopically labelled compound, the metabolite or the prodrug thereof according to claim 25, **characterised in that**:
when n is 0, R^{d} is selected from -C(=O)R^{6A} and -S(=O)₂R^{6A};
R^{6A} is selected from C₁₋₆ alkyl, 3- to 8-membered cycloalkyl and 3- to 8-membered heterocyclyl, wherein the C₁₋₆ alkyl, 3- to 8-membered cycloalkyl, and 3- to 8-membered heterocyclyl are optionally substituted with one or more substituents selected from halogen, hydroxyl, amino, C₁₋₆ alkyl, C₁₋₆ haloalkyl and C₁₋₆ alkoxy; preferably, R^{6A} is selected from - C₂H₄NH₂, 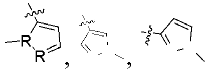 and cyclopropyl.

29. The compound or the pharmaceutically acceptable salt, the ester, the stereoisomer, the tautomer, the polymorph, the hydrate, the solvate, the N-oxide, the isotopically labelled compound, the metabolite or the prodrug thereof according to claim 25 or 28, **characterised in that** R^{d} is selected from - C(=O)C₂H₄NH₂,

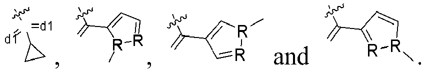

30. The compound or the pharmaceutically acceptable salt, the ester, the stereoisomer, the tautomer, the polymorph, the hydrate, the solvate, the N-oxide, the isotopically labelled compound, the metabolite or the prodrug thereof according to claim 1, **characterised in that** the compound is a compound represented by formula (I-5), 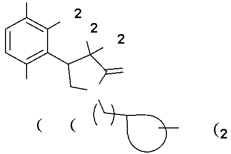 wherein:
R¹ and R² are the same or different, and are each independently selected from a hydrogen atom, C₁₋₄ alkyl, C₂₋₆ alkenyl and halogen; preferably, R¹ and R² are the same or different, and are each independently selected from methyl, a chlorine atom and a fluorine atom;
R^{a} is selected from a hydrogen atom, -C(=O)C₁₋₆ alkyl, -C(=O)-NH(C₁₋₆ alkyl) and -C(=O)-N(C₁₋₆ alkyl)₂; preferably, R^{a} is selected from a hydrogen atom, - C(=O)CH(CH₃)₂ and -C(=O)-N(CH₃)₂; more preferably, R^{a} is a hydrogen atom;
R⁷ and R⁸ are both selected from hydrogen atoms and fluorine atoms; alternatively, R⁷ and R⁸, together with the carbon atom to which they are attached, form 3- to 6-membered cycloalkyl or 3- to 6-membered heterocyclyl; preferably, R⁷ and R⁸ are both hydrogen atoms or fluorine atoms, or, together with the carbon atom to which they are attached, form cyclopropyl;
ring A is selected from 3- to 8-membered cycloalkyl, 3- to 8-membered heterocyclyl, 6- to 12-membered spirocyclyl, 6- to 12-membered spiro heterocyclyl, a 5- to 12-membered bridged ring group, 5- to 12-membered bridged heterocyclyl, 6- to 10-membered aryl, 5- to 10-membered heteroaryl containing at least one nitrogen atom and 6- to 12-membered fused heterocyclyl;
k is selected from 0, 1, 2, 3 and 4;
n is selected from 0, 1, 2 and 3;
R^{6C} is as defined in claim 1.

31. The compound or the pharmaceutically acceptable salt, the ester, the stereoisomer, the tautomer, the polymorph, the hydrate, the solvate, the N-oxide, the isotopically labelled compound, the metabolite or the prodrug thereof according to claim 30, **characterised in that**:
n is 0 or 1, and ring A is selected from 6- to 12-membered spirocyclyl, 6- to 12-membered spiro heterocyclyl, a 5- to 12-membered bridged ring group and 5- to 12-membered bridged heterocyclyl; preferably, ring A is selected from 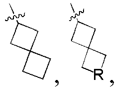 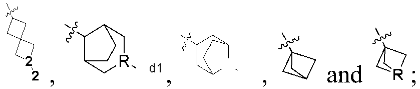 R^{d1} is selected from a hydrogen atom, C₁₋₆ alkyl, C₁₋₆ haloalkyl, 3- to 10-membered cycloalkyl, -C(=O)C₁₋₆ alkyl, -C(=O)C₁₋₆ haloalkyl, -C(=O)C₃₋₆ cycloalkyl, -S(=O)₂C₁₋₆ alkyl, -S(=O)₂C₃₋₆ cycloalkyl, C₂₋₆ alkenyl and -C₁₋₆ alkyl- P(=O)(C₁₋₆ alkyl)₂; preferably, R^{d1} is selected from a hydrogen atom, C₁₋₆ alkyl, -S(=O)₂C₁₋₆ alkyl, -S(=O)₂C₃₋₆ cycloalkyl, C₂₋₆ alkenyl and -C₁₋₆ alkyl- P(=O)(C₁₋₆ alkyl)₂; more preferably, R^{d1} is selected from a hydrogen atom;
R^{6C} is selected from halogen, amino, hydroxyl, cyano, C₁₋₆ alkyl, C₁₋₆ haloalkyl, C₁₋₆ alkoxy, 3- to 8-membered cycloalkoxy, C₁₋₆ haloalkoxy, 3- to 8-membered halocycloalkoxy, C₂₋₆ alkenyl, oxo, -C(=O)R^{6C1}, -NH-C(=O)R^{6C1}, - S(=O)(=NH)R^{6C1}, -S(=O)₂R^{6C1}, -NH-S(=O)₂R^{6C1}, -C(=O)-NR^{6C1}R^{6C2}, -P(=O) R^{6C1}R^{6C2} and -C₁₋₆ alkyl- P(=O) R^{6C1}R^{6C2};
R^{6C1} and R^{6C2} are the same or different, and are each independently selected from a hydrogen atom, C₁₋₆ alkyl, C₁₋₆ haloalkyl, 3- to 8-membered cycloalkyl and 3- to 8-membered heterocyclyl; preferably, R^{6C} is selected from halogen, amino, hydroxyl, C₁₋₆ alkyl, C₁₋₆ haloalkyl and -S(=O)₂ C₁₋₆ alkyl; more preferably, R^{6C} is selected from a fluorine atom, amino, hydroxyl, methyl, trifluoromethyl and - S(=O)₂CH₃;
k is selected from 0, 1, 2, 3 and 4; preferably, k is 0 or 1.

32. The compound or the pharmaceutically acceptable salt, the ester, the stereoisomer, the tautomer, the polymorph, the hydrate, the solvate, the N-oxide, the isotopically labelled compound, the metabolite or the prodrug thereof according to claim 30 or 31, **characterised in that** structural unit 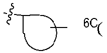 is selected from

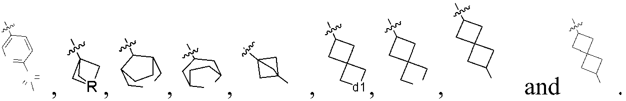

33. The compound or the pharmaceutically acceptable salt, the ester, the stereoisomer, the tautomer, the polymorph, the hydrate, the solvate, the N-oxide, the isotopically labelled compound, the metabolite or the prodrug thereof according to claim 30, **characterised in that**:
n is 0 or 1, and ring A is selected from 3- to 8-membered cycloalkyl, 3- to 8-membered heterocyclyl, 6- to 10-membered aryl, 5- to 10-membered heteroaryl containing at least one nitrogen atom and 6- to 12-membered fused heterocyclyl; preferably, ring A is selected from 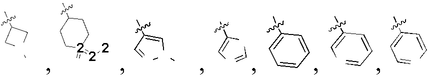 and 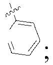
R^{d1} is selected from a hydrogen atom, 3- to 6-membered cycloalkyl, - S(=O)₂C₁₋₆ alkyl, -S(=O)₂C₃₋₆ cycloalkyl, C₂₋₆ alkenyl and -C₁₋₆ alkyl- P(=O)(C₁₋₆ alkyl)₂; more preferably, R^{d1} is selected from cyclopropyl, vinyl, allyl, -S(=O)₂CH₃, -S(=O)₂ cyclopropyl and -CH₂- P(=O)(CH₃)₂;
R^{6C} is selected from C₂₋₆ alkenyl, -S(=O)(=NH)R^{6C1}, -S(=O)₂R^{6C1}, -NH-S(=O)₂R^{6C1}, -P(=O) R^{6C1}R^{6C2} and -C₁₋₆ alkyl- P(=O) R^{6C1}R^{6C2}; R^{6C1} and R^{6C2} are the same or different, and are each independently selected from a hydrogen atom, C₁₋₆ alkyl, C₁₋₆ haloalkyl, 3- to 8-membered cycloalkyl and 3- to 8-membered heterocyclyl; preferably, R^{6C} is selected from -S(=O)₂NH₂ and -S(=O)₂ C₁₋₆ alkyl; more preferably, R^{6C} is selected from -S(=O)₂NH₂ and -S(=O)₂CH₃;
k is selected from 0, 1, 2, 3 and 4; preferably, k is 0 or 1.

34. The compound or the pharmaceutically acceptable salt, the ester, the stereoisomer, the tautomer, the polymorph, the hydrate, the solvate, the N-oxide, the isotopically labelled compound, the metabolite or the prodrug thereof according to claim 30 or 33, **characterised in that** structural unit 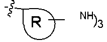 is selected from

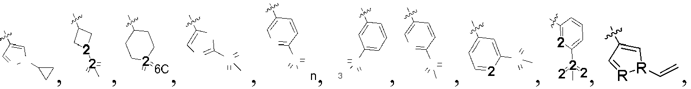

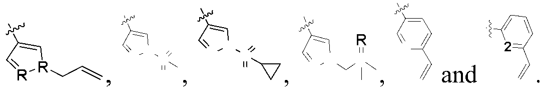

35. The compound or the pharmaceutically acceptable salt, the ester, the stereoisomer, the tautomer, the polymorph, the hydrate, the solvate, the N-oxide, the isotopically labelled compound, the metabolite or the prodrug thereof according to claim 1, **characterised in that** the compound is a compound represented by formula (I-6), 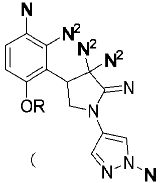
wherein R¹ and R² are the same or different, and are each independently selected from a hydrogen atom, C₁₋₄ alkyl, C₂₋₆ alkenyl and halogen;
Rₐ is selected from a hydrogen atom, -C(=O)C₁₋₆ alkyl, -C(=O)-NH(C₁₋₆ alkyl) and -C(=O)-N(C₁₋₆ alkyl)₂;
R⁷ and R⁸ are both selected from hydrogen atoms, fluorine atoms and methyl; alternatively, R⁷ and R⁸, together with the carbon atom to which they are attached, form 3- to 6-membered cycloalkyl or 3- to 6-membered heterocyclyl;
R^{6D} is selected from C₁₋₆ alkyl, C₁₋₆ haloalkyl, C₂₋₆ alkenyl, 3- to 8-membered cycloalkyl, 3- to 8-membered halocycloalkyl, 3- to 8-membered heterocyclyl, - S(=O)₂-C₁₋₆ alkyl or -S(=O)₂-3- to 8-membered cycloalkyl, wherein the C₁₋₆ alkyl, C₁₋₆ haloalkyl, C₂₋₆ alkenyl, 3- to 8-membered cycloalkyl, 3- to 8-membered halocycloalkyl, 3- to 8-membered heterocyclyl, -S(=O)₂-C₁₋₆ alkyl, and -S(=O)₂-3-to 8-membered cycloalkyl are optionally substituted with one or more substituents selected from: halogen, hydroxyl, amino, cyano, C₁₋₄ alkyl, C₁₋₄ haloalkyl, or C₁₋₄ alkoxy.

36. The compound or the pharmaceutically acceptable salt, the ester, the stereoisomer, the tautomer, the polymorph, the hydrate, the solvate, the N-oxide, the isotopically labelled compound, the metabolite or the prodrug thereof according to claim 35, **characterised in that** R¹ and R² are the same or different, and are each independently selected from methyl, a chlorine atom and a fluorine atom.

37. The compound or the pharmaceutically acceptable salt, the ester, the stereoisomer, the tautomer, the polymorph, the hydrate, the solvate, the N-oxide, the isotopically labelled compound, the metabolite or the prodrug thereof according to claim 35 or 36, **characterised in that** R¹ is a chlorine atom or a fluorine atom, and R² is a chlorine atom or a fluorine atom.

38. The compound or the pharmaceutically acceptable salt, the ester, the stereoisomer, the tautomer, the polymorph, the hydrate, the solvate, the N-oxide, the isotopically labelled compound, the metabolite or the prodrug thereof according to any one of claims 35-37, **characterised in that** R¹ and R² are both chlorine atoms.

39. The compound or the pharmaceutically acceptable salt, the ester, the stereoisomer, the tautomer, the polymorph, the hydrate, the solvate, the N-oxide, the isotopically labelled compound, the metabolite or the prodrug thereof according to any one of claims 35-38, **characterised in that** Rₐ is selected from a hydrogen atom, -C(=O)CH(CH₃)₂ and -C(=O)-N(CH₃)₂.

40. The compound or the pharmaceutically acceptable salt, the ester, the stereoisomer, the tautomer, the polymorph, the hydrate, the solvate, the N-oxide, the isotopically labelled compound, the metabolite or the prodrug thereof according to any one of claims 35-39, **characterised in that** Rₐ is a hydrogen atom.

41. The compound or the pharmaceutically acceptable salt, the ester, the stereoisomer, the tautomer, the polymorph, the hydrate, the solvate, the N-oxide, the isotopically labelled compound, the metabolite or the prodrug thereof according to any one of claims 35-40, **characterised in that** R⁷ and R⁸ are both hydrogen atoms or fluorine atoms; alternatively, R⁷ and R⁸, together with the carbon atom to which they are attached, form cyclopropyl.

42. The compound or the pharmaceutically acceptable salt, the ester, the stereoisomer, the tautomer, the polymorph, the hydrate, the solvate, the N-oxide, the isotopically labelled compound, the metabolite or the prodrug thereof according to any one of claims 35-41, **characterised in that** R⁷ and R⁸ are both hydrogen atoms.

43. The compound or the pharmaceutically acceptable salt, the ester, the stereoisomer, the tautomer, the polymorph, the hydrate, the solvate, the N-oxide, the isotopically labelled compound, the metabolite or the prodrug thereof according to any one of claims 35-42, **characterised in that** R^{6D} is selected from C₁₋₆ alkyl, C₁₋₆ haloalkyl, C₂₋₆ alkenyl, 3- to 8-membered cycloalkyl and -S(=O)₂ -C₁₋₆ alkyl.

44. The compound or the pharmaceutically acceptable salt, the ester, the stereoisomer, the tautomer, the polymorph, the hydrate, the solvate, the N-oxide, the isotopically labelled compound, the metabolite or the prodrug thereof according to any one of claims 35-43, **characterised in that** R^{6D} is selected from methyl, cyclopropyl, vinyl and -S(=O)₂-CH₃.

45. The compound or the pharmaceutically acceptable salt, the ester, the stereoisomer, the tautomer, the polymorph, the hydrate, the solvate, the N-oxide, the isotopically labelled compound, the metabolite or the prodrug thereof according to any one of claims 35-44, **characterised in that** R^{6D} is selected from methyl and cyclopropyl.

46. The compound or the pharmaceutically acceptable salt, the ester, the stereoisomer, the tautomer, the polymorph, the hydrate, the solvate, the N-oxide, the isotopically labelled compound, the metabolite or the prodrug thereof according to any one of the preceding claims, **characterised in that** the compound is any one of the compounds in Table 1.

47. The compound or the pharmaceutically acceptable salt, the ester, the stereoisomer, the tautomer, the polymorph, the hydrate, the solvate, the N-oxide, the isotopically labelled compound, the metabolite or the prodrug thereof according to any one of the preceding claims, **characterised in that** the compound is substituted with an isotope, and preferably, the isotope substitution is deuterium atom substitution.

48. A method for preparing the compound or the pharmaceutically acceptable salt thereof according to any one of the preceding claims, **characterised by** the method comprising: 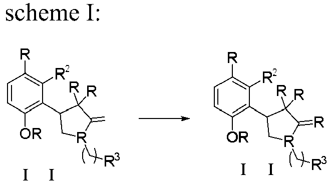
reacting a compound represented by general formula (IA) or a salt thereof with a thiocarbonylating reagent to obtain a compound represented by general formula (1-1) or a pharmaceutically acceptable salt thereof; optionally, the method further comprising the step of removing the phenolic hydroxyl protecting group R^{e} before, simultaneously with or after the reaction of the compound represented by general formula (IA) or the salt thereof with the thiocarbonylating reagent,
wherein:
the thiocarbonylating reagent is an oxygen-sulphur exchange reagent for converting carbonyl in an amide into thiocarbonyl, yielding a corresponding compound, preferably Lawesson's reagent;
R^{e} is a phenolic hydroxyl protecting group, preferably (trimethylsilyl)ethoxymethyl or allyl;
R¹, R², R⁷, R⁸, R^{a}, R^{d} and n are as defined in any one of the preceding claims; 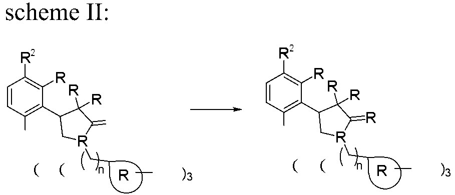
reacting a compound represented by general formula (IB) or a salt thereof with a thiocarbonylating reagent to obtain a compound represented by general formula (I-2) or a pharmaceutically acceptable salt thereof; optionally, the method further comprising the step of removing the phenolic hydroxyl protecting group R^{e} before, simultaneously with or after the reaction of the compound represented by general formula (IB) or the salt thereof with the thiocarbonylating reagent,
wherein:
the thiocarbonylating reagent is an oxygen-sulphur exchange reagent for converting carbonyl in an amide into thiocarbonyl, yielding a corresponding compound, preferably Lawesson's reagent;
R^{e} is a phenolic hydroxyl protecting group, preferably (trimethylsilyl)ethoxymethyl or allyl;
R¹, R², R⁷, R⁸, R^{a}, R^{6C}, ring A, n and k are as defined in any one of the preceding claims; 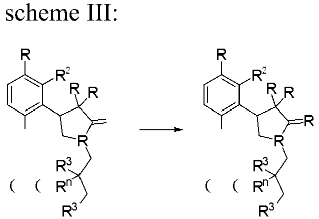
reacting a compound represented by general formula (IC) or a salt thereof with a thiocarbonylating reagent to obtain a compound represented by general formula (I-3) or a pharmaceutically acceptable salt thereof; optionally, the method further comprising the step of removing the phenolic hydroxyl protecting group R^{e} before, simultaneously with or after the reaction of the compound represented by general formula (IC) or the salt thereof with the thiocarbonylating reagent,
wherein:
the thiocarbonylating reagent is an oxygen-sulphur exchange reagent for converting carbonyl in an amide into thiocarbonyl, yielding a corresponding compound, preferably Lawesson's reagent;
R^{e} is a phenolic hydroxyl protecting group, preferably (trimethylsilyl)ethoxymethyl or allyl;
R¹, R², R⁷, R⁸, R^{a}, R^{b}, R^{c} and R^{d} are as defined in any one of the preceding claims; or 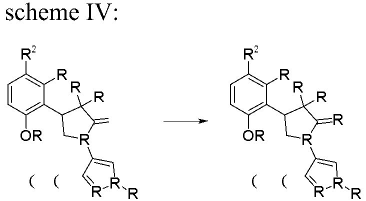
reacting a compound represented by general formula ID or a salt thereof with a thiocarbonylating reagent to obtain a compound represented by general formula (I-6) or a pharmaceutically acceptable salt thereof; optionally, the method further comprising the step of removing the phenolic hydroxyl protecting group R^{e} before, simultaneously with or after the reaction of the compound represented by general formula ID or the salt thereof with the thiocarbonylating reagent,
wherein:
the thiocarbonylating reagent is an oxygen-sulphur exchange reagent for converting carbonyl in an amide into thiocarbonyl, yielding a corresponding compound, preferably Lawesson's reagent;
R^{e} is a phenolic hydroxyl protecting group, preferably (trimethylsilyl)ethoxymethyl or allyl;
R¹, R², R⁷, R⁸, R^{a} and R^{6D} are as defined herein.

49. A pharmaceutical composition, **characterised in that** the pharmaceutical composition comprises the compound or the pharmaceutically acceptable salt, the ester, the stereoisomer, the tautomer, the polymorph, the hydrate, the solvate, the N-oxide, the isotopically labelled compound, the metabolite or the prodrug thereof according to any one of claims 1 to 36, and one or more pharmaceutically acceptable carriers, diluents or excipients.

50. The compound or the pharmaceutically acceptable salt, the ester, the stereoisomer, the tautomer, the polymorph, the hydrate, the solvate, the N-oxide, the isotopically labelled compound, the metabolite or the prodrug thereof according to any one of claims 1 to 47 or the pharmaceutical composition according to claim 49, for use as a medicament.

51. The compound or the pharmaceutically acceptable salt, the ester, the stereoisomer, the tautomer, the polymorph, the hydrate, the solvate, the N-oxide, the isotopically labelled compound, the metabolite or the prodrug thereof according to any one of claims 1 to 47 or the pharmaceutical composition according to claim 49, for use in the treatment or prevention of a disease or symptom in which a Kv1.3 potassium channel plays a role.

52. The compound or the pharmaceutically acceptable salt, the ester, the stereoisomer, the tautomer, the polymorph, the hydrate, the solvate, the N-oxide, the isotopically labelled compound, the metabolite or the prodrug thereof, or an isotope-substituted substance or the pharmaceutical composition for use in the treatment or prevention of the disease or symptom according to claim 40, **characterised in that** the disease or symptom is selected from inflammatory disease or autoimmune disease, transplant rejection, neurological disorder or neurodegenerative disease, cardiovascular disease, metabolic disorder, nephrosis, gastrointestinal disorder, or oncological condition; preferably, the inflammatory disease is selected from atopic dermatitis, arthritis, or psoriasis, the autoimmune disease is selected from rheumatoid arthritis, multiple sclerosis, systemic lupus erythematosus or type I diabetes, the neurodegenerative disease is Alzheimer's disease, the cardiovascular disease is ischemic stroke, the metabolic disorder is selected from obesity or type II diabetes, the nephrosis is selected from chronic kidney disease, nephritis or chronic renal failure, and the gastrointestinal disorder is inflammatory bowel disease.

53. A method for inhibiting a Kv1.3 potassium channel, **characterised by** comprising administering the compound or the pharmaceutically acceptable salt, the ester, the stereoisomer, the tautomer, the polymorph, the hydrate, the solvate, the N-oxide, the isotopically labelled compound, the metabolite or the prodrug thereof according to any one of claims 1 to 47 or the pharmaceutical composition according to claim 49.

54. A method for treating or preventing a disease or symptom in which a Kv1.3 potassium channel plays a role, **characterised by** comprising administering to a subject in need thereof the compound or the pharmaceutically acceptable salt, the ester, the stereoisomer, the tautomer, the polymorph, the hydrate, the solvate, the N-oxide, the isotopically labelled compound, the metabolite or the prodrug thereof according to any one of claims 1 to 47 or the pharmaceutical composition according to claim 49, wherein the disease or symptom is selected from inflammatory disease or autoimmune disease, transplant rejection, neurological disorder or neurodegenerative disease, cardiovascular disease, metabolic disorder, nephrosis, gastrointestinal disorder, or oncological condition; preferably, the inflammatory disease is selected from atopic dermatitis, arthritis, or psoriasis, the autoimmune disease is selected from rheumatoid arthritis, multiple sclerosis, systemic lupus erythematosus or type I diabetes, the neurodegenerative disease is Alzheimer's disease, the cardiovascular disease is ischemic stroke, the metabolic disorder is selected from obesity or type II diabetes, the nephrosis is selected from chronic kidney disease, nephritis or chronic renal failure, and the gastrointestinal disorder is inflammatory bowel disease.

55. A kit, **characterised by** comprising the compound or the pharmaceutically acceptable salt, the ester, the stereoisomer, the tautomer, the polymorph, the hydrate, the solvate, the N-oxide, the isotopically labelled compound, the metabolite or the prodrug thereof according to any one of claims 1 to 47 or the pharmaceutical composition according to claim 49 and instructions for use.
